(19) Europäisches Patentamt / European Patent Office / Office européen des brevets

(11) **EP 3 250 604 B1**

(12) **EUROPEAN PATENT SPECIFICATION**

(45) Date of publication and mention of the grant of the patent:
**21.04.2021  Bulletin 2021/16**

(21) Application number: **16703448.7**

(22) Date of filing: **25.01.2016**

(51) Int Cl.:
*C07K 16/28* (2006.01)    *A61K 39/395* (2006.01)

(86) International application number:
**PCT/EP2016/051467**

(87) International publication number:
**WO 2016/120216 (04.08.2016 Gazette 2016/31)**

(54) **MAB-DRIVEN CHIMERIC ANTIGEN RECEPTOR SYSTEMS FOR SORTING/DEPLETING ENGINEERED IMMUNE CELLS**

MAB-GESTEUERET ANTIGENREZEPTORSYSTEME ZUM SORTIEREN/ABREICHERN VON IMMUNZELLEN

SYSTÈMES DE RÉCEPTEURS D'ANTIGÈNES CHIMÉRIQUES DIRIGÉS PAR DES MAB POUR TRIER/APPAUVRIR LES CELLULES IMMUNITAIRES GÉNÉTIQUEMENT MODIFIÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **26.01.2015   DK 201570044**

(43) Date of publication of application:
**06.12.2017   Bulletin 2017/49**

(73) Proprietors:
• **Cellectis**
  **75013 Paris (FR)**
• **Allogene Therapeutics, Inc.**
  **South San Francisco, CA 94080 (US)**

(72) Inventors:
• **SASU, Barbra Johnson**
  **SanFrancisco, California CA 94110 (US)**
• **RAJPAL, Arvind**
  **San Francisco, CA CA 94127 (US)**
• **DUCHATEAU, Philippe**
  **11500 Saint Louis et Parahou (FR)**
• **JUILLERAT, Alexandre**
  **New York, New York 10028 (US)**
• **VALTON, Julien**
  **94220 Charenton-le-Pont (FR)**

(74) Representative: **Zacco Denmark A/S**
  **Arne Jacobsens Allé 15**
  **2300 Copenhagen S (DK)**

(56) References cited:
  WO-A1-2013/154760    WO-A1-2014/039523
  WO-A1-2014/130635    WO-A1-2014/152177

• **MARC CARTELLIERI ET AL: "A Novel Ex Vivo Isolation and Expansion Procedure for Chimeric Antigen Receptor Engrafted Human T Cells", PLOS ONE, vol. 9, no. 4, 3 April 2014 (2014-04-03), page e93745, XP055153686, DOI: 10.1371/journal.pone.0093745**
• **M. SADELAIN ET AL: "The Basic Principles of Chimeric Antigen Receptor Design", CANCER DISCOVERY, vol. 3, no. 4, 1 April 2013 (2013-04-01), pages 388-398, XP55133523, ISSN: 2159-8274, DOI: 10.1158/2159-8290.CD-12-0548**
• **B. Philip ET AL: "A highly compact epitope-based marker/suicide gene for easier and safer T-cell therapy", Blood, vol. 124, no. 8, 21 August 2014 (2014-08-21), pages 1277-1287, XP055229811, US ISSN: 0006-4971, DOI: 10.1182/blood-2014-01-545020**

**Description**

FIELD OF THE INVENTION

The present invention relates to improved chimeric antigen receptors (CAR) to be used in immunotherapy, the extracellular binding domains (scFv) of which have been modified by insertion of a mAb-specific epitope having the amino acid sequence of SEQ ID NO 35 to allow depletion of the immune cells endowed with said CARs. The present invention relates also to immune cells expressing said CARs and their therapeutic use.

BACKGROUND

Adoptive immunotherapy, which involves the transfer of autologous antigen-specific T cells generated *ex vivo*, is a promising strategy to treat viral infections and cancer. The T cells used for adoptive immunotherapy can be generated either by expansion of antigen-specific T cells or redirection of T cells through genetic engineering (Park, Rosenberg et al. 2011). Transfer of viral antigen specific T cells is a well-established procedure used for the treatment of transplant associated viral infections and rare viral-related malignancies. Similarly, isolation and transfer of tumor specific T cells has been shown to be successful in treating melanoma.

Novel specificities in T cells have been successfully generated through the genetic transfer of transgenic T cell receptors or chimeric antigen receptors (CARs) (Jena, Dotti et al. 2010). CARs are synthetic receptors consisting of a targeting moiety that is associated with one or more signaling domains in a single fusion molecule. In general, the binding moiety of a CAR consists of an antigen-binding domain of a single-chain antibody (scFv), comprising the light and heavy variable fragments of a monoclonal antibody joined by a flexible linker. Binding moieties based on receptor or ligand domains have also been used successfully. The signaling domains for first generation CARs are derived from the cytoplasmic region of the CD3zeta or the Fc receptor gamma chains. First generation CARs have been shown to successfully redirect T cell cytotoxicity, however, they failed to provide prolonged expansion and anti-tumor activity in vivo. Signaling domains from co-stimulatory molecules including CD28, OX-40 (CD134), ICOS and 4-1BB (CD137) have been added alone (second generation) or in combination (third generation) to enhance survival and increase proliferation of CAR modified T cells. CARs have successfully allowed T cells to be redirected against antigens expressed at the surface of tumor cells from various malignancies including lymphomas and solid tumors (Jena, Dotti et al. 2010).

However, despite their unprecedent efficacy for tumor eradication in vivo, CAR T cells can promote acute adverse events after being transferred into patients. Among the well documented adverse events is Graft versus host disease (GvHD), on-target off-tumor activity or aberrant lymphoproliferative capacity due to vector derived insertional mutagenesis. Therefore, there is a need to develop cell specific depletion systems to prevent such deleterious events to occur in vivo.

There are many on-going researches to develop a safer CAR-based immunotherapy, such as on inhibitory signals referred to as immune checkpoints (such as CTLA-4- or PD-1) which are crucial for the maintenance of self-tolerance and also to limit immune-mediated collateral tissue damage (Dolan et al, 2014). Recently, inhibitory chimeric antigen receptors (iCARs) were designed having as objective to put the brakes on T cell function upon encountering off-target cells (Federov et al. 2013). Another system is described in Budde et al. (2013) in which a CD20 Chimeric Antigen Receptor is combined with an inducible caspase 9 (iC9) suicide switch. In the application US 2014/0286987, the latter gene is made functional in the presence of the prodrug AP1903 (tacrolimus) by binding to the mutated FK506-binding protein (FKBP1). A clinical trial is ongoing sponsored by the company Bellicum in which the above capsase technology (CaspaCIDe™) is engineered into GD2 targeted third generation CAR T cells. A similar apoptosis-inducing system based on a multimerizing agent is described in the application WO 2014/152177.

Philip et al (2014) describes the RQR8 system which is being used as compact marker/suicide gene allowing selection of transduced cells. RQR8 derives from the combination of target epitopes from both CD34 and CD20 antigens. This construct allows selection with the clinically approved CliniMACS CD34 system (Miltenyi). Moreover, this RQR8 construct binds the widely used pharmaceutical antibody rituximab, resulting in selective deletion of transgene-expressing cells. Within this system, RQR8 is co-expressed with a CAR in a retroviral vector using the foot-and-mouth disease 2A peptide, resulting thereby into the expression of 2 independent transgenes (RQR8 and CAR) on the surface of the T-cells. This system presents some limitations from the industrial perspective, as first, it requires the cloning large retroviral inserts, and second, to ensure that the transformed cells express both RQR8 and CAR polypeptides, to eliminate possible "false-positive" i.e. T-cells that would not express both polypeptides, in particular the RQR8 suicide gene allowing the depletion of the engineered immune cells in the event of undesirable effects.

The concept of depleting T cells in the context of auto-immune disease and transplantation has been successfully practiced in the clinic for decades. To deplete cell-mediated immunity, including T-cells, immunosuppressive drugs such as glucocorticoids or cytostatics such as alkylating agents (cyclophosphamide, nitrosoureas, platinum compounds...) or antimetabolites (methotrexate, azathioprine, fluorouracil...) are widely used. However, despite their immunosuppressive efficacy, these drugs are not discriminative as they affect the proliferation of all T and B cells. Antibodies are sometimes used as a quick and potent immunosuppressive therapy to prevent the acute rejection reactions as well as a targeted treatment of lymphoproliferative or autoimmune disorders, in particular anti-CD20 monoclonals. *In vivo* elimination of T cell subsets was performed by Benjamin and Waldmann (1986) to determine the role of CD4+ T cells in generating antibody responses to soluble proteins, and by Cobbold et al. (1986) to determine the role of CD4+ and CD8+ T cells in rejecting bone marrow and tissue allografts. In vivo depletion has been performed extensively to study varied topics including control of antiviral cytotoxic T lymphocyte (CTL) responses (Buller et al., 1987). However, the antibodies which have been used so far on T cells direct antigens (CD3, CD4, CD52) that are all broadly present on resting or activated T cells as well as on other cell types. As such, the use of such antibodies would not allow the selective elimination of the engineered immune cells endowed with CARs.

Cartellieri et al. (2014) discloses a method for selective *in vitro* expansion of engineered T lymphocytes.

As presented thereafter, the inventors have sought for an "all-in-one" system which allows an optimized *in vitro* sorting of CAR-expressing immune cells by reducing "false-positive", meanwhile allowing the *in vivo* depletion of the immune cells expressing said CARs in case of adverse clinical event.

## SUMMARY OF THE INVENTION

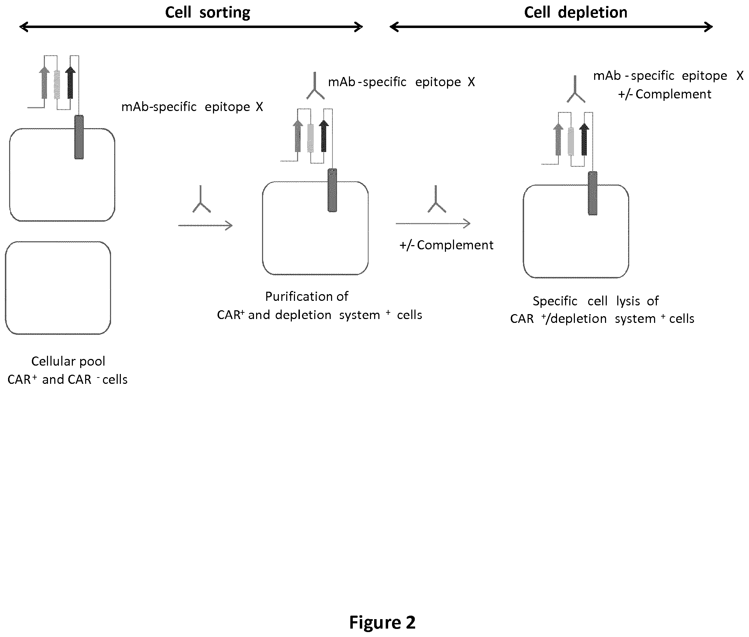

The present invention is drawn to chimeric antigen receptors (CAR), which extracellular binding domain (scFv) is modified in such a way to allow cell depletion (see Figure 2 for illustrative embodiment). This structure named mAb-driven depletion system consists in inserting at least one mAb-specific epitope having the amino acid sequence of SEQ ID NO 35 within the scFv; this epitope having a specificity to be recognized by a specific antibody (preferably mAb). Given the fact that mainly the external ligand binding domain of the CAR is modified to include the epitope, different CAR architectures can be envisioned: single-chain or multi-chain. The chimeric scFv of the invention, which is formed of the VH and VL polypeptides and the specific epitope(s) may itself have different structures depending on the position of insertion of the epitope and the use of linkers.

To further enhance the cytotoxicity of the engineered immune cells, the epitope-specific antibody may be conjugated with a cytotoxic drug. It is also possible to promote CDC cytotoxicity by using engineered antibodies on which are grafted component(s) of the complement system.

Finally, the invention encompasses therapeutic uses where the activation of the engineered immune cells endowed with CARs is modulated by depleting the cells by using an antibody that directs the external ligand binding domain of said CARs.

The invention is defined by the appendant claims.

## BRIEF DESCRIPTION OF THE FIGURES AND TABLE

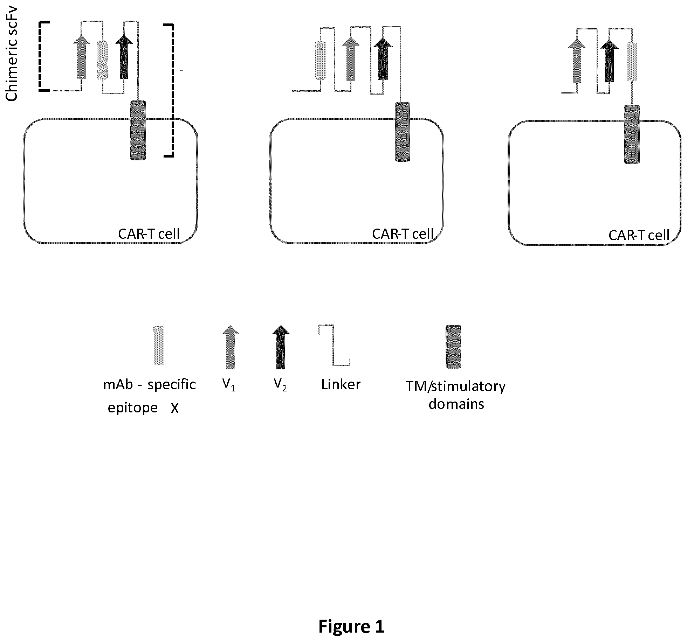

**Figure 1:** Schematic structure of the mAb-driven sorting/depletion system of the invention using here a single-chain CAR scaffold; several configurations are presented for the chimeric scFv with different positions of the VH, VL chains and the mAb-specific epitope.

**Figure 2:** Schematic representation of cell sorting and cell depletion functioning by using the mAb-driven system of the invention. The addition of specific mAb (+/- complement) allows purification of CAR+ T cells by recognizing its epitope within the chimeric scFv. During the cell depletion step, the same specific mAb (+/- complement) by binding to its specific epitope within the chimeric scFv provokes a specific lysis of CAR+ T cells.

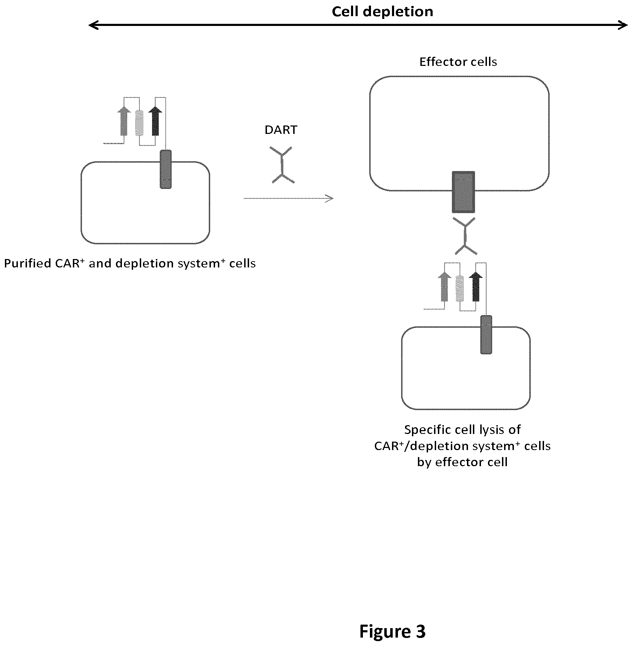

**Figure 3:** Schematic representation of cell depletion using bi-specific antibody. By binding to both CAR-expressing immune cell and to an effector cell, this system allows recruitment of effector cells at the surface of CAR-expressing immune cell and triggers their specific depletion *in vivo.*

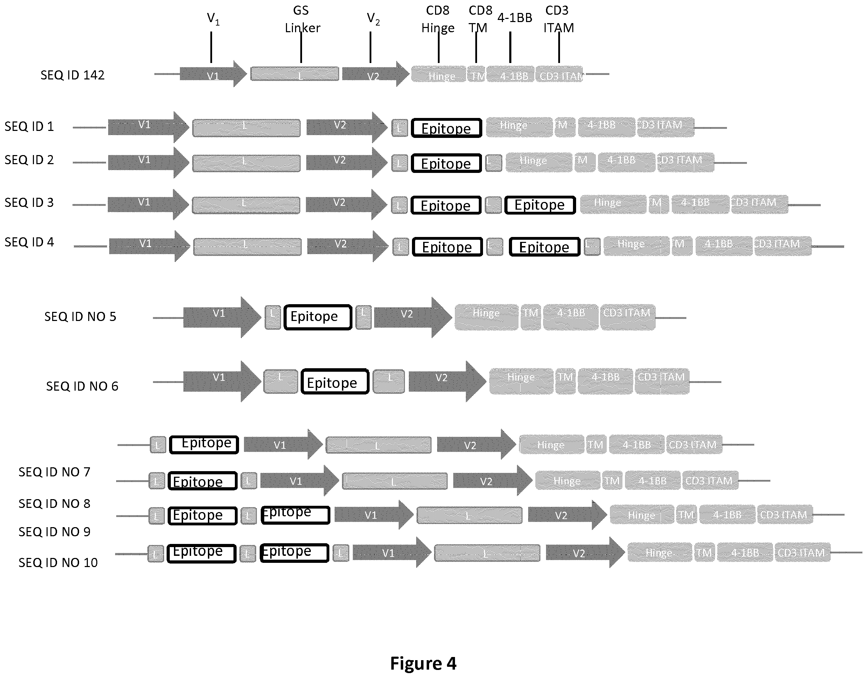

**Figure 4:** CAR architecture for 10 CARs expressing anti-CD123 scFv with CD20 mimotope(s) used in Examples 1-2. A series of 10 chimeric scFv are designed in which one or two copies of the CD20 mimotopes (black box named "mimotope") are inserted between the anti-CD123 scFv and the hinge. As depicted in the figure 4, all the 10 CARs have the same hinge (CD8 hinge), transmembrane domain (CD8 TM), co-stimulatory domain (4-1BB) and stimulatory domain (ITAM CD3 zeta). SEQ ID NO 1-10 comprise the leader sequence MALPVTALLLPLALLLHAARP, which is present when the CAR is initially expressed but which is not part of the CAR expressed at the surface of the cell. Depending on the position of the mimotope(s) in view of the scFv, 3 series of CARs are designed (Anti-CD123 No 1, 2, 3, 4, 5, 6, 7, 8, 9 and 10 corresponding to SEQ ID NO: 1, 2, 3, 4, 5, 6, 7, 8, 9 and 10, respectively):

- first series: SEQ ID NO 1-2 and SEQ ID NO 3-4 correspond to the conformations wherein respectively one and two CD20 mimotope(s) is(are) inserted between the anti-CD123 scFv and the hinge; in SEQ ID NO 2 a GS linker joins the CD20 mimotope to the hinge. In SEQ ID NO 3-4, a GS linker interspaces the two mimotopes and in SEQ ID NO 4 has an extra GS linker between the mimotopes and the hinge.

- second series: SEQ ID NO 5-6 correspond to the conformations wherein one copy of CD20 mimotope is inserted within the anti-CD123 scFv; the differences of sequence coming from the presence of respectively a short GS linker (SEQ ID NO 5) and a long GS linker (SEQ ID NO 6) lying on both sides.
- third series: SEQ ID NO 7 to 10 correspond to the conformations wherein the anti-CD123 scFv is located between the CD mimotope(s) and the hinge. In SEQ ID NO. 7-8 and SEQ ID NO. 9-10 respectively, one copy and two copies of CD20 mimotope is (are) inserted. A GS linker is inserted between the 2 CD20 mimotopes, and for SEQ ID NO.10, a supplementary GS linker joins the mimotopes to the anti-CD123 scFv.

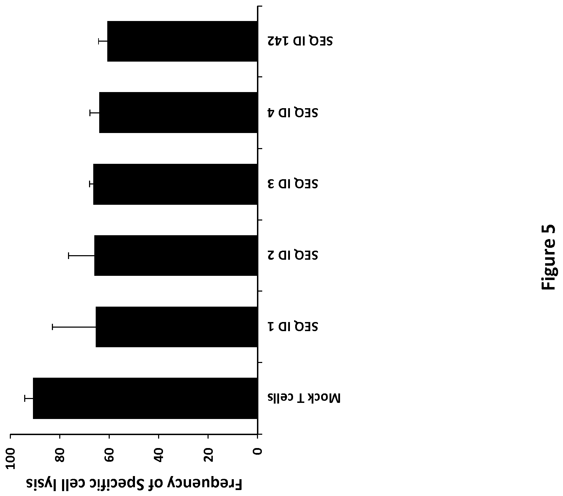

**Figure 5** shows the cytolytic activity of T cells expressing anti-CD123 CAR of SEQ ID NO 1-4 or 142 or control T Cell not expressing any anti CD123 CAR (mock T-cell -transfection step without any mRNA). The cytolytic activity is expressed as the frequency of specific cell lysis as detailed in Example 1.

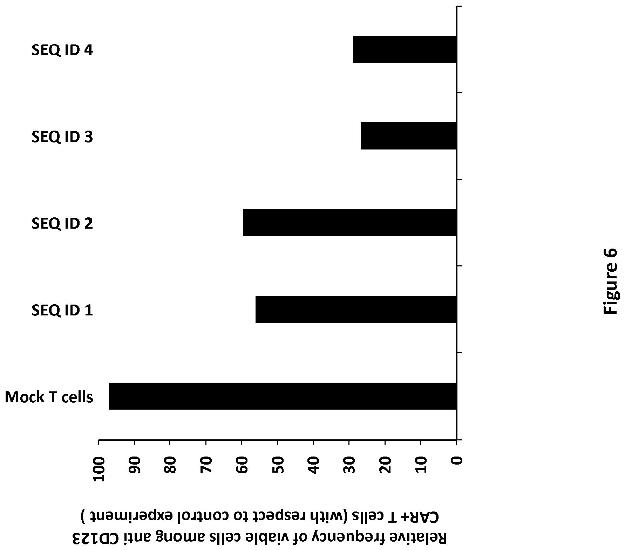

**Figure 6** shows the result of a CDC assay where T cells expressing anti-CD123 CAR of SEQ ID NO 1-4 or control T Cell not expressing any anti CD123 CAR (mock T-cell (transfection step without any mRNA)) are incubated with Rituximab (RTX) and Baby Rabbit Complement BRC). The results are expressed as relative frequency of viable cells among anti-CD123 CAR positive T cells (with respect to control experiment) as detailed in Example 3.

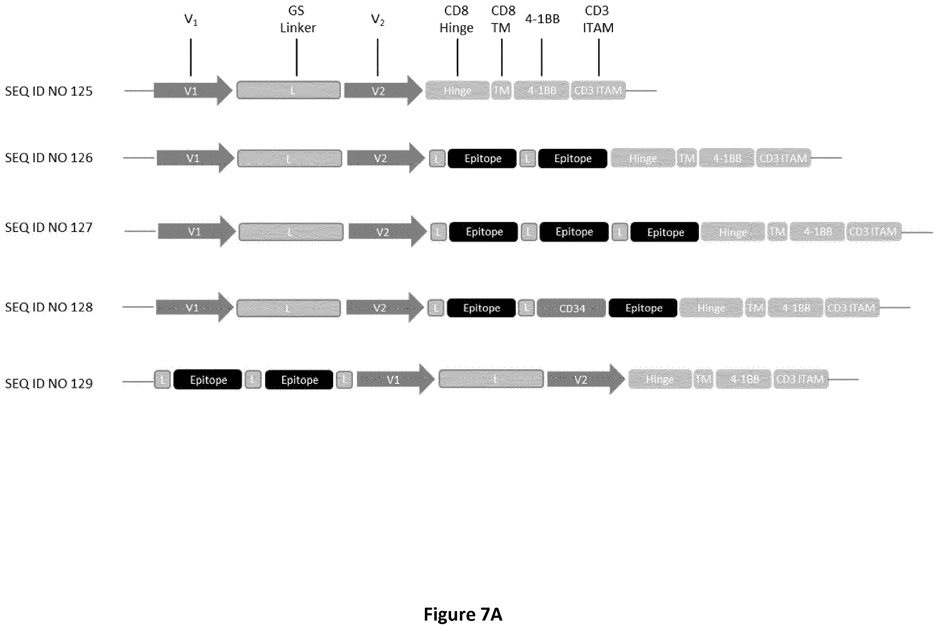

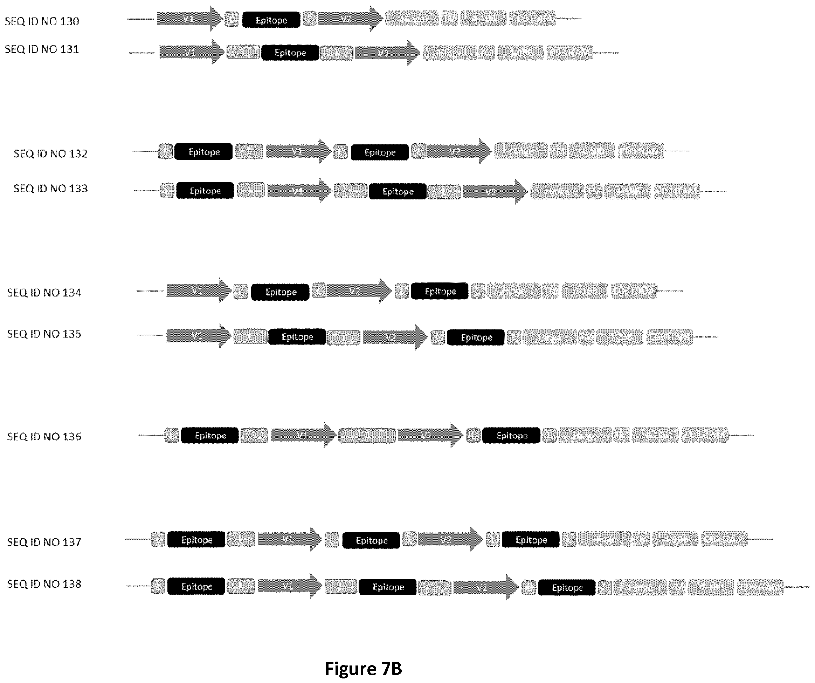

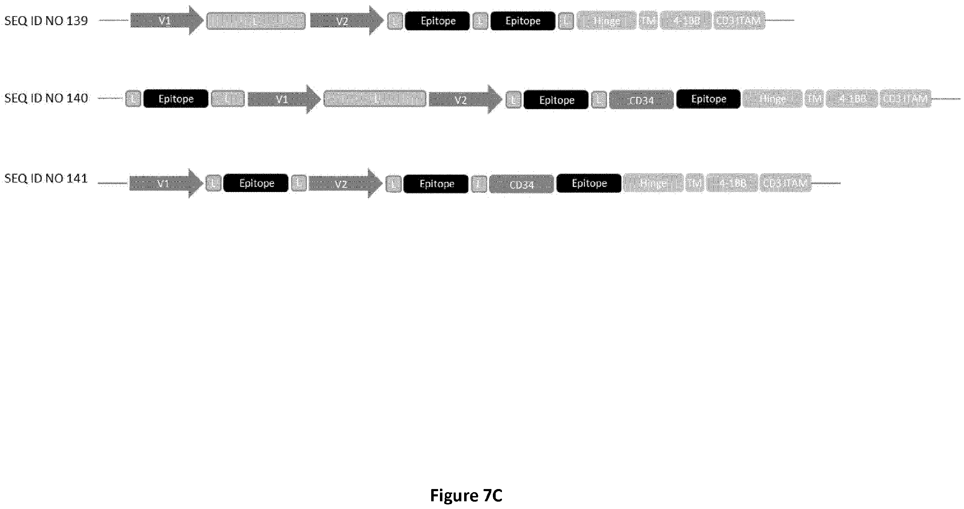

**Figures 7A, 7B and 7C** discloses the general structure of CARs of SEQ ID NO 125 to 141 that have been designed and tested in Examples 4 to 6. An anti BCMA ScFV was used in all these CARs. One, two, three or four epitopes selected from a CD20 mimotope (black box named "mimotope") and or a CD34 epitope (gray box named "CD34") were included at different positions, i.e upstream to the ScFv, downstream to the ScFv or between the variable chains of the ScFv (noted as V1 and V2). As depicted in the Figure 7, all the CARs have the same hinge (CD8 hinge), transmembrane domain (CD8 TM), co-stimulatory domain (4-1BB) and stimulatory domain (ITAM CD3 zeta).

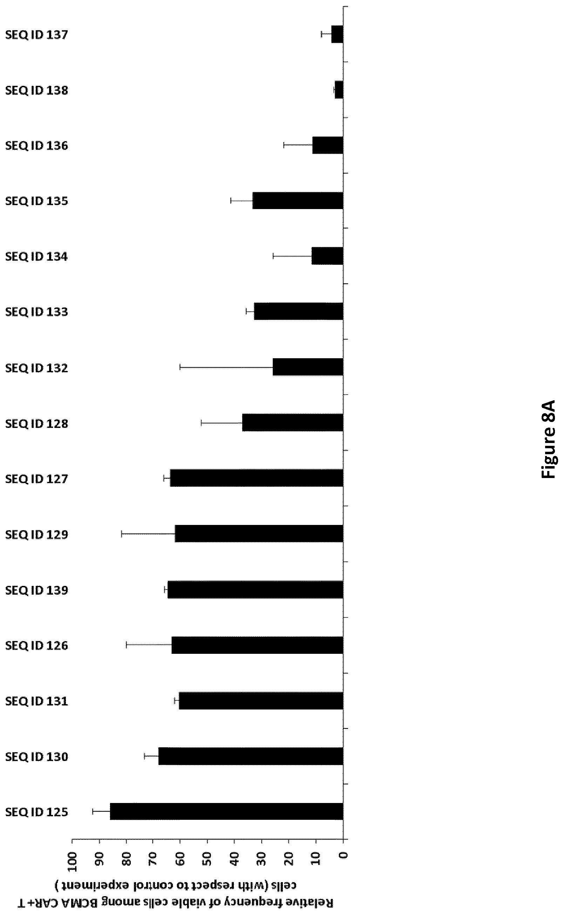

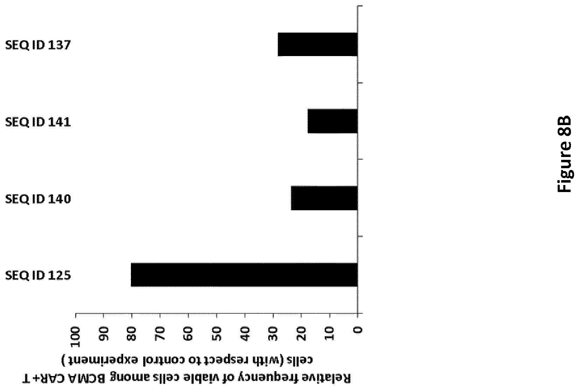

**Figure 8A and 8B** shows the result of a CDC assay where T cells expressing anti-BCMA CAR of SEQ ID NO 125 or 130 to 141 are incubated with RTX and BRC. The results are expressed as relative frequency of viable cells among anti-BCMA CAR positive T cells (with respect to control experiment) as detailed in Example 4.

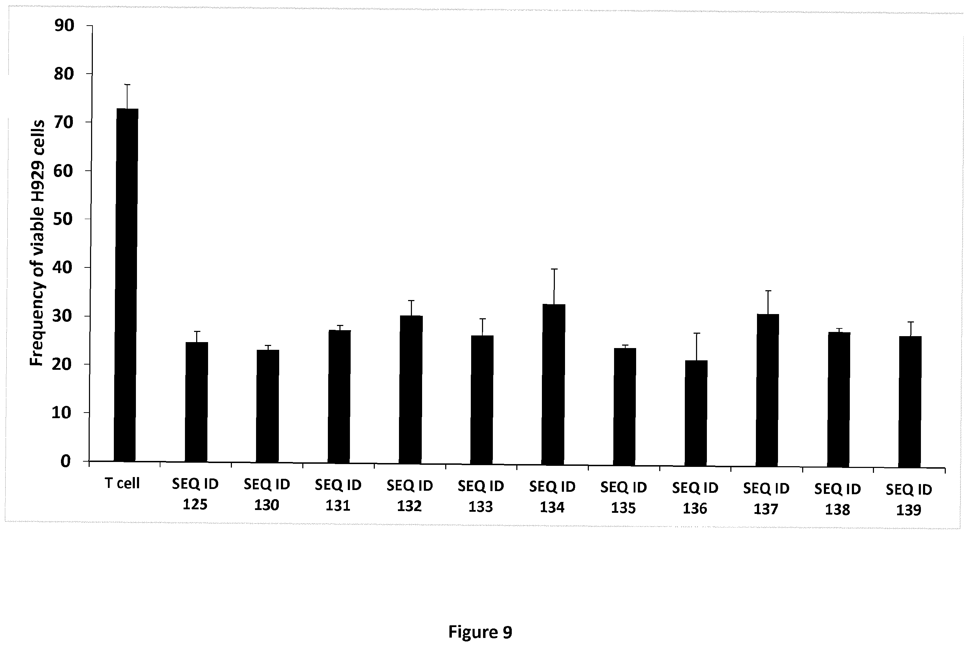

**Figure 9** shows the cytolytic activity of T cells expressing anti-BCMA CAR of SEQ ID NO 125 or 130 to 139 or control T Cell not expressing any anti BCMA CAR (T-cell). The cytolytic activity is expressed as the frequency of viable H929 cells as detailed in Example 4.

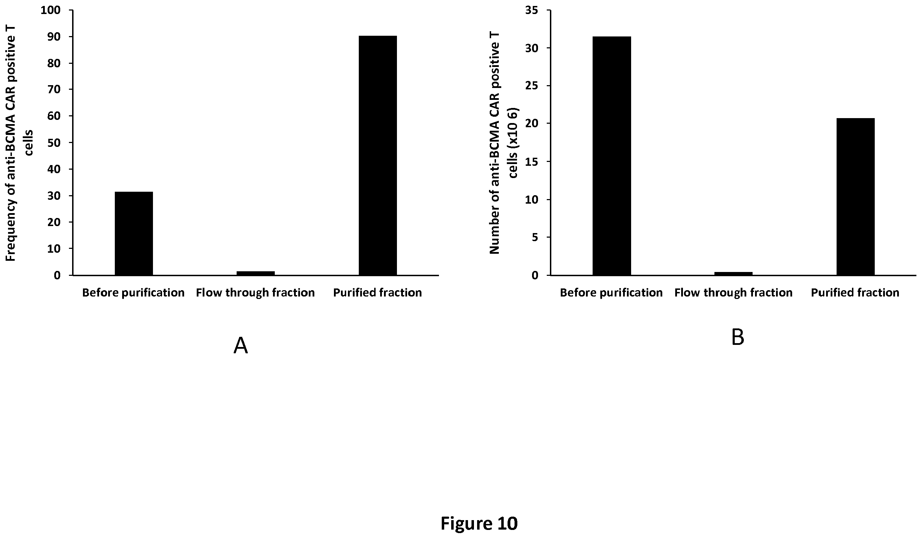

Figure 10A shows the frequency of T-cells expressing CAR of SEQ ID NO 128 comprising a CD34 epitope and two CD20 mimotopes before or after purification with CD34 MicroBead Kit or in the flow through fraction.

**Figure 10B** shows the number of T-cells expressing CAR of SEQ ID NO 128 comprising a CD34 epitope before or after purification with CD34 MicroBead Kit or in the flow through fraction.

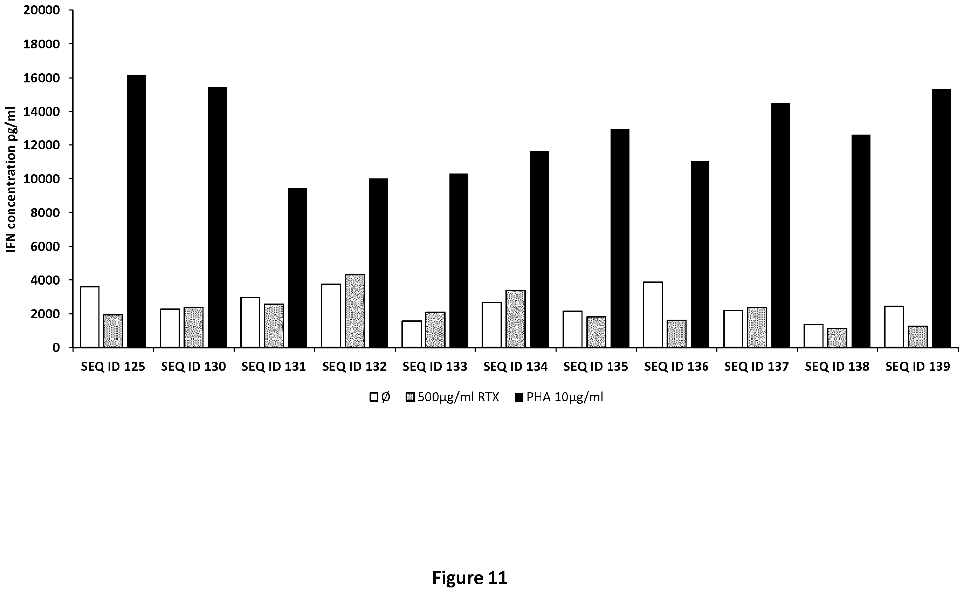

**Figure 11** shows concentration of INF gamma produced by T cell expressing anti-BCMA CAR of SEQ ID NO 125 or 130 to 139 in the presence of RTX, phytohemagglutinin (PHA) or in the absence of both RTX anf PHA.

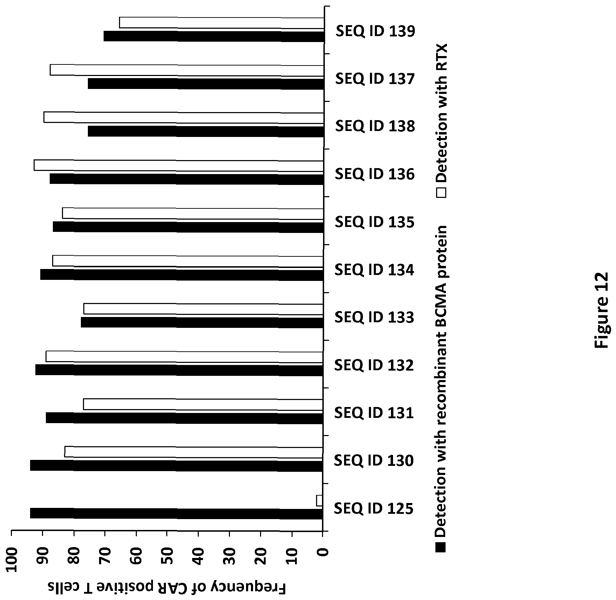

**Figure 12** shows the frequency of CAR positive T-Cell resulting from the detection of T Cell expressing CARs of SEQ ID NO 125 or 130 to 139 using a BCMA-Fc fusion protein and a labeled anti Fc antibody or RTX and a labeled anti Fc antibody.

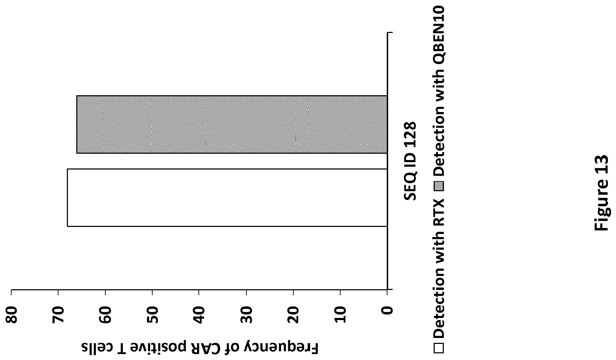

**Figure 13** shows the frequency of CAR positive T-cells resulting from the detection of T Cell expressing CARs of SEQ ID NO 128 using RTX and a labeled anti Fc antibody or labeled QBEND-10 .

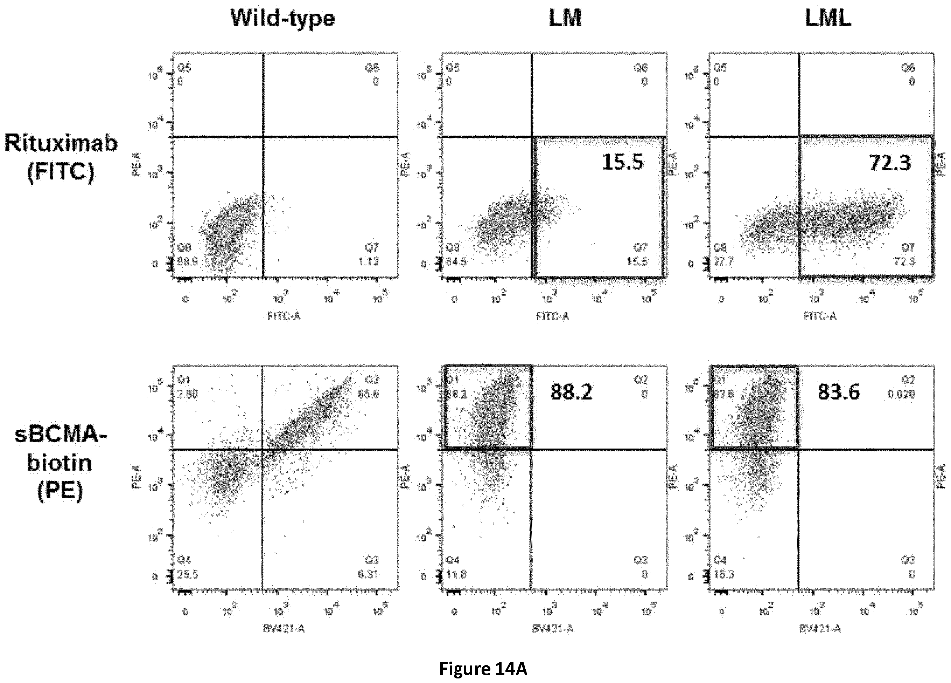

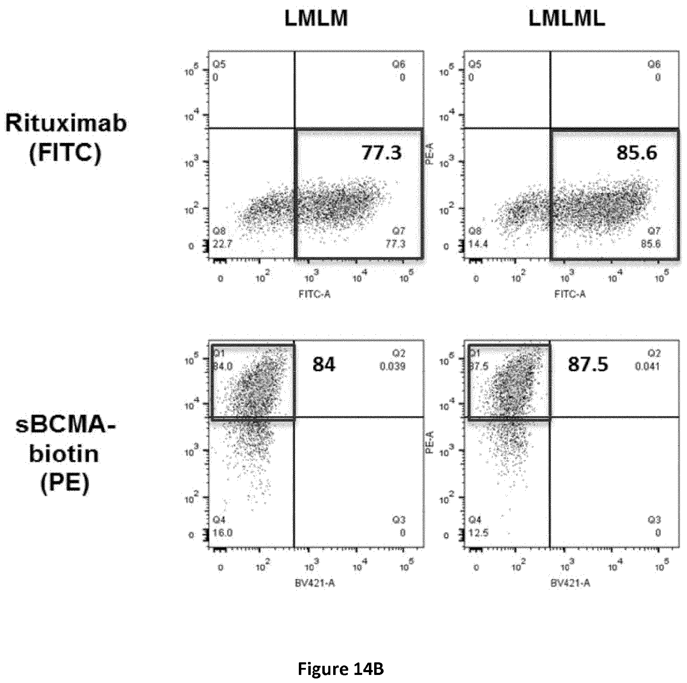

**Figure 14A and 14B** shows the detection of T Cells expressing BCMA CARs of SEQ ID NO 145 (BC30, Wild type), 146 (LM), 147 (LML), 148 (LMLM and-149 (LMLML) containing CD20 mimotopes by flow cytometry. The CAR T cells are detected by flow cytometry using either soluble biotinylated-BCMA protein followed by PE-conjugated streptavidin (sBCMA biotin (PE) or the anti-CD20 antibody rituximab followed by FITC-conjugated anti-human IgG (Rituximab (FITC)).

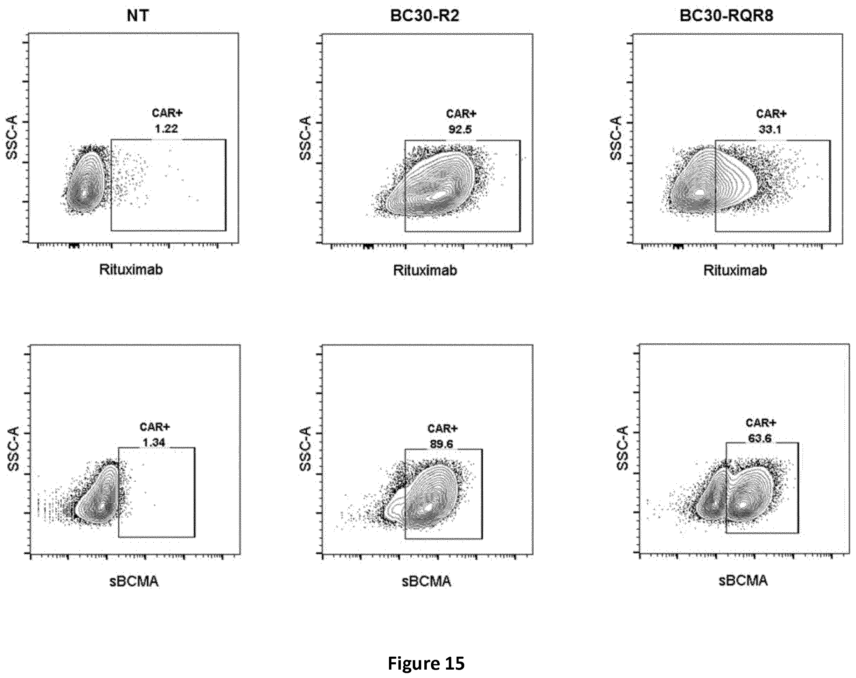

**Figure 15** shows the detection of T cells not transduced, transduced with a lentivirus for the coexpression of an anti BCMA CAR of SEQ ID NO 145 and RQR8 (SEQ ID NO 150) (BC30-RQR8), or BCMA CARs of SEQ ID NO 149 containing CD20 mimotopes by flow cytometry. The CAR-T cells are detected by flow cytometry using either soluble biotinylated-BCMA protein followed by PE-conjugated streptavidin (sBCMA biotin (PE)) or the anti-CD20 antibody rituximab followed by FITC-conjugated anti-human IgG (Rituximab (FITC)).

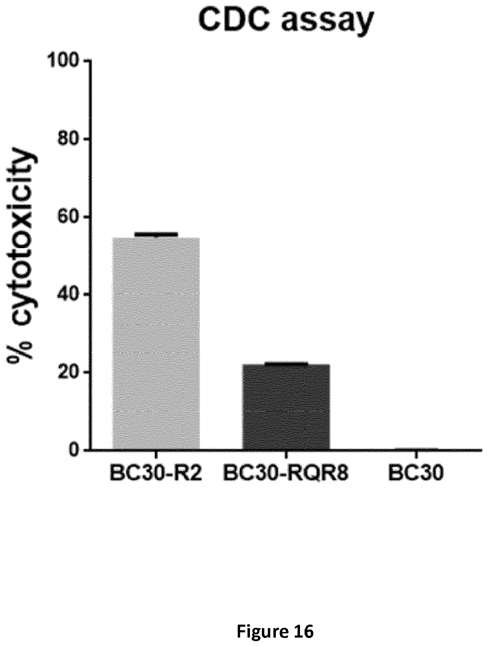

**Figure 16** shows the result of a CDC assay where T cells expressing anti-BCMA CAR of SEQ ID NO 149 (BC30-R2), anti BCMA CAR of SEQ ID NO 145 or coexpressing an anti BCMA CAR of SEQ ID NO 145 and RQR8 (SEQ ID NO 150) (BC30-RQR8) are incubated with RTX and BRC. The percentage of cytotoxicity is determined by flow cytometry analysis using biotinylated BCMA protein. The results are expressed as the frequency of cell lysis among anti-BCMA CAR positive T cells with respect to control (cells incubated with BRC only).

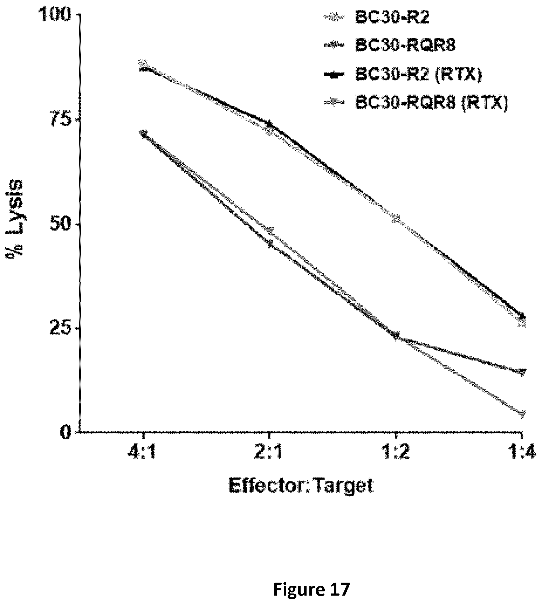

**Figure 17** shows the cytolytic activity of T cells expressing anti-BCMA CAR of SEQ ID NO 149 (BC30-R2) or coexpressing an anti BCMA CAR of SEQ ID NO 145 (BC30) and RQR8 (SEQ ID NO 150) (BC30-RQR8) in the presence/absence of RTX. The cytolytic activity is expressed as the percentage of cell lysis calculated as disclosed in example 7.5 and is determined at different ratio of effector (CAR T cell): target (MM1S cells expressing BCMA).

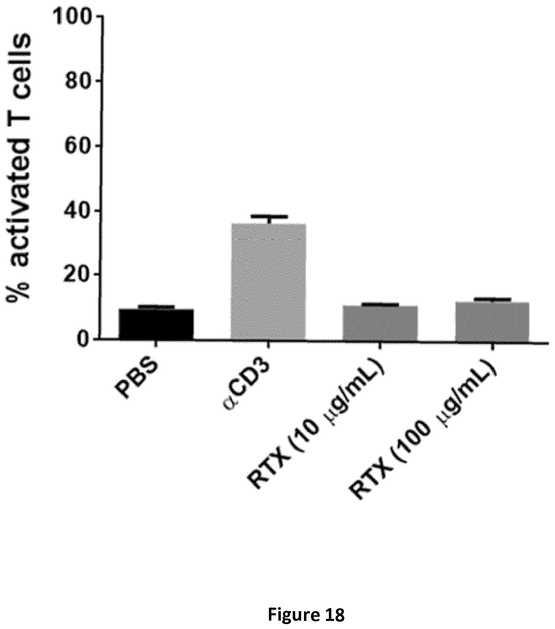

**Figure 18** shows the percentage of activated T cells expressing anti-BCMA CAR of SEQ ID NO 149 in the presence of PBS (control), anti-CD3 OKT3 antibody ($\alpha$CD3), or Rituximab (RTX). T cell activation is assessed by measuring the expression of the activation markers CD25 and CD69 using flow cytometry.

**Table 1:** Listing of the pharmaceutically-approved mAb with their antigenic targets. The sequences of the latter are provided, as well as epitope(s) for some of them.
**Table 2:** Listing of several mimotopes and epitopes corresponding to their mAb which are presented in Example 2.
**Table 3:** Listing of the VH & VL chains of scFv targeting the CD19, CD33, 5T4, ROR1, EGFRvIII, BCMA, CS1 and CD123 antigens.
**Table 4:** Exemplary sequence of CAR components

<u>DETAILED DESCRIPTION OF THE INVENTION</u>

The invention relates to a polypeptide encoding a chimeric antigen receptor (CAR) comprising at least one extracellular binding domain that comprises a scFv formed by at least a VH chain and a VL chain specific to an antigen, preferably a cell surface marker antigen, wherein said extracellular binding domain comprises at least one inserted mAb-specific epitope having the amino acid sequence of SEQ ID NO 35.
In some embodiments, the invention relates to a CAR comprising

- an extracellular domain comprising

    - at least one, preferably one, extracellular binding domain that comprises a scFv formed by at least a VH chain and a VL chain specific to an antigen, preferably a cell surface marker antigen, wherein said extracellular binding domain comprises at least one inserted mAb-specific epitope having the amino acid sequence of SEQ ID NO 35,
    - a hinge,
    - a transmembrane domain, and
    - an intracellular domain.

In embodiments, the CAR of the invention comprises one extracellular binding domain.
By "chimeric scFv" is meant a polypeptide corresponding to a single-chain variable fragment composed of heavy and light chains ($V_H$ and $V_L$, respectively) and of an epitope, which was not originally included in said $V_H$ and $V_L$ chains. The latter epitope is referred to as "mAb-specific epitope" when it has the capacity to be bound specifically by an antibody, in particular a monoclonal antibody. In some embodiments, the mAbs specific epitope is not an epitope recognized by the ScFv. In some embodiments, the mAbs specific epitope is not derived from the extracellular domain of the CAR. The components of this chimeric scFv (i.e. the light and heavy variable fragments of the ligand binding domain and the mAb specific epitope) may be joined together by at least one linker, usually a flexible linker. These components are generally joined to the transmembrane domain of the CAR by a hinge.

*Chimeric scFv conformations*

The structure of the chimeric scFv of the invention can be various as presented in the Figure 1 depending of the position of its main components ($V_H$ and $V_L$ and m-Ab specific epitope).
The chimeric scFv of the invention may have several conformations, at least 9 when considering the number of possible permutations of one $V_H$, one $V_L$ and one epitope.
Preferably, each component (VH, VL and epitope) is interconnected with its neighbor(s) by at least one flexible linker such as presented previously. The suitable combinations according to the invention are the ones which provide a good affinity/specificity in both bindings: between the mAb-specific epitope and the infused mAb, and between the VH &VL chains of the chimeric scFv and the antigen of the cell target ligand.
According to one embodiment, the extracellular-binding domain of the CAR comprises at least two linkers, both of them joining the epitope to the VH and VL chains; and a hinge joining the scFv-epitope to the transmembrane domain of the CAR. For instance, if the projected CAR conformation is such that the mAb-specific epitope is located beside the VH and VL chains, a screening is performed when the CAR is expressed and is tested for cytoxicity and/or mAb depletion.
When the mAb-specific epitope is sought for being located between the VH and VL chains, a screening may be performed by phage display before testing and/or transient expression of the CAR construct. This may be obtained by transfection of mRNA, which is sufficient for a primary cytoxicity and/or mAb depletion test.
In some embodiments, the extracellular binding domain comprises the following sequence (N-term is located on the left hand side):

$V_1$-$L_1$-$V_2$-$(L)_x$-Epitope1-$(L)_x$; $V_1$-$L_1$-$V_2$-$(L)_x$-Epitope1-$(L)_x$-Epitope2-$(L)_x$;
$V_1$-$L_1$-$V_2$-$(L)_x$-Epitope1-$(L)_x$-Epitope2-$(L)_x$-Epitope3-$(L)_x$; $(L)_x$-Epitope1-$(L)_x$-$V_1$-$L_1$-$V_2$;
$(L)_x$-Epitope1-$(L)_x$-Epitope2-$(L)_x$-$V_1$-$L_1$-$V_2$;
Epitope1-$(L)_x$-Epitope2-$(L)_x$-Epitope3-$(L)_x$-$V_1$-$L_1$-$V_2$;$(L)_x$-Epitope1-$(L)_x$-$V_1$-$L_1$-$V_2$-$(L)_x$-Epitope2-$(L)_x$;$(L)_x$-Epitope1-$(L)_x$-
$V_1$-$L_1$-$V_2$-$(L)_x$-Epitope2-$(L)_x$-Epitope3-$(L)_x$;$(L)_x$-Epitope1-$(L)_x$-$V_1$-$L_1$-$V_2$-$(L)_x$-Epitope2-$(L)_x$-Epitope3-$(L)_x$-Epitope4-$(L)_x$;
$(L)_x$-Epitope1-$(L)_x$-Epitope2-$(L)_x$-$V_1$-$L_1$-$V_2$-$(L)_x$-Epitope3-$(L)_x$;
$(L)_x$-Epitope1-$(L)_x$-Epitope2-$(L)_x$-$V_1$-$L_1$-$V_2$-$(L)_x$-Epitope3-$(L)_x$-Epitope4-$(L)_x$; $V_1$-$(L)_x$-Epitope1-$(L)_x$-$V_2$;
$V_1$-$(L)_x$-Epitope1-$(L)_x$-$V_2$-$(L)_x$-Epitope2-$(L)_x$; $V_1$-$(L)_x$-Epitope1-$(L)_x$-$V_2$-$(L)_x$-Epitope2-$(L)_x$-Epitope3-$(L)_x$;
$V_1$-$(L)_x$-Epitope1-$(L)_x$-$V_2$-$(L)_x$-Epitope2-$(L)_x$-Epitope3-$(L)_x$-Epitope4-$(L)_x$; $(L)_x$-Epitope1-$(L)_x$-$V_1$-$(L)_x$-Epitope2-$(L)_x$-$V_2$;
$(L)_x$-Epitope1-$(L)_x$-$V_1$-$(L)_x$-Epitope2-$(L)_x$-$V_2$-$(L)_x$-Epitope3-$(L)_x$; $V_1$-$L_1$-$V_2$-L-Epitope1; $V_1$-$L_1$-$V_2$-L-Epitope1-L;
$V_1$-$L_1$-$V_2$-L-Epitope1-L-Epitope2; $V_1$-$L_1$-$V_2$-L-Epitope1-L-Epitope2-L; $V_1$-$L_1$-$V_2$-L-Epitope1-L-Epitope2-L-Epitope3;
$V_1$-$L_1$-$V_2$-L-Epitope1-L-Epitope2-L-Epitope3-L; $V_1$-$L_1$-$V_2$-Epitope1; $V_1$-$L_1$-$V_2$-Epitope1-L; $V_1$-$L_1$-$V_2$-Epitope1-L-
Epitope2; $V_1$-$L_1$-$V_2$-Epitope1-L-Epitope2-L; $V_1$-$L_1$-$V_2$-Epitope1-L-Epitope2-L-Epitope3; $V_1$-$L_1$-$V_2$-Epitope1-L-Epitope2-
L-Epitope3-L; Epitope1-$V_1$-$L_1$-$V_2$; Epitopel-L-$V_1$-$L_1$-$V_2$; L-Epitopel-$V_1$-$L_1$-$V_2$;
L-Epitope1-L-$V_1$-$L_1$-$V_2$; Epitope1-L-Epitope2-$V_1$-$L_1$-$V_2$; Epitope1-L-Epitope2-L-$V_1$-$L_1$-$V_2$; L-Epitope1-L-Epitope2-
$V_1$-$L_1$-$V_2$; L-Epitope1-L-Epitope2-L-$V_1$-$L_1$-$V_2$; Epitope1-L-Epitope2-Epitope3-$V_1$-$L_1$-$V_2$; Epitope1-L-Epitope2-L-
Epitope3-L-$V_1$-$L_1$-$V_2$; L-Epitope1-L-Epitope2-L-Epitope3-$V_1$-$L_1$-$V_2$; L-Epitope1-L-Epitope2-L-Epitope3-L-$V_1$-$L_1$-$V_2$;
$V_1$-L-Epitope1-L-$V_2$; L-Epitope1-L-$V_1$-L-Epitope2-L-$V_2$; $V_1$-L-Epitope1-L-$V_2$-L-Epitope2-L; $V_1$-L-Epitope1-L-$V_2$-L-
Epitope2-L-Epitope3; $V_1$-L-Epitope1-L-$V_2$-L-Epitope2-Epitope3; $V_1$-L-Epitope1-L-$V_2$-L-Epitope2-L-Epitope3-Epitope4;
L-Epitope1-L-$V_1$-L-Epitope2-L-$V_2$-L-Epitope3-L; Epitope1-L-$V_1$-L-Epitope2-L-$V_2$-L-Epitope3-L; L-Epitope1-L-$V_1$-L-
Epitope2-L-$V_2$-L-Epitope3; L-Epitope1-L-$V_1$-$L_1$-$V_2$-L-Epitope2-L; L-Epitope1-L-$V_1$-$L_1$-$V_2$-L-Epitope2-L-Epitope3; L-
Epitope1-L-$V_1$-$L_1$-$V_2$-L-Epitope2-Epitope3, or Epitope1-L-$V_1$-$L_1$-$V_2$-L-Epitope2-L-Epitope3-Epitope4.
wherein,

$V_1$ and $V_2$ are $V_H$ and $V_L$ of an ScFv (i.e , $V_1$ is $V_L$ and $V_2$ is $V_H$ or $V_1$ is $V_H$ and $V_2$ is $V_L$);
$L_1$ is any linker suitable to link the $V_H$ chain to the $V_L$ chain in an ScFv;
L is a linker, preferably comprising glycine and serine residues, and each occurrence of L in the extracellular binding domain can be identical or different to other occurrence of L in the same extracellular binding domain, and,
x is 0 or 1 and each occurrence of x is independently from the others; and
Epitope 1, Epitope 2 and Epitope 4 are a mAb-specific epitope having the amino acid sequence of SEQ ID NO 35; and Epitope 3 is selected from mAb-specific epitopes having the amino acid sequence of SEQ ID NO 35 or SEQ ID NO 144.

In some embodiments, the extracellular binding domains comprises the following sequence (Nterm is located on the left hand side):
$V_H$-$L_1$-$V_L$-L-Epitope1-L-Epitope2-L; L-Epitope1-L-$V_H$-L-Epitope2-L-$V_L$-L-Epitope3-L; $V_L$-$L^1$-$V_H$-L-Epitope1-L-Epitope2-L; or, L-Epitope1-L-$V_L$-L-Epitope2-L-$V_H$-L-Epitope3-L,
wherein L, $L_1$, Epitope1, Epitope2 and Epitope3 are as defined above.
In some embodiments, $L_1$ is a linker comprising Glycine and/or Serine. In some embodiments, $L_1$ is a linker comprising the amino acid sequence (Gly-Gly-Gly-Ser)$_n$ or (Gly-Gly-Gly-Gly-Ser)$_n$, where n is 1, 2, 3, 4 or 5. In some embodiments $L_1$ is (Gly$_4$Ser)$_4$ or (Gly$_4$Ser)$_3$.
In some embodiments, L is a flexible linker, preferably comprising Glycine and/or Serine. In some embodiments, L has an amino acid sequence selected from SGG, GGS, SGGS, SSGGS, GGGG, SGGGG, GGGGS, SGGGGS, GGGGGS, SGGGGGS, SGGGGG, GSGGGGS, GGGGGGGS, SGGGGGGG, SGGGGGGGS, or SGGGGSGGGGS, preferably SGG, SGGS, SSGGS, GGGG, SGGGGS, SGGGGGS, SGGGGG, GSGGGGS or SGGGGSGGGGS. In some embodiments, when the extracellular binding domain comprises several occurrences of L, all the Ls are identical. In some embodiments, when the extracellular binding domain comprises several occurrences of L, the Ls are not all identical. In some embodiments, L is SGGGGS. In some embodiments, the extracellular binding domain comprises several occurrences of L and all the Ls are SGGGGS.
In some embodiments, Epitope 3 is a mAb-specific epitope having the amino acid sequence of SEQ ID NO 35. In some embodiments, Epitope 3 is an mAb-specific epitope having the amino acid sequence of SEQ ID NO 144.

*Inserted mAb-specific epitope*

The epitope to be inserted within the chimeric scFv is specific to the monoclonal antibody (mAb) which is used for cell sorting and/or cell depletion processes.
The introduced epitope within chimeric scFv is chosen as part of a mAb-specific epitope/epitope-specific mAb couple, on the basis of their approval by National Health Agencies in terms of regulatory/safety. Such couples are presented in the following table 1.

**Table 1:** Listing of pharmaceutically-approved monoclonal antibodies with their antigenic targets.

| Antibody | Indication | Drug bank accession n° (or other n° if stated) | Target/Antigen | SEQ ID NO |
|---|---|---|---|---|
| Murine | | | | |
| Ibritumomab tiuxetan | Non-Hodgkin lymphoma (with yttrium-90 or indium-111) | DB00078 | CD20 | SEQ ID NO 11 |
| Muromonab-CD3 | Transplant rejection | DB00075 | T cell CD3 Receptor | SEQ ID NO 12 |
| Tositumomab | Non-Hodgkin lymphoma | DB00081 | CD20 | SEQ ID NO 11 |
| Chimeric | | | | |
| Abciximab | Cardiovascular disease | DB00054 | inhibition of glycoprotein IIb/IIIa | SEQ ID NO 13 |
| Basiliximab | Transplant rejection | DB00074 | IL-2Ra receptor (CD25) | SEQ ID NO 14 |
| Brentuximab vedotin | Anaplastic large cell lymphoma | DB08870 | CD30 | SEQ ID NO 15 |
| Cetuximab | Colorectal cancer, Head and neck cancer | DB00002 | epidermal growth factor receptor | SEQ ID NO 16 |
| Infliximab | Several autoimmune disorders | DB00065 | inhibition of TNF-$\alpha$ signaling | SEQ ID NO 17 |
| Rituximab | Non-Hodgkin lymphoma | DB00073 | CD20 | SEQ ID NO 11 |
| Humanized | | | | |
| Alemtuzumab | Chronic lymphocytic leukemia | DB00087 | CD52 | SEQ ID NO 18 |
| Bevacizumab | Colorectal cancer, Age related macular degeneration (off-label) | DB00112 | Vascular endothelial growth factor (VEGF) | SEQ ID NO 19 |
| Certolizumab pegol | Crohn's disease | DB08904 | inhibition of TNF-$\alpha$ signaling | SEQ ID NO 17 |
| Daclizumab | Transplant rejection | DB00111 | IL-2Ra receptor (CD25) | SEQ ID NO 14 |
| Eculizumab | Paroxysmal nocturnal hemoglobinuria | DB01257 | Complement system protein | SEQ ID NO 20 |

(continued)

| Humanized | | | | |
|---|---|---|---|---|
| Efalizumab | Psoriasis | DB00095 | CD11a | SEQ ID NO 21 |
| Gemtuzumab | Acute myelogenous leukemia (with calicheamicin) | DB00056 | CD33 | SEQ ID NO 22 |
| Natalizumab | Multiple sclerosis and Crohn's disease | DB00108 | alpha-4 ($\alpha$4) integrin | SEQ ID NO 23 |
| Omalizumab | mainly allergy-related asthma | DB00043 | immunoglobulin E (IgE) | SEQ ID NO 24 |
| Palivizumab | Respiratory Syncytial Virus | DB00110 | an epitope of the RSV F protein | SEQ ID NO 25 |
| Ranibizumab | Macular degeneration | DB01270 | Vascular endothelial growth factor A (VEGF-A) | SEQ ID NO 19 |
| Tocilizumab (or Atlizumab) | Rheumatoid arthritis | DB06273 | Anti- IL-6R | SEQ ID NO 26 |
| Trastuzumab | Breast cancer | DB00072 | ErbB2 | SEQ ID NO 27 |
| Vedolizumab | Crohn's disease, ulcerative colitis | CAS n°943609-66-3 | integrin $\alpha_4\beta_7$ | SEQ ID NO 28 |
| Human | | | | |
| Adalimumab | Several auto-immune disorders | DB00051 | inhibition of TNF-$\alpha$ signaling | SEQ ID NO 17 |
| Belimumab | Systemic lupus erythematosus | DB08879 | inihibition of B-cell activating factor | SEQ ID NO 29 |
| Canakinumab | Cryopyrin-associated periodic syndrome (CAPS) | DB06168 | IL-1$\beta$ | SEQ ID NO 30 |
| Denosumab | Postmenopausal osteoporosis, Solid tumor's bony metastases | DB06643 | RANK Ligand inhibitor | SEQ ID NO 31 |
| Golimumab | Rheumatoid arthritis, Psoriatic arthritis, and Ankylosing spondylitis | DB06674 | TNF-alpha inihibitor | SEQ ID NO 17 |
| Ipilimumab (MDX-101) | Melanoma | DB06186 | blocks CTLA-4 | SEQ ID NO 32 |

(continued)

| Human | | | | |
|---|---|---|---|---|
| Ofatumumab | Chronic lymphocytic leukemia | CAS n° 679818-59-8 | CD20 | SEQ ID NO 11 |
| Panitumumab | Colorectal cancer | DB01269 | epidermal growth factor receptor | SEQ ID NO 16 |
| Ustekinumab | Psoriatic Arthritis, Plaque Psoriasis | DB05679 | IL-12, IL-23 | SEQ ID NO 33 |
| Nivolumab | renal cell carcinoma, lung cancer, melanoma, and advanced or metastatic solid tumors | CAS n°946414-94-4 | PD-1 | SEQ ID NO 34 |

**Table 2:** Examples of mAb-specific epitopes (and their corresponding mAbs) that can be used in the extracellular binding domain of the CAR of the invention such as for example mimotopes and epitope with their corresponding mAb as used in the Examples 1-2

| **Rituximab** | | |
|---|---|---|
| Mimotope | SEQ ID NO 35 | CPYSNPSLC |
| **Palivizumab** | | |
| Epitope | SEQ ID NO 36 | NSELLSLINDMPITNDQKKLMSNN |
| **Cetuximab** | | |
| Mimotope 1 | SEQ ID NO 37 | CQFDLSTRRLKC |
| Mimotope 2 | SEQ ID NO 38 | CQYNLSSRALKC |
| Mimotope 3 | SEQ ID NO 39 | CVWQRWQKSYVC |
| Mimotope 4 | SEQ ID NO 40 | CMWDRFSRWYKC |
| **Nivolumab** | | |
| Epitope 1 | SEQ ID NO 41 | SFVLNWYRMSPSNQTDKLAAFPEDR |
| Epitope 2 | SEQ ID NO 42 | SGTYLCGAISLAPKAQIKE |
| **QBEND-10** | | |
| Epitope | SEQ ID NO 144 | ELPTQGTFSNVSTNVSPAKPTTTA |
| **Alemtuzumab** | | |
| Epitope | SEQ ID NO 174 | GQNDTSQTSSPS |

According to the invention, the at least one epitope introduced within the chimeric scFv is the CD20 antigen of SEQ ID NO 35 and the infused mAb which is being used to target it -for sorting and/or depletion purpose(s) is rituximab.

In some embodiments, the extracellular binding domain of the CAR of the invention comprises one mAb-specific epitope of SEQ ID NO 35, two mAb-specific epitopes of SEQ ID NO 35, three mAb-specific epitopes of SEQ ID NO 35, one mAb-specific epitope of SEQ ID NO 35 and one mAb-specific epitope of SEQ ID NO 144, two mAb-specific epitopes of SEQ ID NO 35 and one mAb-specific epitope of SEQ ID NO 144, three mAb-specific epitopes of SEQ ID NO 35 and one mAb-specific epitope of SEQ ID NO 144.

According to another embodiment, the epitope is a mimotope. As a macromolecule, often a peptide, which mimics the structure of an epitope, the mimotope has the advantage to be smaller than conventional epitope, and therefore may be beneficial for a non-conformational sequence and easier to reproduce in a long polypeptide such a CAR. Mimotopes are known for several pharmaceutically-approved mAb such as two 10 amino acid peptides for cetuximab (Riemer et al., 2005), or a 24 aa for palivizumab (Arbiza et al, 1992). As these mimotopes can be identified by phage display, it is possible to try several of them in order to obtain a sequence which does not perturb the scFv for the same mAb. Furthermore, their use can enhance a complement-dependent cytotoxicity (CDC).

*scFv*

The term "extracellular ligand-binding domain" as used herein is defined as an oligo- or polypeptide that is capable of binding a ligand. Preferably, said domain is sought for being capable of interacting with a cell surface molecule. For example, the extracellular ligand-binding domain may be chosen to recognize a ligand that acts as a cell surface marker on target cells associated with a particular disease state. Thus examples of cell surface markers that may act as ligands include those associated with viral, bacterial and parasitic infections, autoimmune disease and cancer cells. In particular, the extracellular ligand-binding domain can comprise an antigen binding domain derived from an antibody against an antigen of the target. As non-limiting examples, the antigen of the target can be a tumor-associated surface antigen as described above. In some embodiments, the extracellular binding domain is an extracellular ligand-binding domain as defined above. According to the present invention, said extracellular ligand-binding domain is a single chain antibody fragment (scFv) comprising the light ($V_L$) and the heavy ($V_H$) variable fragment of a target antigen specific monoclonal antibody, and an mAb epitope specific antigen. In some embodiments, the extracellular binding domain comprises a single chain antibody fragment (scFv) comprising the light ($V_L$) and the heavy ($V_H$) variable fragment of a cell surface target antigen specific monoclonal antibody.

Other binding domain than scFv can also be used for predefined targeting of lymphocytes, such as camelid single-domain antibody fragments, receptor ligands like a vascular endothelial growth factor polypeptide, an integrin-binding peptide, heregulin or an IL-13 mutein, antibody binding domains, antibody hypervariable loops or CDRs as non-limiting examples.

In another embodiment, said extracellular binding domain can be a DARPin (designed ankyrin repeat protein). DARPins are genetically engineered antibody mimetic proteins typically exhibiting highly specific and high-affinity target protein binding. They are derived from natural ankyrin proteins and comprise at least three, usually four or five repeat motifs of these proteins. DARPins are small, single domain proteins which can be selected to bind any given target protein with high affinity and specificity (Epa, Dolezal et al. 2013; Friedrich, Hanauer et al. 2013; Jost, Schilling et al. 2013). According to the present invention, DARPins can be engineered to comprise multiple antigen recognition sites. Thus, said DARPins can be used to recognize a series of consecutive different antigens as well as a unique antigen. Thus, the present invention relates to a method comprising providing an immune cell, and expressing at the surface of said immune cell chimeric antigen receptor which comprises a designed ankyrin repeat protein capable of recognizing at least one specific ligand, preferably at two specific ligands.

As non-limiting example, the ligand of the target or the antigen recognized by the extracellular binding domain, preferably by the ScFv, can be a tumor-associated surface antigen, such as ErbB2 (HER2/neu), carcinoembryonic antigen (CEA), epithelial cell adhesion molecule (EpCAM), epidermal growth factor receptor (EGFR), EGFR variant III (EGFRvIII), CD19, CD20, CD30, CD40, disialoganglioside GD2, GD3, C-type lectin-like molecule-1 (CLL-1), ductal-epithelial mucine, gp36, TAG-72, glycosphingolipids, glioma-associated antigen, β-human chorionic gonadotropin, alphafetoprotein (AFP), lectin-reactive AFP, thyroglobulin, RAGE-1, MN-CA IX, human telomerase reverse transcriptase, RU1, RU2 (AS), intestinal carboxyl esterase, mut hsp70-2, M-CSF, prostase, prostase specific antigen (PSA), PAP, NY-ESO-1, LAGA-1a, p53, prostein, PSMA, surviving and telomerase, prostate-carcinoma tumor antigen-1 (PCTA-1), MAGE, ELF2M, neutrophil elastase, ephrin B2, CD22, insulin growth factor (IGF1)-I, IGF-II, IGFI receptor, mesothelin, a major histocompatibility complex (MHC) molecule presenting a tumor-specific peptide epitope, 5T4, ROR1, Nkp30, NKG2D, tumor stromal antigens, the extra domain A (EDA) and extra domain B (EDB) of fibronectin and the A1 domain of tenascin-C (TnC A1) and fibroblast associated protein (fap), LRP6, melamona-associated Chondroitin Sulfate Proteoglycan (MCSP), CD38/CS1, MART1, WT1, MUC1, LMP2, Idiotype, NY-ESO-1, Ras mutant, gp100, proteinase 3, bcr-abl, tyrosinase, hTERT, EphA2, ML-TAP, ERG, NA17, PAX3, ALK, Androgen receptor ; a lineage-specific or tissue specific antigen such as CD3, CD4, CD8, CD24, CD25, CD33, CD34, CD70, CD79, CD116, CD117, CD135, CD123, CD133, CD138, CTLA-4, B7-1 (CD80), B7-2 (CD86), endoglin, a major histocompatibility complex (MHC) molecule, BCMA (CD269, TNFRSF 17), FLT-3, or a virus-specific surface antigen such as an HIV-specific antigen (such as HIV gp120); an EBV-specific antigen, a CMV-specific antigen, a HPV-specific antigen, a Lasse Virus-specific antigen, an Influenza Virus-specific antigen as well as any derivate or variant of these surface markers. In specific cases, the ligand that the chimeric antigen receptor recognizes is present on the surface of a target cell, particularly cancer cell or viral cell. In some embodiments, the ligand that the chimeric antigen receptor recognizes is present in a tumor microenvironment. In some aspects of the invention, the ligand that the chimeric antigen receptor recognizes is a growth factor.

In one preferred embodiment, said VH and VL chains have as antigenic target sequence of over 80% identity, preferably over 90%, and more preferably over 95% with SEQ ID NO 43 (CD19 antigen), SEQ ID NO 44 (CD38 antigen), SEQ ID NO 45 (CD123 antigen), SEQ ID NO 46 (CS1 antigen), SEQ ID NO 47 (BCMA antigen), SEQ ID NO 48 (FLT-3 antigen), SEQ ID NO 49 (CD33 antigen), SEQ ID NO 50 (CD70 antigen), SEQ ID NO 51 (EGFR-3v antigen), SEQ ID NO 52 (WT1 antigen).

In one more preferred embodiment, said VH and VL chains have as antigenic target sequence of over 80% identity, preferably over 90%, and more preferably over 95% with or identical to SEQ ID NO 53-64 (CD19 antigen), SEQ ID NO 65-76 (CD33 antigen), SEQ ID NO 77-84 (5T4 antigen), SEQ ID NO 85-90 (ROR1 antigen), SEQ ID NO 91-94 (EGFRvIII antigen), SEQ ID NO 95-102 (BCMA antigen), SEQ ID NO 103-112 (CS1 antigen) and SEQ ID NO 113-124 (CD123 antigen) as follows in Table 3.

In some embodiments, the antigen recognized by the extracellular binding domain, preferably by the ScFv is selected from SEQ ID NO 43 (CD19 antigen), SEQ ID NO 44 (CD38 antigen), SEQ ID NO 45 (CD123 antigen), SEQ ID NO 46 (CS1 antigen), SEQ ID NO 47 (BCMA antigen), SEQ ID NO 48 (FLT-3 antigen), SEQ ID NO 49 (CD33 antigen), SEQ ID NO 50 (CD70 antigen), SEQ ID NO 51 (EGFR-vIII antigen) or SEQ ID NO 52 (WT1 antigen).

In some embodiments, the extracellular binding domain comprises:

- a VH of SEQ ID NO 65 and a VL of SEQ ID NO 66; a VH of SEQ ID NO 67 and a VL of SEQ ID NO 68; a VH of SEQ ID NO 69 and a VL of SEQ ID NO 70; a VH of SEQ ID NO 71 and a VL of SEQ ID NO 72; a VH of SEQ ID NO 77 and a VL of SEQ ID NO 78; a VH of SEQ ID NO 79 and a VL of SEQ ID NO 80;

- a VH of SEQ ID NO 81 and a VL of SEQ ID NO 82; a VH of SEQ ID NO 83 and a VL of SEQ ID NO 84; a VH of SEQ ID NO 85 and a VL of SEQ ID NO 86; a VH of SEQ ID NO 87 and a VL of SEQ ID NO 88; a VH of SEQ ID NO 89 and a VL of SEQ ID NO 90; a VH of SEQ ID NO 91 and a VL of SEQ ID NO 92; a VH of SEQ ID NO 93 and a VL of SEQ ID NO 94; a VH of SEQ ID NO 95 and a VL of SEQ ID NO 96; a VH of SEQ ID NO 97 and a VL of SEQ ID NO 98; a VH of SEQ ID NO 99 and a VL of SEQ ID NO 100; a VH of SEQ ID NO 101 and a VL of SEQ ID NO 102; a VH of SEQ ID NO 103 and a VL of SEQ ID NO 104; a VH of SEQ ID NO 105 and a VL of SEQ ID NO 106; a VH of SEQ ID NO 107 and a VL of SEQ ID NO 108; a VH of SEQ ID NO 109 and a VL of SEQ ID NO 110; a VH of SEQ ID NO 111 and a VL of SEQ ID NO 112; a VH of SEQ ID NO 113 and a VL of SEQ ID NO 114; a VH of SEQ ID NO 115 and a VL of SEQ ID NO 116; a VH of SEQ ID NO 117 and a VL of SEQ ID NO 118; a VH of SEQ ID NO 119 and a VL of SEQ ID NO 120; a VH of SEQ ID NO 121 and a VL of SEQ ID NO 122; a VH of SEQ ID NO 123 and a VL of SEQ ID NO 124; a VH of SEQ ID NO 170 and a VL of SEQ ID NO 171; a VH of SEQ ID NO 172 and a VL of SEQ ID NO 173; or, a VH of SEQ ID NO 186 and a VL of SEQ ID NO 187.

**Table 3**: Listing of the VH & VL chains of scFv targeting the CD19, CD33, 5T4, ROR1, EGFRvIII, BCMA, CS1 and CD123 antigens

| Cell surface antigen | Name of scFV & VH or VL chain | SEQ ID NO | Polypeptide sequence |
|---|---|---|---|
| CD19 | CD19-1 VH chain | 53 | EVKLQESGPGLVAPSQSLSVTCTVSGVSLPDYGVSWIRQPPRKGLEWLGVIWG SETTYYNSALKSRLTIIKDNSKSQVFLKMNSLQTDDTAIYYCAKHYYYGGSYAM DYWGQGTSVTV |
| | CD19-1 VL chain | 54 | DIQMTQTTSSLSASLGDRVTISCRASQDISKYLNWYQQKPDGTVKLLIYHTSRL HSGVPSRFSGSGSGTDYSLTISNLEQEDIATYFCQQGNTLPYTFGGGTKLEIT |
| | CD19-2 VH chain | 55 | EVQLQQSGPELIKPGASVKMSCKASGYTFTSYVMHWVKQKPGQGLEWIGYI NPYNDGTKYNEKFKGKATLTSDKSSSTAYMELSSLTSEDSAVYYCARGTYYYGS RVFDYWGQGTTLTV |
| | CD19-2 VL chain | 56 | DIVMTQAAPSIPVTPGESVSISCRSSKSLLNSNGNTYLYWFLQRPGQSP QLLIYRMSNLASGVPDRFSGSGSGTAFTLRISRVEAEDVGVYYCMQHL EYPFTFGAGTKLELK |

(continued)

| Cell surface antigen | Name of scFV & VH or VL chain | SEQ ID NO | Polypeptide sequence |
|---|---|---|---|
| | 26292 VH chain | 57 | QVQLQQPGAELVRPGASVKLSCKASGYTFTSYWMNWVKQRPDQGLEWIGRI DPYDSETHYNQKFKDKAILTVDKSSSTAYMQLSSLTSEDSAVYYCARGNWDDY WGQGTTLTVSS |
| | 26292 VL chain | 58 | DVQITQSPSYLAASPGETITINCRASKSISKDLAWYQEKPGKTNKLLIYSGSTLQS GIPSRFSGSGSGTDFTLTISSLEPEDFAMYYCQQHNKYPYTFGGGTKLEIK |
| | 32716 VH chain | 59 | QIQLVQSGPELKKPGETVKISCKASGYIFTNYGMNWVKQAPGKSFKWMGWI NTYTGESTYSADFKGRFAFSLETSASTAYLHINDLKNEDTATYFCARSGGYDPM DYWGQGTSVTVSS |
| | 32716 VL chain | 60 | DIVLTQSPASLAVSLGQRATISCRASESVDNYGNTFMHWYQQKPGQPPKLLIY RASNLESGIPARFSGSGSRTDFTLTINPVEADDVATYYCQQSNEDPPTFGAGTK LELK |
| | Klon43 VH chain | 61 | MADYKDIVMTQSHKFMSTSVGDRVNITCKASQNVDSAVAWYQQKPGQSPK ALIYSASYRYSGVPDRFTGRGSGTDFTLTISSVQAEDLAVYYCQQYYSTPWTFG GGTKLEIKR |
| | Klon43 VL chain | 62 | EVKLVESGGGLVQPGGSLSLSCAASGFTFTDYYMSWVRQPPGKALEWLALIRS KADGYTTEYSASVKGRFTLSRDDSQSILYLQMNALRPEDSATYYCARDAAYYSY YSPEGAMDYWGQGTSVTVSS |
| | 12F1 VH chain | 63 | VQLQESGPGLVKPSQSLSLTCSVTDYSITSGYYWNWIRQFPGNKLEWMGYISY DGSNNYNPSLKNRISITRDTSKNQFFLKLSSVTTEDTATYYCSRGEGFYFDSWG QGTTLTVSSARS |
| | 12F1 VL chain | 64 | DIMMSQSPSSLAVSVGEKFTMTCKSSQSLFFGSTQKNYLAWYQQKPGQSPKL LIYWASTRESGVPDRFTGSGSGTDFTLAISSVMPEDLAVYYCQQYYNYPWTFG GGTKLEIK |

(continued)

| Cell surface antigen | Name of scFV & VH or VL chain | SEQ ID NO | Polypeptide sequence |
|---|---|---|---|
| CD33 | M195 VH chain | 65 | EVQLQQSGPELVKPGASVKISCKASGYTFTDYNMHWVKQSHGKSLEWIGYIY PYNGGTGYNQKFKSKATLTVDNSSSTAYMDVRSLTSEDSAVYYCARGRPAMD YWGQGTSVTVS |
| | M195 VL chain | 66 | DIVLTQSPASLAVSLGQRATISCRASESVDNYGISFMNWFQQKPGQPPKLLIYA ASNQGSGVPARFSGSGSGTDFSLNIHPMEEDDTAMYFCQQSKEVPWTFGGG TKLEIK |
| | m2H12 VH chain | 67 | QVQLQQSGPELVRPGTFVKISCKASGYTFTNYDINWVNQRPGQGLEWIGWIY PGDGSTKYNEKFKAKATLTADKSSSTAYLQLNNLTSENSAVYFCASGYEDAMD YWGQGTSVTVSS |
| | m2H12 VL chain | 68 | DIKMTQSPSSMYASLGERVIINCKASQDINSYLSWFQQKPGKSPKTLIYRANRL VDGVPSRFSGSGSGQDYSLTISSLEYEDMGIYYCLQYDEFPLTFGAGTKLELKR |
| | DRB2 VH chain | 69 | EVKLQESGPELVKPGASVKMSCKASGYKFTDYVVHWLKQKPGQGLEWIGYIN PYNDGTKYNEKFKGKATLTSDKSSSTAYMEVSSLTSEDSAVYYCARDYRYEVYG MDYWGQGTSVTVSS |
| | DRB2 VL chain | 70 | DIVLTQSPTIMSASPGERVTMTCTASSSVNYIHWYQQKSGDSPLRWIFDTSKV ASGVPARFSGSGSGTSYSLTISTMEAEDAATYYCQQWRSYPLTFGDGTRLELK RADAAPTVS |
| | My9-6 VH chain | 71 | QVQLQQPGAEVVKPGASVKMSCKASGYTFTSYYIHWIKQTPGQGLEWVGVIY PGNDDISYNQKFKGKATLTADKSSTTAYMQLSSLTSEDSAVYYCAREVRLRYFD VWGAGTTVTVSS |
| | My9-6 VL chain | 72 | NIMLTQSPSSLAVSAGEKVTMSCKSSQSVFFSSSQKNYLAWYQQIPGQSPKLLI YWASTRESGVPDRFTGSGSGTDFTLTISSVQSEDLAIYYCHQYLSSRTFGGGTKL EIKR |
| | M195 VH chain | 73 | EVQLQQSGPELVKPGASVKISCKASGYTFTDYNMHWVKQSHGKSLEWIGYIY PYNGGTGYNQKFKSKATLTVDNSSSTAYMDVRSLTSEDSAVYYCARGRPAMD YWGQGTSVTVS |
| | M195 VL chain | 74 | DIVLTQSPASLAVSLGQRATISCRASESVDNYGISFMNWFQQKPGQPPKLLIYA ASNQGSGVPARFSGSGSGTDFSLNIHPMEEDDTAMYFCQQSKEVPWTFGGG TKLEIK |
| | m2H12 VH chain | 75 | QVQLQQSGPELVRPGTFVKISCKASGYTFTNYDINWVNQRPGQGLEWIGWIY PGDGSTKYNEKFKAKATLTADKSSSTAYLQLNNLTSENSAVYFCASGYEDAMD YWGQGTSVTVSS |
| | m2H12 VL chain | 76 | DIKMTQSPSSMYASLGERVIINCKASQDINSYLSWFQQKPGKSPKTLIYRANRL VDGVPSRFSGSGSGQDYSLTISSLEYEDMGIYYCLQYDEFPLTFGAGTKLELKR |

(continued)

| Cell surface antigen | Name of scFV & VH or VL chain | SEQ ID NO | Polypeptide sequence |
|---|---|---|---|
| 5T4 | H8 heavy chain | 77 | EVQLQQSGPDLVKPGASVKISCKASGYSFTGYYMHWVKQSHGKSLEWIGRIN PNNGVTLYNQKFKDKAILTVDKSSTTAYMELRSLTSEDSAVYYCARSTMITNYV MDYWGQVTSVTVSS |
| | H8 VL chain | 78 | SIVMTQTPTFLLVSAGDRVTITCKASQSVSNDVAWYQQKPGQSPTLLISYTSSR YAGVPDRFIGSGYGTDFTFTISTLQAEDLAVYFCQQDYNSPPTFGGGTKLEIKR |
| | A3 heavy chain | 79 | QIQLVQSGPELKKPGETVKISCKASGYTFTNFGMNWVKQGPGEGLKWMGWI NTNTGEPRYAEEFKGRFAFSLETTASTAYLQINNLKNEDTATYFCARDWDGAY FFDYWGQGTTLTVSS |
| | A3 light chain | 80 | SIVMTQTPKFLLVSAGDRVTITCKASQSVSNDVAWYQQKPGQSPKLLINFATN RYTGVPNRFTGSGYGTDFTFTISTVQAEDLALYFCQQDYSSPWTFGGGTKLEIK |
| | A2 heavy chain | 81 | QVQLQQSRPELVKPGASVKMSCKASGYTFTDYVISWVKQRTGQGLEWIGEIY PGSNSIYYNEKFKGRATLTADKSSSTAYMQLSSLTSEDSAVYFCAMGGNYGFD YWGQGTTLTVSS |
| | A2 light chain | 82 | QIVLTQSPAIMSASLGERVTLTCTASSSVNSNYLHWYQQKPGSSPKLWIYSTSN LASGVPARFSGSGSGTSYSLTISSMEAEDAATYYCHQYHRSPLTFGAGTKLELK |
| | A3 heavy chain | 83 | EVQLVESGGGLVQPKGSLKLSCAASGFTFNTYAMNWVRQAPGKGLEWVARI RSKSNNYATYYADSVKDRFTISRDDSQSMLYLQMNNLKTEDTAMYYCVRQW DYDVRAMNYWGQGTSVTVSS |
| | A3 light chain | 84 | DIVMTQSHIFMSTSVGDRVSITCKASQDVDTAVAWYQQKPGQSPKLLIYWAS TRLTGVPDRFTGSGSGTDFTLTISNVQSEDLADYFCQQYSSYPYTFGGGTKLEIK |
| ROR1 | 2A2 heavy chain | 85 | QVQLQQSGAELVRPGASVTLSCKASGYTFSDYEMHWVIQTPVHGLEWIGAID PETGGTAYNQKFKGKAILTADKSSSTAYMELRSLTSEDSAVYYCTGYYDYDSFT YWGQGTLVTVSA |
| | 2A2 VL chain | 86 | DIVMTQSQKIMSTTVGDRVSITCKASQNVDAAVAWYQQKPGQSPKLLIYSAS NRYTGVPDRFTGSGSGTDFTLTISNMQSEDLADYFCQQYDIYPYTFGGGTKLEI K |
| | 4A5 heavy chain | 87 | EVKLVESGGGLVKPGGSLKLSCAASGFTFSSYAMSWVRQIPEKRLEWVASISR GGTTYYPDSVKGRFTISRDNVRNILYLQMSSLRSEDTAMYYCGRYDYDGYYAM DYWGQGTSVTVSS |
| | 4A5 light chain | 88 | DIKMTQSPSSMYASLGERVTITCKASPDINSYLSWFQQKPGKSPKTLIYRANRL VDGVPSRFSGGGSGQDYSLTINSLEYEDMGIYYCLQYDEFPYTFGGGTKLEMK |
| | D10 heavy chain | 89 | QVQLKESGPGLVAPSQTLSITCTVSGFSLTSYGVHWVRQPPGKGLEWLGVIW AGGFTNYNSALKSRLSISKDNSKSQVLLKMTSLQTDDTAMYYCARRGSSYSMD YWGQGTSVTVSS |
| | D10 light chain | 90 | EIVLSQSPAITAASLGQKVTITCSASSNVSYIHWYQQRSGTSPRPWIYEISKLASG VPVRFSGSGSGTSYSLTISSMEAEDAAIYYCQQWNYPLITFGSGTKLEIQ |

(continued)

| Cell surface antigen | Name of scFV & VH or VL chain | SEQ ID NO | Polypeptide sequence |
|---|---|---|---|
| EGFRvIII | 139-heavy chain | 91 | EVQVLESGGGLVQPGGSLRLSCAASGFTFSSYAMSWVRQAPGKGLEWVSAIS GSGGGSTNYADSVKGRFTISRDNSKNTLYLQMNSLRAEDTAVYYCAGSSGWSE YWGQGTLVTVSS |
| | 139-VL chain | 92 | DIQMTQSPSSLSASVGDRVTITCRASQGIRNNLAWYQQKPGKAPKRLIYAASN LQSGVPSRFTGSGSGTEFTLIVSSLQPEDFATYYCLQHHSYPLTSGGGTKVEIK |
| | MR1-heavy chain | 93 | QVQLQQSGGGLVKPGASLKLSCVTSGFTFRKFGMSWVRQTSDKRLEWVASIS TGGYNTYYSDNVKGRFTISRENAKNTLYLQMSSLKSEDTALYYCTRGYSSTSYA MDYWGQGTTVTV |
| | MR1-light chain | 94 | DIELTQSPASLSVATGEKVTIRCMTSTDIDDDMNWYQQKPGEPPKFLISEGNTL RPGVPSRFSSSGTGTDFVFTIENTLSEDVGDYYCLQSFNVPLTFGDGTKLEKAL |
| BCMA | BCMA-50 VH chain | 95 | QVQLVQSGAEVKKPGASVKVSCKASGYSFPDYYINWVRQAPGQGLEWMGW IYFASGNSEYNQKFTGRVTMTRDTSINTAYMELSSLTSEDTAVYFCASLYDYDW YFDVWGQGTMVTVSS |
| | BCMA-50 VL chain | 96 | DIVMTQTPLSLSVTPGQPASISCKSSQSLVHSNGNTYLHWYLQKPGQSPQLLIY KVSNRFSGVPDRFSGSGSGTDFTLKISRVEAEDVGIYYCSQSSIYPWTFGQGTK LEIK |
| | BCMA-30 VH chain | 97 | QVQLVQSGAEVKKPGASVKVSCKASGYSFPDYYINWVRQAPGQGLEWMGW IYFASGNSEYNQKFTGRVTMTRDTSSSTAYMELSSLRSEDTAVYFCASLYDYD WYFDVWGQGTMVTVSS |
| | BCMA-30 VL chain | 98 | DIVMTQTPLSLSVTPGEPASISCKSSQSLVHSNGNTYLHWYLQKPGQSPQLLIY KVSNRFSGVPDRFSGSGSGADFTLKISRVEAEDVGVYYCAETSHVPWTFGQGT KLEIK |
| | C11D5.3 VH chain | 99 | QIQLVQSGPELKKPGETVKISCKASGYTFTDYSINWVKRAPGKGLKWMGWIN TETREPAYAYDFRGRFAFSLETSASTAYLQINNLKYEDTATYFCALDYSYAMDY WGQGTSVTVSS |
| | C11D5.3 VL chain | 100 | DIVLTGSPPSLAMSLGKRATISCRASESVTILGSHLIHWYQQKPGQPPTLLIQLA SNVQTGVPARFSGSGSRTDFTLTIDPVEEDDVAVYYCLQSRTIPRTFGGGTKLEI K |
| | C13F12.1 VH chain | 101 | QIQLVQSGPELKKPGETVKISCKASGYTFTHYSMNWVKQAPGKGLKWMGRI NTETGEPLYADDFKGRFAFSLETSASTAYLVINNLKNEDTATFFCSNDYLYSCDY WGRGTTLTVSS |
| | C13F12.1 VL chain | 102 | DIVLTQSPPSLAMSLGKRATISCRASESVTILGSHLIYWYQQKPGQPPTLLIQLAS NVQTGVPARFSGSGSRTDFTLTIDPVEEDDVAVYYCLQSRTIPRTFGGGTKLEIK |

(continued)

| Cell surface antigen | Name of scFV & VH or VL chain | SEQ ID NO | Polypeptide sequence |
|---|---|---|---|
| CS1 | Luc63 VH chain | 103 | EVKLLESGGGLVQPGGSLKLSCAASGFDFSRYWMSWVRQAPGKGLEWIGEIN PDSSTINYTPSLKDKFIISRDNAKNTLYLQMSKVRSEDTALYYCARPDGNYWYF DVWGAGTTVTVSS |
| | Luc63 VL chain | 104 | DIVMTQSHKFMSTSVGDRVSITCKASQDVGIAVAWYQQKPGQSPKLLIYWAS TRHTGVPDRFTGSGSGTDFTLTISNVQSEDLADYFCQQYSSYPYTFGGGTKLEI K |
| | Luc90 VH chain | 105 | QVQLQQPGAELVRPGASVKLSCKASGYSFTTYWMNWVKQRPGQGLEWIG MIHPSDSETRLNQKFKDKATLTVDKSSSTAYMQLSSPTSEDSAVYYCARSTMIA TRAMDYWGQGTSVTVSS |
| | Luc90 VL chain | 106 | DIVMTQSQKSMSTSVGDRVSITCKASQDVITGVAWYQQKPGQSPKLLIYSASY RYTGVPDRFTGSGSGTDFTFTISNVQAEDLAVYYCQQHYSTPLTFGAGTKLELK |
| | Luc34 VH chain | 107 | QVQLQQSGAELARPGASVKLSCKASGYTFTSYWMQWVKQRPGQGLEWIGA IYPGDGDTRYTQKFKGKATLTADKSSSTAYMQLSSLASEDSAVYYCARGKVYYG SNPFAYWGQGTLVTVSA |
| | Luc34 VL chain | 108 | DIQMTQSSSYLSVSLGGRVTITCKASDHINNWLAWYQQKPGNAPRLLISGATS LETGVPSRFSGSGSGKDYTLSITSLQTEDVATYYCQQYWSTPWTFGGGTKLEIK |
| | LucX1 VH chain | 109 | QVQLQQSGPELVKPGASVKISCKASGYAFSSSWMNWVKQRPGQGLEWIGRI YPGDGDTKYNGKFKGKATLTADKSSSTAYMQLSSLTSVDSAVYFCARSTMIAT GAMDYWGQGTSVTVSS |
| | LucX1 VL chain | 110 | ETTVTQSPASLSMAIGEKVTIRCITSTDIDDDMNWYQQKPGEPPKLLISEGNTL RPGVPSRFSSSGYGTDFVFTIENMLSEDVADYYCLQSDNLPLTFGGGTKLEIK |
| | LucX2 VH chain | 111 | QVQLQQSGPELVKPGASVKISCKASGYAFSSSWMNWVKQRPGQGLEWIGRI YPGDGDTKYNGKFKGKATLTADKSSSTAYMQLSSLTSVDSAVYFCARSTMIAT GAMDYWGQGTSVTVSS |
| | LucX2 VL chain | 112 | DIVMTQSHKFMSTSVGDRVSITCKASQDVSTAVAWYQQKPGQSPKLLIYSASY RYTGVPDRFTGSGSGTDFTFTISSVQAEDLAVYYCQQHYSTPPYTFGGGTKLEI K |

(continued)

| Cell surface antigen | Name of scFV & VH or VL chain | SEQ ID NO | Polypeptide sequence |
|---|---|---|---|
| CD123 | 7G3 VH chain | 113 | MGWSWIFLFLVSGTGGVLSEVQLQQSGPELVKPGASVKMSCKASGYTFTDYY MKWVKQSHGKSLEWIGDIIPSNGATFYNQKFKGKATLTVDRSSSTAYMHLNS LTSEDSAVYYCTRSHLLRASWFAYWGQGTLVTVSAAS |
| | 7G3 VL chain | 114 | MESQTQVLMSLLFWVSGTCGDFVMTQSPSSLTVTAGEKVTMSCKSSQSLLNS GNQKNYLTWYLQKPGQPPKLLIYWASTRESGVPDRFTGSGSGTDFTLTISSVQ AEDLAVYYCQNDYSYPYTFGGGTKLEIKR |
| | Old4 VH chain | 115 | WTWRFLFVVAAATGVQSQVQLLQSGAEVKKPGSSVKVSCKASGGTFSTYAIS WVRQAPGQGLEWMGGIIPIFGIVNYAQKFQGRVTITADESTSTAYMELSSLRS EDTAVYYCARGGGSGPDVLDIWGQGTMVTVSSAST |
| | Old4 VL chain | 116 | MDMRVPAQLLGLLLLWLPGARCVIWMTQSPSLLSASTGDRVTISCRMSQGIR SYLAWYQQKPGKAPELLIYAASTLQSGVPSRFSGSGSGTDFTLTISSLQSEDFAT YYCQQYYSFPYTFGQGTKLEIKRTV |
| | 26292 VH chain | 117 | QVQLQQPGAELVRPGASVKLSCKASGYTFTSYWMNWVKQRPDQGLEWIGRI DPYDSETHYNQKFKDKAILTVDKSSSTAYMQLSSLTSEDSAVYYCARGNWDDY WGQGTTLTVSS |
| | 26292 VL chain | 118 | DVQITQSPSYLAASPGETITINCRASKSISKDLAWYQEKPGKTNKLLIYSGSTLQS GIPSRFSGSGSGTDFTLTISSLEPEDFAMYYCQQHNKYPYTFGGGTKLEIK |
| | 32716 VH chain | 119 | QIQLVQSGPELKKPGETVKISCKASGYIFTNYGMNWVKQAPGKSFKWMGWI NTYTGESTYSADFKGRFAFSLETSASTAYLHINDLKNEDTATYFCARSGGYDPM DYWGQGTSVTVSS |
| | 32716 VL chain | 120 | DIVLTQSPASLAVSLGQRATISCRASESVDNYGNTFMHWYQQKPGQPPKLLIY RASNLESGIPARFSGSGSRTDFTLTINPVEADDVATYYCQQSNEDPPTFGAGTK LELK |
| | Klon43 VH chain | 121 | EVKLVESGGGLVQPGGSLSLSCAASGFTFTDYYMSWVRQPPGKALEWLALIRS KADGYTTEYSASVKGRFTLSRDDSQSILYLQMNALRPEDSATYYCARDAAYYSY YSPEGAMDYWGQGTSVTVSS |
| | Klon43 VL chain | 122 | MADYKDIVMTQSHKFMSTSVGDRVNITCKASQNVDSAVAWYQQKPGQSPK ALIYSASYRYSGVPDRFTGRGSGTDFTLTISSVQAEDLAVYYCQQYYSTPWTFG GGTKLEIKR |
| | 12F1 VH chain | 123 | VQLQESGPGLVKPSQSLSLTCSVTDYSITSGYYWNWIRQFPGNKLEWMGYISY DGSNNYNPSLKNRISITRDTSKNQFFLKLSSVTTEDTATYYCSRGEGFYFDSWG QGTTLTVSSARS |
| | 12F1 VL chain | 124 | DIMMSQSPSSLAVSVGEKFTMTCKSSQSLFFGSTQKNYLAWYQQKPGQSPKL LIYWASTRESGVPDRFTGSGSGTDFTLAISSVMPEDLAVYYCQQYYNYPWTFG GGTKLEIK |

(continued)

| Cell surface antigen | Name of scFV & VH or VL chain | SEQ ID NO | Polypeptide sequence |
|---|---|---|---|
| FAP | Humanized F19 VH chain | 170 | QVQLVQSGAEVKKPGASVKVSCKTSRYTFTEYTIHWVRQAPGQRLEWIGGIN PNNGIPNYNQKFKGRVTITVDTSASTAYMELSSLRSEDTAVYYCARRRIAYGYD EGHAMDYWGQGTLVTVSS |
| | Humanized F19 VL chain | 171 | DIVMTQSPDSLAVSLGERATINCKSSQSLLYSRNQKNYLAWYQQKPGQPPKLLI FWASTRESGVPDRFSGSGFGTDFTLTISSLQAEDVAVYYCQQYFSYPLTFGQGT KVEIK |
| | FAP5 VH chain | 172 | QVQLQQSGAELARPGASVNLSCKASGYTFTNNGINWLKQRTGQGLEWIGEIY PRSTNTLYNEKFKGKATLTADRSSNTAYMETELRSLTSEDSAVYFCARTLTAPFA FWGQGTLVTVSA |
| | FAP5 VL chain | 173 | QIVLTQSPAIMSASPGEKVTMTCSASSGVNFMHWYQQKSGTSPKRWIFDTSK LASGVPARFSGSGSGTSYSLTISSMEAEDAATYYCQQWSFNPPTFGGGTKLEIK R |
| BCMA | VH | 186 | EVQLLESGGGLVQPGGSLRLSCAASGFTFSSYAMNWVRQAPGKGLE WVSAILSSGGSTYYADSVKGRFTISRDNSKNTLYLQMNSLRAEDTAVYY CARYWPMDIWGQGTLVTV |
| | VL | 187 | EIVLTQSPGTLSLSPGERATLSCRGGQSVSSSYLAWYQQKPGQAPRLL MYDASIRATGIPDRFSGSGSGTDFTLTISRLEPEDFAVYYCQQYQSWPL TFGQGTKVEIK |

In another preferred embodiment, said VH and VL chains have as epitope target sequence of over 80% identity, preferably over 90%, and more preferably over 95% with SEQ ID NO 11 (CD20 antigen).

The extracellular ligand-binding domain can also comprise a peptide binding an antigen of the target, a peptide or a protein binding an antibody that binds an antigen of the target, a peptide or a protein ligand such as a growth factor, a cytokine or a hormone as non-limiting examples binding a receptor on the target, or a domain derived from a receptor such as a growth factor receptor, a cytokine receptor or a hormone receptor as non-limiting examples, binding a peptide or a protein ligand on the target. Preferably the target is a cell or a virus.

The antigen binding domain of the CAR can be any domain that binds to the cell target antigen including but not limited to a monoclonal antibody, a recombinant antibody, a human antibody, a humanized antibody, and a functional fragment thereof.

A humanized antibody can be produced using a variety of techniques known in the art, including but not limited to, CDR-grafting (see, e.g., European Patent No. EP 239,400; International Publication No. WO 91/09967; and U.S. Pat. Nos. 5,225,539, 5,530,101, and 5,585,089), veneering or resurfacing (see, e.g., European Patent Nos. EP 592,106 and EP 519,596; Padlan, 1991, Molecular Immunology, 28(4/5):489-498; Studnicka et al., 1994, Protein Engineering, 7(6):805-814; and Roguska et al., 1994, PNAS, 91:969-973), chain shuffling (see, e.g., U.S. Pat. No. 5,565,332), and techniques disclosed in, e.g., U.S. Patent Application Publication No. US2005/0042664, U.S. Patent Application Publication No. US2005/0048617, U.S. Pat. No. 6,407,213, U.S. Pat. No. 5,766,886, International Publication No. WO 9317105, Tan et al., J. Immunol., 169: 1119-25 (2002), Caldas et al., Protein Eng., 13(5):353-60 (2000), Morea et al., Methods, 20(3):267-79 (2000), Baca et al., J. Biol. Chem., 272(16): 10678-84 (1997), Roguska et al., Protein Eng., 9(10):895-904 (1996), Couto et al., Cancer Res., 55 (23 Supp):5973s-5977s (1995), Couto et al., Cancer Res., 55(8): 1717-22 (1995), Sandhu J S, Gene, 150(2):409-10 (1994), and Pedersen et al., J. Mol. Biol., 235(3):959- 73 (1994. Often, framework residues in the framework regions will be substituted with the corresponding residue from the CDR donor antibody to alter, for example improve, antigen binding. These framework substitutions are identified by methods well-known in the art, e.g., by modeling of the interactions of the CDR and framework residues to identify framework residues important for antigen binding and sequence comparison to identify unusual framework residues at particular positions. (See, e.g., Queen et al., U.S. Pat. No. 5,585,089; and Riechmann et al., 1988, Nature, 332:323).

According to the invention, the scFv may be nanobodies (natural single domain antibodies) which can be obtained by immunization of dromedaries, camels, llamas, alpacas or sharks.

*Linkers within the chimeric scFv*

The flexibility of scFv linker engineering can be combined with the inherent quick and adaptable characters of surface coupling chemistry (e.g., electrostatic, hydrogen bonding, or covalent attachment). Peptide linkers can vary from 10 to 25 amino acids in length and are typically, but not always, composed of hydrophilic amino acids such as glycine (G) and serine (S). Peptide linkers of shorter lengths (0-4 amino acids) have also been used. However, scFv bearing shorter linkers can form multimers. Generally, the (GGGGS)$_3$ peptide is used as an scFv peptide linker. This 15-amino acid linker sequence [designated as the (GGGGS)$_3$ linker] is used in the Recombinant Phage Antibody System (RPAS kit) commercially available from Amersham. Previous study demonstrated that scFvs (MW ~27 000) containing metal-binding amino acids (i.e., cysteine or histidine) in the scFv peptide linker can be directly immobilized onto a gold surface in a favorable antigen-binding orientation at high density that significantly increased assay sensitivity by 3-5-fold over whole IgG or Fab antibody fragments, respectively (Shen Z, Mernaugh RL, Yan H, Yu L, Zhang Y, Zeng X. Anal. Chem. 2005;77:6834-6842; Shen Z, Stryker GA, Mernaugh RL, Yu L, Yan H, Zeng X. Anal. Chem. 2005; 77:797-805).

Amongst other linkers suitable within the present invention are the 15-mer peptide linker (RGRGRGRGRSRGGGS) (Zhihong Shen, Heping Yan, Ying Zhang, Raymond L. Mernaugh, and Xiangqun Zeng (2008), Anal Chem. 80(6): 1910-1917).

In some embodiments, the "linker" as used in the context of a scFv refers to a peptide linker that consists of amino acids such as glycine and/or serine residues used alone or in combination, to link variable heavy and variable light chain regions together. In one embodiment, the flexible polypeptide linker is a Glycine/Serine linker and comprises the amino acid sequence (Gly-Gly-GlySer)$_n$ or (Gly-Gly-Gly-Gly-Ser)$_n$, where n is a positive integer equal to or greater than 1. For example, n=1, n=2, n=3, n=4, n=5, n=6, n=7, n=8, n=9 and n=10. In one embodiment, the flexible polypeptide linkers include, but are not limited to, (Gly$_4$Ser)$_4$ or (Gly$_4$Ser)$_3$. In another embodiment, the linkers include multiple repeats of (Gly$_x$Ser)$_n$, where x=1, 2, 3, 4 or 5 and n is 1, 2, 3, 4, 5, 6, 7, 8, 9 or 10, such as multiple repeat of (GlySer), (Gly$_2$Ser) or (Gly$_5$Ser). Also included within the scope of the invention are linkers described in WO2012/138475.

Chimeric antigen receptor (CAR)

The CAR according to the invention are sought for enabling engineered immune cells to trigger the destruction of pathological cells, in particular malignant cells. They may be designed according to single-chain or multi-chain architectures. In some embodiments, the extracellular ligand-binding domain, transmembrane domain, and intracellular signaling domain are in one polypeptide, i.e., in a single chain. Multi-chain architectures are more particularly disclosed in WO2014039523.

A multi-chain CAR is typically formed of different polypeptides such as:

- one transmembrane polypeptide comprising at least one extracellular ligand-binding domain and;

- one transmembrane polypeptide comprising at least one signal-transducing domain.

The signaling polypeptide is responsible for the activation of at least one of the normal functions of the engineered immune cell. For example, the function of a T cell can be a cytolytic activity or helper activity including the secretion of cytokines. Thus, the term "signaling protein" refers to a protein which transduces the transmitter domain function signal and directs the cell to perform a specialized function. In a particular embodiment, said transmitter domain can be a signaling protein. Transmission of the signals can result from: protein/protein interactions, protein/DNA interaction, protein/RNA interaction, protein/small molecule interaction, post translational protein modification, conformational change, subcellular relocalization.

The signaling protein can activate a gene in the nucleus. Examples of signaling protein can be members of NFAT transcription factor family which are inducible factor that could bind the intereukin-2 promoter in activated T cells. The regulation of NFAT proteins involves metabolites and proteins such as calcium, calcineurin and Homer scaffolding proteins. Said signaling protein can be an activated engineered form of NFAT avoiding regulation by calcineurin and Homer proteins. Said signaling protein can be a NF-κB engineered to avoid sequestration in the cytoplasm by IκB allowing activation of T cells. Said signaling protein can also be the expression of the three IKK subunits (IKKα, IKKβ, IKKy). Reconstituted IKK complex activated NF-κB pathway, by triggering the ubiquitination of the IκB. Also the activation of the JNK signaling could be triggered through the direct expression of signaling protein AP-1 (transcription factor). Said signaling protein can be an engineered transcription activator like effector (TALE) binding domain that will specifically target and activate transcription of the same gene as for the NFAT and NF-kb.

According to the invention, said signaling protein can inhibit a signaling pathway through protein-protein interaction or can activate a gene in the nucleus to inhibit a signaling pathway. Said signaling protein can be vaccinia H1 related proteins (VHR) a member of the mitogen-activated protein kinase phosphatases (MKPs) family which dephosphorylates and inactivates an extracellular signal regulated kinases (ERK) signaling proteins.

According to the invention, signal transducing domain for use in a CAR can be the cytoplasmic sequences of the T cell receptor and co-receptors that act in concert to initiate signal transduction following antigen receptor engagement, as well as any derivate or variant of these sequences and any synthetic sequence that has the same functional capability. Signal transduction domain may comprise two distinct classes of cytoplasmic signaling sequence, those that initiate antigen-dependent primary activation, and those that act in an antigen-independent manner to provide a secondary or co-stimulatory signal.

In particular embodiment the signal transduction domain of the CAR of the present invention comprises a co-stimulatory signal molecule. A co-stimulatory molecule is a cell surface molecule other than an antigen receptor or their ligands that is required for an efficient immune response.

"Co-stimulatory ligand" refers to a molecule on an antigen presenting cell that specifically binds a cognate co-stimulatory molecule on a T cell, thereby providing a signal which, in addition to the primary signal provided by, for instance, binding of a TCR/CD3 complex with an MHC molecule loaded with peptide, mediates a T cell response, including, but not limited to, proliferation activation, differentiation and the like. A "co-stimulatory molecule" refers to the cognate binding partner on a T cell that specifically binds with a co-stimulatory ligand, thereby mediating a co-stimulatory response by the cell, such as, but not limited to proliferation. Co-stimulatory molecules include, but are not limited to an MHC class I molecule, BTLA and Toll ligand receptor.

For instance, a multi-chain CAR can be derived from the structure of a Fc receptor, preferably FcεRI, and comprise at least two of the following components:

a) one polypeptide comprising the transmembrembrane domain of FcRI alpha chain fused to an extracellular ligand-binding domain,

b) one polypeptide comprising a part of N- and C- terminal cytoplasmic tail fused to the transmembrane domain of a FcRI beta chain, and/or

c) two additional polypeptides comprising each one part of an intracytoplasmic tail and/or the transmembrane domain of FcRI gamma chain,

In general, these different polypeptides multimerize together spontaneously to form dimeric, trimeric or tetrameric structures that arise at the cell surface in a juxtamembrane position.

In some embodiments, the invention relates to an immune cell comprising a single-chain CAR as well defined in the prior art, as well as in any of US7446190, WO2008/121420, US8252592, US20140024809, WO2012/079000, WO2014153270, WO2012/099973, WO2014/011988, WO2014/011987, WO2013/067492, WO2013/070468, WO2013/040557, WO2013/126712, WO2013/126729, WO 2013/126726, WO2013/126733, US8399645, US20130266551, US20140023674, WO2014039523, US7514537, US8324353, WO2010/025177, US7446179, WO2010/025177, WO2012/031744, WO2012/136231A1, WO2012/050374A2,WO2013074916, WO/2009/091826A3, WO2013/176915 or WO/2013/059593.

In some embodiments, the invention relates to a CAR comprising

- an extracellular domain comprising

  - at least one extracellular binding domain that comprises a scFv formed by at least a VH chain and a VL chain specific to an antigen, preferably a cell surface marker antigen, wherein said extracellular binding domain comprises at least one mAb-specific epitope, and,
  - a hinge,

- a transmembrane domain, and,
- an intracellular domain.

In one embodiment, the transmembrane domain comprises the transmembrane region(s) of the alpha, beta or zeta chain of the T-cell receptor, PD-1, 4-1BB, OX40, ICOS, CTLA-4, LAG3, 2B4, BTLA4, TIM-3, TIGIT, SIRPA, CD28, CD3 epsilon, CD45, CD4, CD5, CD8, CD9, CD16, CD22, CD33, CD37, CD64, CD80, CD86, CD134, CD137 or CD154. In another embodiment, the hinge is an IgG4 hinge or a CD8 alpha hinge, preferably a CD8 alpha hinge.

The distinguishing features of appropriate transmembrane domains comprise the ability to be expressed at the surface of a cell, preferably in the present invention an immune cell, in particular lymphocyte cells or Natural killer (NK) cells, and to interact together for directing cellular response of immune cell against a predefined target cell. The transmembrane domain can be derived either from a natural or from a synthetic source. The transmembrane domain can be derived from any membrane-bound or transmembrane protein. As non-limiting examples, the transmembrane polypeptide can be a subunit of the T cell receptor such as α, β, γ or δ, polypeptide constituting CD3 complex, IL2 receptor p55 (α chain), p75 (β chain) or γ chain, subunit chain of Fc receptors, in particular Fcγ receptor III or CD proteins. Alternatively the transmembrane domain can be synthetic and can comprise predominantly hydrophobic residues such as leucine and valine. In a preferred embodiment said transmembrane domain is derived from the human CD8 alpha chain (e.g. NP_001139345.1). Said transmembrane domain can also be a CD8 transmembrane domain (alpha and beta chains). Said Transmembrane domain can be engineered to create obligated hetero or homodimers. In particular embodiment said CARs can comprise transmembrane domains or intracellular domains which can only dimerize after ligand recognition. Another example of transmembrane domain can be NKG2-D receptor. NKG2D (natural killer cell group 2D) is a C-type lectin-like receptor expressed on NK cells, γδ-TcR⁺ T cells, and CD8⁺αβ-TcR⁺ T cells (Bauer, Groh et al., 1999, Science 285(5428):727-9. NKG2D is associated with the transmembrane adapter protein DAP10 (Wu, Song et al. 1999, Science 285(5428):730-2), whose cytoplasmic domain binds to the p 85 subunit of the PI-3 kinase.

Said transmembrane domain can also be an integrin. Integrins are heterodimeric integral membrane proteins composed of a α and β chains which combined together form the LFA-1 (integrin lymphocyte function-associated antigen-1) which is expressed on all leukocytes. LFA-1 plays a central role in leukocyte intercellular adhesion through interactions with its ligand, ICAMs 1-3 (intercellular adhesion molecules 1 through 3), and also it has an important role in lymphocyte co-stimulatory signaling (Chen and Flies 2013, Nat Rev Immunol 13(4):227-42). The molecular details of the binding of LAF-1 to its immunoglobulin ICAM-1 are quite known allowing a careful engineering of LAF-1 binding site. The affinity of $\alpha_L$ domain for ICAM-1 is regulated by the displacement of its C-terminal helix which is conformational linked to alterations of specific loops in LAF-1. The active and low conformations differ of 500 and 10,000 folds. It is also interesting to note that two types of antagonists are known for LFA-1 and their mechanism of action is known. Integrin cell surface adhesion receptors can transmit a signal from the outside to inside but also *vice-versa.* There are cytoskeletal proteins as Talin which binds to the integrin tail LFA-1 to transfer a message from inside to outside.

According to one embodiment, the transmembrane domain comprises the transmembrane region of PD-1 or the transmembrane region(s) of CD8 alpha.

In one aspect of the invention, the transmembrane domain is attached to the extracellular domain of the CAR via a hinge e.g., a hinge from a human protein. For example, in one embodiment, the hinge can be a human Ig (immunoglobulin) hinge, e.g., a IgG1 hinge, an IgG4 hinge, or a CD8alpha hinge.

In a preferred embodiment, the hinge of the CAR is a human immunoglobulin hinge.

In a more preferred embodiment, the hinge of the CAR is an IgG4 hinge or a CD8 alpha hinge.

In some embodiments, the hinge is an FcγRIII alpha hinge.

In some embodiments, the hinge is a CD8 alpha hinge.

In some embodiments, the hinge is a CD8 alpha hinge has amino acid sequence with at least about 70%, preferably at least 80%, more preferably at least 90%, 95%, 97%, or 99% sequence identity with an amino acid sequence shown in SEQ. ID NO: 179, 180 or 181.

The term "hinge region" (also named stalk region in the literature) used herein generally means any oligo- or polypeptide that functions to link the transmembrane domain to the extracellular ligand-binding domain. In particular, stalk region are used to provide more flexibility and accessibility for the extracellular ligand-binding domain. A stalk region may comprise up to 300 amino acids, preferably 10 to 100 amino acids and most preferably 25 to 50 amino acids. Stalk region may be derived from all or part of naturally occurring molecules, such as from all or part of the extracellular region of CD8, CD4, CD28 or RTK, or from all or part of an antibody constant region. Alternatively the stalk region may be a synthetic sequence that corresponds to a naturally occurring stalk sequence, or may be an entirely synthetic stalk sequence.

The intracellular domain (also referred to herein as a "cytoplasmic signaling domain" or "an intracellular signaling domain") comprises a functional signaling domain derived from a stimulatory molecule as defined below. In some embodiments, the stimulatory molecule is the zeta chain associated with the T-cell receptor complex. In some embodiments, the cytoplasmic signaling domain further comprises one or more functional signaling domains derived from at least one costimulatory molecule as defined below. In some embodiments, the costimulatory molecule is chosen from 4-1BB (i.e., CD137), CD27 and/or CD28.

The term "stimulatory molecule," refers to a molecule expressed by a T-cell that provides the positive cytoplasmic signaling sequence(s) that regulate positive activation of the TCR complex in a stimulatory way for at least some aspect of the T-cell signaling pathway. In some embodiments, the positive signal is initiated by, for instance, binding of a TCR/CD3 complex with an MHC molecule loaded with peptide, and which leads to mediation of a T-cell response, including, but not limited to, proliferation, activation, differentiation, and the like. A positive cytoplasmic signaling sequence (also referred to as a "positive signaling domain" or positive intracellular signaling domain) that acts in a stimulatory manner may contain a signaling motif which is known as immunoreceptor tyrosine-based activation motif or ITAM. Examples of an ITAM containing positive cytoplasmic signaling sequence includes, but is not limited to, those derived from TCR zeta (or CD3zeta), FcR gamma, FcR beta, CD3 gamma, CD3 delta, CD3 epsilon, CD5, CD22, CD79a, CD79b, CD278 (also known as "ICOS") and CD66d. In some embodiments, the intracellular signaling domain of the CAR can comprise the CD3ζ (zeta) signaling domain which has amino acid sequence with at least about 70%, preferably at least 80%, more preferably at least 90%, 95%, 97%, or 99% sequence identity with an amino acid sequence shown in SEQ. ID NO: 175.

In some aspect, the intracellular signaling domain of the CAR generates a signal that promotes an immune effector function of the CAR containing cell. Examples of immune effector function, e.g., in a CAR T-cell, include cytolytic activity and helper activity, including the secretion of cytokines.

The term "costimulatory molecule" refers to the cognate binding partner on a T-cell that specifically binds with a costimulatory ligand, thereby mediating a costimulatory response by the T-cell, such as, but not limited to, proliferation. Costimulatory molecules are cell surface molecules other than antigen receptors or their ligands that are required for an efficient immune response. Costimulatory molecules include, but are not limited to an MHC class I molecule, BTLA and a Toll ligand receptor, as well as OX40, CD2, CD27, CD28, CDS, ICAM-1, LFA-1 (CD11a/CD18) and 4-IBB (CD137).

A costimulatory intracellular signaling domain can be the intracellular portion of a costimulatory molecule. A costimulatory molecule can be represented in the following protein families: TNF receptor proteins, Immunoglobulin-like proteins, cytokine receptors, integrins, signaling lymphocytic activation molecules (SLAM proteins), and activating NK cell receptors. Examples of such molecules include CD27, CD28, 4-1BB (CD137), OX40, GITR, CD30, CD40, ICOS, BAFFR, HVEM, lymphocyte function-associated antigen-1 (LFA-1), CD2, CD7, LIGHT, NKG2C, SLAMF7, NKp80, CD160, B7-H3, and a ligand that specifically binds with CD83, and the like. In some embodiments, the intracellular signaling domain of the CAR of the invention comprises amino acid sequence which comprises at least 70%, preferably at least 80%, more preferably at least 90%, 95%, 97%, or 99% sequence identity with an amino acid sequence shown in SEQ. ID NO: 176 and SEQ. ID NO: 177.

Table 4 provide exemplary sequence of CAR components

| Domain | Amino Acid Sequence | SEQ ID NO: |
|---|---|---|
| CD8α signal peptide | MALPVTALLLPLALLLHAARP | 178 |
| FcγRIIIα hinge | GLAVSTISSFFPPGYQ | 179 |
| CD8α hinge | TTTPAPRPPTPAPTIASQPLSLRPEACRPAAGGAVHTRGLDFACD | 180 |
| IgG1 hinge | EPKSPDKTHTCPPCPAPPVAGPSVFLFPPKPKDTLMIARTPEVTCVVV DVSHEDPEVKFNWYVDGVEVHNAKTKPREEQYNSTYRVVSVLTVLH QDWLNGKEYKCKVSNKALPAPIEKTISKAKGQPREPQVYTLPPSRDEL TKNQVSLTCLVKGFYPSDIAVEWESNGQPENNYKTTPPVLDSDGSFFL YSKLTVDKSRWQQGNVFSCSVMHEALHNHYTQKSLSLSPGK | 181 |
| CD8α transmembrane (TM) domain | IYIWAPLAGTCGVLLLSLVITLYC | 182 |
| 41BB intracellular signaling domain (ISD) | KRGRKKLLYIFKQPFMRPVQTTQEEDGCSCRFPEEEEGGCEL | 176 |
| CD3ζ intracellular signaling domain (ISD) | RVKFSRSADAPAYQQGQNQLYNELNLGRREEYDVLDKRRGRDPEM GGKPRRKNPQEGLYNELQKDKMAEAYSEIGMKGERRRGKGHDGLY QGLSTATKDTYDALHMQALPPR | 175 |
| FcεRI α-TM-IC (FcεRI α chain transmembrane and intracellular domain) | FFIPLLVVILFAVDTGLFISTQQQVTFLLKIKRTRKGFRLLNPHPKPNPKN N | 183 |

(continued)

| Domain | Amino Acid Sequence | SEQ ID NO: |
|---|---|---|
| FcεRIβ-ΔITAM (FcεRI β chain without ITAM) | MDTESNRRANLALPQEPSSVPAFEVLEISPQEVSSGRLLKSASSPPLHT WLTVLKKEQEFLGVTQILTAMICLCFGTVVCSVLDISHIEGDIFSSFKAG YPFWGAIFFSISGMLSIISERRNATYLVRGSLGANTASSIAGGTGITILII NLKKSLAYIHIHSCQKFFETKCFMASFSTEIVVMMLFLTILGLGSAVSLTI CGAGEELKGNKVPE | 184 |
| 41BB-IC (41BB co-stimulatory domain) | KRGRKKLLYIFKQPFMRPVQTTQEEDGCSCRFPEEEEGGCEL | 177 |
| CD28-IC (CD28 co-stimulatory domain) | RSKRSRGGHSDYMNMTPRRPGPTRKHYQPYAPPRDFAAYRS | 185 |

CARs and immune cells comprising them have been extensively disclosed and can be prepared by the skilled person according to known methods. For example, methodologies to prepare CARs and cells comprising such CARs are disclosed in US7446190, WO2008/121420, US8252592, US20140024809, WO2012/079000, WO2014153270, WO2012/099973, WO2014/011988, WO2014/011987, WO2013/067492, WO2013/070468, WO2013/040557, WO2013/126712, WO2013/126729, WO 2013/126726, WO2013/126733, US8399645, US20130266551, US20140023674, WO2014039523, US7514537, US8324353, WO2010/025177, US7446179, WO2010/025177, WO2012/031744, WO2012/136231A1, WO2012/050374A2, WO2013074916, WO2009/091826A3, WO2013/176915 or WO/2013/059593.

The present invention encompasses a recombinant DNA construct comprising sequences encoding an CAR as defined above, wherein the CAR comprises an extracellular domain such as an antibody fragment that binds specifically to cell target antigen, and wherein the sequence of the extracellular domain is contiguous with and in the same reading frame as a nucleic acid sequence encoding a transmembrane domain and an intracellular domain. An exemplary CAR construct may comprise an optional leader sequence, an extracellular cell target antigen binding domain, a hinge, a transmembrane domain, and an intracellular inhibitory signaling domain

In some embodiments, the invention relates to a recombinant DNA construct comprising sequences encoding a CAR as defined above. In some embodiments, the CAR comprises an extracellular domain comprising

- at least one extracellular binding domain that comprises a scFv formed by at least a VH chain and a VL chain specific to a cell surface marker antigen, wherein said extracellular binding domain comprises at least one inserted mAb-specific epitope having the amino acid sequence of SEQ ID NO 35,
- a hinge,
- a transmembrane domain, and,
- an intracellular domain.

Method for sorting CAR-positive immune cells

According to one aspect, the disclosure relates to a method for *in vitro* sorting CAR-expressing immune cell, comprising contacting a population of said engineered immune with antigen-specific antibody (preferably monoclonal Abs) to collect only cells expressing CAR.

In some instances, the disclosure relates to a method for *in vitro* sorting CAR-expressing immune cell, wherein said CAR comprises at least one extracellular binding domain comprising at least one mAb-specific epitope as described above, comprising

- contacting a population of said immune cells with a monoclonal antibody specific for said mAb-specific epitope to collect only said CAR-expressing immune cell.

In some instances, the disclosure relates to a method for *in vitro* sorting CAR-expressing immune cells, wherein said CAR comprises at least one extracellular binding domain comprising at least one mAb-specific epitope, comprising - contacting a population of said immune cells with a monoclonal antibody (epitope-specific mAb) specific for said mAb-specific epitope,

- selecting the cells that bind to the monoclonal antibody,

to obtain a population of cells enriched in CAR-expressing immune cell.

In some instances, said monoclonal antibody specific for said mAb-specific epitope is conjugated to a fluorophore and the step of selecting the cells that bind to the monoclonal antibody is done by Fluorescence Activated Cell Sorting (FACS).

In some instances, said monoclonal antibody specific for said mAb-specific epitope is conjugated to a magnetic particle and the step of selecting the cells that bind to the monoclonal antibody is done by Magnetic Activated Cell Sorting (MACS).

In some instances, the extracellular binding domain of the CAR comprises a mAb-specific epitope of SEQ ID NO 144.

In some instances, the extracellular binding domain of the CAR comprises a mAb-specific epitope of SEQ ID NO 144 and the antibody used to contact the population of immune cells is QBEND-10. In some instances, the extracellular binding domain of the CAR comprises a mAb-specific epitope of SEQ ID NO 35.

In some instances, the extracellular binding domain of the CAR comprises a mAb-specific epitope of SEQ ID NO 35 and the antibody used to contact the population of immune cells is Rituximab.

In some instances, the population CAR-expressing immune cells obtained when using the method for *in vitro* sorting CAR-expressing immune cells described above, comprises at least 70%, 75%, 80%, 85%, 90%, 95% of CAR-expressing immune cells. In some embodiments, the population CAR-expressing immune cells obtained when using the method for *in vitro* sorting CAR-expressing immune cells described above, comprises at least 85% CAR-expressing immune cells.

In some instances, the population of CAR-expressing immune cells obtained when using the method for *in vitro* sorting CAR-expressing immune cells described above shows increased cytotoxic activity in vitro compared with the initial (non-sorted) cell population using the protocol described in Example 7.5. In a preferred embodiment, said cytotoxic activity in vitro is increased by 10%, 20%, 30% or 50%.

Preferably, the mAbs are previously bound onto a support such as a column or on beads such as routinely realized by the skilled in the art.

According to a favored example, immune cells are T-cells.

According to the disclosure, cells to be administered to the recipient may be enriched *in vitro* from the source population.

Methods of expanding source populations are well known in the art, and may include selecting cells that express an antigen such as CD34 antigen, using combinations of density centrifugation, immuno-magnetic bead purification, affinity chromatography, and fluorescent activated cell sorting, known to those skilled in the art.

*Flow Cytometry*

Flow cytometry is widely used in the art and is a method well known to one of ordinary skill to sort and quantify specific cell types within a population of cells. In general, flow cytometry is a method for quantitating components or structural features of cells primarily by optical means. Since different cell types can be distinguished by quantitating structural features, flow cytometry and cell sorting can be used to count and sort cells of different phenotypes in a mixture. A flow cytometric analysis involves two basic steps: 1) labeling selected cell types with one or more labeled markers, and T) determining the number of labeled cells relative to the total number of cells in the population.

The primary method of labeling cell types is by binding labeled antibodies to markers expressed by the specific cell type. The antibodies are either directly labeled with a fluorescent compound or indirectly labeled using, for example, a fluorescent- labeled second antibody which recognizes the first antibody.

In a preferred instance, the method used for sorting T cells expressing CAR is the Magnetic-Activated Cell Sorting (MACS).

Magnetic-activated cell sorting (MACS) is a method for separation of various cell populations depending on their surface antigens (CD molecules) by using superparamagnetic nanoparticles and columns. It takes only a few simple steps to get pure cell populations Cells in a single-cell suspension are magnetically labeled with microbeads. The sample is applied to a column composed of ferromagnetic spheres, which are covered with a cell-friendly coating allowing fast and gentle separation of cells. The unlabeled cells pass through while the magnetically labeled cells are retained within the column. The flow-through can be collected as the unlabeled cell fraction. After a short washing step, the column is removed from the separator, and the magnetically labeled cells are eluted from the column.

Amongst other technique, FACS is a technique of choice to purify cell populations of known phenotype as very high purity of the desired population can be achieved, or when the target cell population expresses a very low level of the identifying marker, or when cell populations require separation based on differential marker density. In addition, FACS is the only available purification technique to isolate cells based on internal staining or intracellular protein expression, such as a genetically modified fluorescent protein marker. FACS allows the purification of individual cells based on size, granularity and fluorescence. In order to purify cells of interest, they are first stained with fluorescently-tagged monoclonal antibodies (mAb), which recognize specific surface markers on the desired cell population.

Detailed protocol for the purification of specific cell population such as T-cell can be found in Basu S et al. (2010). (Basu S, Campbell HM, Dittel BN, Ray A. Purification of specific cell population by fluorescence activated cell sorting (FACS). J Vis Exp. (41): 1546).

In a preferred instance, the mAb used in the method for sorting T cells expressing the CAR is chosen amongst ibritumomab, tiuxetan, muromonab-CD3, tositumomab, abciximab, basiliximab, brentuximab vedotin, cetuximab, infliximab, rituximab, alemtuzumab, bevacizumab, certolizumab pegol, daclizumab, eculizumab, efalizumab, gemtuzumab, natalizumab, omalizumab, palivizumab, ranibizumab, tocilizumab, trastuzumab, vedolizumab, adalimumab, belimumab, canakinumab, denosumab, golimumab, ipilimumab, ofatumumab, panitumumab, QBEND-10 and ustekinumab.

In a more preferred instance, said mAb is rituximab.
In a more preferred instance, said mAb is QBEND-10.

Method for depleting CAR-expressing immune cells

By *"in vivo* depletion"* is meant in the present invention the administration of a treatment to a mammalian organism aiming to stop the proliferation of CAR-expressing immune cells by inhibition or elimination.

One aspect of the invention is related to an epitope-specific monoclonal antibody (mAb) which specifically recognizes the mAb-specific epitope having the amino acid sequence of SEQ ID NO 35 for use in a method for *in vivo* depleting an engineered immune cell expressing a CAR comprising an m-Ab specific epitope having the amino acid sequence of SEQ ID NO 35, comprising contacting said engineered immune cell or said CAR-expressing immune cell with said epitope-specific mAbs.

Preferably, said immune cells are T-cells and/or the antibody is monoclonal.
According to one embodiment, the *in vivo* depletion of immune engineered cell is performed on engineered immune cell which has been previously sorted using the *in vitro* method of the present disclosure. In this case, this will be the same infused mAb used.

According to a preferred embodiment, the epitope-specific mAb is rituximab.

In some embodiments, the step of contacting said engineered immune cell or said CAR-expressing immune cell with at least one epitope-specific mAb comprises infusing the patient with epitope-specific mAb, preferably rituximab. In some embodiments, the amount of epitope-specific mAb administered to the patient is sufficient to eliminate at least 20%, 30%, 40%, 50%, 60%, 70%, 80% or 90% of the CAR-expressing immune cell in the patient.

In some embodiments, the step of contacting said engineered immune cell or said CAR-expressing immune cell with at least one epitope-specific mAb comprises infusing the patient with $375mg/m^2$ of rituximab, once or several times, preferably once weekly.

In some embodiments, when immune cells expressing a CAR comprising an mAb-specific epitope (CAR-expressing immune cells) are depleted in a CDC assay using epitope-specific mAb, the amount of viable CAR-expressing immune cells decreases, preferably by at least 10%, 20%, 30%, 40%, 50%, 60%, 70%, 80% or 90%. Preferably the CDC assay is the assay disclosed in Example 3, Example 4 or Example 7.4.

To one particular embodiment, the *in vivo* depletion of CAR-engineered immune cells is performed by infusing bi-specific antibodies. By definition, a bispecific monoclonal antibody (BsAb) is an artificial protein that is composed of fragments of two different monoclonal antibodies and consequently binds to two different types of antigen. These BsAbs and their use in immunotherapy have been extensively reviewed in Müller D and Kontermann R.E. (2010) Bispecific Antibodies for Cancer Immunotherapy, BioDrugs 24 (2): 89-98.

By "effector cell", this term includes immune cells such as lymphocytes, macrophages, dendritic cells, natural killer cells (NK Cell), cytotoxic T lymphocytes (CTL).

According to another particular embodiment, the infused bi-specific mAb is able to bind both the mAb-specific epitope borne on engineered immune cells expressing the chimeric scFv and to a surface antigen on an effector and cytotoxic cell. This aspect is presented in Figure 3. By doing so, the depletion of engineered immune cells triggered by the BsAb can occur through antibody-dependent cellular cytotoxicity (ADCC). Such conformation can be found by instance in Deo Y M, Sundarapandiyan K, Keler T, Wallace PK, and Graziano RF, (2000), Journal of Immunology, 165 (10):5954-5961].

According to a particular embodiment, a cytotoxic drug is coupled to the epitope-specific mAbs which are used in to deplete CAR-expressing immune cells. By combining targeting capabilities of monoclonal antibodies with the cancer-killing ability of cytotoxic drugs, antibody-drug conjugate (ADC) allows a sensitive discrimination between healthy and diseased tissue when compared to the use of the drug alone. Market approvals were received for several ADCs; the technology for making them -particularly on linkers- is abundantly presented in the following prior art (Payne, G. (2003) Cancer Cell 3:207-212; Trail et al (2003) Cancer Immunol. Immunother. 52:328-337; Syrigos and Epenetos (1999) Anticancer Research 19:605-614; Niculescu-Duvaz and Springer (1997) Adv. Drug Del. Rev. 26:151-172; U.S. Pat. No. 4,975,278).

According to another particular embodiment, the epitope-specific mAb to be infused is conjugated beforehand with a molecule able to promote complement dependent cytotoxicity (CDC). Therefore, the complement system helps or complements the ability of antibodies to clear pathogens from the organism. When stimulated by one of several, is triggered an activation cascade as a massive amplification of the response and activation of the cell-killing membrane attack complex.

Different molecule may be used to conjugate the mAb, such as glycans [Courtois, A, Gac-Breton, S., Berthou, C., Guézennec, J., Bordron, A. and Boisset, C. (2012), Complement dependent cytotoxicity activity of therapeutic antibody fragments is acquired by immunogenic glycan coupling, Electronic Journal of Biotechnology ISSN: 0717-3458; http://www.ejbiotechnology.info DOI: 10.2225/vol15-issue5).

In some embodiments of the invention, different antibodies are used for sorting and depleting the cells. In some embodiments, the extracellular binding domain comprises at least one mAb-specific epitope having the amino acid sequence of SEQ ID NO 35 and at least one epitope having the amino acid sequence of SEQ ID NO 144 and the mAb used for sorting the cells is QBEND10 and the mAb used to deplete the cell is rituximab.

Methods of engineering immune cells

The inventors developed ex vivo methods of engineering immune cells expressing a chimeric antigen receptor (CAR) as described above, with all components necessary to trigger a cell surface target antigen and to expand/amplify. Further, this CAR has the particularity of to carry a chimeric scFv wherein the scFv is modified to include at least one mAb-specific epitope having the amino acid sequence of SEQ ID NO 35.

In one embodiment, the method for engineering an immune cell chimeric antigen receptor (CAR), comprising at least one extracellular binding domain that comprises a scFv formed by at least a VH chain and a VL chain specific to a cell surface marker antigen and one mAb-specific epitope having the amino acid sequence of SEQ ID NO 35, comprising:

(a) Introducing into said cell at least one polynucleotide encoding said chimeric antigen receptor; and

(b) Expressing said polynucleotide into said cell.

CARs and immune cells comprising them have been extensively disclosed and can be prepared by the skilled person according to known methods. For example, methodologies to prepare CAR and cells comprising such CARs are disclosed earlier. Immune cells comprising a CAR can be prepared by the skilled person according to the methodologies disclosed in the above mentioned references. In a preferred embodiment, immune cells comprising a CAR can be prepared by the skilled person according to the methodologies disclosed in WO2013/176915.

In some embodiments, the immune cell can be derived from an inflammatory T-lymphocyte, a cytotoxic T-lymphocyte, a regulatory T- lymphocyte, or a helper T-lymphocyte.

In some embodiments, the immune cell has been obtained from a healthy donor. In some embodiments, the immune cell has been obtained from a patient.

In some embodiments, the method to engineer cell of the invention further comprises one or more additional genomic modification step. By additional genomic modification step, can be intended the introduction into cells to engineer of one or more protein of interest. Said protein of interest can be a CAR.

In some embodiments, the method of engineering T-cells of invention can comprise:

(a) modifying T-cells by inactivating at least:

- a first gene expressing a target for an immunosuppressive agent, and
- a second gene encoding a component of the T-cell receptor (TCR)

(b) expanding said cells, optionally in presence of said immunosuppressive agent.

An immunosuppressive agent is an agent that suppresses immune function by one of several mechanisms of action. In other words, an immunosuppressive agent is a role played by a compound which is exhibited by a capability to diminish the extent and/or voracity of an immune response. As non-limiting example, an immunosuppressive agent can be a calcineurin inhibitor, a target of rapamycin, an interleukin-2 u-chain blocker, an inhibitor of inosine monophosphate dehydrogenase, an inhibitor of dihydrofolic acid reductase, a corticosteroid or an immunosuppressive antimetabolite.

In a particular embodiment, the genetic modification step of the method relies on the inactivation of one gene selected from the group consisting of CD52, GR, TCR alpha and TCR beta. In another embodiment, the genetic modification step of the method relies on the inactivation of two genes selected from the group consisting of CD52 and GR, CD52 and TCR alpha, CDR52 and TCR beta, GR and TCR alpha, GR and TCR beta, TCR alpha and TCR beta. In another embodiment, the genetic modification step of the method relies on the inactivation of more than two genes. The genetic modification is preferably operated ex-vivo.

The rare-cutting endonucleases used for inactivating the genes in T-cells are preferably Transcription Activator like Effector (TALE), but may be also a Cas9 coupled to a RNA guide as respectively described in WO 2013/176915 and WO 2014/191128.

Delivery methods

The different methods described above involve expressing CAR at the surface of a cell. As non-limiting example, said CAR can be expressed by introducing the latter into a cell. CARs can be introduced as transgene encoded by one plasmid vector. Said plasmid vector can also contain a selection marker which provides for identification and/or selection of cells which received said vector.

Polypeptides may be synthesized *in situ* in the cell as a result of the introduction of polynucleotides encoding said polypeptides into the cell. Alternatively, said polypeptides could be produced outside the cell and then introduced thereto. Methods for introducing a polynucleotide construct into cells are known in the art and including as non-limiting examples stable transformation methods wherein the polynucleotide construct is integrated into the genome of the cell, transient transformation methods wherein the polynucleotide construct is not integrated into the genome of the cell and virus mediated methods. Said polynucleotides may be introduced into a cell by for example, recombinant viral vectors (e.g. retroviruses, adenoviruses), liposome and the like. For example, transient transformation methods include for example microinjection, electroporation or particle bombardment. Said polynucleotides may be included in vectors, more particularly plasmids or virus, in view of being expressed in cells.

Polynucleotides and vectors

In one embodiment, said isolated cell according to the present invention comprises a polynucleotide encoding the chimeric antigen receptor carrying the chimeric scFv.

The present invention also relates to polynucleotides, vectors encoding the above described CAR according to the invention.

The polynucleotide may consist in an expression cassette or expression vector (e.g. a plasmid for introduction into a bacterial host cell, or a viral vector such as a baculovirus vector for transfection of an insect host cell, or a plasmid or viral vector such as a lentivirus for transfection of a mammalian host cell).

Those skilled in the art will recognize that, in view of the degeneracy of the genetic code, considerable sequence variation is possible among these polynucleotide molecules. Preferably, the nucleic acid sequences of the present invention are codon-optimized for expression in mammalian cells, preferably for expression in human cells. Codon-optimization refers to the exchange in a sequence of interest of codons that are generally rare in highly expressed genes of a given species by codons that are generally frequent in highly expressed genes of such species, such codons encoding the amino acids as the codons that are being exchanged.

Therapeutic applications

In another embodiment, isolated cell or immune cell expressing a CAR as described herein obtained by the different methods or cell line derived from said isolated cell as previously described can be used as a medicament.

In another embodiment, said medicament can be used for treating pathologies such as cancer in a patient in need thereof.

In another embodiment, said isolated cell or immune cell expressing a CAR as described herein I according to the invention or cell line derived from said isolated cell can be used in the manufacture of a medicament for treatment of a pathology such as a cancer in a patient in need thereof.

Said treatment can be ameliorating, curative or prophylactic. It may be either part of an autologous immunotherapy or part of an allogenic immunotherapy treatment. By autologous, it is meant that cells, cell line or population of cells used for treating patients are originating from said patient or from a Human Leucocyte Antigen (HLA) compatible donor. By allogeneic is meant that the cells or population of cells used for treating patients are not originating from said patient but from a donor.

Said treatment can be used to treat patients diagnosed with cancer, viral infection, autoimmune disorders or Graft versus Host Disease (GvHD). Cancers that may be treated include tumors that are not vascularized, or not yet substantially vascularized, as well as vascularized tumors. The cancers may comprise non solid tumors (such as hematological tumors, for example, leukemias and lymphomas) or may comprise solid tumors. Types of cancers to be treated with the CAR of the invention include, but are not limited to, carcinoma, blastoma, and sarcoma, and certain leukemia or lymphoid malignancies, benign and malignant tumors, and malignancies e.g., sarcomas, carcinomas, and melanomas. Adult tumors/cancers and pediatric tumors/cancers are also included.

It can be a treatment in combination with one or more therapies against cancer selected from the group of antibodies therapy, chemotherapy, cytokines therapy, dendritic cell therapy, gene therapy, hormone therapy, laser light therapy and radiation therapy.

The administration of the cells or population of cells according to the present invention may be carried out in any convenient manner, including by aerosol inhalation, injection, ingestion, transfusion, implantation or transplantation. The compositions described herein may be administered to a patient subcutaneously, intradermally, intratumorally, intranodally, intramedullary, intramuscularly, by intravenous or intralymphatic injection, or intraperitoneally. In one embodiment, the cell compositions of the present invention are preferably administered by intravenous injection.

The administration of the cells or population of cells can consist of the administration of $10^4$-$10^9$ cells per kg body weight, preferably $10^5$ to $10^6$ cells/kg body weight including all integer values of cell numbers within those ranges. The cells or population of cells can be administered in one or more doses. In another embodiment, said effective amount of cells are administrated as a single dose. In another embodiment, said effective amount of cells are administrated as more than one dose over a period time. Timing of administration is within the judgment of managing physician and depends on the clinical condition of the patient. The cells or population of cells may be obtained from any source, such as a blood bank or a donor. While individual needs vary, determination of optimal ranges of effective amounts of a given cell type for a particular disease or conditions within the skill of the art. An effective amount means an amount which provides a therapeutic or prophylactic benefit. The dosage administrated will be dependent upon the age, health and weight of the recipient, kind of concurrent treatment, if any, frequency of treatment and the nature of the effect desired.

Said effective amount of cells or composition comprising those cells can be administrated parenterally. Said administration can be an intravenous administration. Said administration can be directly done by injection within a tumor.

Cells can be administered to a patient in conjunction with (e.g., before, simultaneously or following) any number of relevant treatment modalities, including but not limited to treatment with agents such as antiviral therapy, cidofovir and interleukin-2, Cytarabine (also known as ARA-C) or nataliziimab treatment for MS patients or efaliztimab treatment for psoriasis patients or other treatments for PML patients. In further embodiments, the T cells of the invention may be used in combination with chemotherapy, radiation, immunosuppressive agents, such as cyclosporin, azathioprine, methotrexate, mycophenolate, and FK506, antibodies, or other immunoablative agents such as CAMPATH, anti-CD3 antibodies or other antibody therapies, cytoxin, fludaribine, cyclosporin, FK506, rapamycin, mycoplienolic acid, steroids, FR901228, cytokines, and irradiation. These drugs inhibit either the calcium dependent phosphatase calcineurin (cyclosporine and FK506) or inhibit the p70S6 kinase that is important for growth factor induced signaling (rapamycin) (Henderson, Naya et al. 1991, Immunology 73(3):316-21; Liu, Albers et al. 1992, 31(16):3896-901; Bierer, Hollander et al. 1993, Curr Opin Immunol 5(5):763-73). In a further instance, the cell compositions of the present invention can be administered to a patient in conjunction with (e.g., before, simultaneously or following) bone marrow transplantation, T cell ablative therapy using either chemotherapy agents such as, fludarabine, external-beam radiation therapy (XRT), cyclophosphamide, or antibodies such as OKT3 or CAMPATH, In another instance, the cell compositions of the present invention are administered following B-cell ablative therapy such as agents that react with CD20, e.g., Rituxan. For example, in one instance, subjects may undergo standard treatment with high dose chemotherapy followed by peripheral blood stem cell transplantation. In certain instances, following the transplant, subjects receive an infusion of the expanded immune cells of the present invention. In an additional embodiment, expanded cells are administered before or following surgery.

**Other definitions**

- Amino acid residues in a polypeptide sequence are designated herein according to the one-letter code, in which, for example, Q means Gln or Glutamine residue, R means Arg or Arginine residue and D means Asp or Aspartic acid residue.

- Nucleotides are designated as follows: one-letter code is used for designating the base of a nucleoside: a is adenine, t is thymine, c is cytosine, and g is guanine. For the degenerated nucleotides, r represents g or a (purine nucleotides), k represents g or t, s represents g or c, w represents a or t, m represents a or c, y represents t or c (pyrimidine nucleotides), d represents g, a or t, v represents g, a or c, b represents g, t or c, h represents a, t or c, and n represents g, a, t or c.

- "As used herein, "nucleic acid" or "polynucleotides" refers to nucleotides and/or polynucleotides, such as deoxyribonucleic acid (DNA) or ribonucleic acid (RNA), oligonucleotides, fragments generated by the polymerase chain reaction (PCR), and fragments generated by any of ligation, scission, endonuclease action, and exonuclease action. Nucleic acid molecules can be composed of monomers that are naturally-occurring nucleotides (such as DNA and RNA), or analogs of naturally-occurring nucleotides (e.g., enantiomeric forms of naturally-occurring nucleotides), or a combination of both. Modified nucleotides can have alterations in sugar moieties and/or in pyrimidine or purine base moieties. Sugar modifications include, for example, replacement of one or more hydroxyl groups with halogens, alkyl groups, amines, and azido groups, or sugars can be functionalized as ethers or esters. Moreover, the entire sugar moiety can be replaced with sterically and electronically similar structures, such as aza-sugars and carbocyclic sugar analogs. Examples of modifications in a base moiety include alkylated purines and pyrimidines, acylated purines or pyrimidines, or other well-known heterocyclic substitutes. Nucleic acid monomers can be linked by phosphodiester bonds or analogs of such linkages. Nucleic acids can be either single stranded or double stranded.

- By chimeric antigen receptor (CAR) is intended molecules that combine a binding domain against a component present on the target cell, for example an antibody-based specificity for a desired antigen (e.g., tumor antigen) with a T cell receptor-activating intracellular domain to generate a chimeric protein that exhibits a specific anti-target cellular immune activity. Generally, CAR consists of an extracellular single chain antibody (scFv) fused to the intracellular signaling domain of the T cell antigen receptor complex zeta chain (scFv:ζ) and have the ability, when expressed in T cells, to redirect antigen recognition based on the monoclonal antibody's specificity.

- By " delivery vector" or " delivery vectors" is intended any delivery vector which can be used in the present invention to put into cell contact (i.e "contacting") or deliver inside cells or subcellular compartments (i.e "introducing") agents/chemicals and molecules (proteins or nucleic acids) needed in the present invention. It includes, but is not limited to liposomal delivery vectors, viral delivery vectors, drug delivery vectors, chemical carriers, polymeric carriers, lipoplexes, polyplexes, dendrimers, microbubbles (ultrasound contrast agents), nanoparticles, emulsions or other appropriate transfer vectors. These delivery vectors allow delivery of molecules, chemicals, macromolecules (genes, proteins), or other vectors such as plasmids, peptides developed by Diatos. In these cases, delivery vectors are molecule carriers. By "delivery vector" or "delivery vectors" are also intended delivery methods to perform transfection.

- The terms "vector" or "vectors" refer to a nucleic acid molecule capable of transporting another nucleic acid to which it has been linked. A "vector" in the present invention includes, but is not limited to, a viral vector, a plasmid, a RNA vector or a linear or circular DNA or RNA molecule which may consists of a chromosomal, non chromosomal, semi-synthetic or synthetic nucleic acids. Preferred vectors are those capable of autonomous replication (episomal vector) and/or expression of nucleic acids to which they are linked (expression vectors). Large numbers of suitable vectors are known to those of skill in the art and commercially available.

Viral vectors include retrovirus, adenovirus, parvovirus (e. g. adenoassociated viruses), coronavirus, negative strand RNA viruses such as orthomyxovirus (e. g., influenza virus), rhabdovirus (e. g., rabies and vesicular stomatitis virus), paramyxovirus (e. g. measles and Sendai), positive strand RNA viruses such as picornavirus and alphavirus, and double-stranded DNA viruses including adenovirus, herpesvirus (e. g., Herpes Simplex virus types 1 and 2, Epstein-Barr virus, cytomegalovirus), and poxvirus (e. g., vaccinia, fowlpox and canarypox). Other viruses include Norwalk virus, togavirus, flavivirus, reoviruses, papovavirus, hepadnavirus, and hepatitis virus, for example. Examples of retroviruses include: avian leukosis-sarcoma, mammalian C-type, B-type viruses, D type viruses, HTLV-BLV group, lentivirus, spumavirus (Coffin, J. M., Retroviridae: The viruses and their replication, In Fundamental Virology, Third Edition, B. N. Fields, et al., Eds., Lippincott-Raven Publishers, Philadelphia, 1996).

- By "lentiviral vector" is meant HIV-Based lentiviral vectors that are very promising for gene delivery because of their relatively large packaging capacity, reduced immunogenicity and their ability to stably transduce with high efficiency a large range of different cell types. Lentiviral vectors are usually generated following transient transfection of three (packaging, envelope and transfer) or more plasmids into producer cells. Like HIV, lentiviral vectors enter the target cell through the interaction of viral surface glycoproteins with receptors on the cell surface. On entry, the viral RNA undergoes reverse transcription, which is mediated by the viral reverse transcriptase complex. The product of reverse transcription is a double-stranded linear viral DNA, which is the substrate for viral integration in the DNA of infected cells. By "integrative lentiviral vectors (or LV)", is meant such vectors as nonlimiting example, that are able to integrate the genome of a target cell. At the opposite by "non-integrative lentiviral vectors (or NILV)" is meant efficient gene delivery vectors that do not integrate the genome of a target cell through the action of the virus integrase.

- Delivery vectors and vectors can be associated or combined with any cellular permeabilization techniques such as sonoporation or electroporation or derivatives of these techniques.

- by "mutation" is intended the substitution, deletion, insertion of up to one, two, three, four, five, six, seven, eight, nine, ten, eleven, twelve, thirteen, fourteen, fifteen, twenty, twenty five, thirty, fourty, fifty, or more nucleotides/amino acids in a polynucleotide (cDNA, gene) or a polypeptide sequence. The mutation can affect the coding sequence of a gene or its regulatory sequence. It may also affect the structure of the genomic sequence or the structure/stability of the encoded mRNA.

- by "functional variant" is intended a catalytically active mutant of a protein or a protein domain; such mutant may have the same activity compared to its parent protein or protein domain or additional properties, or higher or lower activity.

- "identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default setting. For example, polypeptides having at least 70%, 85%, 90%, 95%, 98% or 99% identity to specific polypeptides described herein and preferably exhibiting substantially the same functions, as well as polynucleotide encoding such polypeptides, are contemplated.

- The term "subject" or "patient" as used herein includes all members of the animal kingdom including non-human primates and humans. In some embodiments, the patient is human.

In addition to the preceding features, the invention comprises further features which will emerge from the following examples illustrating the method of *in vitro* sorting or *in vivo* depleting immune cells expressing CAR for immunotherapy, as well as the appended drawings.

**Example 1. Generation of rituximab-driven depletion systems embedded in an anti-CD123 CAR**

All 10 CARs having different conformations in terms of chimeric scFv (anti-CD123 scFv with CD20 mimotope(s)) are depicted in Figure 4: their resulting polypeptide sequences are shown in SEQ ID NO 1 to 10.

The DNA construct of the 10 CARs are transcribed into their corresponding mRNA via in vitro transcription and used to transfect by electroporation primary T cells freshly isolated from buffy coat via a standard ficoll procedure. One day post transfection, T cells were recovered and used to performed a flow based cytotoxicity assay as described as follows.

*Generation of anti CD123 CAR T cells.*

To generate primary T cells expressing anti-CD123 CAR, primary T cells are first purified from buffy-coat samples and activated using Dynabeads human T activator CD3/CD28. 3 days post activation, 1 million of activated T cells are transduced with lentiviral vectors harboring an anti-CD123 CAR expression cassette under the control of the Ef1$\alpha$ promoter, at the multiplicity of infection (MOI) of 1. T cells are kept in culture at 37°C in the presence of 5% $CO_2$, 20 ng/ml IL-2 (final concentration) and 5% human AB serum in X-vivo-15 media (Lonza) for further characterization. 5 days post transduction, cells are used to perform the flow-based cytotoxicity assay.

*Flow-based cytotoxicity assay*

The cytolytic activity and specificity of anti-CD123 CAR T cell are assessed according to the flow cytometry-based cytotoxicity assay as routinely performed (see for example Valton.et Al (2015) Mol Ther; 23(9):1507-1518). This assay consists of labeling $10^4$ CD123 positive tumor cells and $10^4$ CD123-negative control cells with 0.5mM CellTrace™ CFSE and 0.5mM CellTrace™ violet (Life Technology) and co-incubating them with $10^5$ effector CAR T cells (E/T ratio of 10:1) in a final volume of 100 μl X-Vivo-15 media, for 5 H at 37°C. Cells are then recovered and labeled with eFluor780 viability marker before being fixed by 4% PFA as described above. Fixed cells are then analysed by flow cytometry to determine their viability. The frequency of specific cell lysis is calculated and displayed in the following:

$$\text{Frequency of specific cell lysis} = (\text{Via CD123+cells with T / CD123-cells with T}) / (\text{Via CD123+cells / Via CD123-cells})$$

where Via CD123+ with T and Via CD123-with T correspond respectively to the % of viable CD123+ cells and CD123-cells obtained after 5 H in the presence of CAR T cells and where Via CD123+ cells and Via CD123- cells correspond respectively to the % of CD123+ cells and CD123- cells obtained after 5 H in the absence of CAR T cells.

The results show that T cells transfected with engineered anti-CD123 CAR are able to kill CD123-positive tumor cell models. As shown in Figure 5, results from flow-based cytotoxicity assays described above showed that T cells expressing SEQ ID 1-4 displayed the same activity than T cells expressing unmodified anti-CD123 CAR SEQ ID 142 (figure 5). These data suggest that insertion of CD20 mimotope in the sequence of the anti-CD123 CAR (SEQ ID 142) does not significantly impair its ability to specifically recognize the CD123 antigen and to kill CD123-expressing tumor cells. In some embodiments, the CARs of the invention comprising one of two mAb-specific epitope, preferably of SEQ ID NO 35 are able to specifically recognize the antigen targeted by the CAR and to kill cells expressing said antigen.

Consistent with these findings, transfected CAR T cells are tested for their capacity to degranulate when exposed to a CD123 recombinant protein coated on a 96 well plate. Together, ourexperiments are designed to show that insertion of CD20 mimotope in the sequence of the anti-CD123 CAR does not significantly impair its ability to specifically recognize the CD123 antigen.

To demonstrate the ability of rituximab to inhibit T cell cytotoxicity functions through specific recognition of CD20 mimotopes, transfected T cells are incubated in the presence of CD123-positive tumor cells, in the presence or in the absence of rituximab and baby rabbit-complement. The objective is to show that the cytotoxic activity and degranulation capacity of transfected T cells are impaired in the presence of rituximab and baby rabbit complement, indicating further that efficient recognition of engineered anti-CD123 CAR by rituximab leads to T cell depletion.

**Example 2. Flexibility of the mAb-driven depletion systems in the anti-CD123 chimeric antigen receptor.**

To further demonstrate the flexibility of the mAb-driven depletion system, different epitopes or mimotopes (SEQ ID NO 35-42) specific for cetuximab, palivizumab and nivolumab mAbs are inserted within the anti-CD123 CAR constructions using the same procedure and architecture as the one used for the CD20 mimotope described in Example 1. The results aim to show that transfected T cells retain their cytolytic activity and degranulation capacity toward CD123 positive tumor cells. In addition, the experiments are designed also to indicate that transfected T cells are depleted by some of the aforementioned mAbs

**Example 3. Rituximab-dependent depletion of anti-CD123 CAR containing an mAb-driven depletion system.**

To explore the ability of the mAb-driven depletion system to allow depletion of anti-CD123 CAR T cells, transduced T cell expressing a CAR of SEQ ID NO 1, 2, 3 or 4 or an unmodified anti-CD123 CAR (SEQ ID NO 142), were subjected to a complement dependant cytotoxicity assay (CDC).

*CDC assay*

The CDC assay consisted in incubating $0.2 \times 10^6$ transduced T cells either alone, or in the presence of Rituximab (RTX, ROCHE, 400 ng) and Babby Rabbit Complement (BRC, AbD Serotec, ref# C12CA, 100 μL of the solution diluted according to the manufacturer protocol) for 3 hours at 37°C in a final volume of 400 μL of Xvivo 10% FBS. At the end of incubation, anti CD123-CAR T cells were recovered and labeled with recombinant CD123 protein fused to an FC fragment (SEQ ID 143) and a PE labeled anti-FC secondary monoclonal antibody (Jackson ImmunoResearch, ref# 115-115-164, diluted 1/200). Cells were then recovered in PFA 4% before being analyzed by flow cytometry. The flow cytometry gating strategy consisted of determining the viability of anti-CD123 CAR positive T cells (PE positive cells) among the singlet found in the total population of cells. This analysis was performed on cells incubated alone and in the presence of RTX and BRC. Results are expressed as the ratio named "Relative frequency of viable cells among anti-CD123 CAR positive T cells (with respect to control experiment)" described below:

(Frequency of viable cells among anti-CD123 CAR positive T cells obtained in the presence of RTX and BRC) x 100 / (Frequency of viable cells among anti-CD123 CAR positive T cells obtained in the absence of RTX and BRC)

The results showed that all CAR architectures allowed the RTX-dependent depletion of CAR T cells (Figure 6). CAR T cells expressing SEQ ID NO 3 and 4 were more efficiently depleted than the ones expressing SEQ ID NO 1 and 2 suggesting that the number of CD20 mimotope present in the CAR architecture influenced the extent and/or the kinetic of T cells depletion.

In some embodiments, CARs of the invention having architectures illustrated by Figure 4 allow rituximab dependent depletion of CAR T cells. In some embodiments, CAR of the invention comprising at least 2 mAb-specific epitopes, preferably having CAR architecture of SEQ ID NO 3 or 4 are particularly efficiently depleted.

**Example 4. Efficiency of the mAb-driven depletion system in cells expressing an anti-BCMA CAR comprising one or more mAbs specific epitopes in the extracellular domain.**

To explore the ability of the mAb-driven depletion system to allow depletion of anti-BCMA CAR T cells, 15 different CAR architectures (SEQ ID 125-139, Figure 7) were constructed. These architectures were designed to contain 1, 2 or 3 CD20 mimotopes localized at different portions of the extracellular domain of the anti-BCMA CAR (SEQ ID NO 125), i.e in the N terminal domain, in the linker domain separating the V1 and V2 of the ScFv or upstream to the CD8 hinge linking the ScFv to the transmembrane domain of the CAR.

To generate primary T cells expressing anti-BCMA CAR, primary T cells were first purified from buffy-coat samples and activated using Dynabeads human T activator CD3/CD28. 3 days post activation, 5 million of activated T cells were transfected with either 15 or 30 μg of poly adenylated mRNA encoding the different anti-BCMA CAR architectures (SEQ ID 125-139, Figure 7). T cells were then kept in culture at 37°C in the presence of 5% $CO_2$, 20 ng/ml IL-2 (final concentration) and 5% human AB serum in X-vivo-15 media (Lonza) for further characterization. One day post transfection, cells were used to perform the CDC assay, a flow based cytotoxicity assay, a detection assay and an Interferon γ (IFN y) release assay.

*CDC assay*

The CDC assay consisted in incubating 0.2 $10^6$ transfected cells either alone, or in the presence of Rituximab (RTX, ROCHE, 400ng) and Babby Rabbit Complement (BRC, AbD Serotec, ref#C12CA, 100 μL of the solution diluted according to the manufacturer protocol) for 2 hours at 37°C in a final volume of 400 μL of Xvivo 10% FBS. At the end of incubation, anti BCMA-CAR T cells were recovered and labeled with recombinant BCMA protein fused to an FC fragment (SEQ ID NO 151) and a PE labeled anti-FC secondary monoclonal antibody (Jackson ImmunoResearch, ref# 115-115-164, diluted 1/200). Cells were then recovered in PFA 4% before being analyzed by flow cytometry. The flow cytometry gating strategy was to determine the viability of anti-BCMA CAR positive T cells (PE positive cells) among the singlet found in the total population of cells. This analysis was performed on cells incubated alone and in the presence of RTX and BRC. Results are expressed as the ratio named "Relative frequency of viable cells among BCMA CAR positive T cells (with respect to control experiment)" described below:

(Frequency of viable cells among anti-BCMA CAR positive T cells obtained in the presence of RTX and BRC) x 100 / (Frequency of viable cells among anti-BCMA CAR positive T cells obtained in the absence of RTX and BRC)

*Flow-based cytotoxicity assay*

The cytolytic activity and specificity of anti-BCMA CAR T cell were assessed according to the flow cytometry-based cytotoxicity assay reported in Valton.et Al (2015) Mol Ther; 23(9):1507-1518. This assay consisted of labeling BCMA positive tumor target cell (T, H929) with 0.5 mM CellTrace™ CFSE (Life Technology, incubation 10 min 37°C according to manufacturer protocol) and co-incubate them with $10^5$ anti BCMA CAR T effector (E) cells (E/T ratio of 10:1) in a final volume of 100 μl X-Vivo-15 media, for 5 H at 37°C. Cells were then recovered and labeled with eFluor780 viability marker before being fixated by 4% PFA. Fixated cells were then analysed by flow cytometry to determine their viability.

*IFN release assay*

To investigate autoactivation of T cell expressing various anti-BCMA CAR comprising RTX specific epitopes by clinically relevant dose of RTX, primary T cells transfected with mRNA encoding SEQ ID 125, 130-139 were incubated, one day post transfection, for 72 hours in X-vivo-15 medium supplemented with 5% AB serum, 20 ng/mL IL2 in the absence or in the presence of 500 $\mu$g/mL RTX at a concentration of 0.1 $10^6$ cells/wells in a final volume of 100 $\mu$l. CAR T cells were then spun down, the supernatant was recovered and analysed by ELISA (using the Human IFN-gamma Quantikine ELISA Kit, RandD systems, ref # DIF50) to determine the amount of *IFN* released in the culture media. As positive control for CAR T cell activation and *IFN* release, cells were incubated with 10 $\mu$g/mL phytohemagglutinin (PHA).

*Purification of anti-BCMA CAR T cells using Miltenyi CD34 purification kit*

To test the capacity of certain anti-BCMA CAR architectures (containing the CD34 epitope, SEQ ID NO 144 recognized by the QBEND10 antibody) to be purified, 100 $10^6$ primary T cells steadily expressing SEQ ID 128 were purified using the CD34 MicroBead Kit (Miltenyi, ref# 130-046-702) according to the manufacturer protocol.

**Results**

*Depletability of anti-BCMA CAR positive T cells*

The results showed that T cells expressing SEQ ID 126-139 were all depleted to different extents by BRC and RTX in contrast to the unmodified anti-BCMA CAR (SEQ ID NO 125) that was not markedly depleted (Figure 8A). The results show that efficiency of depletion increase along with the number of CD20 mimotopes present in the CAR architecture. In addition, the results show that separating multiple CD20 mimotopes by domain larger than GS linkers, increased the efficiency of depletion as seen when comparing the extents of depletion obtained with T cells expressing SEQ ID NO 127 and SEQ ID NO 137 containing 3 CD20 mimotopes, as well as SEQ ID NO 139 and SEQ ID NO 136 containing 2 CD20 mimotopes (Figure 7-8A).

In some embodiments, the CAR of the invention having CAR architecture of SEQ ID NO126-139 allow rituximab dependent depletion of CAR T cell. In some embodiments the CAR of the invention having CAR architecture such as in SEQ ID, 136, 137, 138, i.e where the CAR comprises at least two identical mAb specific epitope separated by one or more other domains (such as VH, VL, VH-L1-VL...) are particularly efficiently depleted.

*Cytotoxic activity of anti-BCMA CAR + T cells*

The flow-based cytotoxicity assay results indicated that all architectures (SEQ ID 126-139) were able to recognize and kill BCMA-expressing H929 tumor cells to a similar extent than T cells expressing the unmodified version of anti-BCMA CAR architecture (SEQ ID NO 125, Figure 9). Consistent with the results obtained in Example 1, the presence of 1, 2 or 3 mAb specific epitopes and in particular 1, 2 or 3 CD20 mimotopes inside the CAR architecture, did not significantly influence the cytolytic activity of anti-BCMA CAR T cells.

In some embodiments, the CARs of the invention having CAR architectures of SEQ ID NO126-139 have similar cytotoxic activity as compared to a CAR without mAb-specific epitope such as a CAR of SEQ ID 125.

*Anti-BCMA positive CAR T cells sorting from an heterogeneous population of cells*

To test the capacity of T-cell expressing the anti-BCMA CAR of SEQ ID 128 (Figure 7A) to be purified from an heterogeneous population of cells, a bulk population of 100x$10^6$ primary T cells containing 31.5% of CAR positive cells was purified using the CD34 MicroBead Kit according to the manufacturer protocol. The results showed that the purified fraction harbored about 90% of anti-BCMA CAR positive T cells indicating that the purification process was efficient (Figure 10). Out of 31.5x$10^6$ anti-BCMA CAR positive T cells, about 20x$10^6$ anti-BCMA CAR positive T cells were recovered after purification indicating that less than 40% of anti-BCMA CAR positive T cells were lost throughout the purification process.

*IFN $\gamma$ release assay*

The ELISA assay results showed that the presence of one or multiple CD20 mimotopes within the CAR architecture did not influence the propensity of CAR T cells to be activated by RTX (Figure 11). Indeed The results showed that the level of *IFN $\gamma$* released by all architectures in the presence of RTX were similar to the basal level of *IFN $\gamma$* released in the absence of RTX.

**Example 5. Hybrid anti-BCMA chimeric antigen receptor architectures for optimal depletion and purification of CAR T cells.**

To improve the depletability of anti-BCMA CAR T cells and at the same time, allow to sort them, two new hybrid CAR architectures SEQ ID NO 140 and 141 (Figure 7C) were designed. These two architectures contained three CD20 mimotopes separated from one another by protein domains and one CD34 epitope. Their ability to be depleted by RTX and BRC was assessed by CDC assay according to the protocol described in Example 4. The results showed that these two architectures were efficiently depleted to a similar extent than T cells expressing SEQ ID 137 (Figure 8B). Their cytolytic properties were also assessed using the flow-based assay described earlier. The results showed that they share similar cytotoxic activity than the T cells expressing the unmodified anti-BCMA CAR T cells (SEQ ID NO 125) indicating that the presence of CD20 mimotopes and CD34 epitope (SEQ ID NO 35 and 144 respectively) did not negatively impact the cytolytic activity of CAR T cells.

In some embodiments the CAR of the invention having CAR architecture such as in SEQ ID 140, 141, i.e where the CAR comprises at three identical mAb-specific epitope recognized by an approved antibody such as rituximab which can be used for depletion of the cells and one mAb specific eptitope which can be used for purification are particularly efficiently depleted and can also be efficiently purified.

Additional CARs based on the architecture of SEQ ID NO 140 and 141 but comprising VH and VL of ScFv specific for CD19 (CAR of SEQ ID NO 162-163 and 168-169), CD123 (CAR of SEQ ID NO 164-165), CD20 (CAR of SEQ ID NO 166-167) were assembled according to the protocol described in Example 4. Their ability to be depleted by RTX and BRC can be assessed by CDC assay according to the protocol described in Example 4.

**Example 6. Universal detection of CAR T cells bearing a mAb-driven depletion system.**

Monitoring and comparing proliferation of different CAR T cells *in vivo* has been tiedous and cumbersome due to the lack of universal detection system. The ability of different CAR architectures to be detected was tested by flow cytometry using RTX as primary antibody and an FITC-coupled anti-Fab'2 monoclonal antibody (Life technologies, ref# H10101C, diluted 1/200) or using an APC labeled anti-CD34 monoclonal antibody named QBEND10 (Miltenyi Biotec, ref# 130-090-954, diluted 1/25). Results were compared side by side with detection performed with recombinant BCMA protein fused to an FC fragment (SEQ ID NO 151) and a PE labeled anti-FC secondary monoclonal antibody (Jackson ImmunoResearch, ref# 115-115-164, diluted 1/200).

The results, showed that the frequency of positive CAR T cells expressing SEQ ID NO 128 and 130-139 detected with RTX were similar to the ones obtained when they were detected with recombinant BCMA protein (Figure 12). Similar results were found when CAR T cells expressing SEQ ID NO 128 were detected with QBEND10 and rituximab (Figure 13). Altogether, The results showed that the presence of CD20 mimotope or CD34 epitope allow for efficient and universal detection of different CAR architectures.

**Example 7 - anti-BCMA CAR T cells expressing anti BCMA CAR comprising one, two or three mAB specific epitopes**

*7.1 - Plasmids*

The below CD20 mimotope-containing CARs are codon-optimized, synthesized and subcloned into the lentiviral vector pLVX-EF1a-IRES-Puro (Clontech) using the EcoRI (5') and MluI (3') restriction sites (thus removing the IRES-Puro cassette). Lentiviruses are produced using psPAX2, an HIV-1gag-pol packaging plasmid, and pMD2.G, a VSV-G expression plasmid.

BC30 (SEQ ID NO 145) comprises the following domains:
leader-BCMA30 VH-linker-BCMA30 VL-CD8 Hinge-CD8 TM-4-1BB-**CD3z** wherein BCMA30 VH and BCMA30 VL are respectively SEQ ID NO 97 and SEQ ID NO 98.

BC30-LM (SEQ ID NO 146) comprises the following domains:
Leader-BCMA30 VH-linker-BCMA30 VL-linker(L)-Mimotope (M)-CD8 Hinge-CD8 TM-4-1BB-CD3z wherein BCMA30 VH and BCMA30 VL are respectively SEQ ID NO 97 and SEQ ID NO 98 and the mimotope is SEQ ID NO35.

BC30-LML (SEQ ID NO 147) comprises the following domains:
**Leader**-BCMA30 VH-linker-BCMA30 VL-linker(L)-Mimotope (M)-linker(L)-CD8 Hinge-CD8 TM-4-1BB-**CD3z** wherein BCMA30 VH and BCMA30 VL are respectively SEQ ID NO 97 and SEQ ID NO 98 and the mimotope is SEQ ID NO35.

BC30-LMLM (SEQ ID NO 148) comprises the following domains:
Leader-BCMA30 VH-linker-BCMA30 VL-linker(L)-Mimotope (M)-linker(L)-Mimotope (M)-CD8 Hinge-CD8 TM-4-1BB-**CD3z** wherein BCMA30 VH and BCMA30 VL are respectively SEQ ID NO 97 and SEQ ID NO 98 and the mimotopes are both SEQ ID NO 35.

BC30-LMLML (SEQ ID NO 149) comprises the following domains:
Leader-BCMA30 VH-linker-BCMA30 VL-linker(L)-Mimotope (M)-linker(L)-Mimotope (M)-linker(L)-CD8 Hinge-CD8 TM-4-1BB-CD3z wherein BCMA30 VH and BCMA30 VL are respectively SEQ ID NO 97 and SEQ ID NO 98 and the mimotopes are both SEQ ID NO 35.

*7.2 - T cell activation and lentiviral transduction*

Untouched T cells are isolated from human peripheral blood mononuclear cells (PBMCs) using the Pan T Cell isolation kit (Miltenyi Biotec) and activated for three days with antibodies against human CD2, CD3, and CD28 (T Cell activation/expansion kit - Miltenyi Biotec). Lentiviral vectors (LV) are produced by transient transfection of sub-confluent HEK-293T/17 (American Type Culture Collection (ATCC)) cells in 6-well plates. Briefly, pLVX, psPAX2, and pMD2.G plasmids are transfected at a 4:3:1 ratio, respectively, using Lipofectamine 2000 (Invitrogen) following the manufacturer's instructions. The following day, the media is replaced with T cell culture medium (5% human AB serum in X-vivo-15 medium (Lonza)), and 48 h after transfection the LV supernatant is harvested and filtered through a 0.45 $\mu$m syringe filter (Millipore). Activated T cells are seeded at 0.25 x $10^6$ cells/mL in T cell culture medium containing 40 ng/ml IL-2 and transduced by adding an equal volume of fresh LV supernatant. Cells are cultured at 37°C and 5% CO2 for three days and used for flow cytometry analysis or expanded in fresh T cell medium containing 20 ng/ml IL-2.

*7.3 - Detection of BCMA CARs containing CD20 mimotopes by flow cytometry*

To test the utility of intra CAR CD20 mimotopes for detection and tracking of CAR-T cells, flow cytometry analysis is performed on transduced T cells using either biotinylated-BCMA protein , which binds the scFV region of the CAR followed by PE-conjugated streptavidin, or the anti-CD20 antibody rituximab followed by FITC-conjugated anti-human IgG (Rituximab (FITC)). Figures 14A and 14B show that T cells transduced with the different CD20-mimotope-containing CARs are detected with comparable efficiency by flow cytometry using biotinylated-BCMA followed by PE-conjugated streptavidin. Detection of intra CAR CD20 mimotope(s) with rituximab is weak in cells transduced with the LM construct (15.5%) but very high in all other formats tested. For example, the LMLML CAR is detected in 85.6% of the cells, indicating that this format allows the identification of virtually all cells expressing the CAR (Figures 14A and 14B). Thus, the presence of two CD20 epitopes separated by flexible linkers allows for enhanced binding to rituximab and provides an optimum system to detect CAR+ cells.

The functionality of intra CAR CD20 epitopes for CAR-T cell detection is assessed by comparison with the RQR8 marker/suicide gene system (SEQ ID NO 150), which consists of a compact protein containing two CD20 epitopes and a CD34 epitope that is normally co-expressed with the CAR (Philip, Blood 2014). For this experiment, T cells are transduced with a lentivirus that allows the co-expression of the BCMA30 CAR (SEQ ID NO 145) and the RQR8 protein (SEQ ID NO 150) (BC30-RQR8 construct). For comparison, T cells are transduced with the BCMA30 LMLML CAR construct (BC30-R2 construct - SEQ ID NO 149) and analyzed by flow cytometry three days post-transduction. In addition, non-transduced (NT) T cells serve as negative control. Figure 15 aims to show that incorporating the CD20 epitopes in the CAR molecule significantly improves detection of CAR-T cells with the anti-CD20 antibody rituximab. In addition, increased transduction efficiency and CAR expression is observed in cells transduced with the BC30-R2 construct compared with those transduced with the vector encoding RQR8 and the CAR, as indicated by flow cytometry analysis with biotinylated BCMA (Figure 15). Thus, insertion of CD20 epitopes into the CAR molecule enables enhanced transduction, improved detection, and absolute correlation between CAR expression and mAb specific epitope(s) expression.

*7.4 - Intra CAR CD20 epitopes sensitize CAR-T cells to complement-dependent cytotoxicity*

The ability of intra CAR CD20 epitopes to enable selective elimination of CAR-T cells is evaluated in vitro using a CDC assay. The objective is to show that the presence of CD20 epitopes in the CAR molecule renders CAR-T cells highly susceptible to rituximab-mediated depletion. For this experiment, T cells transduced with either the BC30-R2 construct or the BC30-RQR8 construct are mixed with 25% baby-rabbit complement (AbD serotec) in the presence or absence of rituximab (100 $\mu$g/mL) and incubated at 37°C and 5% CO2 for 4 hours. Selective deletion of CAR-T cells is determined by flow cytometry analysis using biotinylated BCMA protein. Figure 16 shows that while both the RQR8 and intra CAR CD20 epitope suicide gene systems enable CAR-T cell depletion, cells transduced with the BC30-R2 construct are depleted more efficiently than those expressing RQR8. As expected, T cells expressing the BCMA30 CAR but no CD20 epitopes (BC30 construct) are spared. These differences may be due to the high expression of the BC30-R2 CAR and the absolute correlation between CAR expression and suicide gene expression.

*7.5 - Incorporation of CD20 epitopes into CARs does not impair the cytolytic activity of CAR-T cells*

The possibility that insertion of CD20 epitopes between the hinge and the scFv regions of the CAR might impair CAR activity is evaluated in a cytotoxicity assay. Briefly, T cells expressing either the BC30-R2 construct or the BC30-RQR8 construct are incubated with Luciferase-positive MM1S target cells at different ratios. For these killing assays, cells are seeded in 96-well white opaque tissue culture plates in a final volume of 100 $\mu$l of 5% human AB serum in X-vivo-15 medium (Lonza). After 4 hours, cells are equilibrated to room temperature and one volume of Bright-Glo™ Reagent (Promega) is added to each well. Luminescence is measured in a GLOMAX 96 microplate luminometer (Promega) and percentage of cell lysis is calculated according to the following formula:

100 x (1 - (Sample lysis - max lysis)/(Spontaneous lysis - max lysis)). Maximum lysis is determined by addition of 8% Triton X-100 (Sigma) to Luc+ MM1S cells. For spontaneous lysis, MM1S cells are incubated in the absence of effector CAR-T cells.

The results show that BC30-R2 CAR-T cells effectively eliminate BCMA-expressing MM1S cells in vitro (Figure 17). Moreover, the cytolytic activity of BC30-R2 CAR-T cells is not influenced by rituximab (100 $\mu$g/mL), which is added to the effector cell population 2 h before these cells are mixed with the target cells (Figure 17). This experiment aims to demonstrate that insertion of CD20 epitopes into the BC30 CAR molecule does not affect its ability to mediate killing of BCMA+ target cells, even in the presence of rituximab.

*7.6 Rituximab binding to intra CAR CD20 epitopes does not lead to CAR-T cell activation*

To investigate if crosslinking of CARs by rituximab might lead to T cell activation due to CAR aggregation on the cell surface, BC30-R2 CAR-T cells are grown in the presence of rituximab. The anti-CD3 OKT3 antibody (eBioscience) causes crosslinking of the T cell receptor (TCR) resulting in cellular activation and proliferation and is used as a positive control. Briefly, BC30-R2 CAR-T cells are cultured in T cell medium in the presence/absence of rituximab for three days. T cell activation is then assessed by measuring the expression of the activation markers CD25 and CD69 using flow cytometry. This experiment shows that the percentage of activated T cells in the presence of RTX is not significantly different from the control (PBS). Therefore, soluble rituximab has no significant effect on the activation state of BC30-R2 CAR-T cells (Figure 18).

REFERENCES

- Arbiza J., Taylor G., López J.A., Furze J., Wyld S., Whyte P., Stott E.J., Wertz G., Sullender W., Trudel M. , et al. (1992), Characterization of two antigenic sites recognized by neutralizing monoclonal antibodies directed against the fusion glycoprotein of human respiratory syncytial virus. J Gen Virol.;73 (9):2225-34).
- Benjamin, RJ and Waldmann, H. (1986). Induction of tolerance by monoclonal antibody therapy. Nature 520: 449-451.
- Boch, J., H. Scholze, et al. (2009). Breaking the code of DNA binding specificity of TAL-type III effectors. Science 326(5959): 1509-12.
- Budde, L.E., Berger C, Lin Y, Wang J, Lin X, Frayo SE, Brouns SA, Spencer DM, Till BG, Jensen MC, Riddell SR (2013). Combining a CD20 chimeric antigen receptor and an inducible caspase 9 suicide switch to improve the efficacy and safety of T cell adoptive immunotherapy for lymphoma. PLoS One. Dec 17;8(12):e82742
- Buller R.M., Holmes K.L., Hügin A., Fredrickson T.N., Morse H.C. (1987). Induction of cytotoxic T cell responses in vivo in the absence of CD4 helper cells. Nature.;328:77-79.
- Cobbold, S.P., Martin, G., Qin, S., and Waldmann, H. (1986). Monoclonal antibodies to promote marrow engraftment and tissue graft rejection. Nature 323:164-166
- Dolan DE, Gupta S. 2014 PD-1 pathway inhibitors: changing the landscape of cancer immunotherapy. Cancer Control. 21(3):231-7.
- Cartellieri. M., Koristka, S., Arndt, C., Feldmann, A., Stamova, S., von Bonin, M., Töpfer, K., Krüger, T., Geib, M., Michalk, I., Temme, A., Bornhäuser, M., Lindemann, D., Ehninger, G., Bachmann, M.P. (2014). A novel ex vivo isolation and expansion procedure for chimeric antigen receptor engrafted human T cells. PloS One 9(4):e93745.
- Epa, V. C., O. Dolezal, et al. (2013). Structural model for the interaction of a designed Ankyrin Repeat Protein with the human epidermal growth factor receptor 2. PLoS One 8(3): e59163. Fedorov V.D., Themeli M and Sadelain M. (2013). PD-1- and CTLA-4-Based Inhibitory Chimeric Antigen Receptors (iCARs) Divert Off-Target Immunotherapy Responses. Sci Transl Med:5 (215), 215
- Friedrich, K., J. R. Hanauer, et al. (2013). DARPin-targeting of measles virus: unique bispecificity, effective oncolysis, and enhanced safety. Mol Ther 21(4): 849-59.

- Jena, B., G. Dotti, et al. (2010). Redirecting T-cell specificity by introducing a tumor-specific chimeric antigen receptor. Blood 116(7): 1035-44.
- Jost, C., J. Schilling, et al. (2013). Structural Basis for Eliciting a Cytotoxic Effect in HER2-Overexpressing Cancer Cells via Binding to the Extracellular Domain of HER2. Structure 21(11): 1979-91.
- Moscou, M. J. and A. J. Bogdanove (2009). A simple cipher governs DNA recognition by TAL effectors. Science 326(5959): 1501.
- Park, T. S., S. A. Rosenberg, et al. (2011). Treating cancer with genetically engineered T cells. Trends Biotechnol 29(11): 550-7.
- Philip B, Kokalaki E, Mekkaoui L, Thomas S, Straathof K, Flutter B, Marin V, Marafioti T, Chakraverty R, Linch D, Quezada SA, Peggs KS, Pule M (2014). A highly compact epitope-based marker/suicide gene for easier and safer T-cell therapy. Blood. 124(8):1277-87
- Riemer A.B., Kurz H., Klinger, M., Scheiner, O., Zielinski, C., and Jensen-Jarolim, E. (2005), Vaccination with cetuximab mimotopes and biological properties of induced anti-epidermal growth factor receptor antibodies, J Natl Cancer Inst.;97(22):1663-70)
- Valton J., Guyot V., Marechal A., Filhol JM., Juillerat A., Duclert A., Duchateau P., Poirot L. (2015) A multidrug resistant engineered CAR T cell for allogeneic combination immunotherapy. Mol Ther; 23(9):1507-1518
- Philip B, Kokalaki E, Mekkaoui L, Thomas S, et al. A highly compact epitope-based marker/suicide gene for easier and safer T-cell therapy. Blood 2014; 124(8):1277-87.

SEQUENCE LISTING

<110> Rinat; Cellectis

<120> mAb-DRIVEN CHIMERIC ANTIGEN RECEPTOR SYSTEMS FOR SORTING/DEPLETING ENGINEERED IMMUNE CELLS

<130> P81500233PCT00

<150> PA201570044
<151> 2015-01-26

<160> 185

<170> PatentIn version 3.5

<210> 1
<211> 511
<212> PRT
<213> artificial sequence

<220>
<223> Anti-CD123 NO1

<400> 1

```
Met Ala Leu Pro Val Thr Ala Leu Leu Leu Pro Leu Ala Leu Leu Leu
1               5                   10                  15

His Ala Ala Arg Pro Gln Ile Gln Leu Val Gln Ser Gly Pro Glu Leu
                20                  25                  30

Lys Lys Pro Gly Glu Thr Val Lys Ile Ser Cys Lys Ala Ser Gly Tyr
            35                  40                  45

Ile Phe Thr Asn Tyr Gly Met Asn Trp Val Lys Gln Ala Pro Gly Lys
        50                  55                  60

Ser Phe Lys Trp Met Gly Trp Ile Asn Thr Tyr Thr Gly Glu Ser Thr
65                  70                  75                  80

Tyr Ser Ala Asp Phe Lys Gly Arg Phe Ala Phe Ser Leu Glu Thr Ser
                85                  90                  95

Ala Ser Thr Ala Tyr Leu His Ile Asn Asp Leu Lys Asn Glu Asp Thr
            100                 105                 110

Ala Thr Tyr Phe Cys Ala Arg Ser Gly Gly Tyr Asp Pro Met Asp Tyr
        115                 120                 125

Trp Gly Gln Gly Thr Ser Val Thr Val Ser Ser Gly Gly Gly Gly Ser
    130                 135                 140

Gly Gly Gly Gly Ser Gly Gly Gly Gly Ser Asp Ile Val Leu Thr Gln
```

145                    150                    155                    160

Ser Pro Ala Ser Leu Ala Val Ser Leu Gly Gln Arg Ala Thr Ile Ser
              165                    170                    175

Cys Arg Ala Ser Glu Ser Val Asp Asn Tyr Gly Asn Thr Phe Met His
              180                    185                    190

Trp Tyr Gln Gln Lys Pro Gly Gln Pro Pro Lys Leu Leu Ile Tyr Arg
              195                    200                    205

Ala Ser Asn Leu Glu Ser Gly Ile Pro Ala Arg Phe Ser Gly Ser Gly
    210                    215                    220

Ser Arg Thr Asp Phe Thr Leu Thr Ile Asn Pro Val Glu Ala Asp Asp
225                    230                    235                    240

Val Ala Thr Tyr Tyr Cys Gln Gln Ser Asn Glu Asp Pro Pro Thr Phe
              245                    250                    255

Gly Ala Gly Thr Lys Leu Glu Leu Lys Arg Ser Asp Pro Gly Ser Gly
              260                    265                    270

Gly Gly Gly Ser Cys Pro Tyr Ser Asn Pro Ser Leu Cys Ala Pro Thr
              275                    280                    285

Thr Thr Pro Ala Pro Arg Pro Pro Thr Pro Ala Pro Thr Ile Ala Ser
    290                    295                    300

Gln Pro Leu Ser Leu Arg Pro Glu Ala Cys Arg Pro Ala Ala Gly Gly
305                    310                    315                    320

Ala Val His Thr Arg Gly Leu Asp Phe Ala Cys Asp Ile Tyr Ile Trp
              325                    330                    335

Ala Pro Leu Ala Gly Thr Cys Gly Val Leu Leu Leu Ser Leu Val Ile
              340                    345                    350

Thr Leu Tyr Cys Arg Arg Gly Arg Lys Lys Leu Leu Tyr Ile Phe Lys
              355                    360                    365

Gln Pro Phe Met Arg Pro Val Gln Thr Thr Gln Glu Glu Asp Gly Cys
    370                    375                    380

Ser Cys Arg Phe Pro Glu Glu Glu Glu Gly Gly Cys Glu Leu Arg Val
385                    390                    395                    400

39

```
Lys Phe Ser Arg Ser Ala Asp Ala Pro Ala Tyr Gln Gln Gly Gln Asn
            405             410             415

Gln Leu Tyr Asn Glu Leu Asn Leu Gly Arg Arg Glu Glu Tyr Asp Val
            420             425             430

Leu Asp Lys Arg Arg Gly Arg Asp Pro Glu Met Gly Gly Lys Pro Arg
            435             440             445

Arg Lys Asn Pro Gln Glu Gly Leu Tyr Asn Glu Leu Gln Lys Asp Lys
        450             455             460

Met Ala Glu Ala Tyr Ser Glu Ile Gly Met Lys Gly Glu Arg Arg Arg
465             470             475             480

Gly Lys Gly His Asp Gly Leu Tyr Gln Gly Leu Ser Thr Ala Thr Lys
            485             490             495

Asp Thr Tyr Asp Ala Leu His Met Gln Ala Leu Pro Pro Arg Glu
            500             505             510
```

<210> 2
<211> 517
<212> PRT
<213> artificial sequence

<220>
<223> Anti-CD123 No2

<400> 2

```
Met Ala Leu Pro Val Thr Ala Leu Leu Leu Pro Leu Ala Leu Leu Leu
1               5               10              15

His Ala Ala Arg Pro Gln Ile Gln Leu Val Gln Ser Gly Pro Glu Leu
            20              25              30

Lys Lys Pro Gly Glu Thr Val Lys Ile Ser Cys Lys Ala Ser Gly Tyr
        35              40              45

Ile Phe Thr Asn Tyr Gly Met Asn Trp Val Lys Gln Ala Pro Gly Lys
    50              55              60

Ser Phe Lys Trp Met Gly Trp Ile Asn Thr Tyr Thr Gly Glu Ser Thr
65              70              75              80

Tyr Ser Ala Asp Phe Lys Gly Arg Phe Ala Phe Ser Leu Glu Thr Ser
            85              90              95

Ala Ser Thr Ala Tyr Leu His Ile Asn Asp Leu Lys Asn Glu Asp Thr
```

100                     105                        110

Ala Thr Tyr Phe Cys Ala Arg Ser Gly Gly Tyr Asp Pro Met Asp Tyr
        115                 120                 125

Trp Gly Gln Gly Thr Ser Val Thr Val Ser Ser Gly Gly Gly Gly Ser
        130                 135                 140

Gly Gly Gly Gly Ser Gly Gly Gly Gly Ser Asp Ile Val Leu Thr Gln
145                 150                 155                 160

Ser Pro Ala Ser Leu Ala Val Ser Leu Gly Gln Arg Ala Thr Ile Ser
                165                 170                 175

Cys Arg Ala Ser Glu Ser Val Asp Asn Tyr Gly Asn Thr Phe Met His
            180                 185                 190

Trp Tyr Gln Gln Lys Pro Gly Gln Pro Pro Lys Leu Leu Ile Tyr Arg
        195                 200                 205

Ala Ser Asn Leu Glu Ser Gly Ile Pro Ala Arg Phe Ser Gly Ser Gly
        210                 215                 220

Ser Arg Thr Asp Phe Thr Leu Thr Ile Asn Pro Val Glu Ala Asp Asp
225                 230                 235                 240

Val Ala Thr Tyr Tyr Cys Gln Gln Ser Asn Glu Asp Pro Pro Thr Phe
                245                 250                 255

Gly Ala Gly Thr Lys Leu Glu Leu Lys Arg Ser Asp Pro Gly Ser Gly
            260                 265                 270

Gly Gly Gly Ser Cys Pro Tyr Ser Asn Pro Ser Leu Cys Ser Gly Gly
        275                 280                 285

Gly Gly Ser Ala Pro Thr Thr Thr Pro Ala Pro Arg Pro Pro Thr Pro
        290                 295                 300

Ala Pro Thr Ile Ala Ser Gln Pro Leu Ser Leu Arg Pro Glu Ala Cys
305                 310                 315                 320

Arg Pro Ala Ala Gly Gly Ala Val His Thr Arg Gly Leu Asp Phe Ala
                325                 330                 335

Cys Asp Ile Tyr Ile Trp Ala Pro Leu Ala Gly Thr Cys Gly Val Leu
        340                 345                 350

42

```
Leu Leu Ser Leu Val Ile Thr Leu Tyr Cys Arg Arg Gly Arg Lys Lys
        355             360             365

Leu Leu Tyr Ile Phe Lys Gln Pro Phe Met Arg Pro Val Gln Thr Thr
        370             375             380

Gln Glu Glu Asp Gly Cys Ser Cys Arg Phe Pro Glu Glu Glu Glu Gly
385             390             395             400

Gly Cys Glu Leu Arg Val Lys Phe Ser Arg Ser Ala Asp Ala Pro Ala
                405             410             415

Tyr Gln Gln Gly Gln Asn Gln Leu Tyr Asn Glu Leu Asn Leu Gly Arg
            420             425             430

Arg Glu Glu Tyr Asp Val Leu Asp Lys Arg Arg Gly Arg Asp Pro Glu
        435             440             445

Met Gly Gly Lys Pro Arg Arg Lys Asn Pro Gln Glu Gly Leu Tyr Asn
        450             455             460

Glu Leu Gln Lys Asp Lys Met Ala Glu Ala Tyr Ser Glu Ile Gly Met
465             470             475             480

Lys Gly Glu Arg Arg Arg Gly Lys Gly His Asp Gly Leu Tyr Gln Gly
                485             490             495

Leu Ser Thr Ala Thr Lys Asp Thr Tyr Asp Ala Leu His Met Gln Ala
            500             505             510

Leu Pro Pro Arg Glu
            515
```

<210> 3
<211> 526
<212> PRT
<213> artificial sequence

<220>
<223> Anti-CD123 No3

<400> 3

```
Met Ala Leu Pro Val Thr Ala Leu Leu Leu Pro Leu Ala Leu Leu Leu
1               5               10              15

His Ala Ala Arg Pro Gln Ile Gln Leu Val Gln Ser Gly Pro Glu Leu
        20              25              30

Lys Lys Pro Gly Glu Thr Val Lys Ile Ser Cys Lys Ala Ser Gly Tyr
```

                    35                          40                          45

Ile Phe Thr Asn Tyr Gly Met Asn Trp Val Lys Gln Ala Pro Gly Lys
    50                  55                  60

Ser Phe Lys Trp Met Gly Trp Ile Asn Thr Tyr Thr Gly Glu Ser Thr
65                  70                  75                  80

Tyr Ser Ala Asp Phe Lys Gly Arg Phe Ala Phe Ser Leu Glu Thr Ser
                85                  90                  95

Ala Ser Thr Ala Tyr Leu His Ile Asn Asp Leu Lys Asn Glu Asp Thr
            100                 105                 110

Ala Thr Tyr Phe Cys Ala Arg Ser Gly Gly Tyr Asp Pro Met Asp Tyr
            115                 120                 125

Trp Gly Gln Gly Thr Ser Val Thr Val Ser Ser Gly Gly Gly Gly Ser
    130                 135                 140

Gly Gly Gly Gly Ser Gly Gly Gly Gly Ser Asp Ile Val Leu Thr Gln
145                 150                 155                 160

Ser Pro Ala Ser Leu Ala Val Ser Leu Gly Gln Arg Ala Thr Ile Ser
            165                 170                 175

Cys Arg Ala Ser Glu Ser Val Asp Asn Tyr Gly Asn Thr Phe Met His
            180                 185                 190

Trp Tyr Gln Gln Lys Pro Gly Gln Pro Pro Lys Leu Leu Ile Tyr Arg
            195                 200                 205

Ala Ser Asn Leu Glu Ser Gly Ile Pro Ala Arg Phe Ser Gly Ser Gly
    210                 215                 220

Ser Arg Thr Asp Phe Thr Leu Thr Ile Asn Pro Val Glu Ala Asp Asp
225                 230                 235                 240

Val Ala Thr Tyr Tyr Cys Gln Gln Ser Asn Glu Asp Pro Pro Thr Phe
            245                 250                 255

Gly Ala Gly Thr Lys Leu Glu Leu Lys Arg Ser Asp Pro Gly Ser Gly
            260                 265                 270

Gly Gly Gly Ser Cys Pro Tyr Ser Asn Pro Ser Leu Cys Ser Gly Gly
            275                 280                 285

```
Gly Gly Ser Cys Pro Tyr Ser Asn Pro Ser Leu Cys Ala Pro Thr Thr
    290             295             300

Thr Pro Ala Pro Arg Pro Pro Thr Pro Ala Pro Thr Ile Ala Ser Gln
305             310             315             320

Pro Leu Ser Leu Arg Pro Glu Ala Cys Arg Pro Ala Ala Gly Gly Ala
                325             330             335

Val His Thr Arg Gly Leu Asp Phe Ala Cys Asp Ile Tyr Ile Trp Ala
            340             345             350

Pro Leu Ala Gly Thr Cys Gly Val Leu Leu Leu Ser Leu Val Ile Thr
            355             360             365

Leu Tyr Cys Arg Arg Gly Arg Lys Lys Leu Leu Tyr Ile Phe Lys Gln
    370             375             380

Pro Phe Met Arg Pro Val Gln Thr Thr Gln Glu Glu Asp Gly Cys Ser
385             390             395             400

Cys Arg Phe Pro Glu Glu Glu Glu Gly Gly Cys Glu Leu Arg Val Lys
            405             410             415

Phe Ser Arg Ser Ala Asp Ala Pro Ala Tyr Gln Gln Gly Gln Asn Gln
            420             425             430

Leu Tyr Asn Glu Leu Asn Leu Gly Arg Arg Glu Glu Tyr Asp Val Leu
            435             440             445

Asp Lys Arg Arg Gly Arg Asp Pro Glu Met Gly Gly Lys Pro Arg Arg
    450             455             460

Lys Asn Pro Gln Glu Gly Leu Tyr Asn Glu Leu Gln Lys Asp Lys Met
465             470             475             480

Ala Glu Ala Tyr Ser Glu Ile Gly Met Lys Gly Glu Arg Arg Arg Gly
            485             490             495

Lys Gly His Asp Gly Leu Tyr Gln Gly Leu Ser Thr Ala Thr Lys Asp
            500             505             510

Thr Tyr Asp Ala Leu His Met Gln Ala Leu Pro Pro Arg Glu
            515             520             525
```

<210> 4
<211> 532

<212> PRT
<213> artificial sequence

<220>
<223> Anti-CD123 No4

<400> 4

```
Met Ala Leu Pro Val Thr Ala Leu Leu Leu Pro Leu Ala Leu Leu Leu
1               5                   10                  15

His Ala Ala Arg Pro Gln Ile Gln Leu Val Gln Ser Gly Pro Glu Leu
            20                  25                  30

Lys Lys Pro Gly Glu Thr Val Lys Ile Ser Cys Lys Ala Ser Gly Tyr
            35                  40                  45

Ile Phe Thr Asn Tyr Gly Met Asn Trp Val Lys Gln Ala Pro Gly Lys
    50                  55                  60

Ser Phe Lys Trp Met Gly Trp Ile Asn Thr Tyr Thr Gly Glu Ser Thr
65                  70                  75                  80

Tyr Ser Ala Asp Phe Lys Gly Arg Phe Ala Phe Ser Leu Glu Thr Ser
                85                  90                  95

Ala Ser Thr Ala Tyr Leu His Ile Asn Asp Leu Lys Asn Glu Asp Thr
            100                 105                 110

Ala Thr Tyr Phe Cys Ala Arg Ser Gly Gly Tyr Asp Pro Met Asp Tyr
            115                 120                 125

Trp Gly Gln Gly Thr Ser Val Thr Val Ser Ser Gly Gly Gly Gly Ser
    130                 135                 140

Gly Gly Gly Gly Ser Gly Gly Gly Gly Ser Asp Ile Val Leu Thr Gln
145                 150                 155                 160

Ser Pro Ala Ser Leu Ala Val Ser Leu Gly Gln Arg Ala Thr Ile Ser
                165                 170                 175

Cys Arg Ala Ser Glu Ser Val Asp Asn Tyr Gly Asn Thr Phe Met His
            180                 185                 190

Trp Tyr Gln Gln Lys Pro Gly Gln Pro Pro Lys Leu Leu Ile Tyr Arg
        195                 200                 205

Ala Ser Asn Leu Glu Ser Gly Ile Pro Ala Arg Phe Ser Gly Ser Gly
    210                 215                 220
```

```
Ser Arg Thr Asp Phe Thr Leu Thr Ile Asn Pro Val Glu Ala Asp Asp
225             230         235             240

Val Ala Thr Tyr Tyr Cys Gln Gln Ser Asn Glu Asp Pro Pro Thr Phe
            245             250             255

Gly Ala Gly Thr Lys Leu Glu Leu Lys Arg Ser Asp Pro Gly Ser Gly
            260             265             270

Gly Gly Gly Ser Cys Pro Tyr Ser Asn Pro Ser Leu Cys Ser Gly Gly
            275             280             285

Gly Gly Ser Cys Pro Tyr Ser Asn Pro Ser Leu Cys Ser Gly Gly Gly
            290             295             300

Gly Ser Ala Pro Thr Thr Thr Pro Ala Pro Arg Pro Pro Thr Pro Ala
305             310             315             320

Pro Thr Ile Ala Ser Gln Pro Leu Ser Leu Arg Pro Glu Ala Cys Arg
            325             330             335

Pro Ala Ala Gly Gly Ala Val His Thr Arg Gly Leu Asp Phe Ala Cys
            340             345             350

Asp Ile Tyr Ile Trp Ala Pro Leu Ala Gly Thr Cys Gly Val Leu Leu
            355             360             365

Leu Ser Leu Val Ile Thr Leu Tyr Cys Arg Arg Gly Arg Lys Lys Leu
            370             375             380

Leu Tyr Ile Phe Lys Gln Pro Phe Met Arg Pro Val Gln Thr Thr Gln
385             390             395             400

Glu Glu Asp Gly Cys Ser Cys Arg Phe Pro Glu Glu Glu Glu Gly Gly
            405             410             415

Cys Glu Leu Arg Val Lys Phe Ser Arg Ser Ala Asp Ala Pro Ala Tyr
            420             425             430

Gln Gln Gly Gln Asn Gln Leu Tyr Asn Glu Leu Asn Leu Gly Arg Arg
            435             440             445

Glu Glu Tyr Asp Val Leu Asp Lys Arg Arg Gly Arg Asp Pro Glu Met
            450             455             460

Gly Gly Lys Pro Arg Arg Lys Asn Pro Gln Glu Gly Leu Tyr Asn Glu
465             470             475             480
```

```
Leu Gln Lys Asp Lys Met Ala Glu Ala Tyr Ser Glu Ile Gly Met Lys
                485             490                 495

Gly Glu Arg Arg Arg Gly Lys Gly His Asp Gly Leu Tyr Gln Gly Leu
            500             505                 510

Ser Thr Ala Thr Lys Asp Thr Tyr Asp Ala Leu His Met Gln Ala Leu
            515             520             525

Pro Pro Arg Glu
        530
```

<210> 5
<211> 495
<212> PRT
<213> artificial sequence

<220>
<223> Anti-CD123 No5

<400> 5

```
Met Ala Leu Pro Val Thr Ala Leu Leu Leu Pro Leu Ala Leu Leu Leu
1               5               10                  15

His Ala Ala Arg Pro Gln Ile Gln Leu Val Gln Ser Gly Pro Glu Leu
            20              25              30

Lys Lys Pro Gly Glu Thr Val Lys Ile Ser Cys Lys Ala Ser Gly Tyr
        35              40              45

Ile Phe Thr Asn Tyr Gly Met Asn Trp Val Lys Gln Ala Pro Gly Lys
        50              55              60

Ser Phe Lys Trp Met Gly Trp Ile Asn Thr Tyr Thr Gly Glu Ser Thr
65              70              75              80

Tyr Ser Ala Asp Phe Lys Gly Arg Phe Ala Phe Ser Leu Glu Thr Ser
            85              90              95

Ala Ser Thr Ala Tyr Leu His Ile Asn Asp Leu Lys Asn Glu Asp Thr
            100             105             110

Ala Thr Tyr Phe Cys Ala Arg Ser Gly Gly Tyr Asp Pro Met Asp Tyr
            115             120             125

Trp Gly Gln Gly Thr Ser Val Thr Val Ser Ser Gly Gly Gly Cys Pro
        130             135             140
```

Tyr Ser Asn Pro Ser Leu Cys Gly Gly Gly Gly Ser Asp Ile Val Leu
145             150             155             160

Thr Gln Ser Pro Ala Ser Leu Ala Val Ser Leu Gly Gln Arg Ala Thr
            165             170             175

Ile Ser Cys Arg Ala Ser Glu Ser Val Asp Asn Tyr Gly Asn Thr Phe
        180             185             190

Met His Trp Tyr Gln Gln Lys Pro Gly Gln Pro Pro Lys Leu Leu Ile
        195             200             205

Tyr Arg Ala Ser Asn Leu Glu Ser Gly Ile Pro Ala Arg Phe Ser Gly
    210             215             220

Ser Gly Ser Arg Thr Asp Phe Thr Leu Thr Ile Asn Pro Val Glu Ala
225             230             235             240

Asp Asp Val Ala Thr Tyr Tyr Cys Gln Gln Ser Asn Glu Asp Pro Pro
            245             250             255

Thr Phe Gly Ala Gly Thr Lys Leu Glu Leu Lys Arg Ser Asp Pro Thr
            260             265             270

Thr Thr Pro Ala Pro Arg Pro Pro Thr Pro Ala Pro Thr Ile Ala Ser
        275             280             285

Gln Pro Leu Ser Leu Arg Pro Glu Ala Cys Arg Pro Ala Ala Gly Gly
    290             295             300

Ala Val His Thr Arg Gly Leu Asp Phe Ala Cys Asp Ile Tyr Ile Trp
305             310             315             320

Ala Pro Leu Ala Gly Thr Cys Gly Val Leu Leu Leu Ser Leu Val Ile
            325             330             335

Thr Leu Tyr Cys Arg Arg Gly Arg Lys Lys Leu Leu Tyr Ile Phe Lys
        340             345             350

Gln Pro Phe Met Arg Pro Val Gln Thr Thr Gln Glu Glu Asp Gly Cys
    355             360             365

Ser Cys Arg Phe Pro Glu Glu Glu Glu Gly Gly Cys Glu Leu Arg Val
    370             375             380

Lys Phe Ser Arg Ser Ala Asp Ala Pro Ala Tyr Gln Gln Gly Gln Asn
385             390             395             400

```
Gln Leu Tyr Asn Glu Leu Asn Leu Gly Arg Arg Glu Glu Tyr Asp Val
            405             410             415

Leu Asp Lys Arg Arg Gly Arg Asp Pro Glu Met Gly Gly Lys Pro Arg
            420             425             430

Arg Lys Asn Pro Gln Glu Gly Leu Tyr Asn Glu Leu Gln Lys Asp Lys
            435             440             445

Met Ala Glu Ala Tyr Ser Glu Ile Gly Met Lys Gly Glu Arg Arg Arg
    450             455             460

Gly Lys Gly His Asp Gly Leu Tyr Gln Gly Leu Ser Thr Ala Thr Lys
465             470             475             480

Asp Thr Tyr Asp Ala Leu His Met Gln Ala Leu Pro Pro Arg Glu
            485             490             495
```

<210> 6
<211> 501
<212> PRT
<213> artificial sequence

<220>
<223> Anti-CD123 No6

<400> 6

```
Met Ala Leu Pro Val Thr Ala Leu Leu Leu Pro Leu Ala Leu Leu Leu
1               5               10              15

His Ala Ala Arg Pro Gln Ile Gln Leu Val Gln Ser Gly Pro Glu Leu
            20              25              30

Lys Lys Pro Gly Glu Thr Val Lys Ile Ser Cys Lys Ala Ser Gly Tyr
            35              40              45

Ile Phe Thr Asn Tyr Gly Met Asn Trp Val Lys Gln Ala Pro Gly Lys
    50              55              60

Ser Phe Lys Trp Met Gly Trp Ile Asn Thr Tyr Thr Gly Glu Ser Thr
65              70              75              80

Tyr Ser Ala Asp Phe Lys Gly Arg Phe Ala Phe Ser Leu Glu Thr Ser
            85              90              95

Ala Ser Thr Ala Tyr Leu His Ile Asn Asp Leu Lys Asn Glu Asp Thr
            100             105             110
```

Ala Thr Tyr Phe Cys Ala Arg Ser Gly Gly Tyr Asp Pro Met Asp Tyr
        115                 120                 125

Trp Gly Gln Gly Thr Ser Val Thr Val Ser Ser Gly Gly Gly Gly
        130                 135                 140

Gly Cys Pro Tyr Ser Asn Pro Ser Leu Cys Gly Gly Gly Gly Gly
        145                 150                 155                 160

Gly Ser Asp Ile Val Leu Thr Gln Ser Pro Ala Ser Leu Ala Val Ser
                165                 170                 175

Leu Gly Gln Arg Ala Thr Ile Ser Cys Arg Ala Ser Glu Ser Val Asp
                180                 185                 190

Asn Tyr Gly Asn Thr Phe Met His Trp Tyr Gln Gln Lys Pro Gly Gln
                195                 200                 205

Pro Pro Lys Leu Leu Ile Tyr Arg Ala Ser Asn Leu Glu Ser Gly Ile
        210                 215                 220

Pro Ala Arg Phe Ser Gly Ser Gly Ser Arg Thr Asp Phe Thr Leu Thr
225                 230                 235                 240

Ile Asn Pro Val Glu Ala Asp Asp Val Ala Thr Tyr Tyr Cys Gln Gln
                245                 250                 255

Ser Asn Glu Asp Pro Pro Thr Phe Gly Ala Gly Thr Lys Leu Glu Leu
                260                 265                 270

Lys Arg Ser Asp Pro Thr Thr Thr Pro Ala Pro Arg Pro Pro Thr Pro
        275                 280                 285

Ala Pro Thr Ile Ala Ser Gln Pro Leu Ser Leu Arg Pro Glu Ala Cys
        290                 295                 300

Arg Pro Ala Ala Gly Gly Ala Val His Thr Arg Gly Leu Asp Phe Ala
305                 310                 315                 320

Cys Asp Ile Tyr Ile Trp Ala Pro Leu Ala Gly Thr Cys Gly Val Leu
                325                 330                 335

Leu Leu Ser Leu Val Ile Thr Leu Tyr Cys Arg Arg Gly Arg Lys Lys
        340                 345                 350

Leu Leu Tyr Ile Phe Lys Gln Pro Phe Met Arg Pro Val Gln Thr Thr
        355                 360                 365

```
Gln Glu Glu Asp Gly Cys Ser Cys Arg Phe Pro Glu Glu Glu Glu Gly
    370             375             380

Gly Cys Glu Leu Arg Val Lys Phe Ser Arg Ser Ala Asp Ala Pro Ala
385             390             395             400

Tyr Gln Gln Gly Gln Asn Gln Leu Tyr Asn Glu Leu Asn Leu Gly Arg
            405             410             415

Arg Glu Glu Tyr Asp Val Leu Asp Lys Arg Arg Gly Arg Asp Pro Glu
        420             425             430

Met Gly Gly Lys Pro Arg Arg Lys Asn Pro Gln Glu Gly Leu Tyr Asn
        435             440             445

Glu Leu Gln Lys Asp Lys Met Ala Glu Ala Tyr Ser Glu Ile Gly Met
    450             455             460

Lys Gly Glu Arg Arg Arg Gly Lys Gly His Asp Gly Leu Tyr Gln Gly
465             470             475             480

Leu Ser Thr Ala Thr Lys Asp Thr Tyr Asp Ala Leu His Met Gln Ala
            485             490             495

Leu Pro Pro Arg Glu
            500
```

<210> 7
<211> 508
<212> PRT
<213> artificial sequence

<220>
<223> Anti-CD123 No7

<400> 7

```
Met Ala Leu Pro Val Thr Ala Leu Leu Leu Pro Leu Ala Leu Leu Leu
1               5               10              15

His Ala Ala Arg Pro Gly Gly Gly Gly Ser Cys Pro Tyr Ser Asn Pro
            20              25              30

Ser Leu Cys Gly Gln Ile Gln Leu Val Gln Ser Gly Pro Glu Leu Lys
            35              40              45

Lys Pro Gly Glu Thr Val Lys Ile Ser Cys Lys Ala Ser Gly Tyr Ile
        50              55              60
```

```
Phe Thr Asn Tyr Gly Met Asn Trp Val Lys Gln Ala Pro Gly Lys Ser
65              70              75              80

Phe Lys Trp Met Gly Trp Ile Asn Thr Tyr Thr Gly Glu Ser Thr Tyr
                85              90              95

Ser Ala Asp Phe Lys Gly Arg Phe Ala Phe Ser Leu Glu Thr Ser Ala
            100             105             110

Ser Thr Ala Tyr Leu His Ile Asn Asp Leu Lys Asn Glu Asp Thr Ala
            115             120             125

Thr Tyr Phe Cys Ala Arg Ser Gly Gly Tyr Asp Pro Met Asp Tyr Trp
        130             135             140

Gly Gln Gly Thr Ser Val Thr Val Ser Ser Gly Gly Gly Gly Ser Gly
145             150             155             160

Gly Gly Gly Ser Gly Gly Gly Gly Ser Asp Ile Val Leu Thr Gln Ser
            165             170             175

Pro Ala Ser Leu Ala Val Ser Leu Gly Gln Arg Ala Thr Ile Ser Cys
            180             185             190

Arg Ala Ser Glu Ser Val Asp Asn Tyr Gly Asn Thr Phe Met His Trp
        195             200             205

Tyr Gln Gln Lys Pro Gly Gln Pro Pro Lys Leu Leu Ile Tyr Arg Ala
    210             215             220

Ser Asn Leu Glu Ser Gly Ile Pro Ala Arg Phe Ser Gly Ser Gly Ser
225             230             235             240

Arg Thr Asp Phe Thr Leu Thr Ile Asn Pro Val Glu Ala Asp Asp Val
            245             250             255

Ala Thr Tyr Tyr Cys Gln Gln Ser Asn Glu Asp Pro Pro Thr Phe Gly
        260             265             270

Ala Gly Thr Lys Leu Glu Leu Lys Arg Ser Asp Pro Thr Thr Thr Pro
        275             280             285

Ala Pro Arg Pro Pro Thr Pro Ala Pro Thr Ile Ala Ser Gln Pro Leu
    290             295             300

Ser Leu Arg Pro Glu Ala Cys Arg Pro Ala Ala Gly Gly Ala Val His
305             310             315             320
```

```
        Thr Arg Gly Leu Asp Phe Ala Cys Asp Ile Tyr Ile Trp Ala Pro Leu
                    325             330             335

        Ala Gly Thr Cys Gly Val Leu Leu Leu Ser Leu Val Ile Thr Leu Tyr
                    340             345             350

        Cys Arg Arg Gly Arg Lys Lys Leu Leu Tyr Ile Phe Lys Gln Pro Phe
                    355             360             365

        Met Arg Pro Val Gln Thr Thr Gln Glu Glu Asp Gly Cys Ser Cys Arg
                    370             375             380

        Phe Pro Glu Glu Glu Glu Gly Gly Cys Glu Leu Arg Val Lys Phe Ser
        385             390             395             400

        Arg Ser Ala Asp Ala Pro Ala Tyr Gln Gln Gly Gln Asn Gln Leu Tyr
                    405             410             415

        Asn Glu Leu Asn Leu Gly Arg Arg Glu Glu Tyr Asp Val Leu Asp Lys
                    420             425             430

        Arg Arg Gly Arg Asp Pro Glu Met Gly Gly Lys Pro Arg Arg Lys Asn
                    435             440             445

        Pro Gln Glu Gly Leu Tyr Asn Glu Leu Gln Lys Asp Lys Met Ala Glu
            450             455             460

        Ala Tyr Ser Glu Ile Gly Met Lys Gly Glu Arg Arg Arg Gly Lys Gly
        465             470             475             480

        His Asp Gly Leu Tyr Gln Gly Leu Ser Thr Ala Thr Lys Asp Thr Tyr
                    485             490             495

        Asp Ala Leu His Met Gln Ala Leu Pro Pro Arg Glu
                    500             505
```

<210> 8
<211> 514
<212> PRT
<213> artificial sequence

<220>
<223> Anti-CD123 No8

<400> 8

```
        Met Ala Leu Pro Val Thr Ala Leu Leu Leu Pro Leu Ala Leu Leu Leu
        1               5               10              15
```

56

His Ala Ala Arg Pro Gly Gly Gly Gly Ser Cys Pro Tyr Ser Asn Pro
                20                      25              30

Ser Leu Cys Ser Gly Gly Gly Gly Ser Gly Gln Ile Gln Leu Val Gln
            35                  40                  45

Ser Gly Pro Glu Leu Lys Lys Pro Gly Glu Thr Val Lys Ile Ser Cys
        50                  55                  60

Lys Ala Ser Gly Tyr Ile Phe Thr Asn Tyr Gly Met Asn Trp Val Lys
65                  70                  75                  80

Gln Ala Pro Gly Lys Ser Phe Lys Trp Met Gly Trp Ile Asn Thr Tyr
                85                  90                  95

Thr Gly Glu Ser Thr Tyr Ser Ala Asp Phe Lys Gly Arg Phe Ala Phe
            100                 105                 110

Ser Leu Glu Thr Ser Ala Ser Thr Ala Tyr Leu His Ile Asn Asp Leu
            115                 120                 125

Lys Asn Glu Asp Thr Ala Thr Tyr Phe Cys Ala Arg Ser Gly Gly Tyr
            130                 135                 140

Asp Pro Met Asp Tyr Trp Gly Gln Gly Thr Ser Val Thr Val Ser Ser
145                 150                 155                 160

Gly Gly Gly Gly Ser Gly Gly Gly Gly Ser Gly Gly Gly Gly Ser Asp
                165                 170                 175

Ile Val Leu Thr Gln Ser Pro Ala Ser Leu Ala Val Ser Leu Gly Gln
            180                 185                 190

Arg Ala Thr Ile Ser Cys Arg Ala Ser Glu Ser Val Asp Asn Tyr Gly
            195                 200                 205

Asn Thr Phe Met His Trp Tyr Gln Gln Lys Pro Gly Gln Pro Pro Lys
210                 215                 220

Leu Leu Ile Tyr Arg Ala Ser Asn Leu Glu Ser Gly Ile Pro Ala Arg
225                 230                 235                 240

Phe Ser Gly Ser Gly Ser Arg Thr Asp Phe Thr Leu Thr Ile Asn Pro
                245                 250                 255

Val Glu Ala Asp Asp Val Ala Thr Tyr Tyr Cys Gln Gln Ser Asn Glu
            260                 265                 270

Asp Pro Pro Thr Phe Gly Ala Gly Thr Lys Leu Glu Leu Lys Arg Ser
275 280 285

Asp Pro Thr Thr Thr Pro Ala Pro Arg Pro Pro Thr Pro Ala Pro Thr
290 295 300

Ile Ala Ser Gln Pro Leu Ser Leu Arg Pro Glu Ala Cys Arg Pro Ala
305 310 315 320

Ala Gly Gly Ala Val His Thr Arg Gly Leu Asp Phe Ala Cys Asp Ile
325 330 335

Tyr Ile Trp Ala Pro Leu Ala Gly Thr Cys Gly Val Leu Leu Leu Ser
340 345 350

Leu Val Ile Thr Leu Tyr Cys Arg Arg Gly Arg Lys Lys Leu Leu Tyr
355 360 365

Ile Phe Lys Gln Pro Phe Met Arg Pro Val Gln Thr Thr Gln Glu Glu
370 375 380

Asp Gly Cys Ser Cys Arg Phe Pro Glu Glu Glu Glu Gly Gly Cys Glu
385 390 395 400

Leu Arg Val Lys Phe Ser Arg Ser Ala Asp Ala Pro Ala Tyr Gln Gln
405 410 415

Gly Gln Asn Gln Leu Tyr Asn Glu Leu Asn Leu Gly Arg Arg Glu Glu
420 425 430

Tyr Asp Val Leu Asp Lys Arg Arg Gly Arg Asp Pro Glu Met Gly Gly
435 440 445

Lys Pro Arg Arg Lys Asn Pro Gln Glu Gly Leu Tyr Asn Glu Leu Gln
450 455 460

Lys Asp Lys Met Ala Glu Ala Tyr Ser Glu Ile Gly Met Lys Gly Glu
465 470 475 480

Arg Arg Arg Gly Lys Gly His Asp Gly Leu Tyr Gln Gly Leu Ser Thr
485 490 495

Ala Thr Lys Asp Thr Tyr Asp Ala Leu His Met Gln Ala Leu Pro Pro
500 505 510

Arg Glu

<210> 9
<211> 523
<212> PRT
<213> artificial sequence

<220>
<223> Anti-CD123 No9

<400> 9

```
Met Ala Leu Pro Val Thr Ala Leu Leu Leu Pro Leu Ala Leu Leu Leu
1               5                   10                  15

His Ala Ala Arg Pro Gly Gly Gly Gly Ser Cys Pro Tyr Ser Asn Pro
                20              25                  30

Ser Leu Cys Ser Gly Gly Gly Gly Ser Cys Pro Tyr Ser Asn Pro Ser
            35              40                  45

Leu Cys Gly Gln Ile Gln Leu Val Gln Ser Gly Pro Glu Leu Lys Lys
        50                  55                  60

Pro Gly Glu Thr Val Lys Ile Ser Cys Lys Ala Ser Gly Tyr Ile Phe
65                  70                  75                  80

Thr Asn Tyr Gly Met Asn Trp Val Lys Gln Ala Pro Gly Lys Ser Phe
                85                  90                  95

Lys Trp Met Gly Trp Ile Asn Thr Tyr Thr Gly Glu Ser Thr Tyr Ser
            100                 105                 110

Ala Asp Phe Lys Gly Arg Phe Ala Phe Ser Leu Glu Thr Ser Ala Ser
            115                 120                 125

Thr Ala Tyr Leu His Ile Asn Asp Leu Lys Asn Glu Asp Thr Ala Thr
    130                 135                 140

Tyr Phe Cys Ala Arg Ser Gly Gly Tyr Asp Pro Met Asp Tyr Trp Gly
145                 150                 155                 160

Gln Gly Thr Ser Val Thr Val Ser Ser Gly Gly Gly Gly Ser Gly Gly
                165                 170                 175

Gly Gly Ser Gly Gly Gly Gly Ser Asp Ile Val Leu Thr Gln Ser Pro
            180                 185                 190

Ala Ser Leu Ala Val Ser Leu Gly Gln Arg Ala Thr Ile Ser Cys Arg
            195                 200                 205
```

```
Ala Ser Glu Ser Val Asp Asn Tyr Gly Asn Thr Phe Met His Trp Tyr
    210                 215                 220

Gln Gln Lys Pro Gly Gln Pro Pro Lys Leu Leu Ile Tyr Arg Ala Ser
225                 230                 235                 240

Asn Leu Glu Ser Gly Ile Pro Ala Arg Phe Ser Gly Ser Gly Ser Arg
                245                 250                 255

Thr Asp Phe Thr Leu Thr Ile Asn Pro Val Glu Ala Asp Asp Val Ala
            260                 265                 270

Thr Tyr Tyr Cys Gln Gln Ser Asn Glu Asp Pro Pro Thr Phe Gly Ala
        275                 280                 285

Gly Thr Lys Leu Glu Leu Lys Arg Ser Asp Pro Thr Thr Thr Pro Ala
    290                 295                 300

Pro Arg Pro Pro Thr Pro Ala Pro Thr Ile Ala Ser Gln Pro Leu Ser
305                 310                 315                 320

Leu Arg Pro Glu Ala Cys Arg Pro Ala Ala Gly Gly Ala Val His Thr
                325                 330                 335

Arg Gly Leu Asp Phe Ala Cys Asp Ile Tyr Ile Trp Ala Pro Leu Ala
            340                 345                 350

Gly Thr Cys Gly Val Leu Leu Leu Ser Leu Val Ile Thr Leu Tyr Cys
        355                 360                 365

Arg Arg Gly Arg Lys Lys Leu Leu Tyr Ile Phe Lys Gln Pro Phe Met
    370                 375                 380

Arg Pro Val Gln Thr Thr Gln Glu Glu Asp Gly Cys Ser Cys Arg Phe
385                 390                 395                 400

Pro Glu Glu Glu Glu Gly Gly Cys Glu Leu Arg Val Lys Phe Ser Arg
                405                 410                 415

Ser Ala Asp Ala Pro Ala Tyr Gln Gln Gly Gln Asn Gln Leu Tyr Asn
            420                 425                 430

Glu Leu Asn Leu Gly Arg Arg Glu Glu Tyr Asp Val Leu Asp Lys Arg
        435                 440                 445

Arg Gly Arg Asp Pro Glu Met Gly Gly Lys Pro Arg Arg Lys Asn Pro
    450                 455                 460
```

60

```
Gln Glu Gly Leu Tyr Asn Glu Leu Gln Lys Asp Lys Met Ala Glu Ala
465             470             475             480

Tyr Ser Glu Ile Gly Met Lys Gly Glu Arg Arg Arg Gly Lys Gly His
                485             490             495

Asp Gly Leu Tyr Gln Gly Leu Ser Thr Ala Thr Lys Asp Thr Tyr Asp
            500             505             510

Ala Leu His Met Gln Ala Leu Pro Pro Arg Glu
            515             520
```

<210> 10
<211> 529
<212> PRT
<213> artificial sequence

<220>
<223> Anti-CD123 No10

<400> 10

```
Met Ala Leu Pro Val Thr Ala Leu Leu Leu Pro Leu Ala Leu Leu Leu
1               5               10                      15

His Ala Ala Arg Pro Gly Gly Gly Gly Ser Cys Pro Tyr Ser Asn Pro
            20              25              30

Ser Leu Cys Ser Gly Gly Gly Gly Ser Cys Pro Tyr Ser Asn Pro Ser
            35              40              45

Leu Cys Ser Gly Gly Gly Gly Ser Gly Gln Ile Gln Leu Val Gln Ser
        50              55              60

Gly Pro Glu Leu Lys Lys Pro Gly Glu Thr Val Lys Ile Ser Cys Lys
65              70              75              80

Ala Ser Gly Tyr Ile Phe Thr Asn Tyr Gly Met Asn Trp Val Lys Gln
                85              90              95

Ala Pro Gly Lys Ser Phe Lys Trp Met Gly Trp Ile Asn Thr Tyr Thr
            100             105             110

Gly Glu Ser Thr Tyr Ser Ala Asp Phe Lys Gly Arg Phe Ala Phe Ser
            115             120             125

Leu Glu Thr Ser Ala Ser Thr Ala Tyr Leu His Ile Asn Asp Leu Lys
        130             135             140
```

61

```
Asn Glu Asp Thr Ala Thr Tyr Phe Cys Ala Arg Ser Gly Gly Tyr Asp
145             150         155             160

Pro Met Asp Tyr Trp Gly Gln Gly Thr Ser Val Thr Val Ser Ser Gly
            165         170             175

Gly Gly Gly Ser Gly Gly Gly Gly Ser Gly Gly Gly Gly Ser Asp Ile
            180             185             190

Val Leu Thr Gln Ser Pro Ala Ser Leu Ala Val Ser Leu Gly Gln Arg
        195             200             205

Ala Thr Ile Ser Cys Arg Ala Ser Glu Ser Val Asp Asn Tyr Gly Asn
    210             215             220

Thr Phe Met His Trp Tyr Gln Gln Lys Pro Gly Gln Pro Pro Lys Leu
225             230             235             240

Leu Ile Tyr Arg Ala Ser Asn Leu Glu Ser Gly Ile Pro Ala Arg Phe
            245             250             255

Ser Gly Ser Gly Ser Arg Thr Asp Phe Thr Leu Thr Ile Asn Pro Val
            260             265             270

Glu Ala Asp Asp Val Ala Thr Tyr Tyr Cys Gln Gln Ser Asn Glu Asp
        275             280             285

Pro Pro Thr Phe Gly Ala Gly Thr Lys Leu Glu Leu Lys Arg Ser Asp
        290             295             300

Pro Thr Thr Thr Pro Ala Pro Arg Pro Pro Thr Pro Ala Pro Thr Ile
305             310             315             320

Ala Ser Gln Pro Leu Ser Leu Arg Pro Glu Ala Cys Arg Pro Ala Ala
            325             330             335

Gly Gly Ala Val His Thr Arg Gly Leu Asp Phe Ala Cys Asp Ile Tyr
            340             345             350

Ile Trp Ala Pro Leu Ala Gly Thr Cys Gly Val Leu Leu Leu Ser Leu
        355             360             365

Val Ile Thr Leu Tyr Cys Arg Arg Gly Arg Lys Lys Leu Leu Tyr Ile
    370             375             380

Phe Lys Gln Pro Phe Met Arg Pro Val Gln Thr Thr Gln Glu Glu Asp
385             390             395             400
```

```
Gly Cys Ser Cys Arg Phe Pro Glu Glu Glu Glu Gly Gly Cys Glu Leu
            405             410             415

Arg Val Lys Phe Ser Arg Ser Ala Asp Ala Pro Ala Tyr Gln Gln Gly
            420             425             430

Gln Asn Gln Leu Tyr Asn Glu Leu Asn Leu Gly Arg Arg Glu Glu Tyr
            435             440             445

Asp Val Leu Asp Lys Arg Arg Gly Arg Asp Pro Glu Met Gly Gly Lys
            450             455             460

Pro Arg Arg Lys Asn Pro Gln Glu Gly Leu Tyr Asn Glu Leu Gln Lys
465             470             475             480

Asp Lys Met Ala Glu Ala Tyr Ser Glu Ile Gly Met Lys Gly Glu Arg
            485             490             495

Arg Arg Gly Lys Gly His Asp Gly Leu Tyr Gln Gly Leu Ser Thr Ala
            500             505             510

Thr Lys Asp Thr Tyr Asp Ala Leu His Met Gln Ala Leu Pro Pro Arg
            515             520             525

Glu
```

<210> 11
<211> 297
<212> PRT
<213> homo sapiens

<400> 11

```
Met Thr Thr Pro Arg Asn Ser Val Asn Gly Thr Phe Pro Ala Glu Pro
1               5               10              15

Met Lys Gly Pro Ile Ala Met Gln Ser Gly Pro Lys Pro Leu Phe Arg
            20              25              30

Arg Met Ser Ser Leu Val Gly Pro Thr Gln Ser Phe Phe Met Arg Glu
            35              40              45

Ser Lys Thr Leu Gly Ala Val Gln Ile Met Asn Gly Leu Phe His Ile
            50              55              60

Ala Leu Gly Gly Leu Leu Met Ile Pro Ala Gly Ile Tyr Ala Pro Ile
65              70              75              80
```

```
        Cys Val Thr Val Trp Tyr Pro Leu Trp Gly Gly Ile Met Tyr Ile Ile
                        85                  90                  95

        Ser Gly Ser Leu Leu Ala Ala Thr Glu Lys Asn Ser Arg Lys Cys Leu
                        100                 105                 110

        Val Lys Gly Lys Met Ile Met Asn Ser Leu Ser Leu Phe Ala Ala Ile
                        115                 120                 125

        Ser Gly Met Ile Leu Ser Ile Met Asp Ile Leu Asn Ile Lys Ile Ser
                        130                 135                 140

        His Phe Leu Lys Met Glu Ser Leu Asn Phe Ile Arg Ala His Thr Pro
        145                 150                 155                 160

        Tyr Ile Asn Ile Tyr Asn Cys Glu Pro Ala Asn Pro Ser Glu Lys Asn
                        165                 170                 175

        Ser Pro Ser Thr Gln Tyr Cys Tyr Ser Ile Gln Ser Leu Phe Leu Gly
                        180                 185                 190

        Ile Leu Ser Val Met Leu Ile Phe Ala Phe Phe Gln Glu Leu Val Ile
                        195                 200                 205

        Ala Gly Ile Val Glu Asn Glu Trp Lys Arg Thr Cys Ser Arg Pro Lys
                        210                 215                 220

        Ser Asn Ile Val Leu Leu Ser Ala Glu Glu Lys Lys Glu Gln Thr Ile
        225                 230                 235                 240

        Glu Ile Lys Glu Glu Val Val Gly Leu Thr Glu Thr Ser Ser Gln Pro
                        245                 250                 255

        Lys Asn Glu Glu Asp Ile Glu Ile Ile Pro Ile Gln Glu Glu Glu Glu
                        260                 265                 270

        Glu Glu Thr Glu Thr Asn Phe Pro Glu Pro Pro Gln Asp Gln Glu Ser
                        275                 280                 285

        Ser Pro Ile Glu Asn Asp Ser Ser Pro
                        290                 295
```

<210> 12
<211> 207
<212> PRT
<213> homo sapiens


<400> 12

```
Met Gln Ser Gly Thr His Trp Arg Val Leu Gly Leu Cys Leu Leu Ser
1               5                   10                  15

Val Gly Val Trp Gly Gln Asp Gly Asn Glu Glu Met Gly Gly Ile Thr
            20                  25                  30

Gln Thr Pro Tyr Lys Val Ser Ile Ser Gly Thr Thr Val Ile Leu Thr
            35                  40                  45

Cys Pro Gln Tyr Pro Gly Ser Glu Ile Leu Trp Gln His Asn Asp Lys
        50                  55                  60

Asn Ile Gly Gly Asp Glu Asp Asp Lys Asn Ile Gly Ser Asp Glu Asp
65                  70                  75                  80

His Leu Ser Leu Lys Glu Phe Ser Glu Leu Glu Gln Ser Gly Tyr Tyr
                85                  90                  95

Val Cys Tyr Pro Arg Gly Ser Lys Pro Glu Asp Ala Asn Phe Tyr Leu
            100                 105                 110

Tyr Leu Arg Ala Arg Val Cys Glu Asn Cys Met Glu Met Asp Val Met
            115                 120                 125

Ser Val Ala Thr Ile Val Ile Val Asp Ile Cys Ile Thr Gly Gly Leu
            130                 135                 140

Leu Leu Leu Val Tyr Tyr Trp Ser Lys Asn Arg Lys Ala Lys Ala Lys
145                 150                 155                 160

Pro Val Thr Arg Gly Ala Gly Ala Gly Gly Arg Gln Arg Gly Gln Asn
                165                 170                 175

Lys Glu Arg Pro Pro Pro Val Pro Asn Pro Asp Tyr Glu Pro Ile Arg
            180                 185                 190

Lys Gly Gln Arg Asp Leu Tyr Ser Gly Leu Asn Gln Arg Arg Ile
            195                 200                 205
```

<210> 13
<211> 1039
<212> PRT
<213> homo sapiens

<400> 13

```
Met Ala Arg Ala Leu Cys Pro Leu Gln Ala Leu Trp Leu Leu Glu Trp
1               5                   10                  15
```

```
Val Leu Leu Leu Leu Gly Pro Cys Ala Ala Pro Pro Ala Trp Ala Leu
        20              25          30

Asn Leu Asp Pro Val Gln Leu Thr Phe Tyr Ala Gly Pro Asn Gly Ser
        35              40          45

Gln Phe Gly Phe Ser Leu Asp Phe His Lys Asp Ser His Gly Arg Val
        50              55          60

Ala Ile Val Val Gly Ala Pro Arg Thr Leu Gly Pro Ser Gln Glu Glu
65              70          75              80

Thr Gly Gly Val Phe Leu Cys Pro Trp Arg Ala Glu Gly Gly Gln Cys
            85          90              95

Pro Ser Leu Leu Phe Asp Leu Arg Asp Glu Thr Arg Asn Val Gly Ser
        100             105             110

Gln Thr Leu Gln Thr Phe Lys Ala Arg Gln Gly Leu Gly Ala Ser Val
        115             120             125

Val Ser Trp Ser Asp Val Ile Val Ala Cys Ala Pro Trp Gln His Trp
    130             135             140

Asn Val Leu Glu Lys Thr Glu Glu Ala Glu Lys Thr Pro Val Gly Ser
145             150             155             160

Cys Phe Leu Ala Gln Pro Glu Ser Gly Arg Arg Ala Glu Tyr Ser Pro
            165             170             175

Cys Arg Gly Asn Thr Leu Ser Arg Ile Tyr Val Glu Asn Asp Phe Ser
        180             185             190

Trp Asp Lys Arg Tyr Cys Glu Ala Gly Phe Ser Ser Val Val Thr Gln
        195             200             205

Ala Gly Glu Leu Val Leu Gly Ala Pro Gly Gly Tyr Tyr Phe Leu Gly
    210             215             220

Leu Leu Ala Gln Ala Pro Val Ala Asp Ile Phe Ser Ser Tyr Arg Pro
225             230             235             240

Gly Ile Leu Leu Trp His Val Ser Ser Gln Ser Leu Ser Phe Asp Ser
            245             250             255

Ser Asn Pro Glu Tyr Phe Asp Gly Tyr Trp Gly Tyr Ser Val Ala Val
```

```
                    260                         265                         270


        Gly Glu Phe Asp Gly Asp Leu Asn Thr Thr Glu Tyr Val Val Gly Ala
                275                 280                 285


        Pro Thr Trp Ser Trp Thr Leu Gly Ala Val Glu Ile Leu Asp Ser Tyr
                290                 295                 300


        Tyr Gln Arg Leu His Arg Leu Arg Gly Glu Gln Met Ala Ser Tyr Phe
        305                 310                 315                 320


        Gly His Ser Val Ala Val Thr Asp Val Asn Gly Asp Gly Arg His Asp
                        325                 330                 335


        Leu Leu Val Gly Ala Pro Leu Tyr Met Glu Ser Arg Ala Asp Arg Lys
                        340                 345                 350


        Leu Ala Glu Val Gly Arg Val Tyr Leu Phe Leu Gln Pro Arg Gly Pro
                355                 360                 365


        His Ala Leu Gly Ala Pro Ser Leu Leu Leu Thr Gly Thr Gln Leu Tyr
                370                 375                 380


        Gly Arg Phe Gly Ser Ala Ile Ala Pro Leu Gly Asp Leu Asp Arg Asp
        385                 390                 395                 400


        Gly Tyr Asn Asp Ile Ala Val Ala Ala Pro Tyr Gly Gly Pro Ser Gly
                        405                 410                 415


        Arg Gly Gln Val Leu Val Phe Leu Gly Gln Ser Glu Gly Leu Arg Ser
                        420                 425                 430


        Arg Pro Ser Gln Val Leu Asp Ser Pro Phe Pro Thr Gly Ser Ala Phe
                435                 440                 445


        Gly Phe Ser Leu Arg Gly Ala Val Asp Ile Asp Asp Asn Gly Tyr Pro
                450                 455                 460


        Asp Leu Ile Val Gly Ala Tyr Gly Ala Asn Gln Val Ala Val Tyr Arg
        465                 470                 475                 480


        Ala Gln Pro Val Val Lys Ala Ser Val Gln Leu Leu Val Gln Asp Ser
                        485                 490                 495


        Leu Asn Pro Ala Val Lys Ser Cys Val Leu Pro Gln Thr Lys Thr Pro
                500                 505                 510
```

Val Ser Cys Phe Asn Ile Gln Met Cys Val Gly Ala Thr Gly His Asn
        515                 520                 525

Ile Pro Gln Lys Leu Ser Leu Asn Ala Glu Leu Gln Leu Asp Arg Gln
        530                 535                 540

Lys Pro Arg Gln Gly Arg Arg Val Leu Leu Leu Gly Ser Gln Gln Ala
545                 550                 555                 560

Gly Thr Thr Leu Asn Leu Asp Leu Gly Gly Lys His Ser Pro Ile Cys
                565                 570                 575

His Thr Thr Met Ala Phe Leu Arg Asp Glu Ala Asp Phe Arg Asp Lys
                580                 585                 590

Leu Ser Pro Ile Val Leu Ser Leu Asn Val Ser Leu Pro Pro Thr Glu
                595                 600                 605

Ala Gly Met Ala Pro Ala Val Val Leu His Gly Asp Thr His Val Gln
        610                 615                 620

Glu Gln Thr Arg Ile Val Leu Asp Cys Gly Glu Asp Asp Val Cys Val
625                 630                 635                 640

Pro Gln Leu Gln Leu Thr Ala Ser Val Thr Gly Ser Pro Leu Leu Val
                645                 650                 655

Gly Ala Asp Asn Val Leu Glu Leu Gln Met Asp Ala Ala Asn Glu Gly
                660                 665                 670

Glu Gly Ala Tyr Glu Ala Glu Leu Ala Val His Leu Pro Gln Gly Ala
        675                 680                 685

His Tyr Met Arg Ala Leu Ser Asn Val Glu Gly Phe Glu Arg Leu Ile
        690                 695                 700

Cys Asn Gln Lys Lys Glu Asn Glu Thr Arg Val Val Leu Cys Glu Leu
705                 710                 715                 720

Gly Asn Pro Met Lys Lys Asn Ala Gln Ile Gly Ile Ala Met Leu Val
                725                 730                 735

Ser Val Gly Asn Leu Glu Glu Ala Gly Glu Ser Val Ser Phe Gln Leu
                740                 745                 750

Gln Ile Arg Ser Lys Asn Ser Gln Asn Pro Asn Ser Lys Ile Val Leu
                755                 760                 765

```
Leu Asp Val Pro Val Arg Ala Glu Ala Gln Val Glu Leu Arg Gly Asn
    770             775             780

Ser Phe Pro Ala Ser Leu Val Val Ala Ala Glu Glu Gly Glu Arg Glu
785             790             795             800

Gln Asn Ser Leu Asp Ser Trp Gly Pro Lys Val Glu His Thr Tyr Glu
            805             810             815

Leu His Asn Asn Gly Pro Gly Thr Val Asn Gly Leu His Leu Ser Ile
            820             825             830

His Leu Pro Gly Gln Ser Gln Pro Ser Asp Leu Leu Tyr Ile Leu Asp
        835             840             845

Ile Gln Pro Gln Gly Gly Leu Gln Cys Phe Pro Gln Pro Pro Val Asn
    850             855             860

Pro Leu Lys Val Asp Trp Gly Leu Pro Ile Pro Ser Pro Ser Pro Ile
865             870             875             880

His Pro Ala His His Lys Arg Asp Arg Arg Gln Ile Phe Leu Pro Glu
            885             890             895

Pro Glu Gln Pro Ser Arg Leu Gln Asp Pro Val Leu Val Ser Cys Asp
            900             905             910

Ser Ala Pro Cys Thr Val Val Gln Cys Asp Leu Gln Glu Met Ala Arg
        915             920             925

Gly Gln Arg Ala Met Val Thr Val Leu Ala Phe Leu Trp Leu Pro Ser
    930             935             940

Leu Tyr Gln Arg Pro Leu Asp Gln Phe Val Leu Gln Ser His Ala Trp
945             950             955             960

Phe Asn Val Ser Ser Leu Pro Tyr Ala Val Pro Pro Leu Ser Leu Pro
            965             970             975

Arg Gly Glu Ala Gln Val Trp Thr Gln Leu Leu Arg Ala Leu Glu Glu
            980             985             990

Arg Ala Ile Pro Ile Trp Trp Val  Leu Val Gly Val Leu  Gly Gly Leu
        995             1000            1005

Leu Leu  Leu Thr Ile Leu Val  Leu Ala Met Trp Lys  Val Gly Phe
    1010            1015            1020
```

```
Phe Lys  Arg Asn Arg Pro Pro  Leu Glu Glu Asp Asp  Glu Glu Gly
    1025                1030                1035
```

Glu

```
<210> 14
<211> 272
<212> PRT
<213> homo sapiens

<400> 14
```

```
Met Asp Ser Tyr Leu Leu Met Trp Gly Leu Leu Thr Phe Ile Met Val
1               5               10              15

Pro Gly Cys Gln Ala Glu Leu Cys Asp Asp Asp Pro Pro Glu Ile Pro
            20              25              30

His Ala Thr Phe Lys Ala Met Ala Tyr Lys Glu Gly Thr Met Leu Asn
            35              40              45

Cys Glu Cys Lys Arg Gly Phe Arg Arg Ile Lys Ser Gly Ser Leu Tyr
    50              55              60

Met Leu Cys Thr Gly Asn Ser Ser His Ser Ser Trp Asp Asn Gln Cys
65              70              75              80

Gln Cys Thr Ser Ser Ala Thr Arg Asn Thr Thr Lys Gln Val Thr Pro
            85              90              95

Gln Pro Glu Glu Gln Lys Glu Arg Lys Thr Thr Glu Met Gln Ser Pro
            100             105             110

Met Gln Pro Val Asp Gln Ala Ser Leu Pro Gly His Cys Arg Glu Pro
        115             120             125

Pro Pro Trp Glu Asn Glu Ala Thr Glu Arg Ile Tyr His Phe Val Val
    130             135             140

Gly Gln Met Val Tyr Tyr Gln Cys Val Gln Gly Tyr Arg Ala Leu His
145             150             155             160

Arg Gly Pro Ala Glu Ser Val Cys Lys Met Thr His Gly Lys Thr Arg
            165             170             175

Trp Thr Gln Pro Gln Leu Ile Cys Thr Gly Glu Met Glu Thr Ser Gln
            180             185             190
```

```
Phe Pro Gly Glu Glu Lys Pro Gln Ala Ser Pro Glu Gly Arg Pro Glu
        195             200             205

Ser Glu Thr Ser Cys Leu Val Thr Thr Thr Asp Phe Gln Ile Gln Thr
        210             215             220

Glu Met Ala Ala Thr Met Glu Thr Ser Ile Phe Thr Thr Glu Tyr Gln
225             230             235             240

Val Ala Val Ala Gly Cys Val Phe Leu Leu Ile Ser Val Leu Leu Leu
            245             250             255

Ser Gly Leu Thr Trp Gln Arg Arg Gln Arg Lys Ser Arg Arg Thr Ile
        260             265             270
```

<210> 15
<211> 595
<212> PRT
<213> homo sapiens

<400> 15

```
Met Arg Val Leu Leu Ala Ala Leu Gly Leu Leu Phe Leu Gly Ala Leu
1               5               10              15

Arg Ala Phe Pro Gln Asp Arg Pro Phe Glu Asp Thr Cys His Gly Asn
        20              25              30

Pro Ser His Tyr Tyr Asp Lys Ala Val Arg Arg Cys Cys Tyr Arg Cys
        35              40              45

Pro Met Gly Leu Phe Pro Thr Gln Gln Cys Pro Gln Arg Pro Thr Asp
        50              55              60

Cys Arg Lys Gln Cys Glu Pro Asp Tyr Tyr Leu Asp Glu Ala Asp Arg
65              70              75              80

Cys Thr Ala Cys Val Thr Cys Ser Arg Asp Asp Leu Val Glu Lys Thr
            85              90              95

Pro Cys Ala Trp Asn Ser Ser Arg Val Cys Glu Cys Arg Pro Gly Met
        100             105             110

Phe Cys Ser Thr Ser Ala Val Asn Ser Cys Ala Arg Cys Phe Phe His
        115             120             125

Ser Val Cys Pro Ala Gly Met Ile Val Lys Phe Pro Gly Thr Ala Gln
        130             135             140
```

```
Lys Asn Thr Val Cys Glu Pro Ala Ser Pro Gly Val Ser Pro Ala Cys
145                 150                 155                 160


Ala Ser Pro Glu Asn Cys Lys Glu Pro Ser Ser Gly Thr Ile Pro Gln
                165                 170                 175


Ala Lys Pro Thr Pro Val Ser Pro Ala Thr Ser Ser Ala Ser Thr Met
            180                 185                 190


Pro Val Arg Gly Gly Thr Arg Leu Ala Gln Glu Ala Ala Ser Lys Leu
            195                 200                 205


Thr Arg Ala Pro Asp Ser Pro Ser Ser Val Gly Arg Pro Ser Ser Asp
        210                 215                 220


Pro Gly Leu Ser Pro Thr Gln Pro Cys Pro Glu Gly Ser Gly Asp Cys
225                 230                 235                 240


Arg Lys Gln Cys Glu Pro Asp Tyr Tyr Leu Asp Glu Ala Gly Arg Cys
                245                 250                 255


Thr Ala Cys Val Ser Cys Ser Arg Asp Asp Leu Val Glu Lys Thr Pro
            260                 265                 270


Cys Ala Trp Asn Ser Ser Arg Thr Cys Glu Cys Arg Pro Gly Met Ile
        275                 280                 285


Cys Ala Thr Ser Ala Thr Asn Ser Cys Ala Arg Cys Val Pro Tyr Pro
    290                 295                 300


Ile Cys Ala Ala Glu Thr Val Thr Lys Pro Gln Asp Met Ala Glu Lys
305                 310                 315                 320


Asp Thr Thr Phe Glu Ala Pro Pro Leu Gly Thr Gln Pro Asp Cys Asn
                325                 330                 335


Pro Thr Pro Glu Asn Gly Glu Ala Pro Ala Ser Thr Ser Pro Thr Gln
            340                 345                 350


Ser Leu Leu Val Asp Ser Gln Ala Ser Lys Thr Leu Pro Ile Pro Thr
        355                 360                 365


Ser Ala Pro Val Ala Leu Ser Ser Thr Gly Lys Pro Val Leu Asp Ala
    370                 375                 380


Gly Pro Val Leu Phe Trp Val Ile Leu Val Leu Val Val Val Val Gly
```

72

385 390 395 400

Ser Ser Ala Phe Leu Leu Cys His Arg Arg Ala Cys Arg Lys Arg Ile
                    405                 410                 415

Arg Gln Lys Leu His Leu Cys Tyr Pro Val Gln Thr Ser Gln Pro Lys
            420                 425                 430

Leu Glu Leu Val Asp Ser Arg Pro Arg Arg Ser Ser Thr Gln Leu Arg
            435                 440                 445

Ser Gly Ala Ser Val Thr Glu Pro Val Ala Glu Glu Arg Gly Leu Met
        450                 455                 460

Ser Gln Pro Leu Met Glu Thr Cys His Ser Val Gly Ala Ala Tyr Leu
465                 470                 475                 480

Glu Ser Leu Pro Leu Gln Asp Ala Ser Pro Ala Gly Gly Pro Ser Ser
                485                 490                 495

Pro Arg Asp Leu Pro Glu Pro Arg Val Ser Thr Glu His Thr Asn Asn
            500                 505                 510

Lys Ile Glu Lys Ile Tyr Ile Met Lys Ala Asp Thr Val Ile Val Gly
            515                 520                 525

Thr Val Lys Ala Glu Leu Pro Glu Gly Arg Gly Leu Ala Gly Pro Ala
        530                 535                 540

Glu Pro Glu Leu Glu Glu Glu Leu Glu Ala Asp His Thr Pro His Tyr
545                 550                 555                 560

Pro Glu Gln Glu Thr Glu Pro Pro Leu Gly Ser Cys Ser Asp Val Met
                565                 570                 575

Leu Ser Val Glu Glu Glu Gly Lys Glu Asp Pro Leu Pro Thr Ala Ala
            580                 585                 590

Ser Gly Lys
        595

<210> 16
<211> 1091
<212> PRT
<213> homo sapiens

<400> 16

73

Met Arg Pro Ser Gly Thr Ala Gly Ala Ala Leu Leu Ala Leu Leu Ala

```
        1                5                      10                     15

        Ala Leu Cys Pro Ala Ser Arg Ala Leu Glu Glu Lys Lys Val Cys Gln
                    20              25                  30

        Gly Thr Ser Asn Lys Leu Thr Gln Leu Gly Thr Phe Glu Asp His Phe
                    35              40                  45

        Leu Ser Leu Gln Arg Met Phe Asn Asn Cys Glu Val Val Leu Gly Asn
                    50              55                  60

        Leu Glu Ile Thr Tyr Val Gln Arg Asn Tyr Asp Leu Ser Phe Leu Lys
        65                  70              75                  80

        Thr Ile Gln Glu Val Ala Gly Tyr Val Leu Ile Ala Leu Asn Thr Val
                        85              90                  95

        Glu Arg Ile Pro Leu Glu Asn Leu Gln Ile Ile Arg Gly Asn Met Tyr
                    100             105             110

        Tyr Glu Asn Ser Tyr Ala Leu Ala Val Leu Ser Asn Tyr Asp Ala Asn
                    115             120             125

        Lys Thr Gly Leu Lys Glu Leu Pro Met Arg Asn Leu Gln Gly Gln Lys
            130             135                 140

        Cys Asp Pro Ser Cys Pro Asn Gly Ser Cys Trp Gly Ala Gly Glu Glu
        145             150             155                 160

        Asn Cys Gln Lys Leu Thr Lys Ile Ile Cys Ala Gln Gln Cys Ser Gly
                    165             170                 175

        Arg Cys Arg Gly Lys Ser Pro Ser Asp Cys Cys His Asn Gln Cys Ala
                180             185             190

        Ala Gly Cys Thr Gly Pro Arg Glu Ser Asp Cys Leu Val Cys Arg Lys
                195             200             205

        Phe Arg Asp Glu Ala Thr Cys Lys Asp Thr Cys Pro Pro Leu Met Leu
            210             215             220

        Tyr Asn Pro Thr Thr Tyr Gln Met Asp Val Asn Pro Glu Gly Lys Tyr
        225             230             235                 240

        Ser Phe Gly Ala Thr Cys Val Lys Lys Cys Pro Arg Asn Tyr Val Val
                    245             250                 255
```

```
Thr Asp His Gly Ser Cys Val Arg Ala Cys Gly Ala Asp Ser Tyr Glu
        260             265             270

Met Glu Glu Asp Gly Val Arg Lys Cys Lys Lys Cys Glu Gly Pro Cys
        275             280             285

Arg Lys Val Cys Asn Gly Ile Gly Ile Gly Glu Phe Lys Asp Ser Leu
        290             295             300

Ser Ile Asn Ala Thr Asn Ile Lys His Phe Lys Asn Cys Thr Ser Ile
305             310             315             320

Ser Gly Asp Leu His Ile Leu Pro Val Ala Phe Arg Gly Asp Ser Phe
                325             330             335

Thr His Thr Pro Pro Leu Asp Pro Gln Glu Leu Asp Ile Leu Lys Thr
                340             345             350

Val Lys Glu Ile Thr Gly Phe Leu Leu Ile Gln Ala Trp Pro Glu Asn
        355             360             365

Arg Thr Asp Leu His Ala Phe Glu Asn Leu Glu Ile Ile Arg Gly Arg
        370             375             380

Thr Lys Gln His Gly Gln Phe Ser Leu Ala Val Val Ser Leu Asn Ile
385             390             395             400

Thr Ser Leu Gly Leu Arg Ser Leu Lys Glu Ile Ser Asp Gly Asp Val
                405             410             415

Ile Ile Ser Gly Asn Lys Asn Leu Cys Tyr Ala Asn Thr Ile Asn Trp
                420             425             430

Lys Lys Leu Phe Gly Thr Ser Gly Gln Lys Thr Lys Ile Ile Ser Asn
        435             440             445

Arg Gly Glu Asn Ser Cys Lys Ala Thr Gly Gln Val Cys His Ala Leu
        450             455             460

Cys Ser Pro Glu Gly Cys Trp Gly Pro Glu Pro Arg Asp Cys Val Ser
465             470             475             480

Cys Arg Asn Val Ser Arg Gly Arg Glu Cys Val Asp Lys Cys Asn Leu
                485             490             495

Leu Glu Gly Glu Pro Arg Glu Phe Val Glu Asn Ser Glu Cys Ile Gln
        500             505             510
```

76

Cys His Pro Glu Cys Leu Pro Gln Ala Met Asn Ile Thr Cys Thr Gly
        515                 520                 525

Arg Gly Pro Asp Asn Cys Ile Gln Cys Ala His Tyr Ile Asp Gly Pro
        530                 535                 540

His Cys Val Lys Thr Cys Pro Ala Gly Val Met Gly Glu Asn Asn Thr
545                 550                 555                 560

Leu Val Trp Lys Tyr Ala Asp Ala Gly His Val Cys His Leu Cys His
                565                 570                 575

Pro Asn Cys Thr Tyr Gly Cys Thr Gly Pro Gly Leu Glu Gly Cys Pro
                580                 585                 590

Thr Asn Gly Pro Lys Ile Pro Ser Ile Ala Thr Gly Met Val Gly Ala
        595                 600                 605

Leu Leu Leu Leu Leu Val Val Ala Leu Gly Ile Gly Leu Phe Met Arg
        610                 615                 620

Arg Arg His Ile Val Arg Lys Arg Thr Leu Arg Arg Leu Leu Gln Glu
625                 630                 635                 640

Arg Glu Leu Val Glu Pro Leu Thr Pro Ser Gly Glu Ala Pro Asn Gln
                645                 650                 655

Ala Leu Leu Arg Ile Leu Lys Glu Thr Glu Phe Lys Lys Ile Lys Val
                660                 665                 670

Leu Gly Ser Gly Ala Phe Gly Thr Val Tyr Lys Gly Leu Trp Ile Pro
        675                 680                 685

Glu Gly Glu Lys Val Lys Ile Pro Val Ala Ile Lys Glu Leu Arg Glu
        690                 695                 700

Ala Thr Ser Pro Lys Ala Asn Lys Glu Ile Leu Asp Glu Ala Tyr Val
705                 710                 715                 720

Met Ala Ser Val Asp Asn Pro His Val Cys Arg Leu Leu Gly Ile Cys
                725                 730                 735

Leu Thr Ser Thr Val Gln Leu Ile Thr Gln Leu Met Pro Phe Gly Cys
        740                 745                 750

Leu Leu Asp Tyr Val Arg Glu His Lys Asp Asn Ile Gly Ser Gln Tyr
        755                 760                 765

77

```
Leu Leu Asn Trp Cys Val Gln Ile Ala Lys Gly Met Asn Tyr Leu Glu
    770                 775             780

Asp Arg Arg Leu Val His Arg Asp Leu Ala Ala Arg Asn Val Leu Val
785             790             795                         800

Lys Thr Pro Gln His Val Lys Ile Thr Asp Phe Gly Leu Ala Lys Leu
            805             810                     815

Leu Gly Ala Glu Glu Lys Glu Tyr His Ala Glu Gly Gly Lys Val Pro
            820             825             830

Ile Lys Trp Met Ala Leu Glu Ser Ile Leu His Arg Ile Tyr Thr His
        835             840             845

Gln Ser Asp Val Trp Ser Tyr Gly Val Thr Val Trp Glu Leu Met Thr
    850             855             860

Phe Gly Ser Lys Pro Tyr Asp Gly Ile Pro Ala Ser Glu Ile Ser Ser
865             870             875                         880

Ile Leu Glu Lys Gly Glu Arg Leu Pro Gln Pro Pro Ile Cys Thr Ile
            885             890                     895

Asp Val Tyr Met Ile Met Val Lys Cys Trp Met Ile Asp Ala Asp Ser
        900             905             910

Arg Pro Lys Phe Arg Glu Leu Ile Ile Glu Phe Ser Lys Met Ala Arg
    915             920             925

Asp Pro Gln Arg Tyr Leu Val Ile Gln Gly Asp Glu Arg Met His Leu
    930             935             940

Pro Ser Pro Thr Asp Ser Asn Phe Tyr Arg Ala Leu Met Asp Glu Glu
945             950             955                         960

Asp Met Asp Asp Val Val Asp Ala Asp Glu Tyr Leu Ile Pro Gln Gln
            965             970                     975

Gly Phe Phe Ser Ser Pro Ser Thr Ser Arg Thr Pro Leu Leu Ser Ser
            980             985                     990

Leu Ser Ala Thr Ser Asn Asn Ser   Thr Val Ala Cys Ile   Asp Arg Asn
    995                 1000                1005

Gly Leu   Gln Ser Cys Pro Ile   Lys Glu Asp Ser Phe   Leu Gln Arg
```

```
              1010                    1015                    1020


       Tyr Ser   Ser Asp Pro Thr Gly   Ala Leu Thr Glu Asp   Ser Ile Asp
           1025                    1030                    1035


       Asp Thr   Phe Leu Pro Val Pro   Gly Glu Trp Leu Val   Trp Lys Gln
           1040                    1045                    1050


       Ser Cys   Ser Ser Thr Ser Ser   Thr His Ser Ala Ala   Ala Ser Leu
           1055                    1060                    1065


       Gln Cys   Pro Ser Gln Val Leu   Pro Pro Ala Ser Pro   Glu Gly Glu
           1070                    1075                    1080


       Thr Val   Ala Asp Leu Gln Thr   Gln
           1085                    1090
```

<210> 17
<211> 233
<212> PRT
<213> homo sapiens

<400> 17

Met Ser Thr Glu Ser Met Ile Arg Asp Val Glu Leu Ala Glu Glu Ala
1               5               10              15

Leu Pro Lys Lys Thr Gly Gly Pro Gln Gly Ser Arg Arg Cys Leu Phe
        20              25              30

Leu Ser Leu Phe Ser Phe Leu Ile Val Ala Gly Ala Thr Thr Leu Phe
        35              40              45

Cys Leu Leu His Phe Gly Val Ile Gly Pro Gln Arg Glu Glu Phe Pro
    50              55              60

Arg Asp Leu Ser Leu Ile Ser Pro Leu Ala Gln Ala Val Arg Ser Ser
65              70              75              80

Ser Arg Thr Pro Ser Asp Lys Pro Val Ala His Val Val Ala Asn Pro
            85              90              95

Gln Ala Glu Gly Gln Leu Gln Trp Leu Asn Arg Arg Ala Asn Ala Leu
            100             105             110

Leu Ala Asn Gly Val Glu Leu Arg Asp Asn Gln Leu Val Val Pro Ser
        115             120             125

Glu Gly Leu Tyr Leu Ile Tyr Ser Gln Val Leu Phe Lys Gly Gln Gly

        130             135             140

Cys Pro Ser Thr His Val Leu Leu Thr His Thr Ile Ser Arg Ile Ala
145             150             155             160

Val Ser Tyr Gln Thr Lys Val Asn Leu Leu Ser Ala Ile Lys Ser Pro
            165             170             175

Cys Gln Arg Glu Thr Pro Glu Gly Ala Glu Ala Lys Pro Trp Tyr Glu
            180             185             190

Pro Ile Tyr Leu Gly Gly Val Phe Gln Leu Glu Lys Gly Asp Arg Leu
            195             200             205

Ser Ala Glu Ile Asn Arg Pro Asp Tyr Leu Asp Phe Ala Glu Ser Gly
    210             215             220

Gln Val Tyr Phe Gly Ile Ile Ala Leu
225             230

<210> 18
<211> 61
<212> PRT
<213> homo sapiens

<400> 18

```
Met Lys Arg Phe Leu Phe Leu Leu Leu Thr Ile Ser Leu Leu Val Met
1               5                   10                  15

Val Gln Ile Gln Thr Gly Leu Ser Gly Gln Asn Asp Thr Ser Gln Thr
            20                  25                  30

Ser Ser Pro Ser Ala Ser Ser Asn Ile Ser Gly Gly Ile Phe Leu Phe
            35                  40                  45

Phe Val Ala Asn Ala Ile Ile His Leu Phe Cys Phe Ser
        50                  55                  60
```

<210> 19
<211> 232
<212> PRT
<213> homo sapiens

<400> 19

```
Met Asn Phe Leu Leu Ser Trp Val His Trp Ser Leu Ala Leu Leu Leu
1               5                   10                  15

Tyr Leu His His Ala Lys Trp Ser Gln Ala Ala Pro Met Ala Glu Gly
            20                  25                  30
```

```
Gly Gly Gln Asn His His Glu Val Val Lys Phe Met Asp Val Tyr Gln
        35              40                  45

Arg Ser Tyr Cys His Pro Ile Glu Thr Leu Val Asp Ile Phe Gln Glu
        50              55                  60

Tyr Pro Asp Glu Ile Glu Tyr Ile Phe Lys Pro Ser Cys Val Pro Leu
65                      70              75                  80

Met Arg Cys Gly Gly Cys Cys Asn Asp Glu Gly Leu Glu Cys Val Pro
            85              90                  95

Thr Glu Glu Ser Asn Ile Thr Met Gln Ile Met Arg Ile Lys Pro His
            100             105                 110

Gln Gly Gln His Ile Gly Glu Met Ser Phe Leu Gln His Asn Lys Cys
        115             120                 125

Glu Cys Arg Pro Lys Lys Asp Arg Ala Arg Gln Glu Lys Lys Ser Val
    130             135                 140

Arg Gly Lys Gly Lys Gly Gln Lys Arg Lys Arg Lys Lys Ser Arg Tyr
145             150                 155                 160

Lys Ser Trp Ser Val Tyr Val Gly Ala Arg Cys Cys Leu Met Pro Trp
            165             170                 175

Ser Leu Pro Gly Pro His Pro Cys Gly Pro Cys Ser Glu Arg Arg Lys
            180             185                 190

His Leu Phe Val Gln Asp Pro Gln Thr Cys Lys Cys Ser Cys Lys Asn
        195             200                 205

Thr Asp Ser Arg Cys Lys Ala Arg Gln Leu Glu Leu Asn Glu Arg Thr
        210             215                 220

Cys Arg Cys Asp Lys Pro Arg Arg
225             230
```

<210> 20  
<211> 1676  
<212> PRT  
<213> homo sapiens

<400> 20

```
Met Gly Leu Leu Gly Ile Leu Cys Phe Leu Ile Phe Leu Gly Lys Thr
1               5               10                  15
```

Trp Gly Gln Glu Gln Thr Tyr Val Ile Ser Ala Pro Lys Ile Phe Arg
                20                      25                  30

Val Gly Ala Ser Glu Asn Ile Val Ile Gln Val Tyr Gly Tyr Thr Glu
                35                      40                  45

Ala Phe Asp Ala Thr Ile Ser Ile Lys Ser Tyr Pro Asp Lys Lys Phe
                50                      55                  60

Ser Tyr Ser Ser Gly His Val His Leu Ser Ser Glu Asn Lys Phe Gln
65                      70                      75                  80

Asn Ser Ala Ile Leu Thr Ile Gln Pro Lys Gln Leu Pro Gly Gly Gln
                        85                      90                  95

Asn Pro Val Ser Tyr Val Tyr Leu Glu Val Val Ser Lys His Phe Ser
                100                     105                 110

Lys Ser Lys Arg Met Pro Ile Thr Tyr Asp Asn Gly Phe Leu Phe Ile
                115                     120                 125

His Thr Asp Lys Pro Val Tyr Thr Pro Asp Gln Ser Val Lys Val Arg
                130                     135                 140

Val Tyr Ser Leu Asn Asp Asp Leu Lys Pro Ala Lys Arg Glu Thr Val
145                     150                     155                 160

Leu Thr Phe Ile Asp Pro Glu Gly Ser Glu Val Asp Met Val Glu Glu
                165                     170                 175

Ile Asp His Ile Gly Ile Ile Ser Phe Pro Asp Phe Lys Ile Pro Ser
                180                     185                 190

Asn Pro Arg Tyr Gly Met Trp Thr Ile Lys Ala Lys Tyr Lys Glu Asp
                195                     200                 205

Phe Ser Thr Thr Gly Thr Ala Tyr Phe Glu Val Lys Glu Tyr Val Leu
                210                     215                 220

Pro His Phe Ser Val Ser Ile Glu Pro Glu Tyr Asn Phe Ile Gly Tyr
225                     230                     235                 240

Lys Asn Phe Lys Asn Phe Glu Ile Thr Ile Lys Ala Arg Tyr Phe Tyr
                        245                     250                 255

Asn Lys Val Val Thr Glu Ala Asp Val Tyr Ile Thr Phe Gly Ile Arg

83

EP 3 250 604 B1

260                          265                          270

Glu Asp Leu Lys Asp Asp Gln Lys Glu Met Met Gln Thr Ala Met Gln
        275                  280                  285

Asn Thr Met Leu Ile Asn Gly Ile Ala Gln Val Thr Phe Asp Ser Glu
        290                  295                  300

Thr Ala Val Lys Glu Leu Ser Tyr Tyr Ser Leu Glu Asp Leu Asn Asn
305                  310                  315                  320

Lys Tyr Leu Tyr Ile Ala Val Thr Val Ile Glu Ser Thr Gly Gly Phe
                325                  330                  335

Ser Glu Glu Ala Glu Ile Pro Gly Ile Lys Tyr Val Leu Ser Pro Tyr
            340                  345                  350

Lys Leu Asn Leu Val Ala Thr Pro Leu Phe Leu Lys Pro Gly Ile Pro
        355                  360                  365

Tyr Pro Ile Lys Val Gln Val Lys Asp Ser Leu Asp Gln Leu Val Gly
    370                  375                  380

Gly Val Pro Val Thr Leu Asn Ala Gln Thr Ile Asp Val Asn Gln Glu
385                  390                  395                  400

Thr Ser Asp Leu Asp Pro Ser Lys Ser Val Thr Arg Val Asp Asp Gly
            405                  410                  415

Val Ala Ser Phe Val Leu Asn Leu Pro Ser Gly Val Thr Val Leu Glu
            420                  425                  430

Phe Asn Val Lys Thr Asp Ala Pro Asp Leu Pro Glu Glu Asn Gln Ala
        435                  440                  445

Arg Glu Gly Tyr Arg Ala Ile Ala Tyr Ser Ser Leu Ser Gln Ser Tyr
    450                  455                  460

Leu Tyr Ile Asp Trp Thr Asp Asn His Lys Ala Leu Leu Val Gly Glu
465                  470                  475                  480

His Leu Asn Ile Ile Val Thr Pro Lys Ser Pro Tyr Ile Asp Lys Ile
                485                  490                  495

Thr His Tyr Asn Tyr Leu Ile Leu Ser Lys Gly Lys Ile Ile His Phe
        500                  505                  510

84

```
Gly Thr Arg Glu Lys Phe Ser Asp Ala Ser Tyr Gln Ser Ile Asn Ile
        515             520             525

Pro Val Thr Gln Asn Met Val Pro Ser Ser Arg Leu Leu Val Tyr Tyr
        530             535             540

Ile Val Thr Gly Glu Gln Thr Ala Glu Leu Val Ser Asp Ser Val Trp
545             550             555             560

Leu Asn Ile Glu Glu Lys Cys Gly Asn Gln Leu Gln Val His Leu Ser
            565             570             575

Pro Asp Ala Asp Ala Tyr Ser Pro Gly Gln Thr Val Ser Leu Asn Met
        580             585             590

Ala Thr Gly Met Asp Ser Trp Val Ala Leu Ala Ala Val Asp Ser Ala
        595             600             605

Val Tyr Gly Val Gln Arg Gly Ala Lys Lys Pro Leu Glu Arg Val Phe
        610             615             620

Gln Phe Leu Glu Lys Ser Asp Leu Gly Cys Gly Ala Gly Gly Gly Leu
625             630             635             640

Asn Asn Ala Asn Val Phe His Leu Ala Gly Leu Thr Phe Leu Thr Asn
            645             650             655

Ala Asn Ala Asp Asp Ser Gln Glu Asn Asp Glu Pro Cys Lys Glu Ile
        660             665             670

Leu Arg Pro Arg Arg Thr Leu Gln Lys Lys Ile Glu Glu Ile Ala Ala
        675             680             685

Lys Tyr Lys His Ser Val Val Lys Lys Cys Cys Tyr Asp Gly Ala Cys
        690             695             700

Val Asn Asn Asp Glu Thr Cys Glu Gln Arg Ala Ala Arg Ile Ser Leu
705             710             715             720

Gly Pro Arg Cys Ile Lys Ala Phe Thr Glu Cys Cys Val Val Ala Ser
            725             730             735

Gln Leu Arg Ala Asn Ile Ser His Lys Asp Met Gln Leu Gly Arg Leu
        740             745             750

His Met Lys Thr Leu Leu Pro Val Ser Lys Pro Glu Ile Arg Ser Tyr
        755             760             765
```

```
Phe Pro Glu Ser Trp Leu Trp Glu Val His Leu Val Pro Arg Arg Lys
    770             775             780

Gln Leu Gln Phe Ala Leu Pro Asp Ser Leu Thr Thr Trp Glu Ile Gln
785             790             795             800

Gly Val Gly Ile Ser Asn Thr Gly Ile Cys Val Ala Asp Thr Val Lys
                805             810             815

Ala Lys Val Phe Lys Asp Val Phe Leu Glu Met Asn Ile Pro Tyr Ser
                820             825             830

Val Val Arg Gly Glu Gln Ile Gln Leu Lys Gly Thr Val Tyr Asn Tyr
        835             840             845

Arg Thr Ser Gly Met Gln Phe Cys Val Lys Met Ser Ala Val Glu Gly
    850             855             860

Ile Cys Thr Ser Glu Ser Pro Val Ile Asp His Gln Gly Thr Lys Ser
865             870             875             880

Ser Lys Cys Val Arg Gln Lys Val Glu Gly Ser Ser Ser His Leu Val
            885             890             895

Thr Phe Thr Val Leu Pro Leu Glu Ile Gly Leu His Asn Ile Asn Phe
        900             905             910

Ser Leu Glu Thr Trp Phe Gly Lys Glu Ile Leu Val Lys Thr Leu Arg
    915             920             925

Val Val Pro Glu Gly Val Lys Arg Glu Ser Tyr Ser Gly Val Thr Leu
    930             935             940

Asp Pro Arg Gly Ile Tyr Gly Thr Ile Ser Arg Arg Lys Glu Phe Pro
945             950             955             960

Tyr Arg Ile Pro Leu Asp Leu Val Pro Lys Thr Glu Ile Lys Arg Ile
            965             970             975

Leu Ser Val Lys Gly Leu Leu Val Gly Glu Ile Leu Ser Ala Val Leu
            980             985             990

Ser Gln Glu Gly Ile Asn Ile Leu  Thr His Leu Pro Lys  Gly Ser Ala
        995             1000            1005

Glu Ala  Glu Leu Met Ser Val  Val Pro Val Phe Tyr  Val Phe His
    1010            1015            1020
```

Tyr Leu Glu Thr Gly Asn His Trp Asn Ile Phe His Ser Asp Pro
    1025              1030              1035

Leu Ile Glu Lys Gln Lys Leu Lys Lys Lys Leu Lys Glu Gly Met
    1040              1045              1050

Leu Ser Ile Met Ser Tyr Arg Asn Ala Asp Tyr Ser Tyr Ser Val
    1055              1060              1065

Trp Lys Gly Gly Ser Ala Ser Thr Trp Leu Thr Ala Phe Ala Leu
    1070              1075              1080

Arg Val Leu Gly Gln Val Asn Lys Tyr Val Glu Gln Asn Gln Asn
    1085              1090              1095

Ser Ile Cys Asn Ser Leu Leu Trp Leu Val Glu Asn Tyr Gln Leu
    1100              1105              1110

Asp Asn Gly Ser Phe Lys Glu Asn Ser Gln Tyr Gln Pro Ile Lys
    1115              1120              1125

Leu Gln Gly Thr Leu Pro Val Glu Ala Arg Glu Asn Ser Leu Tyr
    1130              1135              1140

Leu Thr Ala Phe Thr Val Ile Gly Ile Arg Lys Ala Phe Asp Ile
    1145              1150              1155

Cys Pro Leu Val Lys Ile Asp Thr Ala Leu Ile Lys Ala Asp Asn
    1160              1165              1170

Phe Leu Leu Glu Asn Thr Leu Pro Ala Gln Ser Thr Phe Thr Leu
    1175              1180              1185

Ala Ile Ser Ala Tyr Ala Leu Ser Leu Gly Asp Lys Thr His Pro
    1190              1195              1200

Gln Phe Arg Ser Ile Val Ser Ala Leu Lys Arg Glu Ala Leu Val
    1205              1210              1215

Lys Gly Asn Pro Pro Ile Tyr Arg Phe Trp Lys Asp Asn Leu Gln
    1220              1225              1230

His Lys Asp Ser Ser Val Pro Asn Thr Gly Thr Ala Arg Met Val
    1235              1240              1245

Glu Thr Thr Ala Tyr Ala Leu Leu Thr Ser Leu Asn Leu Lys Asp

                    1250                    1255                    1260


      Ile Asn  Tyr Val Asn Pro Val  Ile Lys Trp Leu Ser  Glu Glu Gln
           1265                    1270                    1275


      Arg Tyr  Gly Gly Gly Phe Tyr  Ser Thr Gln Asp Thr  Ile Asn Ala
           1280                    1285                    1290


      Ile Glu  Gly Leu Thr Glu Tyr  Ser Leu Leu Val Lys  Gln Leu Arg
           1295                    1300                    1305


      Leu Ser  Met Asp Ile Asp Val  Ser Tyr Lys His Lys  Gly Ala Leu
           1310                    1315                    1320


      His Asn  Tyr Lys Met Thr Asp  Lys Asn Phe Leu Gly  Arg Pro Val
           1325                    1330                    1335


      Glu Val  Leu Leu Asn Asp Asp  Leu Ile Val Ser Thr  Gly Phe Gly
           1340                    1345                    1350


      Ser Gly  Leu Ala Thr Val His  Val Thr Thr Val Val  His Lys Thr
           1355                    1360                    1365


      Ser Thr  Ser Glu Glu Val Cys  Ser Phe Tyr Leu Lys  Ile Asp Thr
           1370                    1375                    1380


      Gln Asp  Ile Glu Ala Ser His  Tyr Arg Gly Tyr Gly  Asn Ser Asp
           1385                    1390                    1395


      Tyr Lys  Arg Ile Val Ala Cys  Ala Ser Tyr Lys Pro  Ser Arg Glu
           1400                    1405                    1410


      Glu Ser  Ser Ser Gly Ser Ser  His Ala Val Met Asp  Ile Ser Leu
           1415                    1420                    1425


      Pro Thr  Gly Ile Ser Ala Asn  Glu Glu Asp Leu Lys  Ala Leu Val
           1430                    1435                    1440


      Glu Gly  Val Asp Gln Leu Phe  Thr Asp Tyr Gln Ile  Lys Asp Gly
           1445                    1450                    1455


      His Val  Ile Leu Gln Leu Asn  Ser Ile Pro Ser Ser  Asp Phe Leu
           1460                    1465                    1470


      Cys Val  Arg Phe Arg Ile Phe  Glu Leu Phe Glu Val  Gly Phe Leu
           1475                    1480                    1485

```
        Ser Pro  Ala Thr Phe Thr Val  Tyr Glu Tyr His Arg  Pro Asp Lys
            1490              1495              1500

        Gln Cys  Thr Met Phe Tyr Ser  Thr Ser Asn Ile Lys  Ile Gln Lys
            1505              1510              1515

        Val Cys  Glu Gly Ala Ala Cys  Lys Cys Val Glu Ala  Asp Cys Gly
            1520              1525              1530

        Gln Met  Gln Glu Glu Leu Asp  Leu Thr Ile Ser Ala  Glu Thr Arg
            1535              1540              1545

        Lys Gln  Thr Ala Cys Lys Pro  Glu Ile Ala Tyr Ala  Tyr Lys Val
            1550              1555              1560

        Ser Ile  Thr Ser Ile Thr Val  Glu Asn Val Phe Val  Lys Tyr Lys
            1565              1570              1575

        Ala Thr  Leu Leu Asp Ile Tyr  Lys Thr Gly Glu Ala  Val Ala Glu
            1580              1585              1590

        Lys Asp  Ser Glu Ile Thr Phe  Ile Lys Lys Val Thr  Cys Thr Asn
            1595              1600              1605

        Ala Glu  Leu Val Lys Gly Arg  Gln Tyr Leu Ile Met  Gly Lys Glu
            1610              1615              1620

        Ala Leu  Gln Ile Lys Tyr Asn  Phe Ser Phe Arg Tyr  Ile Tyr Pro
            1625              1630              1635

        Leu Asp  Ser Leu Thr Trp Ile  Glu Tyr Trp Pro Arg  Asp Thr Thr
            1640              1645              1650

        Cys Ser  Ser Cys Gln Ala Phe  Leu Ala Asn Leu Asp  Glu Phe Ala
            1655              1660              1665

        Glu Asp  Ile Phe Leu Asn Gly  Cys
            1670              1675
```

<210> 21
<211> 1170
<212> PRT
<213> homo sapiens

<400> 21

```
        Met Lys Asp Ser Cys Ile Thr Val Met Ala Met Ala Leu Leu Ser Gly
        1           5                   10                      15
```

```
Phe Phe Phe Phe Ala Pro Ala Ser Ser Tyr Asn Leu Asp Val Arg Gly
            20              25                  30

Ala Arg Ser Phe Ser Pro Pro Arg Ala Gly Arg His Phe Gly Tyr Arg
            35              40                  45

Val Leu Gln Val Gly Asn Gly Val Ile Val Gly Ala Pro Gly Glu Gly
            50              55                  60

Asn Ser Thr Gly Ser Leu Tyr Gln Cys Gln Ser Gly Thr Gly His Cys
65              70                  75                  80

Leu Pro Val Thr Leu Arg Gly Ser Asn Tyr Thr Ser Lys Tyr Leu Gly
                85                  90                  95

Met Thr Leu Ala Thr Asp Pro Thr Asp Gly Ser Ile Leu Ala Cys Asp
            100             105                 110

Pro Gly Leu Ser Arg Thr Cys Asp Gln Asn Thr Tyr Leu Ser Gly Leu
            115             120                 125

Cys Tyr Leu Phe Arg Gln Asn Leu Gln Gly Pro Met Leu Gln Gly Arg
            130             135                 140

Pro Gly Phe Gln Glu Cys Ile Lys Gly Asn Val Asp Leu Val Phe Leu
145             150                 155                 160

Phe Asp Gly Ser Met Ser Leu Gln Pro Asp Glu Phe Gln Lys Ile Leu
                165                 170                 175

Asp Phe Met Lys Asp Val Met Lys Lys Leu Ser Asn Thr Ser Tyr Gln
            180             185                 190

Phe Ala Ala Val Gln Phe Ser Thr Ser Tyr Lys Thr Glu Phe Asp Phe
            195             200                 205

Ser Asp Tyr Val Lys Arg Lys Asp Pro Asp Ala Leu Leu Lys His Val
            210             215                 220

Lys His Met Leu Leu Leu Thr Asn Thr Phe Gly Ala Ile Asn Tyr Val
225             230                 235                 240

Ala Thr Glu Val Phe Arg Glu Glu Leu Gly Ala Arg Pro Asp Ala Thr
                245                 250                 255

Lys Val Leu Ile Ile Ile Thr Asp Gly Glu Ala Thr Asp Ser Gly Asn
                260                 265                 270
```

90

```
Ile Asp Ala Ala Lys Asp Ile Ile Arg Tyr Ile Ile Gly Ile Gly Lys
    275                 280                 285

His Phe Gln Thr Lys Glu Ser Gln Glu Thr Leu His Lys Phe Ala Ser
    290                 295                 300

Lys Pro Ala Ser Glu Phe Val Lys Ile Leu Asp Thr Phe Glu Lys Leu
305                 310                 315                 320

Lys Asp Leu Phe Thr Glu Leu Gln Lys Lys Ile Tyr Val Ile Glu Gly
                325                 330                 335

Thr Ser Lys Gln Asp Leu Thr Ser Phe Asn Met Glu Leu Ser Ser Ser
                340                 345                 350

Gly Ile Ser Ala Asp Leu Ser Arg Gly His Ala Val Val Gly Ala Val
            355                 360                 365

Gly Ala Lys Asp Trp Ala Gly Gly Phe Leu Asp Leu Lys Ala Asp Leu
    370                 375                 380

Gln Asp Asp Thr Phe Ile Gly Asn Glu Pro Leu Thr Pro Glu Val Arg
385                 390                 395                 400

Ala Gly Tyr Leu Gly Tyr Thr Val Thr Trp Leu Pro Ser Arg Gln Lys
                405                 410                 415

Thr Ser Leu Leu Ala Ser Gly Ala Pro Arg Tyr Gln His Met Gly Arg
            420                 425                 430

Val Leu Leu Phe Gln Glu Pro Gln Gly Gly Gly His Trp Ser Gln Val
            435                 440                 445

Gln Thr Ile His Gly Thr Gln Ile Gly Ser Tyr Phe Gly Gly Glu Leu
    450                 455                 460

Cys Gly Val Asp Val Asp Gln Asp Gly Glu Thr Glu Leu Leu Leu Ile
465                 470                 475                 480

Gly Ala Pro Leu Phe Tyr Gly Glu Gln Arg Gly Gly Arg Val Phe Ile
                485                 490                 495

Tyr Gln Arg Arg Gln Leu Gly Phe Glu Glu Val Ser Glu Leu Gln Gly
            500                 505                 510

Asp Pro Gly Tyr Pro Leu Gly Arg Phe Gly Glu Ala Ile Thr Ala Leu
            515                 520                 525
```

91

```
Thr Asp Ile Asn Gly Asp Gly Leu Val Asp Val Ala Val Gly Ala Pro
    530             535             540

Leu Glu Glu Gln Gly Ala Val Tyr Ile Phe Asn Gly Arg His Gly Gly
    545             550             555             560

Leu Ser Pro Gln Pro Ser Gln Arg Ile Glu Gly Thr Gln Val Leu Ser
            565             570             575

Gly Ile Gln Trp Phe Gly Arg Ser Ile His Gly Val Lys Asp Leu Glu
            580             585             590

Gly Asp Gly Leu Ala Asp Val Ala Val Gly Ala Glu Ser Gln Met Ile
        595             600             605

Val Leu Ser Ser Arg Pro Val Val Asp Met Val Thr Leu Met Ser Phe
    610             615             620

Ser Pro Ala Glu Ile Pro Val His Glu Val Glu Cys Ser Tyr Ser Thr
625             630             635             640

Ser Asn Lys Met Lys Glu Gly Val Asn Ile Thr Ile Cys Phe Gln Ile
            645             650             655

Lys Ser Leu Ile Pro Gln Phe Gln Gly Arg Leu Val Ala Asn Leu Thr
            660             665             670

Tyr Thr Leu Gln Leu Asp Gly His Arg Thr Arg Arg Arg Gly Leu Phe
            675             680             685

Pro Gly Gly Arg His Glu Leu Arg Arg Asn Ile Ala Val Thr Thr Ser
    690             695             700

Met Ser Cys Thr Asp Phe Ser Phe His Phe Pro Val Cys Val Gln Asp
705             710             715             720

Leu Ile Ser Pro Ile Asn Val Ser Leu Asn Phe Ser Leu Trp Glu Glu
            725             730             735

Glu Gly Thr Pro Arg Asp Gln Arg Ala Gln Gly Lys Asp Ile Pro Pro
            740             745             750

Ile Leu Arg Pro Ser Leu His Ser Glu Thr Trp Glu Ile Pro Phe Glu
            755             760             765

Lys Asn Cys Gly Glu Asp Lys Lys Cys Glu Ala Asn Leu Arg Val Ser
```

92

<pre>
                  770                     775                      780

Phe Ser Pro Ala Arg Ser Arg Ala Leu Arg Leu Thr Ala Phe Ala Ser
785                 790                 795                 800

Leu Ser Val Glu Leu Ser Leu Ser Asn Leu Glu Glu Asp Ala Tyr Trp
                805                 810                 815

Val Gln Leu Asp Leu His Phe Pro Pro Gly Leu Ser Phe Arg Lys Val
            820                 825                 830

Glu Met Leu Lys Pro His Ser Gln Ile Pro Val Ser Cys Glu Glu Leu
        835                 840                 845

Pro Glu Glu Ser Arg Leu Leu Ser Arg Ala Leu Ser Cys Asn Val Ser
    850                 855                 860

Ser Pro Ile Phe Lys Ala Gly His Ser Val Ala Leu Gln Met Met Phe
865                 870                 875                 880

Asn Thr Leu Val Asn Ser Ser Trp Gly Asp Ser Val Glu Leu His Ala
                885                 890                 895

Asn Val Thr Cys Asn Asn Glu Asp Ser Asp Leu Leu Glu Asp Asn Ser
            900                 905                 910

Ala Thr Thr Ile Ile Pro Ile Leu Tyr Pro Ile Asn Ile Leu Ile Gln
        915                 920                 925

Asp Gln Glu Asp Ser Thr Leu Tyr Val Ser Phe Thr Pro Lys Gly Pro
    930                 935                 940

Lys Ile His Gln Val Lys His Met Tyr Gln Val Arg Ile Gln Pro Ser
945                 950                 955                 960

Ile His Asp His Asn Ile Pro Thr Leu Glu Ala Val Val Gly Val Pro
            965                 970                 975

Gln Pro Pro Ser Glu Gly Pro Ile Thr His Gln Trp Ser Val Gln Met
            980                 985                 990

Glu Pro Pro Val Pro Cys His Tyr  Glu Asp Leu Glu Arg  Leu Pro Asp
        995                 1000                1005

Ala Ala  Glu Pro Cys Leu Pro  Gly Ala Leu Phe Arg  Cys Pro Val
    1010                1015                1020
</pre>

```
Val Phe Arg Gln Glu Ile Leu Val Gln Val Ile Gly Thr Leu Glu
    1025            1030            1035

Leu Val Gly Glu Ile Glu Ala Ser Ser Met Phe Ser Leu Cys Ser
    1040            1045            1050

Ser Leu Ser Ile Ser Phe Asn Ser Ser Lys His Phe His Leu Tyr
    1055            1060            1065

Gly Ser Asn Ala Ser Leu Ala Gln Val Val Met Lys Val Asp Val
    1070            1075            1080

Val Tyr Glu Lys Gln Met Leu Tyr Leu Tyr Val Leu Ser Gly Ile
    1085            1090            1095

Gly Gly Leu Leu Leu Leu Leu Leu Ile Phe Ile Val Leu Tyr Lys
    1100            1105            1110

Val Gly Phe Phe Lys Arg Asn Leu Lys Glu Lys Met Glu Ala Gly
    1115            1120            1125

Arg Gly Val Pro Asn Gly Ile Pro Ala Glu Asp Ser Glu Gln Leu
    1130            1135            1140

Ala Ser Gly Gln Glu Ala Gly Asp Pro Gly Cys Leu Lys Pro Leu
    1145            1150            1155

His Glu Lys Asp Ser Glu Ser Gly Gly Gly Lys Asp
    1160            1165            1170
```

<210> 22
<211> 364
<212> PRT
<213> homo sapiens

<400> 22

```
Met Pro Leu Leu Leu Leu Leu Pro Leu Leu Trp Ala Gly Ala Leu Ala
1               5               10                  15

Met Asp Pro Asn Phe Trp Leu Gln Val Gln Glu Ser Val Thr Val Gln
            20              25              30

Glu Gly Leu Cys Val Leu Val Pro Cys Thr Phe Phe His Pro Ile Pro
        35              40              45

Tyr Tyr Asp Lys Asn Ser Pro Val His Gly Tyr Trp Phe Arg Glu Gly
    50              55              60
```

```
Ala Ile Ile Ser Arg Asp Ser Pro Val Ala Thr Asn Lys Leu Asp Gln
65              70          75                      80

Glu Val Gln Glu Glu Thr Gln Gly Arg Phe Arg Leu Leu Gly Asp Pro
            85              90                      95

Ser Arg Asn Asn Cys Ser Leu Ser Ile Val Asp Ala Arg Arg Arg Asp
            100             105             110

Asn Gly Ser Tyr Phe Phe Arg Met Glu Arg Gly Ser Thr Lys Tyr Ser
            115             120             125

Tyr Lys Ser Pro Gln Leu Ser Val His Val Thr Asp Leu Thr His Arg
            130             135             140

Pro Lys Ile Leu Ile Pro Gly Thr Leu Glu Pro Gly His Ser Lys Asn
145             150             155             160

Leu Thr Cys Ser Val Ser Trp Ala Cys Glu Gln Gly Thr Pro Pro Ile
            165             170             175

Phe Ser Trp Leu Ser Ala Ala Pro Thr Ser Leu Gly Pro Arg Thr Thr
            180             185             190

His Ser Ser Val Leu Ile Ile Thr Pro Arg Pro Gln Asp His Gly Thr
            195             200             205

Asn Leu Thr Cys Gln Val Lys Phe Ala Gly Ala Gly Val Thr Thr Glu
            210             215             220

Arg Thr Ile Gln Leu Asn Val Thr Tyr Val Pro Gln Asn Pro Thr Thr
225             230             235             240

Gly Ile Phe Pro Gly Asp Gly Ser Gly Lys Gln Glu Thr Arg Ala Gly
            245             250             255

Val Val His Gly Ala Ile Gly Gly Ala Gly Val Thr Ala Leu Leu Ala
            260             265             270

Leu Cys Leu Cys Leu Ile Phe Phe Ile Val Lys Thr His Arg Arg Lys
            275             280             285

Ala Ala Arg Thr Ala Val Gly Arg Asn Asp Thr His Pro Thr Thr Gly
            290             295             300

Ser Ala Ser Pro Lys His Gln Lys Lys Ser Lys Leu His Gly Pro Thr
305             310             315             320
```

95

```
Glu Thr Ser Ser Cys Ser Gly Ala Ala Pro Thr Val Glu Met Asp Glu
            325                 330                 335

Glu Leu His Tyr Ala Ser Leu Asn Phe His Gly Met Asn Pro Ser Lys
            340                 345                 350

Asp Thr Ser Thr Glu Tyr Ser Glu Val Arg Thr Gln
            355                 360
```

<210> 23
<211> 1032
<212> PRT
<213> homo sapiens

<400> 23

```
Met Ala Trp Glu Ala Arg Arg Glu Pro Gly Pro Arg Arg Ala Ala Val
1               5                   10                  15

Arg Glu Thr Val Met Leu Leu Leu Cys Leu Gly Val Pro Thr Gly Arg
            20                  25                  30

Pro Tyr Asn Val Asp Thr Glu Ser Ala Leu Leu Tyr Gln Gly Pro His
            35                  40                  45

Asn Thr Leu Phe Gly Tyr Ser Val Val Leu His Ser His Gly Ala Asn
        50                  55                  60

Arg Trp Leu Leu Val Gly Ala Pro Thr Ala Asn Trp Leu Ala Asn Ala
65                  70                  75                  80

Ser Val Ile Asn Pro Gly Ala Ile Tyr Arg Cys Arg Ile Gly Lys Asn
                85                  90                  95

Pro Gly Gln Thr Cys Glu Gln Leu Gln Leu Gly Ser Pro Asn Gly Glu
            100                 105                 110

Pro Cys Gly Lys Thr Cys Leu Glu Glu Arg Asp Asn Gln Trp Leu Gly
            115                 120                 125

Val Thr Leu Ser Arg Gln Pro Gly Glu Asn Gly Ser Ile Val Thr Cys
        130                 135                 140

Gly His Arg Trp Lys Asn Ile Phe Tyr Ile Lys Asn Glu Asn Lys Leu
145                 150                 155                 160

Pro Thr Gly Gly Cys Tyr Gly Val Pro Pro Asp Leu Arg Thr Glu Leu
                165                 170                 175
```

Ser Lys Arg Ile Ala Pro Cys Tyr Gln Asp Tyr Val Lys Lys Phe Gly
          180                     185                 190

Glu Asn Phe Ala Ser Cys Gln Ala Gly Ile Ser Ser Phe Tyr Thr Lys
          195                     200                 205

Asp Leu Ile Val Met Gly Ala Pro Gly Ser Ser Tyr Trp Thr Gly Ser
          210                     215                 220

Leu Phe Val Tyr Asn Ile Thr Thr Asn Lys Tyr Lys Ala Phe Leu Asp
225                     230                 235                 240

Lys Gln Asn Gln Val Lys Phe Gly Ser Tyr Leu Gly Tyr Ser Val Gly
                    245                 250                 255

Ala Gly His Phe Arg Ser Gln His Thr Thr Glu Val Val Gly Gly Ala
              260                     265                 270

Pro Gln His Glu Gln Ile Gly Lys Ala Tyr Ile Phe Ser Ile Asp Glu
          275                     280                 285

Lys Glu Leu Asn Ile Leu His Glu Met Lys Gly Lys Lys Leu Gly Ser
          290                     295                 300

Tyr Phe Gly Ala Ser Val Cys Ala Val Asp Leu Asn Ala Asp Gly Phe
305                     310                 315                 320

Ser Asp Leu Leu Val Gly Ala Pro Met Gln Ser Thr Ile Arg Glu Glu
              325                     330                 335

Gly Arg Val Phe Val Tyr Ile Asn Ser Gly Ser Gly Ala Val Met Asn
              340                     345                 350

Ala Met Glu Thr Asn Leu Val Gly Ser Asp Lys Tyr Ala Ala Arg Phe
          355                     360                 365

Gly Glu Ser Ile Val Asn Leu Gly Asp Ile Asp Asn Asp Gly Phe Glu
          370                     375                 380

Asp Val Ala Ile Gly Ala Pro Gln Glu Asp Asp Leu Gln Gly Ala Ile
385                     390                 395                 400

Tyr Ile Tyr Asn Gly Arg Ala Asp Gly Ile Ser Ser Thr Phe Ser Gln
                    405                 410                 415

Arg Ile Glu Gly Leu Gln Ile Ser Lys Ser Leu Ser Met Phe Gly Gln
          420                     425                 430

```
Ser Ile Ser Gly Gln Ile Asp Ala Asp Asn Asn Gly Tyr Val Asp Val
        435             440             445

Ala Val Gly Ala Phe Arg Ser Asp Ser Ala Val Leu Leu Arg Thr Arg
        450             455             460

Pro Val Val Ile Val Asp Ala Ser Leu Ser His Pro Glu Ser Val Asn
465             470             475             480

Arg Thr Lys Phe Asp Cys Val Glu Asn Gly Trp Pro Ser Val Cys Ile
            485             490             495

Asp Leu Thr Leu Cys Phe Ser Tyr Lys Gly Lys Glu Val Pro Gly Tyr
            500             505             510

Ile Val Leu Phe Tyr Asn Met Ser Leu Asp Val Asn Arg Lys Ala Glu
            515             520             525

Ser Pro Pro Arg Phe Tyr Phe Ser Ser Asn Gly Thr Ser Asp Val Ile
    530             535             540

Thr Gly Ser Ile Gln Val Ser Ser Arg Glu Ala Asn Cys Arg Thr His
545             550             555             560

Gln Ala Phe Met Arg Lys Asp Val Arg Asp Ile Leu Thr Pro Ile Gln
            565             570             575

Ile Glu Ala Ala Tyr His Leu Gly Pro His Val Ile Ser Lys Arg Ser
            580             585             590

Thr Glu Glu Phe Pro Pro Leu Gln Pro Ile Leu Gln Gln Lys Lys Glu
            595             600             605

Lys Asp Ile Met Lys Lys Thr Ile Asn Phe Ala Arg Phe Cys Ala His
    610             615             620

Glu Asn Cys Ser Ala Asp Leu Gln Val Ser Ala Lys Ile Gly Phe Leu
625             630             635             640

Lys Pro His Glu Asn Lys Thr Tyr Leu Ala Val Gly Ser Met Lys Thr
            645             650             655

Leu Met Leu Asn Val Ser Leu Phe Asn Ala Gly Asp Asp Ala Tyr Glu
            660             665             670

Thr Thr Leu His Val Lys Leu Pro Val Gly Leu Tyr Phe Ile Lys Ile
```

675　　　　　　　　　680　　　　　　　　　685

Leu Glu Leu Glu Glu Lys Gln Ile Asn Cys Glu Val Thr Asp Asn Ser
　　　690　　　　　　　　695　　　　　　　　700

Gly Val Val Gln Leu Asp Cys Ser Ile Gly Tyr Ile Tyr Val Asp His
705　　　　　　　　710　　　　　　　　715　　　　　　　　720

Leu Ser Arg Ile Asp Ile Ser Phe Leu Leu Asp Val Ser Ser Leu Ser
　　　　　　　　725　　　　　　　　730　　　　　　　　735

Arg Ala Glu Glu Asp Leu Ser Ile Thr Val His Ala Thr Cys Glu Asn
　　　　　　740　　　　　　　　745　　　　　　　　750

Glu Glu Glu Met Asp Asn Leu Lys His Ser Arg Val Thr Val Ala Ile
　　　　　755　　　　　　　　760　　　　　　　　765

Pro Leu Lys Tyr Glu Val Lys Leu Thr Val His Gly Phe Val Asn Pro
　　　770　　　　　　　　775　　　　　　　　780

Thr Ser Phe Val Tyr Gly Ser Asn Asp Glu Asn Glu Pro Glu Thr Cys
785　　　　　　　　790　　　　　　　　795　　　　　　　　800

Met Val Glu Lys Met Asn Leu Thr Phe His Val Ile Asn Thr Gly Asn
　　　　　805　　　　　　　　810　　　　　　　　815

Ser Met Ala Pro Asn Val Ser Val Glu Ile Met Val Pro Asn Ser Phe
　　　　　820　　　　　　　　825　　　　　　　　830

Ser Pro Gln Thr Asp Lys Leu Phe Asn Ile Leu Asp Val Gln Thr Thr
　　　　　835　　　　　　　　840　　　　　　　　845

Thr Gly Glu Cys His Phe Glu Asn Tyr Gln Arg Val Cys Ala Leu Glu
　　　850　　　　　　　　855　　　　　　　　860

Gln Gln Lys Ser Ala Met Gln Thr Leu Lys Gly Ile Val Arg Phe Leu
865　　　　　　　　870　　　　　　　　875　　　　　　　　880

Ser Lys Thr Asp Lys Arg Leu Leu Tyr Cys Ile Lys Ala Asp Pro His
　　　　　885　　　　　　　　890　　　　　　　　895

Cys Leu Asn Phe Leu Cys Asn Phe Gly Lys Met Glu Ser Gly Lys Glu
　　　　　900　　　　　　　　905　　　　　　　　910

Ala Ser Val His Ile Gln Leu Glu Gly Arg Pro Ser Ile Leu Glu Met
　　　　　915　　　　　　　　920　　　　　　　　925

99

```
Asp Glu Thr Ser Ala Leu Lys Phe Glu Ile Arg Ala Thr Gly Phe Pro
    930                 935                 940

Glu Pro Asn Pro Arg Val Ile Glu Leu Asn Lys Asp Glu Asn Val Ala
945                 950                 955                 960

His Val Leu Leu Glu Gly Leu His His Gln Arg Pro Lys Arg Tyr Phe
                965                 970                 975

Thr Ile Val Ile Ile Ser Ser Ser Leu Leu Leu Gly Leu Ile Val Leu
            980                 985                 990

Leu Leu Ile Ser Tyr Val Met Trp Lys Ala Gly Phe Phe Lys Arg Gln
    995                 1000                1005

Tyr Lys Ser Ile Leu Gln Glu Glu Asn Arg Arg Asp Ser Trp Ser
    1010                1015                1020

Tyr Ile Asn Ser Lys Ser Asn Asp Asp
    1025                1030
```

<210> 24
<211> 257
<212> PRT
<213> homo sapiens

<400> 24

```
Met Ala Pro Ala Met Glu Ser Pro Thr Leu Leu Cys Val Ala Leu Leu
1               5               10                  15

Phe Phe Ala Pro Asp Gly Val Leu Ala Val Pro Gln Lys Pro Lys Val
        20                  25                  30

Ser Leu Asn Pro Pro Trp Asn Arg Ile Phe Lys Gly Glu Asn Val Thr
        35                  40                  45

Leu Thr Cys Asn Gly Asn Asn Phe Phe Glu Val Ser Ser Thr Lys Trp
    50                  55                  60

Phe His Asn Gly Ser Leu Ser Glu Glu Thr Asn Ser Ser Leu Asn Ile
65                  70                  75                  80

Val Asn Ala Lys Phe Glu Asp Ser Gly Glu Tyr Lys Cys Gln His Gln
                85                  90                  95

Gln Val Asn Glu Ser Glu Pro Val Tyr Leu Glu Val Phe Ser Asp Trp
            100                 105                 110
```

```
Leu Leu Leu Gln Ala Ser Ala Glu Val Val Met Glu Gly Gln Pro Leu
        115             120             125

Phe Leu Arg Cys His Gly Trp Arg Asn Trp Asp Val Tyr Lys Val Ile
        130             135             140

Tyr Tyr Lys Asp Gly Glu Ala Leu Lys Tyr Trp Tyr Glu Asn His Asn
145             150             155             160

Ile Ser Ile Thr Asn Ala Thr Val Glu Asp Ser Gly Thr Tyr Tyr Cys
            165             170             175

Thr Gly Lys Val Trp Gln Leu Asp Tyr Glu Ser Glu Pro Leu Asn Ile
        180             185             190

Thr Val Ile Lys Ala Pro Arg Glu Lys Tyr Trp Leu Gln Phe Phe Ile
        195             200             205

Pro Leu Leu Val Val Ile Leu Phe Ala Val Asp Thr Gly Leu Phe Ile
    210             215             220

Ser Thr Gln Gln Gln Val Thr Phe Leu Leu Lys Ile Lys Arg Thr Arg
225             230             235             240

Lys Gly Phe Arg Leu Leu Asn Pro His Pro Lys Pro Asn Pro Lys Asn
            245             250             255

Asn
```

<210> 25
<211> 574
<212> PRT
<213> homo sapiens

<400> 25

```
Met Glu Leu Leu Ile Leu Lys Ala Asn Ala Ile Thr Thr Ile Leu Thr
1               5               10              15

Ala Val Thr Phe Cys Phe Ala Ser Gly Gln Asn Ile Thr Glu Glu Phe
        20              25              30

Tyr Gln Ser Thr Cys Ser Ala Val Ser Lys Gly Tyr Leu Ser Ala Leu
        35              40              45

Arg Thr Gly Trp Tyr Thr Ser Val Ile Thr Ile Glu Leu Ser Asn Ile
    50              55              60
```

```
Lys Glu Asn Lys Cys Asn Gly Thr Asp Ala Lys Val Lys Leu Ile Lys
65              70              75              80

Gln Glu Leu Asp Lys Tyr Lys Asn Ala Val Thr Glu Leu Gln Leu Leu
            85              90              95

Met Gln Ser Thr Pro Pro Thr Asn Asn Arg Ala Arg Arg Glu Leu Pro
            100             105             110

Arg Phe Met Asn Tyr Thr Leu Asn Asn Ala Lys Lys Thr Asn Val Thr
        115             120             125

Leu Ser Lys Lys Arg Lys Arg Arg Phe Leu Gly Phe Leu Leu Gly Val
        130             135             140

Gly Ser Ala Ile Ala Ser Gly Val Ala Val Ser Lys Val Leu His Leu
145             150             155             160

Glu Gly Glu Val Asn Lys Ile Lys Ser Ala Leu Leu Ser Thr Asn Lys
            165             170             175

Ala Val Val Ser Leu Ser Asn Gly Val Ser Val Leu Thr Ser Lys Val
            180             185             190

Leu Asp Leu Lys Asn Tyr Ile Asp Lys Gln Leu Leu Pro Ile Val Asn
        195             200             205

Lys Gln Ser Cys Ser Ile Ser Asn Ile Glu Thr Val Ile Glu Phe Gln
    210             215             220

Gln Lys Asn Asn Arg Leu Leu Glu Ile Thr Arg Glu Phe Ser Val Asn
225             230             235             240

Ala Gly Val Thr Thr Pro Val Ser Thr Tyr Met Leu Thr Asn Ser Glu
            245             250             255

Leu Leu Ser Leu Ile Asn Asp Met Pro Ile Thr Asn Asp Gln Lys Lys
        260             265             270

Leu Met Ser Asn Asn Val Gln Ile Val Arg Gln Gln Ser Tyr Ser Ile
        275             280             285

Met Ser Ile Ile Lys Glu Glu Val Leu Ala Tyr Val Val Gln Leu Pro
        290             295             300

Leu Tyr Gly Val Ile Asp Thr Pro Cys Trp Lys Leu His Thr Ser Pro
305             310             315             320
```

```
Leu Cys Thr Thr Asn Thr Lys Glu Gly Ser Asn Ile Cys Leu Thr Arg
            325             330             335

Thr Asp Arg Gly Trp Tyr Cys Asp Asn Ala Gly Ser Val Ser Phe Phe
            340             345             350

Pro Gln Ala Glu Thr Cys Lys Val Gln Ser Asn Arg Val Phe Cys Asp
            355             360             365

Thr Met Asn Ser Leu Thr Leu Pro Ser Glu Ile Asn Leu Cys Asn Val
    370             375             380

Asp Ile Phe Asn Pro Lys Tyr Asp Cys Lys Ile Met Thr Ser Lys Thr
385             390             395             400

Asp Val Ser Ser Ser Val Ile Thr Ser Leu Gly Ala Ile Val Ser Cys
            405             410             415

Tyr Gly Lys Thr Lys Cys Thr Ala Ser Asn Lys Asn Arg Gly Ile Ile
            420             425             430

Lys Thr Phe Ser Asn Gly Cys Asp Tyr Val Ser Asn Lys Gly Met Asp
            435             440             445

Thr Val Ser Val Gly Asn Thr Leu Tyr Tyr Val Asn Lys Gln Glu Gly
    450             455             460

Lys Ser Leu Tyr Val Lys Gly Glu Pro Ile Ile Asn Phe Tyr Asp Pro
465             470             475             480

Leu Val Phe Pro Ser Asp Glu Phe Asp Ala Ser Ile Ser Gln Val Asn
            485             490             495

Glu Lys Ile Asn Gln Ser Leu Ala Phe Ile Arg Lys Ser Asp Glu Leu
            500             505             510

Leu His Asn Val Asn Ala Gly Lys Ser Thr Thr Asn Ile Met Ile Thr
            515             520             525

Thr Ile Ile Ile Val Ile Ile Val Ile Leu Leu Ser Leu Ile Ala Val
    530             535             540

Gly Leu Leu Leu Tyr Cys Lys Ala Arg Ser Thr Pro Val Thr Leu Ser
545             550             555             560

Lys Asp Gln Leu Ser Gly Ile Asn Asn Ile Ala Phe Ser Asn
            565             570
```

<210> 26
<211> 468
<212> PRT
<213> homo sapiens

<400> 26

```
Met Leu Ala Val Gly Cys Ala Leu Leu Ala Ala Leu Leu Ala Ala Pro
1               5                   10                  15

Gly Ala Ala Leu Ala Pro Arg Arg Cys Pro Ala Gln Glu Val Ala Arg
                20                  25                  30

Gly Val Leu Thr Ser Leu Pro Gly Asp Ser Val Thr Leu Thr Cys Pro
                35                  40                  45

Gly Val Glu Pro Glu Asp Asn Ala Thr Val His Trp Val Leu Arg Lys
            50                  55                  60

Pro Ala Ala Gly Ser His Pro Ser Arg Trp Ala Gly Met Gly Arg Arg
65                  70                  75                  80

Leu Leu Leu Arg Ser Val Gln Leu His Asp Ser Gly Asn Tyr Ser Cys
                85                  90                  95

Tyr Arg Ala Gly Arg Pro Ala Gly Thr Val His Leu Leu Val Asp Val
                100                 105                 110

Pro Pro Glu Glu Pro Gln Leu Ser Cys Phe Arg Lys Ser Pro Leu Ser
                115                 120                 125

Asn Val Val Cys Glu Trp Gly Pro Arg Ser Thr Pro Ser Leu Thr Thr
        130                 135                 140

Lys Ala Val Leu Leu Val Arg Lys Phe Gln Asn Ser Pro Ala Glu Asp
145                 150                 155                 160

Phe Gln Glu Pro Cys Gln Tyr Ser Gln Glu Ser Gln Lys Phe Ser Cys
                165                 170                 175

Gln Leu Ala Val Pro Glu Gly Asp Ser Ser Phe Tyr Ile Val Ser Met
                180                 185                 190

Cys Val Ala Ser Ser Val Gly Ser Lys Phe Ser Lys Thr Gln Thr Phe
        195                 200                 205

Gln Gly Cys Gly Ile Leu Gln Pro Asp Pro Pro Ala Asn Ile Thr Val
        210                 215                 220
```

```
Thr Ala Val Ala Arg Asn Pro Arg Trp Leu Ser Val Thr Trp Gln Asp
225                 230         235                 240

Pro His Ser Trp Asn Ser Ser Phe Tyr Arg Leu Arg Phe Glu Leu Arg
                245             250                 255

Tyr Arg Ala Glu Arg Ser Lys Thr Phe Thr Thr Trp Met Val Lys Asp
            260             265             270

Leu Gln His His Cys Val Ile His Asp Ala Trp Ser Gly Leu Arg His
        275             280             285

Val Val Gln Leu Arg Ala Gln Glu Glu Phe Gly Gln Gly Glu Trp Ser
    290             295             300

Glu Trp Ser Pro Glu Ala Met Gly Thr Pro Trp Thr Glu Ser Arg Ser
305             310             315             320

Pro Pro Ala Glu Asn Glu Val Ser Thr Pro Met Gln Ala Leu Thr Thr
            325             330             335

Asn Lys Asp Asp Asp Asn Ile Leu Phe Arg Asp Ser Ala Asn Ala Thr
            340             345             350

Ser Leu Pro Val Gln Asp Ser Ser Ser Val Pro Leu Pro Thr Phe Leu
        355             360             365

Val Ala Gly Gly Ser Leu Ala Phe Gly Thr Leu Leu Cys Ile Ala Ile
    370             375             380

Val Leu Arg Phe Lys Lys Thr Trp Lys Leu Arg Ala Leu Lys Glu Gly
385             390             395             400

Lys Thr Ser Met His Pro Pro Tyr Ser Leu Gly Gln Leu Val Pro Glu
            405             410             415

Arg Pro Arg Pro Thr Pro Val Leu Val Pro Leu Ile Ser Pro Pro Val
            420             425             430

Ser Pro Ser Ser Leu Gly Ser Asp Asn Thr Ser Ser His Asn Arg Pro
        435             440             445

Asp Ala Arg Asp Pro Arg Ser Pro Tyr Asp Ile Ser Asn Thr Asp Tyr
    450             455             460

Phe Phe Pro Arg
```

465

<210> 27
<211> 1255
<212> PRT
<213> homo sapiens

<400> 27

```
Met Glu Leu Ala Ala Leu Cys Arg Trp Gly Leu Leu Leu Ala Leu Leu
1               5               10              15

Pro Pro Gly Ala Ala Ser Thr Gln Val Cys Thr Gly Thr Asp Met Lys
        20              25              30

Leu Arg Leu Pro Ala Ser Pro Glu Thr His Leu Asp Met Leu Arg His
        35              40              45

Leu Tyr Gln Gly Cys Gln Val Val Gln Gly Asn Leu Glu Leu Thr Tyr
    50              55              60

Leu Pro Thr Asn Ala Ser Leu Ser Phe Leu Gln Asp Ile Gln Glu Val
65              70              75              80

Gln Gly Tyr Val Leu Ile Ala His Asn Gln Val Arg Gln Val Pro Leu
            85              90              95

Gln Arg Leu Arg Ile Val Arg Gly Thr Gln Leu Phe Glu Asp Asn Tyr
        100             105             110

Ala Leu Ala Val Leu Asp Asn Gly Asp Pro Leu Asn Asn Thr Thr Pro
        115             120             125

Val Thr Gly Ala Ser Pro Gly Gly Leu Arg Glu Leu Gln Leu Arg Ser
    130             135             140

Leu Thr Glu Ile Leu Lys Gly Gly Val Leu Ile Gln Arg Asn Pro Gln
145             150             155             160

Leu Cys Tyr Gln Asp Thr Ile Leu Trp Lys Asp Ile Phe His Lys Asn
            165             170             175

Asn Gln Leu Ala Leu Thr Leu Ile Asp Thr Asn Arg Ser Arg Ala Cys
        180             185             190

His Pro Cys Ser Pro Met Cys Lys Gly Ser Arg Cys Trp Gly Glu Ser
        195             200             205

Ser Glu Asp Cys Gln Ser Leu Thr Arg Thr Val Cys Ala Gly Gly Cys
```

107

210                     215                     220

Ala Arg Cys Lys Gly Pro Leu Pro Thr Asp Cys Cys His Glu Gln Cys
225             230             235             240

Ala Ala Gly Cys Thr Gly Pro Lys His Ser Asp Cys Leu Ala Cys Leu
                245             250             255

His Phe Asn His Ser Gly Ile Cys Glu Leu His Cys Pro Ala Leu Val
            260             265             270

Thr Tyr Asn Thr Asp Thr Phe Glu Ser Met Pro Asn Pro Glu Gly Arg
            275             280             285

Tyr Thr Phe Gly Ala Ser Cys Val Thr Ala Cys Pro Tyr Asn Tyr Leu
    290             295             300

Ser Thr Asp Val Gly Ser Cys Thr Leu Val Cys Pro Leu His Asn Gln
305             310             315             320

Glu Val Thr Ala Glu Asp Gly Thr Gln Arg Cys Glu Lys Cys Ser Lys
            325             330             335

Pro Cys Ala Arg Val Cys Tyr Gly Leu Gly Met Glu His Leu Arg Glu
            340             345             350

Val Arg Ala Val Thr Ser Ala Asn Ile Gln Glu Phe Ala Gly Cys Lys
    355             360             365

Lys Ile Phe Gly Ser Leu Ala Phe Leu Pro Glu Ser Phe Asp Gly Asp
    370             375             380

Pro Ala Ser Asn Thr Ala Pro Leu Gln Pro Glu Gln Leu Gln Val Phe
385             390             395             400

Glu Thr Leu Glu Glu Ile Thr Gly Tyr Leu Tyr Ile Ser Ala Trp Pro
            405             410             415

Asp Ser Leu Pro Asp Leu Ser Val Phe Gln Asn Leu Gln Val Ile Arg
            420             425             430

Gly Arg Ile Leu His Asn Gly Ala Tyr Ser Leu Thr Leu Gln Gly Leu
    435             440             445

Gly Ile Ser Trp Leu Gly Leu Arg Ser Leu Arg Glu Leu Gly Ser Gly
    450             455             460

108

Leu Ala Leu Ile His His Asn Thr His Leu Cys Phe Val His Thr Val
465             470             475             480

Pro Trp Asp Gln Leu Phe Arg Asn Pro His Gln Ala Leu Leu His Thr
                485             490             495

Ala Asn Arg Pro Glu Asp Glu Cys Val Gly Glu Gly Leu Ala Cys His
                500             505             510

Gln Leu Cys Ala Arg Gly His Cys Trp Gly Pro Gly Pro Thr Gln Cys
                515             520             525

Val Asn Cys Ser Gln Phe Leu Arg Gly Gln Glu Cys Val Glu Glu Cys
                530             535             540

Arg Val Leu Gln Gly Leu Pro Arg Glu Tyr Val Asn Ala Arg His Cys
545             550             555             560

Leu Pro Cys His Pro Glu Cys Gln Pro Gln Asn Gly Ser Val Thr Cys
                565             570             575

Phe Gly Pro Glu Ala Asp Gln Cys Val Ala Cys Ala His Tyr Lys Asp
                580             585             590

Pro Pro Phe Cys Val Ala Arg Cys Pro Ser Gly Val Lys Pro Asp Leu
                595             600             605

Ser Tyr Met Pro Ile Trp Lys Phe Pro Asp Glu Glu Gly Ala Cys Gln
        610             615             620

Pro Cys Pro Ile Asn Cys Thr His Ser Cys Val Asp Leu Asp Asp Lys
625             630             635             640

Gly Cys Pro Ala Glu Gln Arg Ala Ser Pro Leu Thr Ser Ile Ile Ser
                645             650             655

Ala Val Val Gly Ile Leu Leu Val Val Val Leu Gly Val Val Phe Gly
                660             665             670

Ile Leu Ile Lys Arg Arg Gln Gln Lys Ile Arg Lys Tyr Thr Met Arg
        675             680             685

Arg Leu Leu Gln Glu Thr Glu Leu Val Glu Pro Leu Thr Pro Ser Gly
        690             695             700

Ala Met Pro Asn Gln Ala Gln Met Arg Ile Leu Lys Glu Thr Glu Leu
705             710             715             720

```
Arg Lys Val Lys Val Leu Gly Ser Gly Ala Phe Gly Thr Val Tyr Lys
            725             730                 735

Gly Ile Trp Ile Pro Asp Gly Glu Asn Val Lys Ile Pro Val Ala Ile
            740             745                 750

Lys Val Leu Arg Glu Asn Thr Ser Pro Lys Ala Asn Lys Glu Ile Leu
            755             760                 765

Asp Glu Ala Tyr Val Met Ala Gly Val Gly Ser Pro Tyr Val Ser Arg
            770             775                 780

Leu Leu Gly Ile Cys Leu Thr Ser Thr Val Gln Leu Val Thr Gln Leu
785             790             795                 800

Met Pro Tyr Gly Cys Leu Leu Asp His Val Arg Glu Asn Arg Gly Arg
                805             810                 815

Leu Gly Ser Gln Asp Leu Leu Asn Trp Cys Met Gln Ile Ala Lys Gly
                820             825                 830

Met Ser Tyr Leu Glu Asp Val Arg Leu Val His Arg Asp Leu Ala Ala
            835             840                 845

Arg Asn Val Leu Val Lys Ser Pro Asn His Val Lys Ile Thr Asp Phe
            850             855                 860

Gly Leu Ala Arg Leu Leu Asp Ile Asp Glu Thr Glu Tyr His Ala Asp
865             870             875                 880

Gly Gly Lys Val Pro Ile Lys Trp Met Ala Leu Glu Ser Ile Leu Arg
                885             890                 895

Arg Arg Phe Thr His Gln Ser Asp Val Trp Ser Tyr Gly Val Thr Val
            900             905                 910

Trp Glu Leu Met Thr Phe Gly Ala Lys Pro Tyr Asp Gly Ile Pro Ala
            915             920                 925

Arg Glu Ile Pro Asp Leu Leu Glu Lys Gly Glu Arg Leu Pro Gln Pro
            930             935                 940

Pro Ile Cys Thr Ile Asp Val Tyr Met Ile Met Val Lys Cys Trp Met
945             950             955                 960

Ile Asp Ser Glu Cys Arg Pro Arg Phe Arg Glu Leu Val Ser Glu Phe
                965             970                 975
```

Ser Arg Met Ala Arg Asp Pro Gln Arg Phe Val Val Ile Gln Asn Glu
            980                 985                 990

Asp Leu Gly Pro Ala Ser Pro Leu  Asp Ser Thr Phe Tyr  Arg Ser Leu
            995                 1000                1005

Leu Glu  Asp Asp Asp Met Gly  Asp Leu Val Asp Ala  Glu Glu Tyr
         1010                1015                1020

Leu Val  Pro Gln Gln Gly Phe  Phe Cys Pro Asp Pro  Ala Pro Gly
         1025                1030                1035

Ala Gly  Gly Met Val His His  Arg His Arg Ser Ser  Ser Thr Arg
         1040                1045                1050

Ser Gly  Gly Gly Asp Leu Thr  Leu Gly Leu Glu Pro  Ser Glu Glu
         1055                1060                1065

Glu Ala  Pro Arg Ser Pro Leu  Ala Pro Ser Glu Gly  Ala Gly Ser
         1070                1075                1080

Asp Val  Phe Asp Gly Asp Leu  Gly Met Gly Ala Ala  Lys Gly Leu
         1085                1090                1095

Gln Ser  Leu Pro Thr His Asp  Pro Ser Pro Leu Gln  Arg Tyr Ser
         1100                1105                1110

Glu Asp  Pro Thr Val Pro Leu  Pro Ser Glu Thr Asp  Gly Tyr Val
         1115                1120                1125

Ala Pro  Leu Thr Cys Ser Pro  Gln Pro Glu Tyr Val  Asn Gln Pro
         1130                1135                1140

Asp Val  Arg Pro Gln Pro Pro  Ser Pro Arg Glu Gly  Pro Leu Pro
         1145                1150                1155

Ala Ala  Arg Pro Ala Gly Ala  Thr Leu Glu Arg Pro  Lys Thr Leu
         1160                1165                1170

Ser Pro  Gly Lys Asn Gly Val  Val Lys Asp Val Phe  Ala Phe Gly
         1175                1180                1185

Gly Ala  Val Glu Asn Pro Glu  Tyr Leu Thr Pro Gln  Gly Gly Ala
         1190                1195                1200

Ala Pro  Gln Pro His Pro Pro  Pro Ala Phe Ser Pro  Ala Phe Asp

111

```
                    1205                    1210                          1215


            Asn Leu  Tyr Tyr Trp Asp Gln  Asp Pro Pro Glu Arg  Gly Ala Pro
                1220                1225                1230

            Pro Ser  Thr Phe Lys Gly Thr  Pro Thr Ala Glu Asn  Pro Glu Tyr
                1235                1240                1245

            Leu Gly  Leu Asp Val Pro Val
                1250                1255
```

<210> 28
<211> 798
<212> PRT
<213> homo sapiens

<400> 28

```
Met Val Ala Leu Pro Met Val Leu Val Leu Leu Leu Val Leu Ser Arg
1            5               10              15

Gly Glu Ser Glu Leu Asp Ala Lys Ile Pro Ser Thr Gly Asp Ala Thr
         20              25              30

Glu Trp Arg Asn Pro His Leu Ser Met Leu Gly Ser Cys Gln Pro Ala
         35              40              45

Pro Ser Cys Gln Lys Cys Ile Leu Ser His Pro Ser Cys Ala Trp Cys
         50              55              60

Lys Gln Leu Asn Phe Thr Ala Ser Gly Glu Ala Glu Ala Arg Arg Cys
65              70              75              80

Ala Arg Arg Glu Glu Leu Leu Ala Arg Gly Cys Pro Leu Glu Glu Leu
         85              90              95

Glu Glu Pro Arg Gly Gln Gln Glu Val Leu Gln Asp Gln Pro Leu Ser
         100             105             110

Gln Gly Ala Arg Gly Glu Gly Ala Thr Gln Leu Ala Pro Gln Arg Val
         115             120             125

Arg Val Thr Leu Arg Pro Gly Glu Pro Gln Gln Leu Gln Val Arg Phe
         130             135             140

Leu Arg Ala Glu Gly Tyr Pro Val Asp Leu Tyr Tyr Leu Met Asp Leu
145             150             155             160

Ser Tyr Ser Met Lys Asp Asp Leu Glu Arg Val Arg Gln Leu Gly His
```

```
                      165                        170                        175

        Ala Leu Leu Val Arg Leu Gln Glu Val Thr His Ser Val Arg Ile Gly
                    180                    185                    190

        Phe Gly Ser Phe Val Asp Lys Thr Val Leu Pro Phe Val Ser Thr Val
                    195                    200                    205

        Pro Ser Lys Leu Arg His Pro Cys Pro Thr Arg Leu Glu Arg Cys Gln
            210                    215                    220

        Ser Pro Phe Ser Phe His His Val Leu Ser Leu Thr Gly Asp Ala Gln
        225                    230                    235                    240

        Ala Phe Glu Arg Glu Val Gly Arg Gln Ser Val Ser Gly Asn Leu Asp
                        245                    250                    255

        Ser Pro Glu Gly Gly Phe Asp Ala Ile Leu Gln Ala Ala Leu Cys Gln
                    260                    265                    270

        Glu Gln Ile Gly Trp Arg Asn Val Ser Arg Leu Leu Val Phe Thr Ser
                    275                    280                    285

        Asp Asp Thr Phe His Thr Ala Gly Asp Gly Lys Leu Gly Gly Ile Phe
                290                    295                    300

        Met Pro Ser Asp Gly His Cys His Leu Asp Ser Asn Gly Leu Tyr Ser
        305                    310                    315                    320

        Arg Ser Thr Glu Phe Asp Tyr Pro Ser Val Gly Gln Val Ala Gln Ala
                        325                    330                    335

        Leu Ser Ala Ala Asn Ile Gln Pro Ile Phe Ala Val Thr Ser Ala Ala
                    340                    345                    350

        Leu Pro Val Tyr Gln Glu Leu Ser Lys Leu Ile Pro Lys Ser Ala Val
                    355                    360                    365

        Gly Glu Leu Ser Glu Asp Ser Ser Asn Val Val Gln Leu Ile Met Asp
            370                    375                    380

        Ala Tyr Asn Ser Leu Ser Ser Thr Val Thr Leu Glu His Ser Ser Leu
        385                    390                    395                    400

        Pro Pro Gly Val His Ile Ser Tyr Glu Ser Gln Cys Glu Gly Pro Glu
                    405                    410                    415
```

```
Lys Arg Glu Gly Lys Ala Glu Asp Arg Gly Gln Cys Asn His Val Arg
        420             425             430

Ile Asn Gln Thr Val Thr Phe Trp Val Ser Leu Gln Ala Thr His Cys
        435             440             445

Leu Pro Glu Pro His Leu Leu Arg Leu Arg Ala Leu Gly Phe Ser Glu
        450             455             460

Glu Leu Ile Val Glu Leu His Thr Leu Cys Asp Cys Asn Cys Ser Asp
465             470             475             480

Thr Gln Pro Gln Ala Pro His Cys Ser Asp Gly Gln Gly His Leu Gln
                485             490             495

Cys Gly Val Cys Ser Cys Ala Pro Gly Arg Leu Gly Arg Leu Cys Glu
        500             505             510

Cys Ser Val Ala Glu Leu Ser Ser Pro Asp Leu Glu Ser Gly Cys Arg
        515             520             525

Ala Pro Asn Gly Thr Gly Pro Leu Cys Ser Gly Lys Gly His Cys Gln
        530             535             540

Cys Gly Arg Cys Ser Cys Ser Gly Gln Ser Ser Gly His Leu Cys Glu
545             550             555             560

Cys Asp Asp Ala Ser Cys Glu Arg His Glu Gly Ile Leu Cys Gly Gly
                565             570             575

Phe Gly Arg Cys Gln Cys Gly Val Cys His Cys His Ala Asn Arg Thr
        580             585             590

Gly Arg Ala Cys Glu Cys Ser Gly Asp Met Asp Ser Cys Ile Ser Pro
        595             600             605

Glu Gly Gly Leu Cys Ser Gly His Gly Arg Cys Lys Cys Asn Arg Cys
        610             615             620

Gln Cys Leu Asp Gly Tyr Tyr Gly Ala Leu Cys Asp Gln Cys Pro Gly
625             630             635             640

Cys Lys Thr Pro Cys Glu Arg His Arg Asp Cys Ala Glu Cys Gly Ala
                645             650             655

Phe Arg Thr Gly Pro Leu Ala Thr Asn Cys Ser Thr Ala Cys Ala His
        660             665             670
```

```
Thr Asn Val Thr Leu Ala Leu Ala Pro Ile Leu Asp Asp Gly Trp Cys
        675             680             685

Lys Glu Arg Thr Leu Asp Asn Gln Leu Phe Phe Phe Leu Val Glu Asp
        690             695             700

Asp Ala Arg Gly Thr Val Val Leu Arg Val Arg Pro Gln Glu Lys Gly
705             710             715             720

Ala Asp His Thr Gln Ala Ile Val Leu Gly Cys Val Gly Gly Ile Val
        725             730             735

Ala Val Gly Leu Gly Leu Val Leu Ala Tyr Arg Leu Ser Val Glu Ile
        740             745             750

Tyr Asp Arg Arg Glu Tyr Ser Arg Phe Glu Lys Glu Gln Gln Gln Leu
        755             760             765

Asn Trp Lys Gln Asp Ser Asn Pro Leu Tyr Lys Ser Ala Ile Thr Thr
        770             775             780

Thr Ile Asn Pro Arg Phe Gln Glu Ala Asp Ser Pro Thr Leu
785             790             795
```

<210> 29
<211> 285
<212> PRT
<213> homo sapiens

<400> 29

```
Met Asp Asp Ser Thr Glu Arg Glu Gln Ser Arg Leu Thr Ser Cys Leu
1               5               10              15

Lys Lys Arg Glu Glu Met Lys Leu Lys Glu Cys Val Ser Ile Leu Pro
        20              25              30

Arg Lys Glu Ser Pro Ser Val Arg Ser Ser Lys Asp Gly Lys Leu Leu
        35              40              45

Ala Ala Thr Leu Leu Leu Ala Leu Leu Ser Cys Cys Leu Thr Val Val
        50              55              60

Ser Phe Tyr Gln Val Ala Ala Leu Gln Gly Asp Leu Ala Ser Leu Arg
65              70              75              80

Ala Glu Leu Gln Gly His His Ala Glu Lys Leu Pro Ala Gly Ala Gly
                85              90              95
```

```
Ala Pro Lys Ala Gly Leu Glu Glu Ala Pro Ala Val Thr Ala Gly Leu
        100                 105                 110

Lys Ile Phe Glu Pro Pro Ala Pro Gly Glu Gly Asn Ser Ser Gln Asn
        115                 120                 125

Ser Arg Asn Lys Arg Ala Val Gln Gly Pro Glu Glu Thr Val Thr Gln
        130                 135                 140

Asp Cys Leu Gln Leu Ile Ala Asp Ser Glu Thr Pro Thr Ile Gln Lys
145                 150                 155                 160

Gly Ser Tyr Thr Phe Val Pro Trp Leu Leu Ser Phe Lys Arg Gly Ser
                165                 170                 175

Ala Leu Glu Glu Lys Glu Asn Lys Ile Leu Val Lys Glu Thr Gly Tyr
        180                 185                 190

Phe Phe Ile Tyr Gly Gln Val Leu Tyr Thr Asp Lys Thr Tyr Ala Met
        195                 200                 205

Gly His Leu Ile Gln Arg Lys Lys Val His Val Phe Gly Asp Glu Leu
    210                 215                 220

Ser Leu Val Thr Leu Phe Arg Cys Ile Gln Asn Met Pro Glu Thr Leu
225                 230                 235                 240

Pro Asn Asn Ser Cys Tyr Ser Ala Gly Ile Ala Lys Leu Glu Glu Gly
                245                 250                 255

Asp Glu Leu Gln Leu Ala Ile Pro Arg Glu Asn Ala Gln Ile Ser Leu
        260                 265                 270

Asp Gly Asp Val Thr Phe Phe Gly Ala Leu Lys Leu Leu
        275                 280                 285
```

<210> 30
<211> 269
<212> PRT
<213> homo sapiens

<400> 30

```
Met Ala Glu Val Pro Glu Leu Ala Ser Glu Met Met Ala Tyr Tyr Ser
1                   5                   10                  15

Gly Asn Glu Asp Asp Leu Phe Phe Glu Ala Asp Gly Pro Lys Gln Met
            20                  25                  30
```

```
Lys Cys Ser Phe Gln Asp Leu Asp Leu Cys Pro Leu Asp Gly Gly Ile
        35                  40                  45

Gln Leu Arg Ile Ser Asp His His Tyr Ser Lys Gly Phe Arg Gln Ala
        50                  55                  60

Ala Ser Val Val Val Ala Met Asp Lys Leu Arg Lys Met Leu Val Pro
65                  70                  75                  80

Cys Pro Gln Thr Phe Gln Glu Asn Asp Leu Ser Thr Phe Phe Pro Phe
            85                  90                  95

Ile Phe Glu Glu Glu Pro Ile Phe Phe Asp Thr Trp Asp Asn Glu Ala
            100                 105                 110

Tyr Val His Asp Ala Pro Val Arg Ser Leu Asn Cys Thr Leu Arg Asp
        115                 120                 125

Ser Gln Gln Lys Ser Leu Val Met Ser Gly Pro Tyr Glu Leu Lys Ala
        130                 135                 140

Leu His Leu Gln Gly Gln Asp Met Glu Gln Gln Val Val Phe Ser Met
145                 150                 155                 160

Ser Phe Val Gln Gly Glu Glu Ser Asn Asp Lys Ile Pro Val Ala Leu
            165                 170                 175

Gly Leu Lys Glu Lys Asn Leu Tyr Leu Ser Cys Val Leu Lys Asp Asp
            180                 185                 190

Lys Pro Thr Leu Gln Leu Glu Ser Val Asp Pro Lys Asn Tyr Pro Lys
            195                 200                 205

Lys Lys Met Glu Lys Arg Phe Val Phe Asn Lys Ile Glu Ile Asn Asn
        210                 215                 220

Lys Leu Glu Phe Glu Ser Ala Gln Phe Pro Asn Trp Tyr Ile Ser Thr
225                 230                 235                 240

Ser Gln Ala Glu Asn Met Pro Val Phe Leu Gly Gly Thr Lys Gly Gly
            245                 250                 255

Gln Asp Ile Thr Asp Phe Thr Met Gln Phe Val Ser Ser
            260                 265
```

<210> 31
<211> 317

<212> PRT
<213> homo sapiens

<400> 31

```
Met Arg Arg Ala Ser Arg Asp Tyr Thr Lys Tyr Leu Arg Gly Ser Glu
1               5                   10              15

Glu Met Gly Gly Gly Pro Gly Ala Pro His Glu Gly Pro Leu His Ala
            20              25                  30

Pro Pro Pro Pro Ala Pro His Gln Pro Pro Ala Ala Ser Arg Ser Met
        35              40                  45

Phe Val Ala Leu Leu Gly Leu Gly Leu Gly Gln Val Val Cys Ser Val
    50              55                  60

Ala Leu Phe Phe Tyr Phe Arg Ala Gln Met Asp Pro Asn Arg Ile Ser
65              70                  75                  80

Glu Asp Gly Thr His Cys Ile Tyr Arg Ile Leu Arg Leu His Glu Asn
            85                  90                  95

Ala Asp Phe Gln Asp Thr Thr Leu Glu Ser Gln Asp Thr Lys Leu Ile
            100             105             110

Pro Asp Ser Cys Arg Arg Ile Lys Gln Ala Phe Gln Gly Ala Val Gln
        115             120             125

Lys Glu Leu Gln His Ile Val Gly Ser Gln His Ile Arg Ala Glu Lys
    130             135             140

Ala Met Val Asp Gly Ser Trp Leu Asp Leu Ala Lys Arg Ser Lys Leu
145             150             155             160

Glu Ala Gln Pro Phe Ala His Leu Thr Ile Asn Ala Thr Asp Ile Pro
            165             170             175

Ser Gly Ser His Lys Val Ser Leu Ser Ser Trp Tyr His Asp Arg Gly
            180             185             190

Trp Ala Lys Ile Ser Asn Met Thr Phe Ser Asn Gly Lys Leu Ile Val
        195             200             205

Asn Gln Asp Gly Phe Tyr Tyr Leu Tyr Ala Asn Ile Cys Phe Arg His
    210             215             220

His Glu Thr Ser Gly Asp Leu Ala Thr Glu Tyr Leu Gln Leu Met Val
225             230             235             240
```

```
Tyr Val Thr Lys Thr Ser Ile Lys Ile Pro Ser Ser His Thr Leu Met
            245             250             255

Lys Gly Gly Ser Thr Lys Tyr Trp Ser Gly Asn Ser Glu Phe His Phe
            260             265             270

Tyr Ser Ile Asn Val Gly Gly Phe Phe Lys Leu Arg Ser Gly Glu Glu
            275             280             285

Ile Ser Ile Glu Val Ser Asn Pro Ser Leu Leu Asp Pro Asp Gln Asp
            290             295             300

Ala Thr Tyr Phe Gly Ala Phe Lys Val Arg Asp Ile Asp
305             310             315
```

<210> 32
<211> 223
<212> PRT
<213> homo sapiens

<400> 32

```
Met Ala Cys Leu Gly Phe Gln Arg His Lys Ala Gln Leu Asn Leu Ala
1               5               10              15

Thr Arg Thr Trp Pro Cys Thr Leu Leu Phe Phe Leu Leu Phe Ile Pro
            20              25              30

Val Phe Cys Lys Ala Met His Val Ala Gln Pro Ala Val Val Leu Ala
            35              40              45

Ser Ser Arg Gly Ile Ala Ser Phe Val Cys Glu Tyr Ala Ser Pro Gly
    50              55              60

Lys Ala Thr Glu Val Arg Val Thr Val Leu Arg Gln Ala Asp Ser Gln
65              70              75              80

Val Thr Glu Val Cys Ala Ala Thr Tyr Met Met Gly Asn Glu Leu Thr
            85              90              95

Phe Leu Asp Asp Ser Ile Cys Thr Gly Thr Ser Ser Gly Asn Gln Val
            100             105             110

Asn Leu Thr Ile Gln Gly Leu Arg Ala Met Asp Thr Gly Leu Tyr Ile
            115             120             125

Cys Lys Val Glu Leu Met Tyr Pro Pro Pro Tyr Tyr Leu Gly Ile Gly
            130             135             140
```

```
Asn Gly Thr Gln Ile Tyr Val Ile Asp Pro Glu Pro Cys Pro Asp Ser
145             150             155             160

Asp Phe Leu Leu Trp Ile Leu Ala Ala Val Ser Ser Gly Leu Phe Phe
                165             170             175

Tyr Ser Phe Leu Leu Thr Ala Val Ser Leu Ser Lys Met Leu Lys Lys
            180             185             190

Arg Ser Pro Leu Thr Thr Gly Val Tyr Val Lys Met Pro Pro Thr Glu
        195             200             205

Pro Glu Cys Glu Lys Gln Phe Gln Pro Tyr Phe Ile Pro Ile Asn
    210             215             220
```

<210> 33
<211> 189
<212> PRT
<213> homo sapiens

<400> 33

```
Met Leu Gly Ser Arg Ala Val Met Leu Leu Leu Leu Leu Pro Trp Thr
1           5               10              15

Ala Gln Gly Arg Ala Val Pro Gly Gly Ser Ser Pro Ala Trp Thr Gln
            20              25              30

Cys Gln Gln Leu Ser Gln Lys Leu Cys Thr Leu Ala Trp Ser Ala His
        35              40              45

Pro Leu Val Gly His Met Asp Leu Arg Glu Glu Gly Asp Glu Glu Thr
    50              55              60

Thr Asn Asp Val Pro His Ile Gln Cys Gly Asp Gly Cys Asp Pro Gln
65              70              75              80

Gly Leu Arg Asp Asn Ser Gln Phe Cys Leu Gln Arg Ile His Gln Gly
            85              90              95

Leu Ile Phe Tyr Glu Lys Leu Leu Gly Ser Asp Ile Phe Thr Gly Glu
            100             105             110

Pro Ser Leu Leu Pro Asp Ser Pro Val Gly Gln Leu His Ala Ser Leu
        115             120             125

Leu Gly Leu Ser Gln Leu Leu Gln Pro Glu Gly His His Trp Glu Thr
        130             135             140
```

```
Gln Gln Ile Pro Ser Leu Ser Pro Ser Gln Pro Trp Gln Arg Leu Leu
145             150             155                 160

Leu Arg Phe Lys Ile Leu Arg Ser Leu Gln Ala Phe Val Ala Val Ala
            165             170             175

Ala Arg Val Phe Ala His Gly Ala Ala Thr Leu Ser Pro
            180             185
```

<210> 34
<211> 288
<212> PRT
<213> homo sapiens

<400> 34

```
Met Gln Ile Pro Gln Ala Pro Trp Pro Val Val Trp Ala Val Leu Gln
1               5               10              15

Leu Gly Trp Arg Pro Gly Trp Phe Leu Asp Ser Pro Asp Arg Pro Trp
            20              25              30

Asn Pro Pro Thr Phe Ser Pro Ala Leu Leu Val Val Thr Glu Gly Asp
            35              40              45

Asn Ala Thr Phe Thr Cys Ser Phe Ser Asn Thr Ser Glu Ser Phe Val
    50              55              60

Leu Asn Trp Tyr Arg Met Ser Pro Ser Asn Gln Thr Asp Lys Leu Ala
65              70              75              80

Ala Phe Pro Glu Asp Arg Ser Gln Pro Gly Gln Asp Cys Arg Phe Arg
            85              90              95

Val Thr Gln Leu Pro Asn Gly Arg Asp Phe His Met Ser Val Val Arg
            100             105             110

Ala Arg Arg Asn Asp Ser Gly Thr Tyr Leu Cys Gly Ala Ile Ser Leu
            115             120             125

Ala Pro Lys Ala Gln Ile Lys Glu Ser Leu Arg Ala Glu Leu Arg Val
            130             135             140

Thr Glu Arg Arg Ala Glu Val Pro Thr Ala His Pro Ser Pro Ser Pro
145             150             155             160

Arg Pro Ala Gly Gln Phe Gln Thr Leu Val Val Gly Val Val Gly Gly
            165             170             175
```

```
Leu Leu Gly Ser Leu Val Leu Leu Val Trp Val Leu Ala Val Ile Cys
            180                 185                 190

Ser Arg Ala Ala Arg Gly Thr Ile Gly Ala Arg Arg Thr Gly Gln Pro
            195                 200                 205

Leu Lys Glu Asp Pro Ser Ala Val Pro Val Phe Ser Val Asp Tyr Gly
            210                 215                 220

Glu Leu Asp Phe Gln Trp Arg Glu Lys Thr Pro Glu Pro Pro Val Pro
225                 230                 235                 240

Cys Val Pro Glu Gln Thr Glu Tyr Ala Thr Ile Val Phe Pro Ser Gly
                245                 250                 255

Met Gly Thr Ser Ser Pro Ala Arg Arg Gly Ser Ala Asp Gly Pro Arg
            260                 265                 270

Ser Ala Gln Pro Leu Arg Pro Glu Asp Gly His Cys Ser Trp Pro Leu
            275                 280                 285
```

<210> 35
<211> 9
<212> PRT
<213> artificial sequence

<220>
<223> mAb-specific epitope

<400> 35

```
Cys Pro Tyr Ser Asn Pro Ser Leu Cys
1                 5
```

<210> 36
<211> 24
<212> PRT
<213> homo sapiens

<400> 36

```
Asn Ser Glu Leu Leu Ser Leu Ile Asn Asp Met Pro Ile Thr Asn Asp
1                 5                 10                  15

Gln Lys Lys Leu Met Ser Asn Asn
            20
```

<210> 37
<211> 12
<212> PRT
<213> artificial sequence

<220>
<223> mAb-specific epitope

<400> 37

```
Cys Gln Phe Asp Leu Ser Thr Arg Arg Leu Lys Cys
1               5                   10
```

<210> 38
<211> 12
<212> PRT
<213> artificial sequence

<220>
<223> mAb-specific epitope

<400> 38

```
Cys Gln Tyr Asn Leu Ser Ser Arg Ala Leu Lys Cys
1               5                   10
```

<210> 39
<211> 12
<212> PRT
<213> artificial sequence

<220>
<223> mAb-specific epitope

<400> 39

```
Cys Val Trp Gln Arg Trp Gln Lys Ser Tyr Val Cys
1               5                   10
```

<210> 40
<211> 12
<212> PRT
<213> artificial sequence

<220>
<223> mAb-specific epitope

<400> 40

```
Cys Met Trp Asp Arg Phe Ser Arg Trp Tyr Lys Cys
1               5                   10
```

<210> 41
<211> 25
<212> PRT
<213> homo sapiens

<400> 41

```
Ser Phe Val Leu Asn Trp Tyr Arg Met Ser Pro Ser Asn Gln Thr Asp
1               5                   10                  15
```

Lys Leu Ala Ala Phe Pro Glu Asp Arg
                20                  25

<210> 42
<211> 19
<212> PRT
<213> homo sapiens

<400> 42

Ser Gly Thr Tyr Leu Cys Gly Ala Ile Ser Leu Ala Pro Lys Ala Gln
1               5               10              15

Ile Lys Glu

<210> 43
<211> 557
<212> PRT
<213> murine

<400> 43

Met Pro Pro Pro Arg Leu Leu Phe Phe Leu Leu Phe Leu Thr Pro Met
1               5               10              15

Glu Val Arg Pro Glu Glu Pro Leu Val Val Lys Val Glu Glu Gly Asp
            20              25              30

Asn Ala Val Leu Gln Cys Leu Lys Gly Thr Ser Asp Gly Pro Thr Gln
            35              40              45

Gln Leu Thr Trp Ser Arg Glu Ser Pro Leu Lys Pro Phe Leu Lys Leu
        50              55              60

Ser Leu Gly Leu Pro Gly Leu Gly Ile His Met Arg Pro Leu Ala Ile
65              70              75              80

Trp Leu Phe Ile Phe Asn Val Ser Gln Gln Met Gly Gly Phe Tyr Leu
                85              90              95

Cys Gln Pro Gly Pro Pro Ser Glu Lys Ala Trp Gln Pro Gly Trp Thr
            100             105             110

Val Asn Val Glu Gly Ser Gly Glu Leu Phe Arg Trp Asn Val Ser Asp
            115             120             125

Leu Gly Gly Leu Gly Cys Gly Leu Lys Asn Arg Ser Ser Glu Gly Pro
        130             135             140

Ser Ser Pro Ser Gly Lys Leu Met Ser Pro Lys Leu Tyr Val Trp Ala

|     | 145 |     |     |     | 150 |     |     |     | 155 |     |     |     | 160 |
| --- | --- | --- | --- | --- | --- | --- | --- | --- | --- | --- | --- | --- | --- |

Lys Asp Arg Pro Glu Ile Trp Glu Gly Glu Pro Pro Cys Leu Pro Pro
    165             170         175

Arg Asp Ser Leu Asn Gln Ser Leu Ser Gln Asp Leu Thr Met Ala Pro
    180             185         190

Gly Ser Thr Leu Trp Leu Ser Cys Gly Val Pro Pro Asp Ser Val Ser
    195             200         205

Arg Gly Pro Leu Ser Trp Thr His Val His Pro Lys Gly Pro Lys Ser
    210             215         220

Leu Leu Ser Leu Glu Leu Lys Asp Asp Arg Pro Ala Arg Asp Met Trp
225             230         235             240

Val Met Glu Thr Gly Leu Leu Leu Pro Arg Ala Thr Ala Gln Asp Ala
            245             250         255

Gly Lys Tyr Tyr Cys His Arg Gly Asn Leu Thr Met Ser Phe His Leu
            260             265         270

Glu Ile Thr Ala Arg Pro Val Leu Trp His Trp Leu Leu Arg Thr Gly
    275             280         285

Gly Trp Lys Val Ser Ala Val Thr Leu Ala Tyr Leu Ile Phe Cys Leu
    290             295         300

Cys Ser Leu Val Gly Ile Leu His Leu Gln Arg Ala Leu Val Leu Arg
305             310         315             320

Arg Lys Arg Lys Arg Met Thr Asp Pro Thr Arg Arg Phe Phe Lys Val
            325             330         335

Thr Pro Pro Pro Gly Ser Gly Pro Gln Asn Gln Tyr Gly Asn Val Leu
            340             345         350

Ser Leu Pro Thr Pro Thr Ser Gly Leu Gly Arg Ala Gln Arg Trp Ala
            355             360         365

Ala Gly Leu Gly Gly Thr Ala Pro Ser Tyr Gly Asn Pro Ser Ser Asp
    370             375         380

Val Gln Ala Asp Gly Ala Leu Gly Ser Arg Ser Pro Pro Gly Val Gly
385             390         395             400

Pro Glu Glu Glu Glu Gly Glu Gly Tyr Glu Glu Pro Asp Ser Glu Glu
                405                    410                  415

Asp Ser Glu Phe Tyr Glu Asn Asp Ser Asn Leu Gly Gln Asp Gln Leu
            420                425                430

Ser Gln Asp Gly Ser Gly Tyr Glu Asn Pro Glu Asp Glu Pro Leu Gly
            435              440              445

Pro Glu Asp Glu Asp Ser Phe Ser Asn Ala Glu Ser Tyr Glu Asn Glu
    450                455              460

Asp Glu Glu Leu Thr Gln Pro Val Ala Arg Thr Met Asp Phe Leu Ser
465                470              475              480

Pro His Gly Ser Ala Trp Asp Pro Ser Arg Glu Ala Thr Ser Leu Ala
            485              490              495

Gly Ser Gln Ser Tyr Glu Asp Met Arg Gly Ile Leu Tyr Ala Ala Pro
            500              505              510

Gln Leu Arg Ser Ile Arg Gly Gln Pro Gly Pro Asn His Glu Glu Asp
            515              520              525

Ala Asp Ser Tyr Glu Asn Met Asp Asn Pro Asp Gly Pro Asp Pro Ala
    530                535              540

Trp Gly Gly Gly Gly Arg Met Gly Thr Trp Ser Thr Arg
545              550              555

<210> 44
<211> 300
<212> PRT
<213> murine

<400> 44

Met Ala Asn Cys Glu Phe Ser Pro Val Ser Gly Asp Lys Pro Cys Cys
1              5                10              15

Arg Leu Ser Arg Arg Ala Gln Leu Cys Leu Gly Val Ser Ile Leu Val
            20              25              30

Leu Ile Leu Val Val Val Leu Ala Val Val Val Pro Arg Trp Arg Gln
            35              40              45

Gln Trp Ser Gly Pro Gly Thr Thr Lys Arg Phe Pro Glu Thr Val Leu
            50              55              60

```
Ala Arg Cys Val Lys Tyr Thr Glu Ile His Pro Glu Met Arg His Val
65              70              75                      80

Asp Cys Gln Ser Val Trp Asp Ala Phe Lys Gly Ala Phe Ile Ser Lys
            85              90                      95

His Pro Cys Asn Ile Thr Glu Glu Asp Tyr Gln Pro Leu Met Lys Leu
                100             105             110

Gly Thr Gln Thr Val Pro Cys Asn Lys Ile Leu Leu Trp Ser Arg Ile
        115             120             125

Lys Asp Leu Ala His Gln Phe Thr Gln Val Gln Arg Asp Met Phe Thr
    130             135             140

Leu Glu Asp Thr Leu Leu Gly Tyr Leu Ala Asp Asp Leu Thr Trp Cys
145             150             155             160

Gly Glu Phe Asn Thr Ser Lys Ile Asn Tyr Gln Ser Cys Pro Asp Trp
                165             170             175

Arg Lys Asp Cys Ser Asn Asn Pro Val Ser Val Phe Trp Lys Thr Val
            180             185             190

Ser Arg Arg Phe Ala Glu Ala Ala Cys Asp Val Val His Val Met Leu
        195             200             205

Asn Gly Ser Arg Ser Lys Ile Phe Asp Lys Asn Ser Thr Phe Gly Ser
    210             215             220

Val Glu Val His Asn Leu Gln Pro Glu Lys Val Gln Thr Leu Glu Ala
225             230             235             240

Trp Val Ile His Gly Gly Arg Glu Asp Ser Arg Asp Leu Cys Gln Asp
            245             250             255

Pro Thr Ile Lys Glu Leu Glu Ser Ile Ile Ser Lys Arg Asn Ile Gln
        260             265             270

Phe Ser Cys Lys Asn Ile Tyr Arg Pro Asp Lys Phe Leu Gln Cys Val
    275             280             285

Lys Asn Pro Glu Asp Ser Ser Cys Thr Ser Glu Ile
    290             295             300
```

<210> 45
<211> 378

<212> PRT
<213> murine

<400> 45

Met Val Leu Leu Trp Leu Thr Leu Leu Leu Ile Ala Leu Pro Cys Leu
1                 5                 10                15

Leu Gln Thr Lys Glu Asp Pro Asn Pro Pro Ile Thr Asn Leu Arg Met
            20                25                30

Lys Ala Lys Ala Gln Gln Leu Thr Trp Asp Leu Asn Arg Asn Val Thr
            35                40                45

Asp Ile Glu Cys Val Lys Asp Ala Asp Tyr Ser Met Pro Ala Val Asn
    50                55                60

Asn Ser Tyr Cys Gln Phe Gly Ala Ile Ser Leu Cys Glu Val Thr Asn
65                70                75                80

Tyr Thr Val Arg Val Ala Asn Pro Pro Phe Ser Thr Trp Ile Leu Phe
            85                90                95

Pro Glu Asn Ser Gly Lys Pro Trp Ala Gly Ala Glu Asn Leu Thr Cys
            100               105               110

Trp Ile His Asp Val Asp Phe Leu Ser Cys Ser Trp Ala Val Gly Pro
        115               120               125

Gly Ala Pro Ala Asp Val Gln Tyr Asp Leu Tyr Leu Asn Val Ala Asn
    130               135               140

Arg Arg Gln Gln Tyr Glu Cys Leu His Tyr Lys Thr Asp Ala Gln Gly
145               150               155               160

Thr Arg Ile Gly Cys Arg Phe Asp Asp Ile Ser Arg Leu Ser Ser Gly
            165               170               175

Ser Gln Ser Ser His Ile Leu Val Arg Gly Arg Ser Ala Ala Phe Gly
            180               185               190

Ile Pro Cys Thr Asp Lys Phe Val Val Phe Ser Gln Ile Glu Ile Leu
        195               200               205

Thr Pro Pro Asn Met Thr Ala Lys Cys Asn Lys Thr His Ser Phe Met
    210               215               220

His Trp Lys Met Arg Ser His Phe Asn Arg Lys Phe Arg Tyr Glu Leu
225               230               235               240

```
        Gln Ile Gln Lys Arg Met Gln Pro Val Ile Thr Glu Gln Val Arg Asp
                    245             250                 255

        Arg Thr Ser Phe Gln Leu Leu Asn Pro Gly Thr Tyr Thr Val Gln Ile
                    260             265                 270

        Arg Ala Arg Glu Arg Val Tyr Glu Phe Leu Ser Ala Trp Ser Thr Pro
                    275             280                 285

        Gln Arg Phe Glu Cys Asp Gln Glu Glu Gly Ala Asn Thr Arg Ala Trp
                    290             295                 300

        Arg Thr Ser Leu Leu Ile Ala Leu Gly Thr Leu Leu Ala Leu Val Cys
        305                 310                 315                 320

        Val Phe Val Ile Cys Arg Arg Tyr Leu Val Met Gln Arg Leu Phe Pro
                    325             330                 335

        Arg Ile Pro His Met Lys Asp Pro Ile Gly Asp Ser Phe Gln Asn Asp
                    340             345                 350

        Lys Leu Val Val Trp Glu Ala Gly Lys Ala Gly Leu Glu Glu Cys Leu
                    355             360                 365

        Val Thr Glu Val Gln Val Val Gln Lys Thr
                    370             375
```

<210> 46
<211> 335
<212> PRT
<213> murine

<400> 46

```
        Met Ala Gly Ser Pro Thr Cys Leu Thr Leu Ile Tyr Ile Leu Trp Gln
        1               5                   10                  15

        Leu Thr Gly Ser Ala Ala Ser Gly Pro Val Lys Glu Leu Val Gly Ser
                    20              25                  30

        Val Gly Gly Ala Val Thr Phe Pro Leu Lys Ser Lys Val Lys Gln Val
                    35              40                  45

        Asp Ser Ile Val Trp Thr Phe Asn Thr Thr Pro Leu Val Thr Ile Gln
            50              55                  60

        Pro Glu Gly Gly Thr Ile Ile Val Thr Gln Asn Arg Asn Arg Glu Arg
        65              70                  75                  80
```

```
Val Asp Phe Pro Asp Gly Gly Tyr Ser Leu Lys Leu Ser Lys Leu Lys
            85              90              95

Lys Asn Asp Ser Gly Ile Tyr Tyr Val Gly Ile Tyr Ser Ser Ser Leu
            100             105             110

Gln Gln Pro Ser Thr Gln Glu Tyr Val Leu His Val Tyr Glu His Leu
            115             120             125

Ser Lys Pro Lys Val Thr Met Gly Leu Gln Ser Asn Lys Asn Gly Thr
    130             135             140

Cys Val Thr Asn Leu Thr Cys Cys Met Glu His Gly Glu Glu Asp Val
145             150             155             160

Ile Tyr Thr Trp Lys Ala Leu Gly Gln Ala Ala Asn Glu Ser His Asn
            165             170             175

Gly Ser Ile Leu Pro Ile Ser Trp Arg Trp Gly Glu Ser Asp Met Thr
            180             185             190

Phe Ile Cys Val Ala Arg Asn Pro Val Ser Arg Asn Phe Ser Ser Pro
            195             200             205

Ile Leu Ala Arg Lys Leu Cys Glu Gly Ala Ala Asp Asp Pro Asp Ser
    210             215             220

Ser Met Val Leu Leu Cys Leu Leu Leu Val Pro Leu Leu Leu Ser Leu
225             230             235             240

Phe Val Leu Gly Leu Phe Leu Trp Phe Leu Lys Arg Glu Arg Gln Glu
            245             250             255

Glu Tyr Ile Glu Glu Lys Lys Arg Val Asp Ile Cys Arg Glu Thr Pro
            260             265             270

Asn Ile Cys Pro His Ser Gly Glu Asn Thr Glu Tyr Asp Thr Ile Pro
            275             280             285

His Thr Asn Arg Thr Ile Leu Lys Glu Asp Pro Ala Asn Thr Val Tyr
    290             295             300

Ser Thr Val Glu Ile Pro Lys Lys Met Glu Asn Pro His Ser Leu Leu
305             310             315             320

Thr Met Pro Asp Thr Pro Arg Leu Phe Ala Tyr Glu Asn Val Ile
            325             330             335
```

<210> 47
<211> 184
<212> PRT
<213> murine

<400> 47

```
Met Leu Gln Met Ala Gly Gln Cys Ser Gln Asn Glu Tyr Phe Asp Ser
1               5               10              15

Leu Leu His Ala Cys Ile Pro Cys Gln Leu Arg Cys Ser Ser Asn Thr
            20              25              30

Pro Pro Leu Thr Cys Gln Arg Tyr Cys Asn Ala Ser Val Thr Asn Ser
            35              40              45

Val Lys Gly Thr Asn Ala Ile Leu Trp Thr Cys Leu Gly Leu Ser Leu
        50              55              60

Ile Ile Ser Leu Ala Val Phe Val Leu Met Phe Leu Leu Arg Lys Ile
65              70              75              80

Asn Ser Glu Pro Leu Lys Asp Glu Phe Lys Asn Thr Gly Ser Gly Leu
                85              90              95

Leu Gly Met Ala Asn Ile Asp Leu Glu Lys Ser Arg Thr Gly Asp Glu
            100             105             110

Ile Ile Leu Pro Arg Gly Leu Glu Tyr Thr Val Glu Glu Cys Thr Cys
            115             120             125

Glu Asp Cys Ile Lys Ser Lys Pro Lys Val Asp Ser Asp His Cys Phe
        130             135             140

Pro Leu Pro Ala Met Glu Glu Gly Ala Thr Ile Leu Val Thr Thr Lys
145             150             155             160

Thr Asn Asp Tyr Cys Lys Ser Leu Pro Ala Ala Leu Ser Ala Thr Glu
                165             170             175

Ile Glu Lys Ser Ile Ser Ala Arg
            180
```

<210> 48
<211> 993
<212> PRT
<213> murine

<400> 48

```
Met Pro Ala Leu Ala Arg Asp Gly Gly Gln Leu Pro Leu Leu Val Val
1               5               10              15

Phe Ser Ala Met Ile Phe Gly Thr Ile Thr Asn Gln Asp Leu Pro Val
            20              25              30

Ile Lys Cys Val Leu Ile Asn His Lys Asn Asn Asp Ser Ser Val Gly
            35              40              45

Lys Ser Ser Ser Tyr Pro Met Val Ser Glu Ser Pro Glu Asp Leu Gly
        50              55              60

Cys Ala Leu Arg Pro Gln Ser Ser Gly Thr Val Tyr Glu Ala Ala Ala
65              70              75              80

Val Glu Val Asp Val Ser Ala Ser Ile Thr Leu Gln Val Leu Val Asp
                85              90              95

Ala Pro Gly Asn Ile Ser Cys Leu Trp Val Phe Lys His Ser Ser Leu
            100             105             110

Asn Cys Gln Pro His Phe Asp Leu Gln Asn Arg Gly Val Val Ser Met
            115             120             125

Val Ile Leu Lys Met Thr Glu Thr Gln Ala Gly Glu Tyr Leu Leu Phe
    130             135             140

Ile Gln Ser Glu Ala Thr Asn Tyr Thr Ile Leu Phe Thr Val Ser Ile
145             150             155             160

Arg Asn Thr Leu Leu Tyr Thr Leu Arg Arg Pro Tyr Phe Arg Lys Met
                165             170             175

Glu Asn Gln Asp Ala Leu Val Cys Ile Ser Glu Ser Val Pro Glu Pro
            180             185             190

Ile Val Glu Trp Val Leu Cys Asp Ser Gln Gly Glu Ser Cys Lys Glu
        195             200             205

Glu Ser Pro Ala Val Val Lys Lys Glu Glu Lys Val Leu His Glu Leu
        210             215             220

Phe Gly Thr Asp Ile Arg Cys Cys Ala Arg Asn Glu Leu Gly Arg Glu
225             230             235             240

Cys Thr Arg Leu Phe Thr Ile Asp Leu Asn Gln Thr Pro Gln Thr Thr
            245             250             255
```

```
Leu Pro Gln Leu Phe Leu Lys Val Gly Glu Pro Leu Trp Ile Arg Cys
        260                 265                 270

Lys Ala Val His Val Asn His Gly Phe Gly Leu Thr Trp Glu Leu Glu
        275                 280                 285

Asn Lys Ala Leu Glu Glu Gly Asn Tyr Phe Glu Met Ser Thr Tyr Ser
        290                 295                 300

Thr Asn Arg Thr Met Ile Arg Ile Leu Phe Ala Phe Val Ser Ser Val
305                 310                 315                 320

Ala Arg Asn Asp Thr Gly Tyr Tyr Thr Cys Ser Ser Ser Lys His Pro
                325                 330                 335

Ser Gln Ser Ala Leu Val Thr Ile Val Glu Lys Gly Phe Ile Asn Ala
                340                 345                 350

Thr Asn Ser Ser Glu Asp Tyr Glu Ile Asp Gln Tyr Glu Glu Phe Cys
                355                 360                 365

Phe Ser Val Arg Phe Lys Ala Tyr Pro Gln Ile Arg Cys Thr Trp Thr
        370                 375                 380

Phe Ser Arg Lys Ser Phe Pro Cys Glu Gln Lys Gly Leu Asp Asn Gly
385                 390                 395                 400

Tyr Ser Ile Ser Lys Phe Cys Asn His Lys His Gln Pro Gly Glu Tyr
                405                 410                 415

Ile Phe His Ala Glu Asn Asp Asp Ala Gln Phe Thr Lys Met Phe Thr
                420                 425                 430

Leu Asn Ile Arg Arg Lys Pro Gln Val Leu Ala Glu Ala Ser Ala Ser
        435                 440                 445

Gln Ala Ser Cys Phe Ser Asp Gly Tyr Pro Leu Pro Ser Trp Thr Trp
        450                 455                 460

Lys Lys Cys Ser Asp Lys Ser Pro Asn Cys Thr Glu Glu Ile Thr Glu
465                 470                 475                 480

Gly Val Trp Asn Arg Lys Ala Asn Arg Lys Val Phe Gly Gln Trp Val
                485                 490                 495

Ser Ser Ser Thr Leu Asn Met Ser Glu Ala Ile Lys Gly Phe Leu Val
```

500    505    510

Lys Cys Cys Ala Tyr Asn Ser Leu Gly Thr Ser Cys Glu Thr Ile Leu
  515    520    525

Leu Asn Ser Pro Gly Pro Phe Pro Phe Ile Gln Asp Asn Ile Ser Phe
  530    535    540

Tyr Ala Thr Ile Gly Val Cys Leu Leu Phe Ile Val Val Leu Thr Leu
545    550    555    560

Leu Ile Cys His Lys Tyr Lys Lys Gln Phe Arg Tyr Glu Ser Gln Leu
  565    570    575

Gln Met Val Gln Val Thr Gly Ser Ser Asp Asn Glu Tyr Phe Tyr Val
  580    585    590

Asp Phe Arg Glu Tyr Glu Tyr Asp Leu Lys Trp Glu Phe Pro Arg Glu
  595    600    605

Asn Leu Glu Phe Gly Lys Val Leu Gly Ser Gly Ala Phe Gly Lys Val
  610    615    620

Met Asn Ala Thr Ala Tyr Gly Ile Ser Lys Thr Gly Val Ser Ile Gln
625    630    635    640

Val Ala Val Lys Met Leu Lys Glu Lys Ala Asp Ser Ser Glu Arg Glu
  645    650    655

Ala Leu Met Ser Glu Leu Lys Met Met Thr Gln Leu Gly Ser His Glu
  660    665    670

Asn Ile Val Asn Leu Leu Gly Ala Cys Thr Leu Ser Gly Pro Ile Tyr
  675    680    685

Leu Ile Phe Glu Tyr Cys Cys Tyr Gly Asp Leu Leu Asn Tyr Leu Arg
  690    695    700

Ser Lys Arg Glu Lys Phe His Arg Thr Trp Thr Glu Ile Phe Lys Glu
705    710    715    720

His Asn Phe Ser Phe Tyr Pro Thr Phe Gln Ser His Pro Asn Ser Ser
  725    730    735

Met Pro Gly Ser Arg Glu Val Gln Ile His Pro Asp Ser Asp Gln Ile
  740    745    750

```
Ser Gly Leu His Gly Asn Ser Phe His Ser Glu Asp Glu Ile Glu Tyr
        755             760             765

Glu Asn Gln Lys Arg Leu Glu Glu Glu Asp Leu Asn Val Leu Thr
        770             775             780

Phe Glu Asp Leu Leu Cys Phe Ala Tyr Gln Val Ala Lys Gly Met Glu
785             790             795             800

Phe Leu Glu Phe Lys Ser Cys Val His Arg Asp Leu Ala Ala Arg Asn
            805             810             815

Val Leu Val Thr His Gly Lys Val Val Lys Ile Cys Asp Phe Gly Leu
            820             825             830

Ala Arg Asp Ile Met Ser Asp Ser Asn Tyr Val Val Arg Gly Asn Ala
            835             840             845

Arg Leu Pro Val Lys Trp Met Ala Pro Glu Ser Leu Phe Glu Gly Ile
    850             855             860

Tyr Thr Ile Lys Ser Asp Val Trp Ser Tyr Gly Ile Leu Leu Trp Glu
865             870             875             880

Ile Phe Ser Leu Gly Val Asn Pro Tyr Pro Gly Ile Pro Val Asp Ala
            885             890             895

Asn Phe Tyr Lys Leu Ile Gln Asn Gly Phe Lys Met Asp Gln Pro Phe
        900             905             910

Tyr Ala Thr Glu Glu Ile Tyr Ile Ile Met Gln Ser Cys Trp Ala Phe
        915             920             925

Asp Ser Arg Lys Arg Pro Ser Phe Pro Asn Leu Thr Ser Phe Leu Gly
    930             935             940

Cys Gln Leu Ala Asp Ala Glu Glu Ala Met Tyr Gln Asn Val Asp Gly
945             950             955             960

Arg Val Ser Glu Cys Pro His Thr Tyr Gln Asn Arg Arg Pro Phe Ser
            965             970             975

Arg Glu Met Asp Leu Gly Leu Leu Ser Pro Gln Ala Gln Val Glu Asp
        980             985             990

Ser
```

<210> 49
<211> 364
<212> PRT
<213> murine

<400> 49

```
Met Pro Leu Leu Leu Leu Leu Pro Leu Leu Trp Ala Gly Ala Leu Ala
1               5                   10                  15

Met Asp Pro Asn Phe Trp Leu Gln Val Gln Glu Ser Val Thr Val Gln
            20                  25                  30

Glu Gly Leu Cys Val Leu Val Pro Cys Thr Phe Phe His Pro Ile Pro
            35                  40                  45

Tyr Tyr Asp Lys Asn Ser Pro Val His Gly Tyr Trp Phe Arg Glu Gly
        50                  55                  60

Ala Ile Ile Ser Arg Asp Ser Pro Val Ala Thr Asn Lys Leu Asp Gln
65                  70                  75                  80

Glu Val Gln Glu Glu Thr Gln Gly Arg Phe Arg Leu Leu Gly Asp Pro
                85                  90                  95

Ser Arg Asn Asn Cys Ser Leu Ser Ile Val Asp Ala Arg Arg Arg Asp
            100                 105                 110

Asn Gly Ser Tyr Phe Phe Arg Met Glu Arg Gly Ser Thr Lys Tyr Ser
            115                 120                 125

Tyr Lys Ser Pro Gln Leu Ser Val His Val Thr Asp Leu Thr His Arg
        130                 135                 140

Pro Lys Ile Leu Ile Pro Gly Thr Leu Glu Pro Gly His Ser Lys Asn
145                 150                 155                 160

Leu Thr Cys Ser Val Ser Trp Ala Cys Glu Gln Gly Thr Pro Pro Ile
            165                 170                 175

Phe Ser Trp Leu Ser Ala Ala Pro Thr Ser Leu Gly Pro Arg Thr Thr
            180                 185                 190

His Ser Ser Val Leu Ile Ile Thr Pro Arg Pro Gln Asp His Gly Thr
            195                 200                 205

Asn Leu Thr Cys Gln Val Lys Phe Ala Gly Ala Gly Val Thr Thr Glu
            210                 215                 220
```

```
Arg Thr Ile Gln Leu Asn Val Thr Tyr Val Pro Gln Asn Pro Thr Thr
225             230             235             240

Gly Ile Phe Pro Gly Asp Gly Ser Gly Lys Gln Glu Thr Arg Ala Gly
            245             250             255

Val Val His Gly Ala Ile Gly Gly Ala Gly Val Thr Ala Leu Leu Ala
            260             265             270

Leu Cys Leu Cys Leu Ile Phe Phe Ile Val Lys Thr His Arg Arg Lys
        275             280             285

Ala Ala Arg Thr Ala Val Gly Arg Asn Asp Thr His Pro Thr Thr Gly
        290             295             300

Ser Ala Ser Pro Lys His Gln Lys Lys Ser Lys Leu His Gly Pro Thr
305             310             315             320

Glu Thr Ser Ser Cys Ser Gly Ala Ala Pro Thr Val Glu Met Asp Glu
            325             330             335

Glu Leu His Tyr Ala Ser Leu Asn Phe His Gly Met Asn Pro Ser Lys
            340             345             350

Asp Thr Ser Thr Glu Tyr Ser Glu Val Arg Thr Gln
            355             360
```

<210> 50
<211> 193
<212> PRT
<213> murine

<400> 50

```
Met Pro Glu Glu Gly Ser Gly Cys Ser Val Arg Arg Arg Pro Tyr Gly
1               5               10              15

Cys Val Leu Arg Ala Ala Leu Val Pro Leu Val Ala Gly Leu Val Ile
        20              25              30

Cys Leu Val Val Cys Ile Gln Arg Phe Ala Gln Ala Gln Gln Gln Leu
        35              40              45

Pro Leu Glu Ser Leu Gly Trp Asp Val Ala Glu Leu Gln Leu Asn His
    50              55              60

Thr Gly Pro Gln Gln Asp Pro Arg Leu Tyr Trp Gln Gly Gly Pro Ala
65              70              75              80
```

```
Leu Gly Arg Ser Phe Leu His Gly Pro Glu Leu Asp Lys Gly Gln Leu
                85                  90                  95

Arg Ile His Arg Asp Gly Ile Tyr Met Val His Ile Gln Val Thr Leu
        100                 105                 110

Ala Ile Cys Ser Ser Thr Thr Ala Ser Arg His His Pro Thr Thr Leu
        115                 120                 125

Ala Val Gly Ile Cys Ser Pro Ala Ser Arg Ser Ile Ser Leu Leu Arg
    130                 135                 140

Leu Ser Phe His Gln Gly Cys Thr Ile Ala Ser Gln Arg Leu Thr Pro
145                 150                 155                 160

Leu Ala Arg Gly Asp Thr Leu Cys Thr Asn Leu Thr Gly Thr Leu Leu
                165                 170                 175

Pro Ser Arg Asn Thr Asp Glu Thr Phe Phe Gly Val Gln Trp Val Arg
                180                 185                 190

Pro
```

<210> 51
<211> 943
<212> PRT
<213> murine

<400> 51

```
Met Arg Pro Ser Gly Thr Ala Gly Ala Ala Leu Leu Ala Leu Leu Ala
1               5                   10                  15

Ala Leu Cys Pro Ala Ser Arg Ala Leu Glu Glu Lys Lys Gly Asn Tyr
            20                  25                  30

Val Val Thr Asp His Gly Ser Cys Val Arg Ala Cys Gly Ala Asp Ser
            35                  40                  45

Tyr Glu Met Glu Glu Asp Gly Val Arg Lys Cys Lys Lys Cys Glu Gly
    50                  55                  60

Pro Cys Arg Lys Val Cys Asn Gly Ile Gly Ile Gly Glu Phe Lys Asp
65                  70                  75                  80

Ser Leu Ser Ile Asn Ala Thr Asn Ile Lys His Phe Lys Asn Cys Thr
                85                  90                  95
```

```
Ser Ile Ser Gly Asp Leu His Ile Leu Pro Val Ala Phe Arg Gly Asp
            100                 105                 110

Ser Phe Thr His Thr Pro Pro Leu Asp Pro Gln Glu Leu Asp Ile Leu
            115                 120                 125

Lys Thr Val Lys Glu Ile Thr Gly Phe Leu Leu Ile Gln Ala Trp Pro
            130                 135                 140

Glu Asn Arg Thr Asp Leu His Ala Phe Glu Asn Leu Glu Ile Ile Arg
145                 150                 155                 160

Gly Arg Thr Lys Gln His Gly Gln Phe Ser Leu Ala Val Val Ser Leu
                165                 170                 175

Asn Ile Thr Ser Leu Gly Leu Arg Ser Leu Lys Glu Ile Ser Asp Gly
            180                 185                 190

Asp Val Ile Ile Ser Gly Asn Lys Asn Leu Cys Tyr Ala Asn Thr Ile
            195                 200                 205

Asn Trp Lys Lys Leu Phe Gly Thr Ser Gly Gln Lys Thr Lys Ile Ile
            210                 215                 220

Ser Asn Arg Gly Glu Asn Ser Cys Lys Ala Thr Gly Gln Val Cys His
225                 230                 235                 240

Ala Leu Cys Ser Pro Glu Gly Cys Trp Gly Pro Glu Pro Arg Asp Cys
                245                 250                 255

Val Ser Cys Arg Asn Val Ser Arg Gly Arg Glu Cys Val Asp Lys Cys
                260                 265                 270

Asn Leu Leu Glu Gly Glu Pro Arg Glu Phe Val Glu Asn Ser Glu Cys
            275                 280                 285

Ile Gln Cys His Pro Glu Cys Leu Pro Gln Ala Met Asn Ile Thr Cys
            290                 295                 300

Thr Gly Arg Gly Pro Asp Asn Cys Ile Gln Cys Ala His Tyr Ile Asp
305                 310                 315                 320

Gly Pro His Cys Val Lys Thr Cys Pro Ala Gly Val Met Gly Glu Asn
                325                 330                 335

Asn Thr Leu Val Trp Lys Tyr Ala Asp Ala Gly His Val Cys His Leu
            340                 345                 350
```

```
Cys His Pro Asn Cys Thr Tyr Gly Cys Thr Gly Pro Gly Leu Glu Gly
        355                 360             365

Cys Pro Thr Asn Gly Pro Lys Ile Pro Ser Ile Ala Thr Gly Met Val
        370                 375             380

Gly Ala Leu Leu Leu Leu Leu Val Val Ala Leu Gly Ile Gly Leu Phe
385                 390             395                 400

Met Arg Arg Arg His Ile Val Arg Lys Arg Thr Leu Arg Arg Leu Leu
                405             410                 415

Gln Glu Arg Glu Leu Val Glu Pro Leu Thr Pro Ser Gly Glu Ala Pro
            420             425             430

Asn Gln Ala Leu Leu Arg Ile Leu Lys Glu Thr Glu Phe Lys Lys Ile
        435             440             445

Lys Val Leu Gly Ser Gly Ala Phe Gly Thr Val Tyr Lys Gly Leu Trp
    450             455             460

Ile Pro Glu Gly Glu Lys Val Lys Ile Pro Val Ala Ile Lys Glu Leu
465             470             475             480

Arg Glu Ala Thr Ser Pro Lys Ala Asn Lys Glu Ile Leu Asp Glu Ala
            485             490             495

Tyr Val Met Ala Ser Val Asp Asn Pro His Val Cys Arg Leu Leu Gly
        500             505             510

Ile Cys Leu Thr Ser Thr Val Gln Leu Ile Thr Gln Leu Met Pro Phe
    515             520             525

Gly Cys Leu Leu Asp Tyr Val Arg Glu His Lys Asp Asn Ile Gly Ser
    530             535             540

Gln Tyr Leu Leu Asn Trp Cys Val Gln Ile Ala Lys Gly Met Asn Tyr
545             550             555             560

Leu Glu Asp Arg Arg Leu Val His Arg Asp Leu Ala Ala Arg Asn Val
            565             570             575

Leu Val Lys Thr Pro Gln His Val Lys Ile Thr Asp Phe Gly Leu Ala
        580             585             590

Lys Leu Leu Gly Ala Glu Glu Lys Glu Tyr His Ala Glu Gly Gly Lys
```

595                    600                    605

Val Pro Ile Lys Trp Met Ala Leu Glu Ser Ile Leu His Arg Ile Tyr
    610             615             620

Thr His Gln Ser Asp Val Trp Ser Tyr Gly Val Thr Val Trp Glu Leu
625             630             635             640

Met Thr Phe Gly Ser Lys Pro Tyr Asp Gly Ile Pro Ala Ser Glu Ile
            645             650             655

Ser Ser Ile Leu Glu Lys Gly Glu Arg Leu Pro Gln Pro Pro Ile Cys
            660             665             670

Thr Ile Asp Val Tyr Met Ile Met Val Lys Cys Trp Met Ile Asp Ala
            675             680             685

Asp Ser Arg Pro Lys Phe Arg Glu Leu Ile Ile Glu Phe Ser Lys Met
    690             695             700

Ala Arg Asp Pro Gln Arg Tyr Leu Val Ile Gln Gly Asp Glu Arg Met
705             710             715             720

His Leu Pro Ser Pro Thr Asp Ser Asn Phe Tyr Arg Ala Leu Met Asp
            725             730             735

Glu Glu Asp Met Asp Asp Val Val Asp Ala Asp Glu Tyr Leu Ile Pro
            740             745             750

Gln Gln Gly Phe Phe Ser Ser Pro Ser Thr Ser Arg Thr Pro Leu Leu
            755             760             765

Ser Ser Leu Ser Ala Thr Ser Asn Asn Ser Thr Val Ala Cys Ile Asp
    770             775             780

Arg Asn Gly Leu Gln Ser Cys Pro Ile Lys Glu Asp Ser Phe Leu Gln
785             790             795             800

Arg Tyr Ser Ser Asp Pro Thr Gly Ala Leu Thr Glu Asp Ser Ile Asp
            805             810             815

Asp Thr Phe Leu Pro Val Pro Glu Tyr Ile Asn Gln Ser Val Pro Lys
    820             825             830

Arg Pro Ala Gly Ser Val Gln Asn Pro Val Tyr His Asn Gln Pro Leu
    835             840             845

```
Asn Pro Ala Pro Ser Arg Asp Pro His Tyr Gln Asp Pro His Ser Thr
    850             855         860

Ala Val Gly Asn Pro Glu Tyr Leu Asn Thr Val Gln Pro Thr Cys Val
865             870             875                 880

Asn Ser Thr Phe Asp Ser Pro Ala His Trp Ala Gln Lys Gly Ser His
            885             890             895

Gln Ile Ser Leu Asp Asn Pro Asp Tyr Gln Gln Asp Phe Phe Pro Lys
        900             905             910

Glu Ala Lys Pro Asn Gly Ile Phe Lys Gly Ser Thr Ala Glu Asn Ala
        915             920             925

Glu Tyr Leu Arg Val Ala Pro Gln Ser Ser Glu Phe Ile Gly Ala
    930             935             940
```

<210> 52
<211> 497
<212> PRT
<213> murine

<400> 52

```
Met Gln Asp Pro Ala Ser Thr Cys Val Pro Glu Pro Ala Ser Gln His
1               5               10              15

Thr Leu Arg Ser Gly Pro Gly Cys Leu Gln Gln Pro Glu Gln Gln Gly
        20              25              30

Val Arg Asp Pro Gly Gly Ile Trp Ala Lys Leu Gly Ala Ala Glu Ala
        35              40              45

Ser Ala Glu Arg Leu Gln Gly Arg Arg Ser Arg Gly Ala Ser Gly Ser
    50              55              60

Glu Pro Gln Gln Met Gly Ser Asp Val Arg Asp Leu Asn Ala Leu Leu
65              70              75                  80

Pro Ala Val Pro Ser Leu Gly Gly Gly Gly Gly Cys Ala Leu Pro Val
            85              90              95

Ser Gly Ala Ala Gln Trp Ala Pro Val Leu Asp Phe Ala Pro Pro Gly
        100             105             110

Ala Ser Ala Tyr Gly Ser Leu Gly Gly Pro Ala Pro Pro Pro Ala Pro
        115             120             125
```

```
Pro Pro Pro Pro Pro Pro Pro Pro His Ser Phe Ile Lys Gln Glu Pro
    130                 135             140

Ser Trp Gly Gly Ala Glu Pro His Glu Glu Gln Cys Leu Ser Ala Phe
145             150             155                 160

Thr Val His Phe Ser Gly Gln Phe Thr Gly Thr Ala Gly Ala Cys Arg
                165             170                 175

Tyr Gly Pro Phe Gly Pro Pro Pro Ser Gln Ala Ser Ser Gly Gln
            180             185             190

Ala Arg Met Phe Pro Asn Ala Pro Tyr Leu Pro Ser Cys Leu Glu Ser
        195             200             205

Gln Pro Ala Ile Arg Asn Gln Gly Tyr Ser Thr Val Thr Phe Asp Gly
    210             215             220

Thr Pro Ser Tyr Gly His Thr Pro Ser His His Ala Ala Gln Phe Pro
225             230             235                 240

Asn His Ser Phe Lys His Glu Asp Pro Met Gly Gln Gln Gly Ser Leu
            245             250             255

Gly Glu Gln Gln Tyr Ser Val Pro Pro Pro Val Tyr Gly Cys His Thr
        260             265             270

Pro Thr Asp Ser Cys Thr Gly Ser Gln Ala Leu Leu Leu Arg Thr Pro
        275             280             285

Tyr Ser Ser Asp Asn Leu Tyr Gln Met Thr Ser Gln Leu Glu Cys Met
    290             295             300

Thr Trp Asn Gln Met Asn Leu Gly Ala Thr Leu Lys Gly His Ser Thr
305             310             315                 320

Gly Tyr Glu Ser Asp Asn His Thr Thr Pro Ile Leu Cys Gly Ala Gln
            325             330             335

Tyr Arg Ile His Thr His Gly Val Phe Arg Gly Ile Gln Asp Val Arg
        340             345             350

Arg Val Pro Gly Val Ala Pro Thr Leu Val Arg Ser Ala Ser Glu Thr
        355             360             365

Ser Glu Lys Arg Pro Phe Met Cys Ala Tyr Pro Gly Cys Asn Lys Arg
370             375             380
```

```
Tyr Phe Lys Leu Ser His Leu Gln Met His Ser Arg Lys His Thr Gly
385                 390             395                     400

Glu Lys Pro Tyr Gln Cys Asp Phe Lys Asp Cys Glu Arg Arg Phe Ser
                405             410                     415

Arg Ser Asp Gln Leu Lys Arg His Gln Arg Arg His Thr Gly Val Lys
                420             425             430

Pro Phe Gln Cys Lys Thr Cys Gln Arg Lys Phe Ser Arg Ser Asp His
                435             440             445

Leu Lys Thr His Thr Arg Thr His Thr Gly Glu Lys Pro Phe Ser Cys
        450             455             460

Arg Trp Pro Ser Cys Gln Lys Lys Phe Ala Arg Ser Asp Glu Leu Val
465             470             475                     480

Arg His His Asn Met His Gln Arg Asn Met Thr Lys Leu Gln Leu Ala
                485             490             495

Leu
```

<210> 53
<211> 118
<212> PRT
<213> artificial sequence

<220>
<223> CD19-1 VH chain

<400> 53

```
Glu Val Lys Leu Gln Glu Ser Gly Pro Gly Leu Val Ala Pro Ser Gln
1               5               10                      15

Ser Leu Ser Val Thr Cys Thr Val Ser Gly Val Ser Leu Pro Asp Tyr
                20              25                      30

Gly Val Ser Trp Ile Arg Gln Pro Pro Arg Lys Gly Leu Glu Trp Leu
                35              40              45

Gly Val Ile Trp Gly Ser Glu Thr Thr Tyr Tyr Asn Ser Ala Leu Lys
        50              55              60

Ser Arg Leu Thr Ile Ile Lys Asp Asn Ser Lys Ser Gln Val Phe Leu
65              70              75                      80
```

```
Lys Met Asn Ser Leu Gln Thr Asp Asp Thr Ala Ile Tyr Tyr Cys Ala
                85                  90                  95

Lys His Tyr Tyr Tyr Gly Gly Ser Tyr Ala Met Asp Tyr Trp Gly Gln
            100                 105             110

Gly Thr Ser Val Thr Val
            115
```

<210> 54
<211> 107
<212> PRT
<213> artificial sequence

<220>
<223> CD19-1 VL chain

<400> 54

```
Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Ser Lys Tyr
            20                  25                  30

Leu Asn Trp Tyr Gln Gln Lys Pro Asp Gly Thr Val Lys Leu Leu Ile
            35                  40                  45

Tyr His Thr Ser Arg Leu His Ser Gly Val Pro Ser Arg Phe Ser Gly
        50                  55                  60

Ser Gly Ser Gly Thr Asp Tyr Ser Leu Thr Ile Ser Asn Leu Glu Gln
65                  70                  75                  80

Glu Asp Ile Ala Thr Tyr Phe Cys Gln Gln Gly Asn Thr Leu Pro Tyr
                85                  90                  95

Thr Phe Gly Gly Gly Thr Lys Leu Glu Ile Thr
            100                 105
```

<210> 55
<211> 119
<212> PRT
<213> artificial sequence

<220>
<223> CD19-2 VH chain

<400> 55

```
Glu Val Gln Leu Gln Gln Ser Gly Pro Glu Leu Ile Lys Pro Gly Ala
1               5                   10                  15

Ser Val Lys Met Ser Cys Lys Ala Ser Gly Tyr Thr Phe Thr Ser Tyr
            20                  25                  30

Val Met His Trp Val Lys Gln Lys Pro Gly Gln Gly Leu Glu Trp Ile
        35                  40                  45

Gly Tyr Ile Asn Pro Tyr Asn Asp Gly Thr Lys Tyr Asn Glu Lys Phe
    50                  55                  60

Lys Gly Lys Ala Thr Leu Thr Ser Asp Lys Ser Ser Ser Thr Ala Tyr
65                  70                  75                  80

Met Glu Leu Ser Ser Leu Thr Ser Glu Asp Ser Ala Val Tyr Tyr Cys
            85                  90                  95

Ala Arg Gly Thr Tyr Tyr Tyr Gly Ser Arg Val Phe Asp Tyr Trp Gly
        100                 105                 110

Gln Gly Thr Thr Leu Thr Val
        115
```

<210> 56
<211> 112
<212> PRT
<213> artificial sequence

<220>
<223> CD19-2 VL chain

<400> 56

```
Asp Ile Val Met Thr Gln Ala Ala Pro Ser Ile Pro Val Thr Pro Gly
1               5               10              15

Glu Ser Val Ser Ile Ser Cys Arg Ser Ser Lys Ser Leu Leu Asn Ser
            20              25              30

Asn Gly Asn Thr Tyr Leu Tyr Trp Phe Leu Gln Arg Pro Gly Gln Ser
        35              40              45

Pro Gln Leu Leu Ile Tyr Arg Met Ser Asn Leu Ala Ser Gly Val Pro
    50              55              60

Asp Arg Phe Ser Gly Ser Gly Ser Gly Thr Ala Phe Thr Leu Arg Ile
65              70              75              80

Ser Arg Val Glu Ala Glu Asp Val Gly Val Tyr Tyr Cys Met Gln His
        85              90              95

Leu Glu Tyr Pro Phe Thr Phe Gly Ala Gly Thr Lys Leu Glu Leu Lys
        100             105             110
```

<210> 57
<211> 115
<212> PRT
<213> murine

<400> 57

```
Gln Val Gln Leu Gln Gln Pro Gly Ala Glu Leu Val Arg Pro Gly Ala
1               5               10                  15

Ser Val Lys Leu Ser Cys Lys Ala Ser Gly Tyr Thr Phe Thr Ser Tyr
            20              25                  30

Trp Met Asn Trp Val Lys Gln Arg Pro Asp Gln Gly Leu Glu Trp Ile
        35              40                  45

Gly Arg Ile Asp Pro Tyr Asp Ser Glu Thr His Tyr Asn Gln Lys Phe
    50              55                  60

Lys Asp Lys Ala Ile Leu Thr Val Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Gly Asn Trp Asp Asp Tyr Trp Gly Gln Gly Thr Thr Leu Thr
        100                 105                 110

Val Ser Ser
        115
```

<210> 58
<211> 107
<212> PRT
<213> murine

<400> 58

```
Asp Val Gln Ile Thr Gln Ser Pro Ser Tyr Leu Ala Ala Ser Pro Gly
1               5               10                  15

Glu Thr Ile Thr Ile Asn Cys Arg Ala Ser Lys Ser Ile Ser Lys Asp
            20              25                  30

Leu Ala Trp Tyr Gln Glu Lys Pro Gly Lys Thr Asn Lys Leu Leu Ile
        35              40                  45

Tyr Ser Gly Ser Thr Leu Gln Ser Gly Ile Pro Ser Arg Phe Ser Gly
```

Ser Gly Ser Gly Thr Asp Phe Thr Leu Thr Ile Ser Ser Leu Glu Pro
65                    70                    75                    80

Glu Asp Phe Ala Met Tyr Tyr Cys Gln Gln His Asn Lys Tyr Pro Tyr
                85                    90                    95

Thr Phe Gly Gly Gly Thr Lys Leu Glu Ile Lys
            100                   105

<210> 59
<211> 118
<212> PRT
<213> murine

<400> 59

Gln Ile Gln Leu Val Gln Ser Gly Pro Glu Leu Lys Lys Pro Gly Glu
1                   5                   10                    15

Thr Val Lys Ile Ser Cys Lys Ala Ser Gly Tyr Ile Phe Thr Asn Tyr
            20                    25                    30

Gly Met Asn Trp Val Lys Gln Ala Pro Gly Lys Ser Phe Lys Trp Met
            35                    40                    45

Gly Trp Ile Asn Thr Tyr Thr Gly Glu Ser Thr Tyr Ser Ala Asp Phe
        50                    55                    60

Lys Gly Arg Phe Ala Phe Ser Leu Glu Thr Ser Ala Ser Thr Ala Tyr
65                    70                    75                    80

Leu His Ile Asn Asp Leu Lys Asn Glu Asp Thr Ala Thr Tyr Phe Cys
                85                    90                    95

Ala Arg Ser Gly Gly Tyr Asp Pro Met Asp Tyr Trp Gly Gln Gly Thr
            100                   105                   110

Ser Val Thr Val Ser Ser
            115

<210> 60
<211> 111
<212> PRT
<213> murine

<400> 60

```
Asp Ile Val Leu Thr Gln Ser Pro Ala Ser Leu Ala Val Ser Leu Gly
1               5               10              15

Gln Arg Ala Thr Ile Ser Cys Arg Ala Ser Glu Ser Val Asp Asn Tyr
            20              25              30

Gly Asn Thr Phe Met His Trp Tyr Gln Gln Lys Pro Gly Gln Pro Pro
            35              40              45

Lys Leu Leu Ile Tyr Arg Ala Ser Asn Leu Glu Ser Gly Ile Pro Ala
    50              55              60

Arg Phe Ser Gly Ser Gly Ser Arg Thr Asp Phe Thr Leu Thr Ile Asn
65              70              75              80

Pro Val Glu Ala Asp Asp Val Ala Thr Tyr Tyr Cys Gln Gln Ser Asn
                85              90              95

Glu Asp Pro Pro Thr Phe Gly Ala Gly Thr Lys Leu Glu Leu Lys
            100             105             110
```

<210> 61
<211> 113
<212> PRT
<213> murine

<400> 61

```
Met Ala Asp Tyr Lys Asp Ile Val Met Thr Gln Ser His Lys Phe Met
1               5                   10                  15

Ser Thr Ser Val Gly Asp Arg Val Asn Ile Thr Cys Lys Ala Ser Gln
            20                  25                  30

Asn Val Asp Ser Ala Val Ala Trp Tyr Gln Gln Lys Pro Gly Gln Ser
            35                  40                  45

Pro Lys Ala Leu Ile Tyr Ser Ala Ser Tyr Arg Tyr Ser Gly Val Pro
    50                  55                  60

Asp Arg Phe Thr Gly Arg Gly Ser Gly Thr Asp Phe Thr Leu Thr Ile
65                  70                  75                  80

Ser Ser Val Gln Ala Glu Asp Leu Ala Val Tyr Tyr Cys Gln Gln Tyr
                85                  90                  95

Tyr Ser Thr Pro Trp Thr Phe Gly Gly Gly Thr Lys Leu Glu Ile Lys
                100                 105                 110

Arg
```

<210> 62
<211> 127
<212> PRT
<213> murine

<400> 62

EP 3 250 604 B1

Glu Val Lys Leu Val Glu Ser Gly Gly Gly Leu Val Gln Pro Gly Gly
1               5                   10                  15

Ser Leu Ser Leu Ser Cys Ala Ala Ser Gly Phe Thr Phe Thr Asp Tyr
            20                  25                  30

Tyr Met Ser Trp Val Arg Gln Pro Pro Gly Lys Ala Leu Glu Trp Leu
            35                  40                  45

Ala Leu Ile Arg Ser Lys Ala Asp Gly Tyr Thr Thr Glu Tyr Ser Ala
    50                  55                  60

Ser Val Lys Gly Arg Phe Thr Leu Ser Arg Asp Asp Ser Gln Ser Ile
65                  70                  75                  80

Leu Tyr Leu Gln Met Asn Ala Leu Arg Pro Glu Asp Ser Ala Thr Tyr
                85                  90                  95

Tyr Cys Ala Arg Asp Ala Ala Tyr Tyr Ser Tyr Tyr Ser Pro Glu Gly
            100                 105                 110

Ala Met Asp Tyr Trp Gly Gln Gly Thr Ser Val Thr Val Ser Ser
            115                 120                 125

<210> 63
<211> 119
<212> PRT
<213> murine

<400> 63

Val Gln Leu Gln Glu Ser Gly Pro Gly Leu Val Lys Pro Ser Gln Ser
1               5                   10                  15

Leu Ser Leu Thr Cys Ser Val Thr Asp Tyr Ser Ile Thr Ser Gly Tyr
            20                  25                  30

Tyr Trp Asn Trp Ile Arg Gln Phe Pro Gly Asn Lys Leu Glu Trp Met
            35                  40                  45

Gly Tyr Ile Ser Tyr Asp Gly Ser Asn Asn Tyr Asn Pro Ser Leu Lys
            50                  55                  60

155

```
Asn Arg Ile Ser Ile Thr Arg Asp Thr Ser Lys Asn Gln Phe Phe Leu
65              70              75                          80

Lys Leu Ser Ser Val Thr Thr Glu Asp Thr Ala Thr Tyr Tyr Cys Ser
                85              90                  95

Arg Gly Glu Gly Phe Tyr Phe Asp Ser Trp Gly Gln Gly Thr Thr Leu
                100             105                 110

Thr Val Ser Ser Ala Arg Ser
            115
```

<210> 64
<211> 113
<212> PRT
<213> murine

<400> 64

```
Asp Ile Met Met Ser Gln Ser Pro Ser Ser Leu Ala Val Ser Val Gly
1               5               10                  15

Glu Lys Phe Thr Met Thr Cys Lys Ser Ser Gln Ser Leu Phe Phe Gly
            20              25                  30

Ser Thr Gln Lys Asn Tyr Leu Ala Trp Tyr Gln Gln Lys Pro Gly Gln
            35              40                  45

Ser Pro Lys Leu Leu Ile Tyr Trp Ala Ser Thr Arg Glu Ser Gly Val
            50              55              60

Pro Asp Arg Phe Thr Gly Ser Gly Ser Gly Thr Asp Phe Thr Leu Ala
65              70              75                          80

Ile Ser Ser Val Met Pro Glu Asp Leu Ala Val Tyr Tyr Cys Gln Gln
                85              90                  95

Tyr Tyr Asn Tyr Pro Trp Thr Phe Gly Gly Gly Thr Lys Leu Glu Ile
            100             105                 110

Lys
```

<210> 65
<211> 115
<212> PRT
<213> murine

<400> 65
```

```
Glu Val Gln Leu Gln Gln Ser Gly Pro Glu Leu Val Lys Pro Gly Ala
1               5               10                  15

Ser Val Lys Ile Ser Cys Lys Ala Ser Gly Tyr Thr Phe Thr Asp Tyr
            20              25                  30

Asn Met His Trp Val Lys Gln Ser His Gly Lys Ser Leu Glu Trp Ile
            35              40                  45

Gly Tyr Ile Tyr Pro Tyr Asn Gly Gly Thr Gly Tyr Asn Gln Lys Phe
    50              55                  60

Lys Ser Lys Ala Thr Leu Thr Val Asp Asn Ser Ser Ser Thr Ala Tyr
65              70              75                  80

Met Asp Val Arg Ser Leu Thr Ser Glu Asp Ser Ala Val Tyr Tyr Cys
            85              90                  95

Ala Arg Gly Arg Pro Ala Met Asp Tyr Trp Gly Gln Gly Thr Ser Val
            100             105                 110

Thr Val Ser
        115
```

<210> 66
<211> 111
<212> PRT
<213> murine

<400> 66

```
Asp Ile Val Leu Thr Gln Ser Pro Ala Ser Leu Ala Val Ser Leu Gly
1               5               10                  15

Gln Arg Ala Thr Ile Ser Cys Arg Ala Ser Glu Ser Val Asp Asn Tyr
            20              25                  30

Gly Ile Ser Phe Met Asn Trp Phe Gln Gln Lys Pro Gly Gln Pro Pro
            35              40                  45

Lys Leu Leu Ile Tyr Ala Ala Ser Asn Gln Gly Ser Gly Val Pro Ala
    50              55                  60

Arg Phe Ser Gly Ser Gly Ser Gly Thr Asp Phe Ser Leu Asn Ile His
65              70              75                  80

Pro Met Glu Glu Asp Asp Thr Ala Met Tyr Phe Cys Gln Gln Ser Lys
            85              90                  95
```

Glu Val Pro Trp Thr Phe Gly Gly Gly Thr Lys Leu Glu Ile Lys
100                     105               110

<210> 67
<211> 117
<212> PRT
<213> murine

<400> 67

Gln Val Gln Leu Gln Gln Ser Gly Pro Glu Leu Val Arg Pro Gly Thr
1               5               10              15

Phe Val Lys Ile Ser Cys Lys Ala Ser Gly Tyr Thr Phe Thr Asn Tyr
            20              25              30

Asp Ile Asn Trp Val Asn Gln Arg Pro Gly Gln Gly Leu Glu Trp Ile
            35              40              45

Gly Trp Ile Tyr Pro Gly Asp Gly Ser Thr Lys Tyr Asn Glu Lys Phe
    50              55              60

Lys Ala Lys Ala Thr Leu Thr Ala Asp Lys Ser Ser Ser Thr Ala Tyr
65              70              75              80

Leu Gln Leu Asn Asn Leu Thr Ser Glu Asn Ser Ala Val Tyr Phe Cys
            85              90              95

Ala Ser Gly Tyr Glu Asp Ala Met Asp Tyr Trp Gly Gln Gly Thr Ser
            100             105             110

Val Thr Val Ser Ser
            115

<210> 68
<211> 108
<212> PRT
<213> murine

<400> 68

```
Asp Ile Lys Met Thr Gln Ser Pro Ser Ser Met Tyr Ala Ser Leu Gly
1               5               10              15

Glu Arg Val Ile Ile Asn Cys Lys Ala Ser Gln Asp Ile Asn Ser Tyr
            20              25              30

Leu Ser Trp Phe Gln Gln Lys Pro Gly Lys Ser Pro Lys Thr Leu Ile
        35              40              45

Tyr Arg Ala Asn Arg Leu Val Asp Gly Val Pro Ser Arg Phe Ser Gly

    50              55              60

Ser Gly Ser Gly Gln Asp Tyr Ser Leu Thr Ile Ser Ser Leu Glu Tyr
65              70              75              80

Glu Asp Met Gly Ile Tyr Tyr Cys Leu Gln Tyr Asp Glu Phe Pro Leu
            85              90              95

Thr Phe Gly Ala Gly Thr Lys Leu Glu Leu Lys Arg
            100             105
```

<210> 69
<211> 120
<212> PRT
<213> murine

<400> 69

```
Glu Val Lys Leu Gln Glu Ser Gly Pro Glu Leu Val Lys Pro Gly Ala
1               5               10              15

Ser Val Lys Met Ser Cys Lys Ala Ser Gly Tyr Lys Phe Thr Asp Tyr
            20              25              30

Val Val His Trp Leu Lys Gln Lys Pro Gly Gln Gly Leu Glu Trp Ile
        35              40              45

Gly Tyr Ile Asn Pro Tyr Asn Asp Gly Thr Lys Tyr Asn Glu Lys Phe
    50              55              60

Lys Gly Lys Ala Thr Leu Thr Ser Asp Lys Ser Ser Ser Thr Ala Tyr
65              70              75              80

Met Glu Val Ser Ser Leu Thr Ser Glu Asp Ser Ala Val Tyr Tyr Cys
                85              90              95

Ala Arg Asp Tyr Arg Tyr Glu Val Tyr Gly Met Asp Tyr Trp Gly Gln
        100             105             110

Gly Thr Ser Val Thr Val Ser Ser
        115             120
```

<210> 70
<211> 115
<212> PRT
<213> murine

<400> 70

```
Asp Ile Val Leu Thr Gln Ser Pro Thr Ile Met Ser Ala Ser Pro Gly
1               5               10              15
```

```
Glu Arg Val Thr Met Thr Cys Thr Ala Ser Ser Ser Val Asn Tyr Ile
            20              25              30

His Trp Tyr Gln Gln Lys Ser Gly Asp Ser Pro Leu Arg Trp Ile Phe
            35              40              45

Asp Thr Ser Lys Val Ala Ser Gly Val Pro Ala Arg Phe Ser Gly Ser
            50              55              60

Gly Ser Gly Thr Ser Tyr Ser Leu Thr Ile Ser Thr Met Glu Ala Glu
65                  70              75              80

Asp Ala Ala Thr Tyr Tyr Cys Gln Gln Trp Arg Ser Tyr Pro Leu Thr
                85              90              95

Phe Gly Asp Gly Thr Arg Leu Glu Leu Lys Arg Ala Asp Ala Ala Pro
            100             105             110

Thr Val Ser
            115
```

<210> 71
<211> 118
<212> PRT
<213> murine

<400> 71

```
Gln Val Gln Leu Gln Gln Pro Gly Ala Glu Val Val Lys Pro Gly Ala
1               5               10              15

Ser Val Lys Met Ser Cys Lys Ala Ser Gly Tyr Thr Phe Thr Ser Tyr
            20              25              30

Tyr Ile His Trp Ile Lys Gln Thr Pro Gly Gln Gly Leu Glu Trp Val
            35              40              45

Gly Val Ile Tyr Pro Gly Asn Asp Asp Ile Ser Tyr Asn Gln Lys Phe
            50              55              60

Lys Gly Lys Ala Thr Leu Thr Ala Asp Lys Ser Ser Thr Thr Ala Tyr
65                  70              75              80

Met Gln Leu Ser Ser Leu Thr Ser Glu Asp Ser Ala Val Tyr Tyr Cys
                85              90              95

Ala Arg Glu Val Arg Leu Arg Tyr Phe Asp Val Trp Gly Ala Gly Thr
            100             105             110
```

```
                         Thr Val Thr Val Ser Ser
                             115
```

<210> 72
<211> 113
<212> PRT
<213> murine

<400> 72

```
        Asn Ile Met Leu Thr Gln Ser Pro Ser Ser Leu Ala Val Ser Ala Gly
        1               5               10              15

        Glu Lys Val Thr Met Ser Cys Lys Ser Ser Gln Ser Val Phe Phe Ser
                    20              25              30

        Ser Ser Gln Lys Asn Tyr Leu Ala Trp Tyr Gln Gln Ile Pro Gly Gln
                35              40              45

        Ser Pro Lys Leu Leu Ile Tyr Trp Ala Ser Thr Arg Glu Ser Gly Val
            50              55              60

        Pro Asp Arg Phe Thr Gly Ser Gly Ser Gly Thr Asp Phe Thr Leu Thr
        65              70              75              80

        Ile Ser Ser Val Gln Ser Glu Asp Leu Ala Ile Tyr Tyr Cys His Gln
                        85              90              95

        Tyr Leu Ser Ser Arg Thr Phe Gly Gly Gly Thr Lys Leu Glu Ile Lys
                    100             105             110

        Arg
```

<210> 73
<211> 115
<212> PRT
<213> murine

<400> 73

```
        Glu Val Gln Leu Gln Gln Ser Gly Pro Glu Leu Val Lys Pro Gly Ala
        1               5               10              15

        Ser Val Lys Ile Ser Cys Lys Ala Ser Gly Tyr Thr Phe Thr Asp Tyr
                    20              25              30

        Asn Met His Trp Val Lys Gln Ser His Gly Lys Ser Leu Glu Trp Ile
                    35              40              45
```

```
Gly Tyr Ile Tyr Pro Tyr Asn Gly Gly Thr Gly Tyr Asn Gln Lys Phe
    50                  55                  60

Lys Ser Lys Ala Thr Leu Thr Val Asp Asn Ser Ser Ser Thr Ala Tyr
    65              70                  75                  80

Met Asp Val Arg Ser Leu Thr Ser Glu Asp Ser Ala Val Tyr Tyr Cys
                85                  90                  95

Ala Arg Gly Arg Pro Ala Met Asp Tyr Trp Gly Gln Gly Thr Ser Val
            100                 105                 110

Thr Val Ser
        115
```

<210> 74
<211> 111
<212> PRT
<213> murine

<400> 74

```
Asp Ile Val Leu Thr Gln Ser Pro Ala Ser Leu Ala Val Ser Leu Gly
1               5                   10                  15

Gln Arg Ala Thr Ile Ser Cys Arg Ala Ser Glu Ser Val Asp Asn Tyr
            20                  25                  30

Gly Ile Ser Phe Met Asn Trp Phe Gln Gln Lys Pro Gly Gln Pro Pro
            35                  40                  45

Lys Leu Leu Ile Tyr Ala Ala Ser Asn Gln Gly Ser Gly Val Pro Ala
    50                  55                  60

Arg Phe Ser Gly Ser Gly Ser Gly Thr Asp Phe Ser Leu Asn Ile His
    65              70                  75                  80

Pro Met Glu Glu Asp Asp Thr Ala Met Tyr Phe Cys Gln Gln Ser Lys
                85                  90                  95

Glu Val Pro Trp Thr Phe Gly Gly Gly Thr Lys Leu Glu Ile Lys
            100                 105                 110
```

<210> 75
<211> 117
<212> PRT
<213> murine

<400> 75

```
Gln Val Gln Leu Gln Gln Ser Gly Pro Glu Leu Val Arg Pro Gly Thr
1               5               10              15

Phe Val Lys Ile Ser Cys Lys Ala Ser Gly Tyr Thr Phe Thr Asn Tyr
        20              25              30

Asp Ile Asn Trp Val Asn Gln Arg Pro Gly Gln Gly Leu Glu Trp Ile
        35              40              45

Gly Trp Ile Tyr Pro Gly Asp Gly Ser Thr Lys Tyr Asn Glu Lys Phe
    50              55              60

Lys Ala Lys Ala Thr Leu Thr Ala Asp Lys Ser Ser Ser Thr Ala Tyr
65              70              75              80

Leu Gln Leu Asn Asn Leu Thr Ser Glu Asn Ser Ala Val Tyr Phe Cys
            85              90              95

Ala Ser Gly Tyr Glu Asp Ala Met Asp Tyr Trp Gly Gln Gly Thr Ser
        100             105             110

Val Thr Val Ser Ser
        115
```

<210> 76
<211> 108
<212> PRT
<213> murine

<400> 76

```
Asp Ile Lys Met Thr Gln Ser Pro Ser Ser Met Tyr Ala Ser Leu Gly
1               5               10              15

Glu Arg Val Ile Ile Asn Cys Lys Ala Ser Gln Asp Ile Asn Ser Tyr
        20              25              30

Leu Ser Trp Phe Gln Gln Lys Pro Gly Lys Ser Pro Lys Thr Leu Ile
        35              40              45

Tyr Arg Ala Asn Arg Leu Val Asp Gly Val Pro Ser Arg Phe Ser Gly
    50              55              60

Ser Gly Ser Gly Gln Asp Tyr Ser Leu Thr Ile Ser Ser Leu Glu Tyr
65              70              75              80

Glu Asp Met Gly Ile Tyr Tyr Cys Leu Gln Tyr Asp Glu Phe Pro Leu
            85              90              95
```

```
               Thr Phe Gly Ala Gly Thr Lys Leu Glu Leu Lys Arg
                       100                 105
```

<210> 77
<211> 120
<212> PRT
<213> murine

<400> 77

```
        Glu Val Gln Leu Gln Gln Ser Gly Pro Asp Leu Val Lys Pro Gly Ala
        1               5               10              15

        Ser Val Lys Ile Ser Cys Lys Ala Ser Gly Tyr Ser Phe Thr Gly Tyr
                    20              25              30

        Tyr Met His Trp Val Lys Gln Ser His Gly Lys Ser Leu Glu Trp Ile
                    35              40              45

        Gly Arg Ile Asn Pro Asn Asn Gly Val Thr Leu Tyr Asn Gln Lys Phe
            50              55              60

        Lys Asp Lys Ala Ile Leu Thr Val Asp Lys Ser Ser Thr Thr Ala Tyr
        65              70              75              80

        Met Glu Leu Arg Ser Leu Thr Ser Glu Asp Ser Ala Val Tyr Tyr Cys
                        85              90              95

        Ala Arg Ser Thr Met Ile Thr Asn Tyr Val Met Asp Tyr Trp Gly Gln
                    100             105             110

        Val Thr Ser Val Thr Val Ser Ser
                    115             120
```

<210> 78
<211> 108
<212> PRT
<213> murine

<400> 78

```
Ser Ile Val Met Thr Gln Thr Pro Thr Phe Leu Leu Val Ser Ala Gly
1               5               10              15

Asp Arg Val Thr Ile Thr Cys Lys Ala Ser Gln Ser Val Ser Asn Asp
            20              25              30

Val Ala Trp Tyr Gln Gln Lys Pro Gly Gln Ser Pro Thr Leu Leu Ile
        35              40              45

Ser Tyr Thr Ser Ser Arg Tyr Ala Gly Val Pro Asp Arg Phe Ile Gly

    50              55              60

Ser Gly Tyr Gly Thr Asp Phe Thr Phe Thr Ile Ser Thr Leu Gln Ala
65              70              75              80

Glu Asp Leu Ala Val Tyr Phe Cys Gln Gln Asp Tyr Asn Ser Pro Pro
            85              90              95

Thr Phe Gly Gly Gly Thr Lys Leu Glu Ile Lys Arg
            100             105
```

<210> 79
<211> 119
<212> PRT
<213> murine

<400> 79

Gln Ile Gln Leu Val Gln Ser Gly Pro Glu Leu Lys Lys Pro Gly Glu
1                   5                   10                  15

Thr Val Lys Ile Ser Cys Lys Ala Ser Gly Tyr Thr Phe Thr Asn Phe
            20                  25                  30

Gly Met Asn Trp Val Lys Gln Gly Pro Gly Glu Gly Leu Lys Trp Met
        35                  40                  45

Gly Trp Ile Asn Thr Asn Thr Gly Glu Pro Arg Tyr Ala Glu Glu Phe
    50                  55                  60

Lys Gly Arg Phe Ala Phe Ser Leu Glu Thr Thr Ala Ser Thr Ala Tyr
65                  70                  75                  80

Leu Gln Ile Asn Asn Leu Lys Asn Glu Asp Thr Ala Thr Tyr Phe Cys
                85                  90                  95

Ala Arg Asp Trp Asp Gly Ala Tyr Phe Phe Asp Tyr Trp Gly Gln Gly
            100                 105                 110

Thr Thr Leu Thr Val Ser Ser
            115

<210> 80
<211> 107
<212> PRT
<213> murine

<400> 80

Ser Ile Val Met Thr Gln Thr Pro Lys Phe Leu Leu Val Ser Ala Gly
1                   5                   10                  15

```
Asp Arg Val Thr Ile Thr Cys Lys Ala Ser Gln Ser Val Ser Asn Asp
            20              25              30

Val Ala Trp Tyr Gln Gln Lys Pro Gly Gln Ser Pro Lys Leu Leu Ile
            35              40              45

Asn Phe Ala Thr Asn Arg Tyr Thr Gly Val Pro Asn Arg Phe Thr Gly
        50              55              60

Ser Gly Tyr Gly Thr Asp Phe Thr Phe Thr Ile Ser Thr Val Gln Ala
65              70              75              80

Glu Asp Leu Ala Leu Tyr Phe Cys Gln Gln Asp Tyr Ser Ser Pro Trp
                85              90              95

Thr Phe Gly Gly Gly Thr Lys Leu Glu Ile Lys
            100             105
```

<210> 81
<211> 117
<212> PRT
<213> murine

<400> 81

```
Gln Val Gln Leu Gln Gln Ser Arg Pro Glu Leu Val Lys Pro Gly Ala
1               5               10              15

Ser Val Lys Met Ser Cys Lys Ala Ser Gly Tyr Thr Phe Thr Asp Tyr
            20              25              30

Val Ile Ser Trp Val Lys Gln Arg Thr Gly Gln Gly Leu Glu Trp Ile
            35              40              45

Gly Glu Ile Tyr Pro Gly Ser Asn Ser Ile Tyr Tyr Asn Glu Lys Phe
        50              55              60

Lys Gly Arg Ala Thr Leu Thr Ala Asp Lys Ser Ser Ser Thr Ala Tyr
65              70              75              80

Met Gln Leu Ser Ser Leu Thr Ser Glu Asp Ser Ala Val Tyr Phe Cys
                85              90              95

Ala Met Gly Gly Asn Tyr Gly Phe Asp Tyr Trp Gly Gln Gly Thr Thr
            100             105             110

Leu Thr Val Ser Ser
            115
```

<210> 82
<211> 108
<212> PRT
<213> murine

<400> 82

```
Gln Ile Val Leu Thr Gln Ser Pro Ala Ile Met Ser Ala Ser Leu Gly
1               5                   10                  15

Glu Arg Val Thr Leu Thr Cys Thr Ala Ser Ser Ser Val Asn Ser Asn
            20                  25                  30

Tyr Leu His Trp Tyr Gln Gln Lys Pro Gly Ser Ser Pro Lys Leu Trp
        35                  40                  45

Ile Tyr Ser Thr Ser Asn Leu Ala Ser Gly Val Pro Ala Arg Phe Ser
    50                  55                  60

Gly Ser Gly Ser Gly Thr Ser Tyr Ser Leu Thr Ile Ser Ser Met Glu
65                  70                  75                  80

Ala Glu Asp Ala Ala Thr Tyr Tyr Cys His Gln Tyr His Arg Ser Pro
                85                  90                  95

Leu Thr Phe Gly Ala Gly Thr Lys Leu Glu Leu Lys
            100                 105
```

<210> 83
<211> 122
<212> PRT
<213> murine

<400> 83

```
Glu Val Gln Leu Val Glu Ser Gly Gly Gly Leu Val Gln Pro Lys Gly
1               5                   10                  15

Ser Leu Lys Leu Ser Cys Ala Ala Ser Gly Phe Thr Phe Asn Thr Tyr
            20                  25                  30

Ala Met Asn Trp Val Arg Gln Ala Pro Gly Lys Gly Leu Glu Trp Val
        35                  40                  45

Ala Arg Ile Arg Ser Lys Ser Asn Asn Tyr Ala Thr Tyr Tyr Ala Asp
    50                  55                  60

Ser Val Lys Asp Arg Phe Thr Ile Ser Arg Asp Asp Ser Gln Ser Met
65                  70                  75                  80
```

```
        Leu Tyr Leu Gln Met Asn Asn Leu Lys Thr Glu Asp Thr Ala Met Tyr
                        85                  90                  95


        Tyr Cys Val Arg Gln Trp Asp Tyr Asp Val Arg Ala Met Asn Tyr Trp
                        100                 105                 110


        Gly Gln Gly Thr Ser Val Thr Val Ser Ser
                        115                 120
```

<210> 84
<211> 107
<212> PRT
<213> murine

<400> 84

```
        Asp Ile Val Met Thr Gln Ser His Ile Phe Met Ser Thr Ser Val Gly
        1                   5                   10                  15


        Asp Arg Val Ser Ile Thr Cys Lys Ala Ser Gln Asp Val Asp Thr Ala
                        20                  25                  30


        Val Ala Trp Tyr Gln Gln Lys Pro Gly Gln Ser Pro Lys Leu Leu Ile
                        35                  40                  45


        Tyr Trp Ala Ser Thr Arg Leu Thr Gly Val Pro Asp Arg Phe Thr Gly
                50                  55                  60


        Ser Gly Ser Gly Thr Asp Phe Thr Leu Thr Ile Ser Asn Val Gln Ser
        65                  70                  75                  80


        Glu Asp Leu Ala Asp Tyr Phe Cys Gln Gln Tyr Ser Ser Tyr Pro Tyr
                        85                  90                  95


        Thr Phe Gly Gly Gly Thr Lys Leu Glu Ile Lys
                        100                 105
```

<210> 85
<211> 118
<212> PRT
<213> murine

<400> 85

```
        Gln Val Gln Leu Gln Gln Ser Gly Ala Glu Leu Val Arg Pro Gly Ala
        1                   5                   10                  15


        Ser Val Thr Leu Ser Cys Lys Ala Ser Gly Tyr Thr Phe Ser Asp Tyr
                        20                  25                  30
```

170

```
          Glu Met His Trp Val Ile Gln Thr Pro Val His Gly Leu Glu Trp Ile
                  35                  40              45

          Gly Ala Ile Asp Pro Glu Thr Gly Gly Thr Ala Tyr Asn Gln Lys Phe
                  50                  55              60

          Lys Gly Lys Ala Ile Leu Thr Ala Asp Lys Ser Ser Ser Thr Ala Tyr
                  65                  70              75              80

          Met Glu Leu Arg Ser Leu Thr Ser Glu Asp Ser Ala Val Tyr Tyr Cys
                          85                  90                  95

          Thr Gly Tyr Tyr Asp Tyr Asp Ser Phe Thr Tyr Trp Gly Gln Gly Thr
                      100                 105                 110

          Leu Val Thr Val Ser Ala
                  115
```

<210> 86
<211> 107
<212> PRT
<213> murine

<400> 86

```
          Asp Ile Val Met Thr Gln Ser Gln Lys Ile Met Ser Thr Thr Val Gly
          1                   5                   10                  15

          Asp Arg Val Ser Ile Thr Cys Lys Ala Ser Gln Asn Val Asp Ala Ala
                          20                  25                  30

          Val Ala Trp Tyr Gln Gln Lys Pro Gly Gln Ser Pro Lys Leu Leu Ile
                      35                  40                  45

          Tyr Ser Ala Ser Asn Arg Tyr Thr Gly Val Pro Asp Arg Phe Thr Gly
                  50                  55                  60

          Ser Gly Ser Gly Thr Asp Phe Thr Leu Thr Ile Ser Asn Met Gln Ser
          65                  70                  75                  80

          Glu Asp Leu Ala Asp Tyr Phe Cys Gln Gln Tyr Asp Ile Tyr Pro Tyr
                          85                  90                  95

          Thr Phe Gly Gly Gly Thr Lys Leu Glu Ile Lys
                      100                 105
```

<210> 87
<211> 119
<212> PRT

171

<213> murine

<400> 87

```
Glu Val Lys Leu Val Glu Ser Gly Gly Gly Leu Val Lys Pro Gly Gly
1               5                   10                  15

Ser Leu Lys Leu Ser Cys Ala Ala Ser Gly Phe Thr Phe Ser Ser Tyr
            20                  25                  30

Ala Met Ser Trp Val Arg Gln Ile Pro Glu Lys Arg Leu Glu Trp Val
            35                  40                  45

Ala Ser Ile Ser Arg Gly Gly Thr Thr Tyr Tyr Pro Asp Ser Val Lys
        50                  55                  60

Gly Arg Phe Thr Ile Ser Arg Asp Asn Val Arg Asn Ile Leu Tyr Leu
65                  70                  75                  80

Gln Met Ser Ser Leu Arg Ser Glu Asp Thr Ala Met Tyr Tyr Cys Gly
                85                  90                  95

Arg Tyr Asp Tyr Asp Gly Tyr Tyr Ala Met Asp Tyr Trp Gly Gln Gly
            100                 105                 110

Thr Ser Val Thr Val Ser Ser
            115
```

<210> 88
<211> 107
<212> PRT
<213> murine

<400> 88

```
Asp Ile Lys Met Thr Gln Ser Pro Ser Ser Met Tyr Ala Ser Leu Gly
1               5                   10                  15

Glu Arg Val Thr Ile Thr Cys Lys Ala Ser Pro Asp Ile Asn Ser Tyr
            20                  25                  30

Leu Ser Trp Phe Gln Gln Lys Pro Gly Lys Ser Pro Lys Thr Leu Ile
            35                  40                  45

Tyr Arg Ala Asn Arg Leu Val Asp Gly Val Pro Ser Arg Phe Ser Gly
    50                  55                  60

Gly Gly Ser Gly Gln Asp Tyr Ser Leu Thr Ile Asn Ser Leu Glu Tyr
65                  70                  75                  80

Glu Asp Met Gly Ile Tyr Tyr Cys Leu Gln Tyr Asp Glu Phe Pro Tyr
                85                  90                  95

    Thr Phe Gly Gly Gly Thr Lys Leu Glu Met Lys
                100                 105
```

<210> 89
<211> 117
<212> PRT
<213> murine

<400> 89

```
Gln Val Gln Leu Lys Glu Ser Gly Pro Gly Leu Val Ala Pro Ser Gln
1               5               10              15

Thr Leu Ser Ile Thr Cys Thr Val Ser Gly Phe Ser Leu Thr Ser Tyr
            20              25              30

Gly Val His Trp Val Arg Gln Pro Pro Gly Lys Gly Leu Glu Trp Leu
        35              40              45

Gly Val Ile Trp Ala Gly Gly Phe Thr Asn Tyr Asn Ser Ala Leu Lys
    50              55              60

Ser Arg Leu Ser Ile Ser Lys Asp Asn Ser Lys Ser Gln Val Leu Leu
65              70              75              80

Lys Met Thr Ser Leu Gln Thr Asp Asp Thr Ala Met Tyr Tyr Cys Ala
            85              90              95

Arg Arg Gly Ser Ser Tyr Ser Met Asp Tyr Trp Gly Gln Gly Thr Ser
        100             105             110

Val Thr Val Ser Ser
        115
```

<210> 90
<211> 106
<212> PRT
<213> murine

<400> 90

```
Glu Ile Val Leu Ser Gln Ser Pro Ala Ile Thr Ala Ala Ser Leu Gly
1               5               10              15

Gln Lys Val Thr Ile Thr Cys Ser Ala Ser Ser Asn Val Ser Tyr Ile
            20              25              30

His Trp Tyr Gln Gln Arg Ser Gly Thr Ser Pro Arg Pro Trp Ile Tyr
        35              40              45
```

```
        Glu Ile Ser Lys Leu Ala Ser Gly Val Pro Val Arg Phe Ser Gly Ser
            50                  55                  60

        Gly Ser Gly Thr Ser Tyr Ser Leu Thr Ile Ser Ser Met Glu Ala Glu
        65                  70                  75                  80

        Asp Ala Ala Ile Tyr Tyr Cys Gln Gln Trp Asn Tyr Pro Leu Ile Thr
                        85                  90                  95

        Phe Gly Ser Gly Thr Lys Leu Glu Ile Gln
                    100                 105
```

<210> 91
<211> 116
<212> PRT
<213> murine

<400> 91

```
        Glu Val Gln Val Leu Glu Ser Gly Gly Gly Leu Val Gln Pro Gly Gly
        1                   5                   10                  15

        Ser Leu Arg Leu Ser Cys Ala Ala Ser Gly Phe Thr Phe Ser Ser Tyr
                    20                  25                  30

        Ala Met Ser Trp Val Arg Gln Ala Pro Gly Lys Gly Leu Glu Trp Val
                    35                  40                  45

        Ser Ala Ile Ser Gly Ser Gly Gly Ser Thr Asn Tyr Ala Asp Ser Val
            50                  55                  60

        Lys Gly Arg Phe Thr Ile Ser Arg Asp Asn Ser Lys Asn Thr Leu Tyr
        65                  70                  75                  80

        Leu Gln Met Asn Ser Leu Arg Ala Glu Asp Thr Ala Val Tyr Tyr Cys
                        85                  90                  95

        Ala Gly Ser Ser Gly Trp Ser Glu Tyr Trp Gly Gln Gly Thr Leu Val
                    100                 105                 110

        Thr Val Ser Ser
                    115
```

<210> 92
<211> 107
<212> PRT
<213> murine

<400> 92

```
Asp Ile Gln Met Thr Gln Ser Pro Ser Ser Leu Ser Ala Ser Val Gly
1               5               10              15

Asp Arg Val Thr Ile Thr Cys Arg Ala Ser Gln Gly Ile Arg Asn Asn
            20              25              30

Leu Ala Trp Tyr Gln Gln Lys Pro Gly Lys Ala Pro Lys Arg Leu Ile
            35              40              45

Tyr Ala Ala Ser Asn Leu Gln Ser Gly Val Pro Ser Arg Phe Thr Gly
    50              55              60

Ser Gly Ser Gly Thr Glu Phe Thr Leu Ile Val Ser Ser Leu Gln Pro
65              70              75              80

Glu Asp Phe Ala Thr Tyr Tyr Cys Leu Gln His His Ser Tyr Pro Leu
            85              90              95

Thr Ser Gly Gly Gly Thr Lys Val Glu Ile Lys
        100             105
```

<210> 93
<211> 118
<212> PRT
<213> murine

<400> 93

```
Gln Val Gln Leu Gln Gln Ser Gly Gly Gly Leu Val Lys Pro Gly Ala
1               5               10              15

Ser Leu Lys Leu Ser Cys Val Thr Ser Gly Phe Thr Phe Arg Lys Phe
            20              25              30

Gly Met Ser Trp Val Arg Gln Thr Ser Asp Lys Arg Leu Glu Trp Val
            35              40              45

Ala Ser Ile Ser Thr Gly Gly Tyr Asn Thr Tyr Tyr Ser Asp Asn Val
    50              55              60

Lys Gly Arg Phe Thr Ile Ser Arg Glu Asn Ala Lys Asn Thr Leu Tyr
65              70              75              80

Leu Gln Met Ser Ser Leu Lys Ser Glu Asp Thr Ala Leu Tyr Tyr Cys
            85              90              95

Thr Arg Gly Tyr Ser Ser Thr Ser Tyr Ala Met Asp Tyr Trp Gly Gln
        100             105             110
```

```
                    Gly Thr Thr Val Thr Val
                        115
```

<210> 94
<211> 108
<212> PRT
<213> murine

<400> 94

```
        Asp Ile Glu Leu Thr Gln Ser Pro Ala Ser Leu Ser Val Ala Thr Gly
        1               5                   10                  15

        Glu Lys Val Thr Ile Arg Cys Met Thr Ser Thr Asp Ile Asp Asp Asp
                        20                  25                  30

        Met Asn Trp Tyr Gln Gln Lys Pro Gly Glu Pro Pro Lys Phe Leu Ile
                    35                  40                  45

        Ser Glu Gly Asn Thr Leu Arg Pro Gly Val Pro Ser Arg Phe Ser Ser
            50                  55                  60

        Ser Gly Thr Gly Thr Asp Phe Val Phe Thr Ile Glu Asn Thr Leu Ser
        65                  70                  75                  80

        Glu Asp Val Gly Asp Tyr Tyr Cys Leu Gln Ser Phe Asn Val Pro Leu
                        85                  90                  95

        Thr Phe Gly Asp Gly Thr Lys Leu Glu Lys Ala Leu
                    100                 105
```

<210> 95
<211> 119
<212> PRT
<213> murine

<400> 95

```
        Gln Val Gln Leu Val Gln Ser Gly Ala Glu Val Lys Lys Pro Gly Ala
        1               5                   10                  15

        Ser Val Lys Val Ser Cys Lys Ala Ser Gly Tyr Ser Phe Pro Asp Tyr
                        20                  25                  30

        Tyr Ile Asn Trp Val Arg Gln Ala Pro Gly Gln Gly Leu Glu Trp Met
                    35                  40                  45

        Gly Trp Ile Tyr Phe Ala Ser Gly Asn Ser Glu Tyr Asn Gln Lys Phe
            50                  55                  60
```

```
Thr Gly Arg Val Thr Met Thr Arg Asp Thr Ser Ile Asn Thr Ala Tyr
65              70              75              80

Met Glu Leu Ser Ser Leu Thr Ser Glu Asp Thr Ala Val Tyr Phe Cys
                85              90              95

Ala Ser Leu Tyr Asp Tyr Asp Trp Tyr Phe Asp Val Trp Gly Gln Gly
            100             105             110

Thr Met Val Thr Val Ser Ser
            115
```

<210> 96
<211> 112
<212> PRT
<213> murine

<400> 96

```
Asp Ile Val Met Thr Gln Thr Pro Leu Ser Leu Ser Val Thr Pro Gly
1               5               10              15

Gln Pro Ala Ser Ile Ser Cys Lys Ser Ser Gln Ser Leu Val His Ser
            20              25              30

Asn Gly Asn Thr Tyr Leu His Trp Tyr Leu Gln Lys Pro Gly Gln Ser
            35              40              45

Pro Gln Leu Leu Ile Tyr Lys Val Ser Asn Arg Phe Ser Gly Val Pro
        50              55              60

Asp Arg Phe Ser Gly Ser Gly Ser Gly Thr Asp Phe Thr Leu Lys Ile
65              70              75              80

Ser Arg Val Glu Ala Glu Asp Val Gly Ile Tyr Tyr Cys Ser Gln Ser
                85              90              95

Ser Ile Tyr Pro Trp Thr Phe Gly Gln Gly Thr Lys Leu Glu Ile Lys
            100             105             110
```

<210> 97
<211> 119
<212> PRT
<213> murine

<400> 97

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Ser Phe Pro Asp Tyr
            20                  25                  30

Tyr Ile Asn Trp Val Arg Gln Ala Pro Gly Gln Gly Leu Glu Trp Met
            35                  40                  45

Gly Trp Ile Tyr Phe Ala Ser Gly Asn Ser Glu Tyr Asn Gln Lys Phe
        50                  55                  60

Thr Gly Arg Val Thr Met Thr Arg Asp Thr Ser Ser Ser Thr Ala Tyr
65                  70                  75                  80

Met Glu Leu Ser Ser Leu Arg Ser Glu Asp Thr Ala Val Tyr Phe Cys
                85                  90                  95

Ala Ser Leu Tyr Asp Tyr Asp Trp Tyr Phe Asp Val Trp Gly Gln Gly
            100                 105                 110

Thr Met Val Thr Val Ser Ser
            115
```

<210> 98
<211> 112
<212> PRT
<213> murine

<400> 98

```
Gln Val Gln Leu Val Gln Ser Gly Ala Glu Val Lys Lys Pro Gly Ala
1               5                   10                  15
```

```
Asp Ile Val Met Thr Gln Thr Pro Leu Ser Leu Ser Val Thr Pro Gly
1               5               10              15

Glu Pro Ala Ser Ile Ser Cys Lys Ser Ser Gln Ser Leu Val His Ser
                20              25              30

Asn Gly Asn Thr Tyr Leu His Trp Tyr Leu Gln Lys Pro Gly Gln Ser
        35              40              45

Pro Gln Leu Leu Ile Tyr Lys Val Ser Asn Arg Phe Ser Gly Val Pro
    50              55              60

Asp Arg Phe Ser Gly Ser Gly Ser Gly Ala Asp Phe Thr Leu Lys Ile
65              70              75              80

Ser Arg Val Glu Ala Glu Asp Val Gly Val Tyr Tyr Cys Ala Glu Thr
                85              90              95

Ser His Val Pro Trp Thr Phe Gly Gln Gly Thr Lys Leu Glu Ile Lys
                100             105             110
```

<210> 99
<211> 117
<212> PRT
<213> murine

<400> 99

```
Gln Ile Gln Leu Val Gln Ser Gly Pro Glu Leu Lys Lys Pro Gly Glu
1               5               10                  15

Thr Val Lys Ile Ser Cys Lys Ala Ser Gly Tyr Thr Phe Thr Asp Tyr
            20                  25                  30

Ser Ile Asn Trp Val Lys Arg Ala Pro Gly Lys Gly Leu Lys Trp Met
            35                  40                  45

Gly Trp Ile Asn Thr Glu Thr Arg Glu Pro Ala Tyr Ala Tyr Asp Phe
        50                  55                  60

Arg Gly Arg Phe Ala Phe Ser Leu Glu Thr Ser Ala Ser Thr Ala Tyr
65                  70                  75                  80

Leu Gln Ile Asn Asn Leu Lys Tyr Glu Asp Thr Ala Thr Tyr Phe Cys
                85                  90                  95

Ala Leu Asp Tyr Ser Tyr Ala Met Asp Tyr Trp Gly Gln Gly Thr Ser
            100                 105                 110

Val Thr Val Ser Ser
            115
```

<210> 100
<211> 111
<212> PRT
<213> murine

<400> 100

```
Asp Ile Val Leu Thr Gly Ser Pro Pro Ser Leu Ala Met Ser Leu Gly
1               5               10                  15

Lys Arg Ala Thr Ile Ser Cys Arg Ala Ser Glu Ser Val Thr Ile Leu
            20                  25                  30

Gly Ser His Leu Ile His Trp Tyr Gln Gln Lys Pro Gly Gln Pro Pro
            35                  40                  45

Thr Leu Leu Ile Gln Leu Ala Ser Asn Val Gln Thr Gly Val Pro Ala
        50                  55                  60

Arg Phe Ser Gly Ser Gly Ser Arg Thr Asp Phe Thr Leu Thr Ile Asp
65                  70                  75                  80
```

```
        Pro Val Glu Glu Asp Asp Val Ala Val Tyr Tyr Cys Leu Gln Ser Arg
                        85              90                  95
```

```
        Thr Ile Pro Arg Thr Phe Gly Gly Gly Thr Lys Leu Glu Ile Lys
                    100             105             110
```

<210> 101
<211> 117
<212> PRT
<213> murine

<400> 101

```
        Gln Ile Gln Leu Val Gln Ser Gly Pro Glu Leu Lys Lys Pro Gly Glu
        1                   5               10                  15
```

```
        Thr Val Lys Ile Ser Cys Lys Ala Ser Gly Tyr Thr Phe Thr His Tyr
                        20                  25                  30
```

```
        Ser Met Asn Trp Val Lys Gln Ala Pro Gly Lys Gly Leu Lys Trp Met
                        35                  40                  45
```

```
        Gly Arg Ile Asn Thr Glu Thr Gly Glu Pro Leu Tyr Ala Asp Asp Phe
                50                  55                  60
```

```
        Lys Gly Arg Phe Ala Phe Ser Leu Glu Thr Ser Ala Ser Thr Ala Tyr
        65                  70                  75                  80
```

```
        Leu Val Ile Asn Asn Leu Lys Asn Glu Asp Thr Ala Thr Phe Phe Cys
                        85                  90                  95
```

```
        Ser Asn Asp Tyr Leu Tyr Ser Cys Asp Tyr Trp Gly Arg Gly Thr Thr
                    100             105             110
```

```
        Leu Thr Val Ser Ser
                    115
```

<210> 102
<211> 111
<212> PRT
<213> murine

<400> 102

```
        Asp Ile Val Leu Thr Gln Ser Pro Pro Ser Leu Ala Met Ser Leu Gly
        1                   5               10                  15
```

```
        Lys Arg Ala Thr Ile Ser Cys Arg Ala Ser Glu Ser Val Thr Ile Leu
                        20                  25                  30
```

```
        Gly Ser His Leu Ile Tyr Trp Tyr Gln Gln Lys Pro Gly Gln Pro Pro
                35                  40                  45


        Thr Leu Leu Ile Gln Leu Ala Ser Asn Val Gln Thr Gly Val Pro Ala
                50                  55                  60


        Arg Phe Ser Gly Ser Gly Ser Arg Thr Asp Phe Thr Leu Thr Ile Asp
                65                  70                  75                  80


        Pro Val Glu Glu Asp Asp Val Ala Val Tyr Tyr Cys Leu Gln Ser Arg
                            85                  90                  95


        Thr Ile Pro Arg Thr Phe Gly Gly Gly Thr Lys Leu Glu Ile Lys
                        100                 105                 110
```

<210> 103
<211> 119
<212> PRT
<213> murine

<400> 103

```
        Glu Val Lys Leu Leu Glu Ser Gly Gly Gly Leu Val Gln Pro Gly Gly
        1               5                   10                  15


        Ser Leu Lys Leu Ser Cys Ala Ala Ser Gly Phe Asp Phe Ser Arg Tyr
                        20                  25                  30


        Trp Met Ser Trp Val Arg Gln Ala Pro Gly Lys Gly Leu Glu Trp Ile
                    35                  40                  45


        Gly Glu Ile Asn Pro Asp Ser Ser Thr Ile Asn Tyr Thr Pro Ser Leu
                50                  55                  60


        Lys Asp Lys Phe Ile Ile Ser Arg Asp Asn Ala Lys Asn Thr Leu Tyr
        65                  70                  75                  80


        Leu Gln Met Ser Lys Val Arg Ser Glu Asp Thr Ala Leu Tyr Tyr Cys
                            85                  90                  95


        Ala Arg Pro Asp Gly Asn Tyr Trp Tyr Phe Asp Val Trp Gly Ala Gly
                        100                 105                 110


        Thr Thr Val Thr Val Ser Ser
                        115
```

<210> 104
<211> 107
<212> PRT
```
```

<213> murine

<400> 104

```
Asp Ile Val Met Thr Gln Ser His Lys Phe Met Ser Thr Ser Val Gly
1               5               10              15

Asp Arg Val Ser Ile Thr Cys Lys Ala Ser Gln Asp Val Gly Ile Ala
                20              25              30

Val Ala Trp Tyr Gln Gln Lys Pro Gly Gln Ser Pro Lys Leu Leu Ile
            35              40              45

Tyr Trp Ala Ser Thr Arg His Thr Gly Val Pro Asp Arg Phe Thr Gly
        50              55              60

Ser Gly Ser Gly Thr Asp Phe Thr Leu Thr Ile Ser Asn Val Gln Ser
65              70              75              80

Glu Asp Leu Ala Asp Tyr Phe Cys Gln Gln Tyr Ser Ser Tyr Pro Tyr
                85              90                  95

Thr Phe Gly Gly Gly Thr Lys Leu Glu Ile Lys
            100             105
```

<210> 105
<211> 120
<212> PRT
<213> murine

<400> 105

```
Gln Val Gln Leu Gln Gln Pro Gly Ala Glu Leu Val Arg Pro Gly Ala
1               5               10              15

Ser Val Lys Leu Ser Cys Lys Ala Ser Gly Tyr Ser Phe Thr Thr Tyr
                20              25              30

Trp Met Asn Trp Val Lys Gln Arg Pro Gly Gln Gly Leu Glu Trp Ile
            35              40              45

Gly Met Ile His Pro Ser Asp Ser Glu Thr Arg Leu Asn Gln Lys Phe
        50              55              60

Lys Asp Lys Ala Thr Leu Thr Val Asp Lys Ser Ser Ser Thr Ala Tyr
65              70              75              80

Met Gln Leu Ser Ser Pro Thr Ser Glu Asp Ser Ala Val Tyr Tyr Cys
                85              90                  95
```

Ala Arg Ser Thr Met Ile Ala Thr Arg Ala Met Asp Tyr Trp Gly Gln
          100                   105                   110

Gly Thr Ser Val Thr Val Ser Ser
          115                   120

<210> 106
<211> 107
<212> PRT
<213> murine

<400> 106

Asp Ile Val Met Thr Gln Ser Gln Lys Ser Met Ser Thr Ser Val Gly
1                   5                   10                  15

Asp Arg Val Ser Ile Thr Cys Lys Ala Ser Gln Asp Val Ile Thr Gly
          20                  25                  30

Val Ala Trp Tyr Gln Gln Lys Pro Gly Gln Ser Pro Lys Leu Leu Ile
          35                  40                  45

Tyr Ser Ala Ser Tyr Arg Tyr Thr Gly Val Pro Asp Arg Phe Thr Gly
          50                  55                  60

Ser Gly Ser Gly Thr Asp Phe Thr Phe Thr Ile Ser Asn Val Gln Ala
65                  70                  75                  80

Glu Asp Leu Ala Val Tyr Tyr Cys Gln Gln His Tyr Ser Thr Pro Leu
                    85                  90                  95

Thr Phe Gly Ala Gly Thr Lys Leu Glu Leu Lys
          100                   105

<210> 107
<211> 121
<212> PRT
<213> murine

<400> 107

185

```
Gln Val Gln Leu Gln Gln Ser Gly Ala Glu Leu Ala Arg Pro Gly Ala
1               5                   10                  15

Ser Val Lys Leu Ser Cys Lys Ala Ser Gly Tyr Thr Phe Thr Ser Tyr
            20                  25                  30

Trp Met Gln Trp Val Lys Gln Arg Pro Gly Gln Gly Leu Glu Trp Ile
        35                  40                  45

Gly Ala Ile Tyr Pro Gly Asp Gly Asp Thr Arg Tyr Thr Gln Lys Phe

    50              55                  60

Lys Gly Lys Ala Thr Leu Thr Ala Asp Lys Ser Ser Ser Thr Ala Tyr
65              70                  75                  80

Met Gln Leu Ser Ser Leu Ala Ser Glu Asp Ser Ala Val Tyr Tyr Cys
            85                  90                  95

Ala Arg Gly Lys Val Tyr Tyr Gly Ser Asn Pro Phe Ala Tyr Trp Gly
            100                 105                 110

Gln Gly Thr Leu Val Thr Val Ser Ala
            115                 120
```

<210> 108
<211> 107
<212> PRT
<213> murine

<400> 108

```
Asp Ile Gln Met Thr Gln Ser Ser Ser Tyr Leu Ser Val Ser Leu Gly
1               5               10              15

Gly Arg Val Thr Ile Thr Cys Lys Ala Ser Asp His Ile Asn Asn Trp
            20              25              30

Leu Ala Trp Tyr Gln Gln Lys Pro Gly Asn Ala Pro Arg Leu Leu Ile
            35              40              45

Ser Gly Ala Thr Ser Leu Glu Thr Gly Val Pro Ser Arg Phe Ser Gly
    50              55              60

Ser Gly Ser Gly Lys Asp Tyr Thr Leu Ser Ile Thr Ser Leu Gln Thr
65              70              75              80

Glu Asp Val Ala Thr Tyr Tyr Cys Gln Gln Tyr Trp Ser Thr Pro Trp
            85              90              95

Thr Phe Gly Gly Gly Thr Lys Leu Glu Ile Lys
        100             105
```

<210> 109
<211> 120
<212> PRT
<213> murine

<400> 109

```
Gln Val Gln Leu Gln Gln Ser Gly Pro Glu Leu Val Lys Pro Gly Ala
1               5               10              15
```

```
Ser Val Lys Ile Ser Cys Lys Ala Ser Gly Tyr Ala Phe Ser Ser Ser
            20              25                  30

Trp Met Asn Trp Val Lys Gln Arg Pro Gly Gln Gly Leu Glu Trp Ile
            35              40                  45

Gly Arg Ile Tyr Pro Gly Asp Gly Asp Thr Lys Tyr Asn Gly Lys Phe
            50              55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Val Asp Ser Ala Val Tyr Phe Cys
                85                  90                  95

Ala Arg Ser Thr Met Ile Ala Thr Gly Ala Met Asp Tyr Trp Gly Gln
            100                 105                 110

Gly Thr Ser Val Thr Val Ser Ser
            115                 120
```

<210> 110
<211> 107
<212> PRT
<213> murine

<400> 110

```
Glu Thr Thr Val Thr Gln Ser Pro Ala Ser Leu Ser Met Ala Ile Gly
1               5                   10                  15

Glu Lys Val Thr Ile Arg Cys Ile Thr Ser Thr Asp Ile Asp Asp Asp
            20              25                  30

Met Asn Trp Tyr Gln Gln Lys Pro Gly Glu Pro Pro Lys Leu Leu Ile
            35              40                  45

Ser Glu Gly Asn Thr Leu Arg Pro Gly Val Pro Ser Arg Phe Ser Ser
    50                  55                  60

Ser Gly Tyr Gly Thr Asp Phe Val Phe Thr Ile Glu Asn Met Leu Ser
65                  70                  75                  80

Glu Asp Val Ala Asp Tyr Tyr Cys Leu Gln Ser Asp Asn Leu Pro Leu
                85                  90                  95

Thr Phe Gly Gly Gly Thr Lys Leu Glu Ile Lys
            100                 105
```

<210> 111
<211> 120
<212> PRT
<213> murine

<400> 111

```
Gln Val Gln Leu Gln Gln Ser Gly Pro Glu Leu Val Lys Pro Gly Ala
1               5                   10                  15

Ser Val Lys Ile Ser Cys Lys Ala Ser Gly Tyr Ala Phe Ser Ser Ser
            20                  25                  30

Trp Met Asn Trp Val Lys Gln Arg Pro Gly Gln Gly Leu Glu Trp Ile
        35                  40                  45

Gly Arg Ile Tyr Pro Gly Asp Gly Asp Thr Lys Tyr Asn Gly Lys Phe
        50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Val Asp Ser Ala Val Tyr Phe Cys
                85                  90                  95

Ala Arg Ser Thr Met Ile Ala Thr Gly Ala Met Asp Tyr Trp Gly Gln
            100                 105                 110

Gly Thr Ser Val Thr Val Ser Ser
            115                 120
```

<210> 112
<211> 108
<212> PRT
<213> murine

<400> 112

```
Asp Ile Val Met Thr Gln Ser His Lys Phe Met Ser Thr Ser Val Gly
1               5                   10                  15

Asp Arg Val Ser Ile Thr Cys Lys Ala Ser Gln Asp Val Ser Thr Ala
            20                  25                  30

Val Ala Trp Tyr Gln Gln Lys Pro Gly Gln Ser Pro Lys Leu Leu Ile
        35                  40                  45

Tyr Ser Ala Ser Tyr Arg Tyr Thr Gly Val Pro Asp Arg Phe Thr Gly
        50                  55                  60
```

```
Ser Gly Ser Gly Thr Asp Phe Thr Phe Thr Ile Ser Ser Val Gln Ala
65          70          75                80

Glu Asp Leu Ala Val Tyr Tyr Cys Gln Gln His Tyr Ser Thr Pro Pro
            85              90              95

Tyr Thr Phe Gly Gly Gly Thr Lys Leu Glu Ile Lys
        100             105
```

<210> 113
<211> 141
<212> PRT
<213> murine

<400> 113

```
Met Gly Trp Ser Trp Ile Phe Leu Phe Leu Val Ser Gly Thr Gly Gly
1           5               10              15

Val Leu Ser Glu Val Gln Leu Gln Gln Ser Gly Pro Glu Leu Val Lys
        20              25              30

Pro Gly Ala Ser Val Lys Met Ser Cys Lys Ala Ser Gly Tyr Thr Phe
        35              40              45

Thr Asp Tyr Tyr Met Lys Trp Val Lys Gln Ser His Gly Lys Ser Leu
        50              55              60

Glu Trp Ile Gly Asp Ile Ile Pro Ser Asn Gly Ala Thr Phe Tyr Asn
65          70              75              80

Gln Lys Phe Lys Gly Lys Ala Thr Leu Thr Val Asp Arg Ser Ser Ser
            85              90              95

Thr Ala Tyr Met His Leu Asn Ser Leu Thr Ser Glu Asp Ser Ala Val
        100             105             110

Tyr Tyr Cys Thr Arg Ser His Leu Leu Arg Ala Ser Trp Phe Ala Tyr
        115             120             125

Trp Gly Gln Gly Thr Leu Val Thr Val Ser Ala Ala Ser
        130             135             140
```

<210> 114
<211> 134
<212> PRT
<213> murine

<400> 114

```
Met Glu Ser Gln Thr Gln Val Leu Met Ser Leu Leu Phe Trp Val Ser
1               5               10              15

Gly Thr Cys Gly Asp Phe Val Met Thr Gln Ser Pro Ser Ser Leu Thr
            20              25              30

Val Thr Ala Gly Glu Lys Val Thr Met Ser Cys Lys Ser Ser Gln Ser
            35              40              45

Leu Leu Asn Ser Gly Asn Gln Lys Asn Tyr Leu Thr Trp Tyr Leu Gln
        50              55              60

Lys Pro Gly Gln Pro Pro Lys Leu Leu Ile Tyr Trp Ala Ser Thr Arg
65              70              75              80

Glu Ser Gly Val Pro Asp Arg Phe Thr Gly Ser Gly Ser Gly Thr Asp
                85              90              95

Phe Thr Leu Thr Ile Ser Ser Val Gln Ala Glu Asp Leu Ala Val Tyr
            100             105             110

Tyr Cys Gln Asn Asp Tyr Ser Tyr Pro Tyr Thr Phe Gly Gly Gly Thr
        115             120             125

Lys Leu Glu Ile Lys Arg
        130
```

<210> 115
<211> 140
<212> PRT
<213> murine

<400> 115

```
Trp Thr Trp Arg Phe Leu Phe Val Val Ala Ala Ala Thr Gly Val Gln
1               5               10              15

Ser Gln Val Gln Leu Leu Gln Ser Gly Ala Glu Val Lys Lys Pro Gly
            20              25              30

Ser Ser Val Lys Val Ser Cys Lys Ala Ser Gly Gly Thr Phe Ser Thr
            35              40              45

Tyr Ala Ile Ser Trp Val Arg Gln Ala Pro Gly Gln Gly Leu Glu Trp
        50              55              60

Met Gly Gly Ile Ile Pro Ile Phe Gly Ile Val Asn Tyr Ala Gln Lys
65              70              75              80
```

Phe Gln Gly Arg Val Thr Ile Thr Ala Asp Glu Ser Thr Ser Thr Ala
                    85                  90                  95

Tyr Met Glu Leu Ser Ser Leu Arg Ser Glu Asp Thr Ala Val Tyr Tyr
                100                 105                 110

Cys Ala Arg Gly Gly Gly Ser Gly Pro Asp Val Leu Asp Ile Trp Gly
                115                 120                 125

Gln Gly Thr Met Val Thr Val Ser Ser Ala Ser Thr
        130                 135                 140

<210> 116
<211> 132
<212> PRT
<213> murine

<400> 116

Met Asp Met Arg Val Pro Ala Gln Leu Leu Gly Leu Leu Leu Leu Trp
1                   5                   10                  15

Leu Pro Gly Ala Arg Cys Val Ile Trp Met Thr Gln Ser Pro Ser Leu
                20                  25                  30

Leu Ser Ala Ser Thr Gly Asp Arg Val Thr Ile Ser Cys Arg Met Ser
                35                  40                  45

Gln Gly Ile Arg Ser Tyr Leu Ala Trp Tyr Gln Gln Lys Pro Gly Lys
        50                  55                  60

Ala Pro Glu Leu Leu Ile Tyr Ala Ala Ser Thr Leu Gln Ser Gly Val
65                  70                  75                  80

Pro Ser Arg Phe Ser Gly Ser Gly Ser Gly Thr Asp Phe Thr Leu Thr
                85                  90                  95

Ile Ser Ser Leu Gln Ser Glu Asp Phe Ala Thr Tyr Tyr Cys Gln Gln
                100                 105                 110

Tyr Tyr Ser Phe Pro Tyr Thr Phe Gly Gln Gly Thr Lys Leu Glu Ile
                115                 120                 125

Lys Arg Thr Val
        130

<210> 117
<211> 115
<212> PRT

<213> murine

<400> 117

```
        Gln Val Gln Leu Gln Gln Pro Gly Ala Glu Leu Val Arg Pro Gly Ala
        1               5                   10                  15

        Ser Val Lys Leu Ser Cys Lys Ala Ser Gly Tyr Thr Phe Thr Ser Tyr
                    20                  25                  30

        Trp Met Asn Trp Val Lys Gln Arg Pro Asp Gln Gly Leu Glu Trp Ile
                    35                  40                  45

        Gly Arg Ile Asp Pro Tyr Asp Ser Glu Thr His Tyr Asn Gln Lys Phe
                50                  55                  60

        Lys Asp Lys Ala Ile Leu Thr Val Asp Lys Ser Ser Ser Thr Ala Tyr
        65                  70                  75                  80

        Met Gln Leu Ser Ser Leu Thr Ser Glu Asp Ser Ala Val Tyr Tyr Cys
                        85                  90                  95

        Ala Arg Gly Asn Trp Asp Asp Tyr Trp Gly Gln Gly Thr Thr Leu Thr
                    100                 105                 110

        Val Ser Ser
                115
```

<210> 118
<211> 107
<212> PRT
<213> murine

<400> 118

```
Asp Val Gln Ile Thr Gln Ser Pro Ser Tyr Leu Ala Ala Ser Pro Gly
1               5                   10                  15

Glu Thr Ile Thr Ile Asn Cys Arg Ala Ser Lys Ser Ile Ser Lys Asp
            20                  25                  30

Leu Ala Trp Tyr Gln Glu Lys Pro Gly Lys Thr Asn Lys Leu Leu Ile
            35                  40                  45

Tyr Ser Gly Ser Thr Leu Gln Ser Gly Ile Pro Ser Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Met Tyr Tyr Cys Gln Gln His Asn Lys Tyr Pro Tyr
                    85                  90                  95

            Thr Phe Gly Gly Gly Thr Lys Leu Glu Ile Lys
                        100                 105
```

<210> 119
<211> 118
<212> PRT
<213> murine

<400> 119

```
Gln Ile Gln Leu Val Gln Ser Gly Pro Glu Leu Lys Lys Pro Gly Glu
1               5               10              15

Thr Val Lys Ile Ser Cys Lys Ala Ser Gly Tyr Ile Phe Thr Asn Tyr
            20              25              30

Gly Met Asn Trp Val Lys Gln Ala Pro Gly Lys Ser Phe Lys Trp Met
        35              40              45

Gly Trp Ile Asn Thr Tyr Thr Gly Glu Ser Thr Tyr Ser Ala Asp Phe
    50              55              60

Lys Gly Arg Phe Ala Phe Ser Leu Glu Thr Ser Ala Ser Thr Ala Tyr
65              70              75              80

Leu His Ile Asn Asp Leu Lys Asn Glu Asp Thr Ala Thr Tyr Phe Cys
            85              90              95

Ala Arg Ser Gly Gly Tyr Asp Pro Met Asp Tyr Trp Gly Gln Gly Thr
        100             105             110

Ser Val Thr Val Ser Ser
        115
```

<210> 120
<211> 111
<212> PRT
<213> murine

<400> 120

```
Asp Ile Val Leu Thr Gln Ser Pro Ala Ser Leu Ala Val Ser Leu Gly
1               5               10              15

Gln Arg Ala Thr Ile Ser Cys Arg Ala Ser Glu Ser Val Asp Asn Tyr
            20              25              30

Gly Asn Thr Phe Met His Trp Tyr Gln Gln Lys Pro Gly Gln Pro Pro
        35              40              45
```

```
Lys Leu Leu Ile Tyr Arg Ala Ser Asn Leu Glu Ser Gly Ile Pro Ala
    50              55              60

Arg Phe Ser Gly Ser Gly Ser Arg Thr Asp Phe Thr Leu Thr Ile Asn
65              70              75                          80

Pro Val Glu Ala Asp Asp Val Ala Thr Tyr Tyr Cys Gln Gln Ser Asn
                85              90                      95

Glu Asp Pro Pro Thr Phe Gly Ala Gly Thr Lys Leu Glu Leu Lys
            100             105             110
```

<210> 121
<211> 127
<212> PRT
<213> murine

<400> 121

```
Glu Val Lys Leu Val Glu Ser Gly Gly Gly Leu Val Gln Pro Gly Gly
1               5               10              15

Ser Leu Ser Leu Ser Cys Ala Ala Ser Gly Phe Thr Phe Thr Asp Tyr
            20              25              30

Tyr Met Ser Trp Val Arg Gln Pro Pro Gly Lys Ala Leu Glu Trp Leu
        35              40              45

Ala Leu Ile Arg Ser Lys Ala Asp Gly Tyr Thr Thr Glu Tyr Ser Ala
    50              55              60

Ser Val Lys Gly Arg Phe Thr Leu Ser Arg Asp Asp Ser Gln Ser Ile
65              70              75                          80

Leu Tyr Leu Gln Met Asn Ala Leu Arg Pro Glu Asp Ser Ala Thr Tyr
                85              90                      95

Tyr Cys Ala Arg Asp Ala Ala Tyr Tyr Ser Tyr Tyr Ser Pro Glu Gly
            100             105             110

Ala Met Asp Tyr Trp Gly Gln Gly Thr Ser Val Thr Val Ser Ser
        115             120             125
```

<210> 122
<211> 113
<212> PRT
<213> murine

<400> 122

```
Met Ala Asp Tyr Lys Asp Ile Val Met Thr Gln Ser His Lys Phe Met
1               5                   10                  15

Ser Thr Ser Val Gly Asp Arg Val Asn Ile Thr Cys Lys Ala Ser Gln
            20                  25                  30

Asn Val Asp Ser Ala Val Ala Trp Tyr Gln Gln Lys Pro Gly Gln Ser
            35                  40                  45

Pro Lys Ala Leu Ile Tyr Ser Ala Ser Tyr Arg Tyr Ser Gly Val Pro
    50                  55                  60

Asp Arg Phe Thr Gly Arg Gly Ser Gly Thr Asp Phe Thr Leu Thr Ile
65                  70                  75                  80

Ser Ser Val Gln Ala Glu Asp Leu Ala Val Tyr Tyr Cys Gln Gln Tyr
                85                  90                  95

Tyr Ser Thr Pro Trp Thr Phe Gly Gly Gly Thr Lys Leu Glu Ile Lys
            100                 105                 110

Arg
```

<210> 123
<211> 119
<212> PRT
<213> murine

<400> 123

```
Val Gln Leu Gln Glu Ser Gly Pro Gly Leu Val Lys Pro Ser Gln Ser
1               5                   10                  15

Leu Ser Leu Thr Cys Ser Val Thr Asp Tyr Ser Ile Thr Ser Gly Tyr
            20                  25                  30

Tyr Trp Asn Trp Ile Arg Gln Phe Pro Gly Asn Lys Leu Glu Trp Met
            35                  40                  45

Gly Tyr Ile Ser Tyr Asp Gly Ser Asn Asn Tyr Asn Pro Ser Leu Lys
    50                  55                  60

Asn Arg Ile Ser Ile Thr Arg Asp Thr Ser Lys Asn Gln Phe Phe Leu
65                  70                  75                  80

Lys Leu Ser Ser Val Thr Thr Glu Asp Thr Ala Thr Tyr Tyr Cys Ser
                85                  90                  95
```

```
Arg Gly Glu Gly Phe Tyr Phe Asp Ser Trp Gly Gln Gly Thr Thr Leu
            100                 105                 110

Thr Val Ser Ser Ala Arg Ser
            115
```

<210> 124
<211> 113
<212> PRT
<213> murine

<400> 124

```
Asp Ile Met Met Ser Gln Ser Pro Ser Ser Leu Ala Val Ser Val Gly
1               5                   10                  15

Glu Lys Phe Thr Met Thr Cys Lys Ser Ser Gln Ser Leu Phe Phe Gly
            20                  25                  30

Ser Thr Gln Lys Asn Tyr Leu Ala Trp Tyr Gln Gln Lys Pro Gly Gln
            35                  40                  45

Ser Pro Lys Leu Leu Ile Tyr Trp Ala Ser Thr Arg Glu Ser Gly Val
            50                  55                  60

Pro Asp Arg Phe Thr Gly Ser Gly Ser Gly Thr Asp Phe Thr Leu Ala
65                  70                  75                  80

Ile Ser Ser Val Met Pro Glu Asp Leu Ala Val Tyr Tyr Cys Gln Gln
                85                  90                  95

Tyr Tyr Asn Tyr Pro Trp Thr Phe Gly Gly Gly Thr Lys Leu Glu Ile
            100                 105                 110

Lys
```

<210> 125
<211> 464
<212> PRT
<213> artificial sequence

<220>
<223> anti-BCMA-CAR

<400> 125

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
```

<pre>
                20                        25                        30

        Ala Met Asn Trp Val Arg Gln Ala Pro Gly Lys Gly Leu Glu Trp Val
            35                      40                      45

        Ser Ala Ile Leu Ser Ser Gly Gly Ser Thr Tyr Tyr Ala Asp Ser Val
            50                      55                      60

        Lys Gly Arg Phe Thr Ile Ser Arg Asp Asn Ser Lys Asn Thr Leu Tyr
        65                      70                      75              80

        Leu Gln Met Asn Ser Leu Arg Ala Glu Asp Thr Ala Val Tyr Tyr Cys
                        85                      90                      95

        Ala Arg Tyr Trp Pro Met Asp Ile Trp Gly Gln Gly Thr Leu Val Thr
                    100                     105                     110

        Val Ser Ser Gly Gly Gly Gly Ser Gly Gly Gly Gly Ser Gly Gly Gly
            115                     120                     125

        Gly Ser Glu Ile Val Leu Thr Gln Ser Pro Gly Thr Leu Ser Leu Ser
            130                     135                     140

        Pro Gly Glu Arg Ala Thr Leu Ser Cys Arg Gly Gly Gln Ser Val Ser
        145                     150                     155                 160

        Ser Ser Tyr Leu Ala Trp Tyr Gln Gln Lys Pro Gly Gln Ala Pro Arg
                    165                     170                     175

        Leu Leu Met Tyr Asp Ala Ser Ile Arg Ala Thr Gly Ile Pro Asp Arg
                    180                     185                     190

        Phe Ser Gly Ser Gly Ser Gly Thr Asp Phe Thr Leu Thr Ile Ser Arg
                    195                     200                     205

        Leu Glu Pro Glu Asp Phe Ala Val Tyr Tyr Cys Gln Gln Tyr Gln Ser
            210                     215                     220

        Trp Pro Leu Thr Phe Gly Gln Gly Thr Lys Val Glu Ile Lys Ala Pro
        225                     230                     235                 240

        Thr Thr Thr Pro Ala Pro Arg Pro Pro Thr Pro Ala Pro Thr Ile Ala
                    245                     250                     255

        Ser Gln Pro Leu Ser Leu Arg Pro Glu Ala Cys Arg Pro Ala Ala Gly
                    260                     265                     270
</pre>

```
Gly Ala Val His Thr Arg Gly Leu Asp Phe Ala Cys Asp Ile Tyr Ile
        275                 280             285

Trp Ala Pro Leu Ala Gly Thr Cys Gly Val Leu Leu Leu Ser Leu Val
        290             295             300

Ile Thr Leu Tyr Cys Arg Arg Gly Arg Lys Lys Leu Leu Tyr Ile Phe
305                 310             315                 320

Lys Gln Pro Phe Met Arg Pro Val Gln Thr Thr Gln Glu Glu Asp Gly
                325             330             335

Cys Ser Cys Arg Phe Pro Glu Glu Glu Glu Gly Gly Cys Glu Leu Arg
        340             345             350

Val Lys Phe Ser Arg Ser Ala Asp Ala Pro Ala Tyr Gln Gln Gly Gln
        355             360             365

Asn Gln Leu Tyr Asn Glu Leu Asn Leu Gly Arg Arg Glu Glu Tyr Asp
    370             375             380

Val Leu Asp Lys Arg Arg Gly Arg Asp Pro Glu Met Gly Gly Lys Pro
385                 390             395                 400

Arg Arg Lys Asn Pro Gln Glu Gly Leu Tyr Asn Glu Leu Gln Lys Asp
                405             410             415

Lys Met Ala Glu Ala Tyr Ser Glu Ile Gly Met Lys Gly Glu Arg Arg
            420             425             430

Arg Gly Lys Gly His Asp Gly Leu Tyr Gln Gly Leu Ser Thr Ala Thr
            435             440             445

Lys Asp Thr Tyr Asp Ala Leu His Met Gln Ala Leu Pro Pro Arg Glu
    450             455             460
```

<210> 126
<211> 498
<212> PRT
<213> artificial sequence

<220>
<223> anti-BCMA-CAR

<400> 126

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr

|  | | 20 | | | | | | 25 | | | | | | 30 | |
|---|---|---|---|---|---|---|---|---|---|---|---|---|---|---|---|

Ala Met Asn Trp Val Arg Gln Ala Pro Gly Lys Gly Leu Glu Trp Val
        35              40              45

Ser Ala Ile Leu Ser Ser Gly Gly Ser Thr Tyr Tyr Ala Asp Ser Val
    50          55              60

Lys Gly Arg Phe Thr Ile Ser Arg Asp Asn Ser Lys Asn Thr Leu Tyr
65              70              75              80

Leu Gln Met Asn Ser Leu Arg Ala Glu Asp Thr Ala Val Tyr Tyr Cys
            85              90              95

Ala Arg Tyr Trp Pro Met Asp Ile Trp Gly Gln Gly Thr Leu Val Thr
        100             105             110

Val Ser Ser Gly Gly Gly Gly Ser Gly Gly Gly Gly Ser Gly Gly Gly
    115             120             125

Gly Ser Glu Ile Val Leu Thr Gln Ser Pro Gly Thr Leu Ser Leu Ser
    130             135             140

Pro Gly Glu Arg Ala Thr Leu Ser Cys Arg Gly Gly Gln Ser Val Ser
145             150             155             160

Ser Ser Tyr Leu Ala Trp Tyr Gln Gln Lys Pro Gly Gln Ala Pro Arg
            165             170             175

Leu Leu Met Tyr Asp Ala Ser Ile Arg Ala Thr Gly Ile Pro Asp Arg
            180             185             190

Phe Ser Gly Ser Gly Ser Gly Thr Asp Phe Thr Leu Thr Ile Ser Arg
            195             200             205

Leu Glu Pro Glu Asp Phe Ala Val Tyr Tyr Cys Gln Gln Tyr Gln Ser
    210             215             220

Trp Pro Leu Thr Phe Gly Gln Gly Thr Lys Val Glu Ile Lys Ser Asp
225             230             235             240

Pro Gly Ser Gly Gly Gly Gly Ser Cys Pro Tyr Ser Asn Pro Ser Leu
            245             250             255

Cys Ser Gly Gly Gly Gly Ser Cys Pro Tyr Ser Asn Pro Ser Leu Cys
            260             265             270

```
        Ala Pro Thr Thr Thr Pro Ala Pro Arg Pro Pro Thr Pro Ala Pro Thr
            275             280             285

        Ile Ala Ser Gln Pro Leu Ser Leu Arg Pro Glu Ala Cys Arg Pro Ala
            290             295             300

        Ala Gly Gly Ala Val His Thr Arg Gly Leu Asp Phe Ala Cys Asp Ile
            305             310             315             320

        Tyr Ile Trp Ala Pro Leu Ala Gly Thr Cys Gly Val Leu Leu Leu Ser
                    325             330             335

        Leu Val Ile Thr Leu Tyr Cys Arg Arg Gly Arg Lys Lys Leu Leu Tyr
                    340             345             350

        Ile Phe Lys Gln Pro Phe Met Arg Pro Val Gln Thr Thr Gln Glu Glu
                    355             360             365

        Asp Gly Cys Ser Cys Arg Phe Pro Glu Glu Glu Glu Gly Gly Cys Glu
                    370             375             380

        Leu Arg Val Lys Phe Ser Arg Ser Ala Asp Ala Pro Ala Tyr Gln Gln
            385             390             395             400

        Gly Gln Asn Gln Leu Tyr Asn Glu Leu Asn Leu Gly Arg Arg Glu Glu
                    405             410             415

        Tyr Asp Val Leu Asp Lys Arg Arg Gly Arg Asp Pro Glu Met Gly Gly
                    420             425             430

        Lys Pro Arg Arg Lys Asn Pro Gln Glu Gly Leu Tyr Asn Glu Leu Gln
                    435             440             445

        Lys Asp Lys Met Ala Glu Ala Tyr Ser Glu Ile Gly Met Lys Gly Glu
            450             455             460

        Arg Arg Arg Gly Lys Gly His Asp Gly Leu Tyr Gln Gly Leu Ser Thr
            465             470             475             480

        Ala Thr Lys Asp Thr Tyr Asp Ala Leu His Met Gln Ala Leu Pro Pro
                    485             490             495

        Arg Glu
```

<210> 127
<211> 513
<212> PRT

<213> artificial sequence

<220>
<223> anti-BCMA-CAR

<400> 127

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Asn Trp Val Arg Gln Ala Pro Gly Lys Gly Leu Glu Trp Val
        35                  40                  45

Ser Ala Ile Leu Ser Ser Gly Gly Ser Thr Tyr Tyr Ala Asp Ser Val
        50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Tyr Trp Pro Met Asp Ile Trp Gly Gln Gly Thr Leu Val Thr
            100                 105                 110

Val Ser Ser Gly Gly Gly Gly Ser Gly Gly Gly Gly Ser Gly Gly Gly
        115                 120                 125

Gly Ser Glu Ile Val Leu Thr Gln Ser Pro Gly Thr Leu Ser Leu Ser
        130                 135                 140

Pro Gly Glu Arg Ala Thr Leu Ser Cys Arg Gly Gly Gln Ser Val Ser
145                 150                 155                 160

Ser Ser Tyr Leu Ala Trp Tyr Gln Gln Lys Pro Gly Gln Ala Pro Arg
            165                 170                 175

Leu Leu Met Tyr Asp Ala Ser Ile Arg Ala Thr Gly Ile Pro Asp Arg
            180                 185                 190

Phe Ser Gly Ser Gly Ser Gly Thr Asp Phe Thr Leu Thr Ile Ser Arg
            195                 200                 205

Leu Glu Pro Glu Asp Phe Ala Val Tyr Tyr Cys Gln Gln Tyr Gln Ser
        210                 215                 220
```

```
Trp Pro Leu Thr Phe Gly Gln Gly Thr Lys Val Glu Ile Lys Ser Asp
225             230         235             240

Pro Gly Ser Gly Gly Gly Gly Ser Cys Pro Tyr Ser Asn Pro Ser Leu
            245             250             255

Cys Ser Gly Gly Gly Gly Ser Cys Pro Tyr Ser Asn Pro Ser Leu Cys
            260             265             270

Ser Gly Gly Gly Gly Ser Cys Pro Tyr Ser Asn Pro Ser Leu Cys Ala
        275             280             285

Pro Thr Thr Thr Pro Ala Pro Arg Pro Pro Thr Pro Ala Pro Thr Ile
    290             295             300

Ala Ser Gln Pro Leu Ser Leu Arg Pro Glu Ala Cys Arg Pro Ala Ala
305             310             315             320

Gly Gly Ala Val His Thr Arg Gly Leu Asp Phe Ala Cys Asp Ile Tyr
            325             330             335

Ile Trp Ala Pro Leu Ala Gly Thr Cys Gly Val Leu Leu Leu Ser Leu
            340             345             350

Val Ile Thr Leu Tyr Cys Arg Arg Gly Arg Lys Lys Leu Leu Tyr Ile
        355             360             365

Phe Lys Gln Pro Phe Met Arg Pro Val Gln Thr Thr Gln Glu Glu Asp
    370             375             380

Gly Cys Ser Cys Arg Phe Pro Glu Glu Glu Glu Gly Gly Cys Glu Leu
385             390             395             400

Arg Val Lys Phe Ser Arg Ser Ala Asp Ala Pro Ala Tyr Gln Gln Gly
            405             410             415

Gln Asn Gln Leu Tyr Asn Glu Leu Asn Leu Gly Arg Arg Glu Glu Tyr
        420             425             430

Asp Val Leu Asp Lys Arg Arg Gly Arg Asp Pro Glu Met Gly Gly Lys
        435             440             445

Pro Arg Arg Lys Asn Pro Gln Glu Gly Leu Tyr Asn Glu Leu Gln Lys
    450             455             460

Asp Lys Met Ala Glu Ala Tyr Ser Glu Ile Gly Met Lys Gly Glu Arg
465             470             475             480
```

205

Arg Arg Gly Lys Gly His Asp Gly Leu Tyr Gln Gly Leu Ser Thr Ala
485          490          495

Thr Lys Asp Thr Tyr Asp Ala Leu His Met Gln Ala Leu Pro Pro Arg
500          505          510

Glu

<210> 128
<211> 522
<212> PRT
<213> artificial sequence

<220>
<223> anti-BCMA-CAR

<400> 128

Glu Val Gln Leu Leu Glu Ser Gly Gly Gly Leu Val Gln Pro Gly Gly
1          5          10          15

Ser Leu Arg Leu Ser Cys Ala Ala Ser Gly Phe Thr Phe Ser Ser Tyr
20          25          30

Ala Met Asn Trp Val Arg Gln Ala Pro Gly Lys Gly Leu Glu Trp Val
35          40          45

Ser Ala Ile Leu Ser Ser Gly Gly Ser Thr Tyr Tyr Ala Asp Ser Val
50          55          60

Lys Gly Arg Phe Thr Ile Ser Arg Asp Asn Ser Lys Asn Thr Leu Tyr
65          70          75          80

Leu Gln Met Asn Ser Leu Arg Ala Glu Asp Thr Ala Val Tyr Tyr Cys
85          90          95

Ala Arg Tyr Trp Pro Met Asp Ile Trp Gly Gln Gly Thr Leu Val Thr
100          105          110

Val Ser Ser Gly Gly Gly Gly Ser Gly Gly Gly Gly Ser Gly Gly Gly
115          120          125

Gly Ser Glu Ile Val Leu Thr Gln Ser Pro Gly Thr Leu Ser Leu Ser
130          135          140

Pro Gly Glu Arg Ala Thr Leu Ser Cys Arg Gly Gly Gln Ser Val Ser
145          150          155          160

```
Ser Ser Tyr Leu Ala Trp Tyr Gln Gln Lys Pro Gly Gln Ala Pro Arg
            165                 170                 175

Leu Leu Met Tyr Asp Ala Ser Ile Arg Ala Thr Gly Ile Pro Asp Arg
            180                 185                 190

Phe Ser Gly Ser Gly Ser Gly Thr Asp Phe Thr Leu Thr Ile Ser Arg
            195                 200                 205

Leu Glu Pro Glu Asp Phe Ala Val Tyr Tyr Cys Gln Gln Tyr Gln Ser
    210                 215                 220

Trp Pro Leu Thr Phe Gly Gln Gly Thr Lys Val Glu Ile Lys Ser Asp
225                 230                 235                 240

Pro Gly Ser Gly Gly Gly Gly Ser Cys Pro Tyr Ser Asn Pro Ser Leu
                245                 250                 255

Cys Ser Gly Gly Gly Gly Ser Glu Leu Pro Thr Gln Gly Thr Phe Ser
                260                 265                 270

Asn Val Ser Thr Asn Val Ser Pro Ala Lys Pro Thr Thr Thr Ala Cys
            275                 280                 285

Pro Tyr Ser Asn Pro Ser Leu Cys Ala Pro Thr Thr Thr Pro Ala Pro
            290                 295                 300

Arg Pro Pro Thr Pro Ala Pro Thr Ile Ala Ser Gln Pro Leu Ser Leu
305                 310                 315                 320

Arg Pro Glu Ala Cys Arg Pro Ala Ala Gly Gly Ala Val His Thr Arg
                325                 330                 335

Gly Leu Asp Phe Ala Cys Asp Ile Tyr Ile Trp Ala Pro Leu Ala Gly
            340                 345                 350

Thr Cys Gly Val Leu Leu Leu Ser Leu Val Ile Thr Leu Tyr Cys Arg
            355                 360                 365

Arg Gly Arg Lys Lys Leu Leu Tyr Ile Phe Lys Gln Pro Phe Met Arg
            370                 375                 380

Pro Val Gln Thr Thr Gln Glu Glu Asp Gly Cys Ser Cys Arg Phe Pro
385                 390                 395                 400

Glu Glu Glu Glu Gly Gly Cys Glu Leu Arg Val Lys Phe Ser Arg Ser
                405                 410                 415
```

```
Ala Asp Ala Pro Ala Tyr Gln Gln Gly Gln Asn Gln Leu Tyr Asn Glu
            420             425             430

Leu Asn Leu Gly Arg Arg Glu Glu Tyr Asp Val Leu Asp Lys Arg Arg
            435             440             445

Gly Arg Asp Pro Glu Met Gly Gly Lys Pro Arg Arg Lys Asn Pro Gln
            450             455             460

Glu Gly Leu Tyr Asn Glu Leu Gln Lys Asp Lys Met Ala Glu Ala Tyr
465             470             475             480

Ser Glu Ile Gly Met Lys Gly Glu Arg Arg Arg Gly Lys Gly His Asp
                485             490             495

Gly Leu Tyr Gln Gly Leu Ser Thr Ala Thr Lys Asp Thr Tyr Asp Ala
            500             505             510

Leu His Met Gln Ala Leu Pro Pro Arg Glu
            515             520
```

<210> 129
<211> 500
<212> PRT
<213> artificial sequence

<220>
<223> anti-BCMA-CAR

<400> 129

```
Gly Gly Gly Gly Ser Cys Pro Tyr Ser Asn Pro Ser Leu Cys Ser Gly
1               5               10              15

Gly Gly Gly Ser Cys Pro Tyr Ser Asn Pro Ser Leu Cys Ser Gly Gly
            20              25              30

Gly Gly Ser Glu Val Gln Leu Leu Glu Ser Gly Gly Gly Leu Val Gln
            35              40              45

Pro Gly Gly Ser Leu Arg Leu Ser Cys Ala Ala Ser Gly Phe Thr Phe
            50              55              60

Ser Ser Tyr Ala Met Asn Trp Val Arg Gln Ala Pro Gly Lys Gly Leu
65              70              75              80

Glu Trp Val Ser Ala Ile Leu Ser Ser Gly Gly Ser Thr Tyr Tyr Ala
                85              90              95
```

```
Asp Ser Val Lys Gly Arg Phe Thr Ile Ser Arg Asp Asn Ser Lys Asn
        100                 105                 110

Thr Leu Tyr Leu Gln Met Asn Ser Leu Arg Ala Glu Asp Thr Ala Val
        115                 120                 125

Tyr Tyr Cys Ala Arg Tyr Trp Pro Met Asp Ile Trp Gly Gln Gly Thr
        130                 135                 140

Leu Val Thr Val Ser Ser Gly Gly Gly Gly Ser Gly Gly Gly Gly Ser
145                 150                 155                 160

Gly Gly Gly Gly Ser Glu Ile Val Leu Thr Gln Ser Pro Gly Thr Leu
                165                 170                 175

Ser Leu Ser Pro Gly Glu Arg Ala Thr Leu Ser Cys Arg Gly Gly Gln
        180                 185                 190

Ser Val Ser Ser Ser Tyr Leu Ala Trp Tyr Gln Gln Lys Pro Gly Gln
        195                 200                 205

Ala Pro Arg Leu Leu Met Tyr Asp Ala Ser Ile Arg Ala Thr Gly Ile
        210                 215                 220

Pro Asp Arg Phe Ser Gly Ser Gly Ser Gly Thr Asp Phe Thr Leu Thr
225                 230                 235                 240

Ile Ser Arg Leu Glu Pro Glu Asp Phe Ala Val Tyr Tyr Cys Gln Gln
                245                 250                 255

Tyr Gln Ser Trp Pro Leu Thr Phe Gly Gln Gly Thr Lys Val Glu Ile
        260                 265                 270

Lys Ser Asp Pro Thr Thr Thr Pro Ala Pro Arg Pro Pro Thr Pro Ala
        275                 280                 285

Pro Thr Ile Ala Ser Gln Pro Leu Ser Leu Arg Pro Glu Ala Cys Arg
        290                 295                 300

Pro Ala Ala Gly Gly Ala Val His Thr Arg Gly Leu Asp Phe Ala Cys
305                 310                 315                 320

Asp Ile Tyr Ile Trp Ala Pro Leu Ala Gly Thr Cys Gly Val Leu Leu
                325                 330                 335

Leu Ser Leu Val Ile Thr Leu Tyr Cys Arg Arg Gly Arg Lys Lys Leu
        340                 345                 350
```

```
Leu Tyr Ile Phe Lys Gln Pro Phe Met Arg Pro Val Gln Thr Thr Gln
        355             360             365

Glu Glu Asp Gly Cys Ser Cys Arg Phe Pro Glu Glu Glu Glu Gly Gly
        370             375             380

Cys Glu Leu Arg Val Lys Phe Ser Arg Ser Ala Asp Ala Pro Ala Tyr
385             390             395             400

Gln Gln Gly Gln Asn Gln Leu Tyr Asn Glu Leu Asn Leu Gly Arg Arg
                405             410             415

Glu Glu Tyr Asp Val Leu Asp Lys Arg Arg Gly Arg Asp Pro Glu Met
            420             425             430

Gly Gly Lys Pro Arg Arg Lys Asn Pro Gln Glu Gly Leu Tyr Asn Glu
            435             440             445

Leu Gln Lys Asp Lys Met Ala Glu Ala Tyr Ser Glu Ile Gly Met Lys
        450             455             460

Gly Glu Arg Arg Arg Gly Lys Gly His Asp Gly Leu Tyr Gln Gly Leu
465             470             475             480

Ser Thr Ala Thr Lys Asp Thr Tyr Asp Ala Leu His Met Gln Ala Leu
            485             490             495

Pro Pro Arg Glu
            500
```

<210> 130
<211> 466
<212> PRT
<213> artificial sequence

<220>
<223> anti-BCMA-CAR

<400> 130

```
Glu Val Gln Leu Leu Glu Ser Gly Gly Gly Leu Val Gln Pro Gly Gly
1               5               10              15

Ser Leu Arg Leu Ser Cys Ala Ala Ser Gly Phe Thr Phe Ser Ser Tyr
        20              25              30

Ala Met Asn Trp Val Arg Gln Ala Pro Gly Lys Gly Leu Glu Trp Val
        35              40              45
```

```
Ser Ala Ile Leu Ser Ser Gly Gly Ser Thr Tyr Tyr Ala Asp Ser Val
    50              55              60

Lys Gly Arg Phe Thr Ile Ser Arg Asp Asn Ser Lys Asn Thr Leu Tyr
65              70              75              80

Leu Gln Met Asn Ser Leu Arg Ala Glu Asp Thr Ala Val Tyr Tyr Cys
            85              90              95

Ala Arg Tyr Trp Pro Met Asp Ile Trp Gly Gln Gly Thr Leu Val Thr
        100             105             110

Val Ser Ser Gly Gly Ser Cys Pro Tyr Ser Asn Pro Ser Leu Cys Ser
    115             120             125

Gly Gly Ser Glu Ile Val Leu Thr Gln Ser Pro Gly Thr Leu Ser Leu
    130             135             140

Ser Pro Gly Glu Arg Ala Thr Leu Ser Cys Arg Gly Gly Gln Ser Val
145             150             155             160

Ser Ser Ser Tyr Leu Ala Trp Tyr Gln Gln Lys Pro Gly Gln Ala Pro
            165             170             175

Arg Leu Leu Met Tyr Asp Ala Ser Ile Arg Ala Thr Gly Ile Pro Asp
            180             185             190

Arg Phe Ser Gly Ser Gly Ser Gly Thr Asp Phe Thr Leu Thr Ile Ser
        195             200             205

Arg Leu Glu Pro Glu Asp Phe Ala Val Tyr Tyr Cys Gln Gln Tyr Gln
210             215             220

Ser Trp Pro Leu Thr Phe Gly Gln Gly Thr Lys Val Glu Ile Lys Ser
225             230             235             240

Asp Pro Thr Thr Thr Pro Ala Pro Arg Pro Pro Thr Pro Ala Pro Thr
            245             250             255

Ile Ala Ser Gln Pro Leu Ser Leu Arg Pro Glu Ala Cys Arg Pro Ala
        260             265             270

Ala Gly Gly Ala Val His Thr Arg Gly Leu Asp Phe Ala Cys Asp Ile
        275             280             285

Tyr Ile Trp Ala Pro Leu Ala Gly Thr Cys Gly Val Leu Leu Leu Ser
    290             295             300
```

211

```
Leu Val Ile Thr Leu Tyr Cys Arg Arg Gly Arg Lys Lys Leu Leu Tyr
305             310             315                 320


Ile Phe Lys Gln Pro Phe Met Arg Pro Val Gln Thr Thr Gln Glu Glu
            325             330                 335


Asp Gly Cys Ser Cys Arg Phe Pro Glu Glu Glu Glu Gly Gly Cys Glu
            340             345             350


Leu Arg Val Lys Phe Ser Arg Ser Ala Asp Ala Pro Ala Tyr Gln Gln
            355             360             365


Gly Gln Asn Gln Leu Tyr Asn Glu Leu Asn Leu Gly Arg Arg Glu Glu
        370             375             380


Tyr Asp Val Leu Asp Lys Arg Arg Gly Arg Asp Pro Glu Met Gly Gly
385             390             395                 400


Lys Pro Arg Arg Lys Asn Pro Gln Glu Gly Leu Tyr Asn Glu Leu Gln
                405             410             415


Lys Asp Lys Met Ala Glu Ala Tyr Ser Glu Ile Gly Met Lys Gly Glu
            420             425             430


Arg Arg Arg Gly Lys Gly His Asp Gly Leu Tyr Gln Gly Leu Ser Thr
            435             440             445


Ala Thr Lys Asp Thr Tyr Asp Ala Leu His Met Gln Ala Leu Pro Pro
    450             455             460


Arg Glu
465
```

<210> 131
<211> 472
<212> PRT
<213> artificial sequence

<220>
<223> anti-BCMA-CAR

<400> 131

```
Glu Val Gln Leu Leu Glu Ser Gly Gly Gly Leu Val Gln Pro Gly Gly
1               5               10                  15


Ser Leu Arg Leu Ser Cys Ala Ala Ser Gly Phe Thr Phe Ser Ser Tyr
            20              25              30
```

```
Ala Met Asn Trp Val Arg Gln Ala Pro Gly Lys Gly Leu Glu Trp Val
        35                  40                  45

Ser Ala Ile Leu Ser Ser Gly Gly Ser Thr Tyr Tyr Ala Asp Ser Val
        50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Tyr Trp Pro Met Asp Ile Trp Gly Gln Gly Thr Leu Val Thr
            100                 105                 110

Val Ser Ser Gly Gly Gly Gly Gly Ser Cys Pro Tyr Ser Asn Pro Ser
            115                 120                 125

Leu Cys Ser Gly Gly Gly Gly Gly Ser Glu Ile Val Leu Thr Gln Ser
        130                 135                 140

Pro Gly Thr Leu Ser Leu Ser Pro Gly Glu Arg Ala Thr Leu Ser Cys
145                 150                 155                 160

Arg Gly Gly Gln Ser Val Ser Ser Ser Tyr Leu Ala Trp Tyr Gln Gln
                165                 170                 175

Lys Pro Gly Gln Ala Pro Arg Leu Leu Met Tyr Asp Ala Ser Ile Arg
            180                 185                 190

Ala Thr Gly Ile Pro Asp Arg Phe Ser Gly Ser Gly Ser Gly Thr Asp
            195                 200                 205

Phe Thr Leu Thr Ile Ser Arg Leu Glu Pro Glu Asp Phe Ala Val Tyr
        210                 215                 220

Tyr Cys Gln Gln Tyr Gln Ser Trp Pro Leu Thr Phe Gly Gln Gly Thr
225                 230                 235                 240

Lys Val Glu Ile Lys Ser Asp Pro Thr Thr Thr Pro Ala Pro Arg Pro
                245                 250                 255

Pro Thr Pro Ala Pro Thr Ile Ala Ser Gln Pro Leu Ser Leu Arg Pro
            260                 265                 270

Glu Ala Cys Arg Pro Ala Ala Gly Gly Ala Val His Thr Arg Gly Leu
            275                 280                 285
```

Asp Phe Ala Cys Asp Ile Tyr Ile Trp Ala Pro Leu Ala Gly Thr Cys
290 295 300

Gly Val Leu Leu Leu Ser Leu Val Ile Thr Leu Tyr Cys Arg Arg Gly
305 310 315 320

Arg Lys Lys Leu Leu Tyr Ile Phe Lys Gln Pro Phe Met Arg Pro Val
325 330 335

Gln Thr Thr Gln Glu Glu Asp Gly Cys Ser Cys Arg Phe Pro Glu Glu
340 345 350

Glu Glu Gly Gly Cys Glu Leu Arg Val Lys Phe Ser Arg Ser Ala Asp
355 360 365

Ala Pro Ala Tyr Gln Gln Gly Gln Asn Gln Leu Tyr Asn Glu Leu Asn
370 375 380

Leu Gly Arg Arg Glu Glu Tyr Asp Val Leu Asp Lys Arg Arg Gly Arg
385 390 395 400

Asp Pro Glu Met Gly Gly Lys Pro Arg Arg Lys Asn Pro Gln Glu Gly
405 410 415

Leu Tyr Asn Glu Leu Gln Lys Asp Lys Met Ala Glu Ala Tyr Ser Glu
420 425 430

Ile Gly Met Lys Gly Glu Arg Arg Arg Gly Lys Gly His Asp Gly Leu
435 440 445

Tyr Gln Gly Leu Ser Thr Ala Thr Lys Asp Thr Tyr Asp Ala Leu His
450 455 460

Met Gln Ala Leu Pro Pro Arg Glu
465 470

<210> 132
<211> 491
<212> PRT
<213> artificial sequence

<220>
<223> anti-BCMA-CAR

<400> 132

Gly Gly Gly Gly Ser Cys Pro Tyr Ser Asn Pro Ser Leu Cys Ser Gly
1 5 10 15

Gly Gly Gly Ser Gly Gly Gly Gly Ser Glu Val Gln Leu Leu Glu Ser
                  20                    25                    30

Gly Gly Gly Leu Val Gln Pro Gly Gly Ser Leu Arg Leu Ser Cys Ala
                  35                    40                    45

Ala Ser Gly Phe Thr Phe Ser Ser Tyr Ala Met Asn Trp Val Arg Gln
                  50                    55                    60

Ala Pro Gly Lys Gly Leu Glu Trp Val Ser Ala Ile Leu Ser Ser Gly
65                    70                    75                    80

Gly Ser Thr Tyr Tyr Ala Asp Ser Val Lys Gly Arg Phe Thr Ile Ser
                  85                    90                    95

Arg Asp Asn Ser Lys Asn Thr Leu Tyr Leu Gln Met Asn Ser Leu Arg
                  100                   105                   110

Ala Glu Asp Thr Ala Val Tyr Tyr Cys Ala Arg Tyr Trp Pro Met Asp
                  115                   120                   125

Ile Trp Gly Gln Gly Thr Leu Val Thr Val Ser Ser Gly Gly Ser Cys
         130                   135                   140

Pro Tyr Ser Asn Pro Ser Leu Cys Ser Gly Gly Ser Glu Ile Val Leu
145                   150                   155                   160

Thr Gln Ser Pro Gly Thr Leu Ser Leu Ser Pro Gly Glu Arg Ala Thr
                  165                   170                   175

Leu Ser Cys Arg Gly Gly Gln Ser Val Ser Ser Ser Tyr Leu Ala Trp
                  180                   185                   190

Tyr Gln Gln Lys Pro Gly Gln Ala Pro Arg Leu Leu Met Tyr Asp Ala
                  195                   200                   205

Ser Ile Arg Ala Thr Gly Ile Pro Asp Arg Phe Ser Gly Ser Gly Ser
         210                   215                   220

Gly Thr Asp Phe Thr Leu Thr Ile Ser Arg Leu Glu Pro Glu Asp Phe
225                   230                   235                   240

Ala Val Tyr Tyr Cys Gln Gln Tyr Gln Ser Trp Pro Leu Thr Phe Gly
                  245                   250                   255

Gln Gly Thr Lys Val Glu Ile Lys Ser Asp Pro Thr Thr Thr Pro Ala
                  260                   265                   270

```
Pro Arg Pro Pro Thr Pro Ala Pro Thr Ile Ala Ser Gln Pro Leu Ser
        275             280             285

Leu Arg Pro Glu Ala Cys Arg Pro Ala Ala Gly Gly Ala Val His Thr
        290             295             300

Arg Gly Leu Asp Phe Ala Cys Asp Ile Tyr Ile Trp Ala Pro Leu Ala
        305             310             315             320

Gly Thr Cys Gly Val Leu Leu Leu Ser Leu Val Ile Thr Leu Tyr Cys
                325             330             335

Arg Arg Gly Arg Lys Lys Leu Leu Tyr Ile Phe Lys Gln Pro Phe Met
            340             345             350

Arg Pro Val Gln Thr Thr Gln Glu Glu Asp Gly Cys Ser Cys Arg Phe
        355             360             365

Pro Glu Glu Glu Glu Gly Gly Cys Glu Leu Arg Val Lys Phe Ser Arg
        370             375             380

Ser Ala Asp Ala Pro Ala Tyr Gln Gln Gly Gln Asn Gln Leu Tyr Asn
385             390             395             400

Glu Leu Asn Leu Gly Arg Arg Glu Glu Tyr Asp Val Leu Asp Lys Arg
                405             410             415

Arg Gly Arg Asp Pro Glu Met Gly Gly Lys Pro Arg Arg Lys Asn Pro
            420             425             430

Gln Glu Gly Leu Tyr Asn Glu Leu Gln Lys Asp Lys Met Ala Glu Ala
            435             440             445

Tyr Ser Glu Ile Gly Met Lys Gly Glu Arg Arg Arg Gly Lys Gly His
        450             455             460

Asp Gly Leu Tyr Gln Gly Leu Ser Thr Ala Thr Lys Asp Thr Tyr Asp
465             470             475             480

Ala Leu His Met Gln Ala Leu Pro Pro Arg Glu
                485             490
```

<210> 133
<211> 497
<212> PRT
<213> artificial sequence

<220>

<223> anti-BCMA-CAR

<400> 133

Gly Gly Gly Gly Ser Cys Pro Tyr Ser Asn Pro Ser Leu Cys Ser Gly
1               5                   10              15

Gly Gly Gly Ser Gly Gly Gly Gly Ser Glu Val Gln Leu Leu Glu Ser
        20                  25              30

Gly Gly Gly Leu Val Gln Pro Gly Gly Ser Leu Arg Leu Ser Cys Ala
        35              40              45

Ala Ser Gly Phe Thr Phe Ser Ser Tyr Ala Met Asn Trp Val Arg Gln
    50              55              60

Ala Pro Gly Lys Gly Leu Glu Trp Val Ser Ala Ile Leu Ser Ser Gly
65              70              75              80

Gly Ser Thr Tyr Tyr Ala Asp Ser Val Lys Gly Arg Phe Thr Ile Ser
            85              90              95

Arg Asp Asn Ser Lys Asn Thr Leu Tyr Leu Gln Met Asn Ser Leu Arg
            100             105             110

Ala Glu Asp Thr Ala Val Tyr Tyr Cys Ala Arg Tyr Trp Pro Met Asp
        115             120             125

Ile Trp Gly Gln Gly Thr Leu Val Thr Val Ser Ser Gly Gly Gly Gly
    130             135             140

Gly Ser Cys Pro Tyr Ser Asn Pro Ser Leu Cys Ser Gly Gly Gly Gly
145             150             155             160

Gly Ser Glu Ile Val Leu Thr Gln Ser Pro Gly Thr Leu Ser Leu Ser
            165             170             175

Pro Gly Glu Arg Ala Thr Leu Ser Cys Arg Gly Gly Gln Ser Val Ser
            180             185             190

Ser Ser Tyr Leu Ala Trp Tyr Gln Gln Lys Pro Gly Gln Ala Pro Arg
        195             200             205

Leu Leu Met Tyr Asp Ala Ser Ile Arg Ala Thr Gly Ile Pro Asp Arg
    210             215             220

Phe Ser Gly Ser Gly Ser Gly Thr Asp Phe Thr Leu Thr Ile Ser Arg
225             230             235             240

```
Leu Glu Pro Glu Asp Phe Ala Val Tyr Tyr Cys Gln Gln Tyr Gln Ser
              245             250                 255

Trp Pro Leu Thr Phe Gly Gln Gly Thr Lys Val Glu Ile Lys Ser Asp
            260             265             270

Pro Thr Thr Thr Pro Ala Pro Arg Pro Pro Thr Pro Ala Pro Thr Ile
        275             280             285

Ala Ser Gln Pro Leu Ser Leu Arg Pro Glu Ala Cys Arg Pro Ala Ala
    290             295             300

Gly Gly Ala Val His Thr Arg Gly Leu Asp Phe Ala Cys Asp Ile Tyr
305             310             315             320

Ile Trp Ala Pro Leu Ala Gly Thr Cys Gly Val Leu Leu Leu Ser Leu
            325             330             335

Val Ile Thr Leu Tyr Cys Arg Arg Gly Arg Lys Lys Leu Leu Tyr Ile
            340             345             350

Phe Lys Gln Pro Phe Met Arg Pro Val Gln Thr Thr Gln Glu Glu Asp
        355             360             365

Gly Cys Ser Cys Arg Phe Pro Glu Glu Glu Glu Gly Gly Cys Glu Leu
    370             375             380

Arg Val Lys Phe Ser Arg Ser Ala Asp Ala Pro Ala Tyr Gln Gln Gly
385             390             395             400

Gln Asn Gln Leu Tyr Asn Glu Leu Asn Leu Gly Arg Arg Glu Glu Tyr
            405             410             415

Asp Val Leu Asp Lys Arg Arg Gly Arg Asp Pro Glu Met Gly Gly Lys
            420             425             430

Pro Arg Arg Lys Asn Pro Gln Glu Gly Leu Tyr Asn Glu Leu Gln Lys
        435             440             445

Asp Lys Met Ala Glu Ala Tyr Ser Glu Ile Gly Met Lys Gly Glu Arg
    450             455             460

Arg Arg Gly Lys Gly His Asp Gly Leu Tyr Gln Gly Leu Ser Thr Ala
465             470             475             480

Thr Lys Asp Thr Tyr Asp Ala Leu His Met Gln Ala Leu Pro Pro Arg
            485             490             495
```

Glu

<210> 134
<211> 490
<212> PRT
<213> artificial sequence

<220>
<223> anti-BCMA-CAR

<400> 134

```
Glu Val Gln Leu Leu Glu Ser Gly Gly Gly Leu Val Gln Pro Gly Gly
1               5               10              15

Ser Leu Arg Leu Ser Cys Ala Ala Ser Gly Phe Thr Phe Ser Ser Tyr
            20              25              30

Ala Met Asn Trp Val Arg Gln Ala Pro Gly Lys Gly Leu Glu Trp Val
            35              40              45

Ser Ala Ile Leu Ser Ser Gly Gly Ser Thr Tyr Tyr Ala Asp Ser Val
        50              55              60

Lys Gly Arg Phe Thr Ile Ser Arg Asp Asn Ser Lys Asn Thr Leu Tyr
65              70              75              80

Leu Gln Met Asn Ser Leu Arg Ala Glu Asp Thr Ala Val Tyr Tyr Cys
                85              90              95

Ala Arg Tyr Trp Pro Met Asp Ile Trp Gly Gln Gly Thr Leu Val Thr
            100             105             110

Val Ser Ser Gly Gly Ser Cys Pro Tyr Ser Asn Pro Ser Leu Cys Ser
        115             120             125

Gly Gly Ser Glu Ile Val Leu Thr Gln Ser Pro Gly Thr Leu Ser Leu
        130             135             140

Ser Pro Gly Glu Arg Ala Thr Leu Ser Cys Arg Gly Gly Gln Ser Val
145             150             155             160

Ser Ser Ser Tyr Leu Ala Trp Tyr Gln Gln Lys Pro Gly Gln Ala Pro
                165             170             175

Arg Leu Leu Met Tyr Asp Ala Ser Ile Arg Ala Thr Gly Ile Pro Asp
            180             185             190
```

Arg Phe Ser Gly Ser Gly Ser Gly Thr Asp Phe Thr Leu Thr Ile Ser
        195                 200             205

Arg Leu Glu Pro Glu Asp Phe Ala Val Tyr Tyr Cys Gln Gln Tyr Gln
        210             215                 220

Ser Trp Pro Leu Thr Phe Gly Gln Gly Thr Lys Val Glu Ile Lys Ser
225             230                 235                     240

Asp Pro Gly Ser Gly Gly Gly Gly Ser Cys Pro Tyr Ser Asn Pro Ser
                245                 250                 255

Leu Cys Ser Gly Gly Gly Gly Ser Ala Pro Thr Thr Thr Pro Ala Pro
            260             265                 270

Arg Pro Pro Thr Pro Ala Pro Thr Ile Ala Ser Gln Pro Leu Ser Leu
        275             280                 285

Arg Pro Glu Ala Cys Arg Pro Ala Ala Gly Gly Ala Val His Thr Arg
        290             295                 300

Gly Leu Asp Phe Ala Cys Asp Ile Tyr Ile Trp Ala Pro Leu Ala Gly
305             310                 315                     320

Thr Cys Gly Val Leu Leu Leu Ser Leu Val Ile Thr Leu Tyr Cys Arg
                325                 330                 335

Arg Gly Arg Lys Lys Leu Leu Tyr Ile Phe Lys Gln Pro Phe Met Arg
            340                 345                 350

Pro Val Gln Thr Thr Gln Glu Glu Asp Gly Cys Ser Cys Arg Phe Pro
        355                 360                 365

Glu Glu Glu Glu Gly Gly Cys Glu Leu Arg Val Lys Phe Ser Arg Ser
        370                 375                 380

Ala Asp Ala Pro Ala Tyr Gln Gln Gly Gln Asn Gln Leu Tyr Asn Glu
385                 390                 395                     400

Leu Asn Leu Gly Arg Arg Glu Glu Tyr Asp Val Leu Asp Lys Arg Arg
                405                 410                 415

Gly Arg Asp Pro Glu Met Gly Gly Lys Pro Arg Arg Lys Asn Pro Gln
            420                 425                 430

Glu Gly Leu Tyr Asn Glu Leu Gln Lys Asp Lys Met Ala Glu Ala Tyr
            435                 440                 445

222

```
            Ser Glu Ile Gly Met Lys Gly Glu Arg Arg Arg Gly Lys Gly His Asp
                450                 455                 460

            Gly Leu Tyr Gln Gly Leu Ser Thr Ala Thr Lys Asp Thr Tyr Asp Ala
                465                 470                 475                 480

            Leu His Met Gln Ala Leu Pro Pro Arg Glu
                            485                 490
```

<210> 135
<211> 496
<212> PRT
<213> artificial sequence

<220>
<223> anti-BCMA-CAR

<400> 135

```
            Glu Val Gln Leu Leu Glu Ser Gly Gly Gly Leu Val Gln Pro Gly Gly
            1                 5                   10                  15

            Ser Leu Arg Leu Ser Cys Ala Ala Ser Gly Phe Thr Phe Ser Ser Tyr
                        20                  25                  30

            Ala Met Asn Trp Val Arg Gln Ala Pro Gly Lys Gly Leu Glu Trp Val
                        35                  40                  45

            Ser Ala Ile Leu Ser Ser Gly Gly Ser Thr Tyr Tyr Ala Asp Ser Val
                50                  55                  60

            Lys Gly Arg Phe Thr Ile Ser Arg Asp Asn Ser Lys Asn Thr Leu Tyr
            65                  70                  75                  80

            Leu Gln Met Asn Ser Leu Arg Ala Glu Asp Thr Ala Val Tyr Tyr Cys
                            85                  90                  95

            Ala Arg Tyr Trp Pro Met Asp Ile Trp Gly Gln Gly Thr Leu Val Thr
                        100                 105                 110

            Val Ser Ser Gly Gly Gly Gly Gly Ser Cys Pro Tyr Ser Asn Pro Ser
                        115                 120                 125

            Leu Cys Ser Gly Gly Gly Gly Ser Glu Ile Val Leu Thr Gln Ser
                130                 135                 140

            Pro Gly Thr Leu Ser Leu Ser Pro Gly Glu Arg Ala Thr Leu Ser Cys
                145                 150                 155                 160
```

Arg Gly Gly Gln Ser Val Ser Ser Ser Tyr Leu Ala Trp Tyr Gln Gln
                165            170            175

Lys Pro Gly Gln Ala Pro Arg Leu Leu Met Tyr Asp Ala Ser Ile Arg
                180            185            190

Ala Thr Gly Ile Pro Asp Arg Phe Ser Gly Ser Gly Ser Gly Thr Asp
                195            200            205

Phe Thr Leu Thr Ile Ser Arg Leu Glu Pro Glu Asp Phe Ala Val Tyr
      210            215            220

Tyr Cys Gln Gln Tyr Gln Ser Trp Pro Leu Thr Phe Gly Gln Gly Thr
225               230            235            240

Lys Val Glu Ile Lys Ser Asp Pro Gly Ser Gly Gly Gly Gly Ser Cys
                245            250            255

Pro Tyr Ser Asn Pro Ser Leu Cys Ser Gly Gly Gly Gly Ser Ala Pro
                260            265            270

Thr Thr Thr Pro Ala Pro Arg Pro Pro Thr Pro Ala Pro Thr Ile Ala
                275            280            285

Ser Gln Pro Leu Ser Leu Arg Pro Glu Ala Cys Arg Pro Ala Ala Gly
      290            295            300

Gly Ala Val His Thr Arg Gly Leu Asp Phe Ala Cys Asp Ile Tyr Ile
305               310            315            320

Trp Ala Pro Leu Ala Gly Thr Cys Gly Val Leu Leu Leu Ser Leu Val
                325            330            335

Ile Thr Leu Tyr Cys Arg Arg Gly Arg Lys Lys Leu Leu Tyr Ile Phe
                340            345            350

Lys Gln Pro Phe Met Arg Pro Val Gln Thr Thr Gln Glu Glu Asp Gly
                355            360            365

Cys Ser Cys Arg Phe Pro Glu Glu Glu Gly Gly Cys Glu Leu Arg
      370            375            380

Val Lys Phe Ser Arg Ser Ala Asp Ala Pro Ala Tyr Gln Gln Gly Gln
385               390            395            400

Asn Gln Leu Tyr Asn Glu Leu Asn Leu Gly Arg Arg Glu Glu Tyr Asp
                405            410            415

```
Val Leu Asp Lys Arg Arg Gly Arg Asp Pro Glu Met Gly Gly Lys Pro
            420             425             430

Arg Arg Lys Asn Pro Gln Glu Gly Leu Tyr Asn Glu Leu Gln Lys Asp
            435             440             445

Lys Met Ala Glu Ala Tyr Ser Glu Ile Gly Met Lys Gly Glu Arg Arg
            450             455             460

Arg Gly Lys Gly His Asp Gly Leu Tyr Gln Gly Leu Ser Thr Ala Thr
465             470             475             480

Lys Asp Thr Tyr Asp Ala Leu His Met Gln Ala Leu Pro Pro Arg Glu
            485             490             495
```

<210> 136
<211> 514
<212> PRT
<213> artificial sequence

<220>
<223> anti-BCMA-CAR

<400> 136

```
Gly Gly Gly Gly Ser Cys Pro Tyr Ser Asn Pro Ser Leu Cys Ser Gly
1           5               10              15

Gly Gly Gly Ser Gly Gly Gly Gly Ser Glu Val Gln Leu Leu Glu Ser
            20              25              30

Gly Gly Gly Leu Val Gln Pro Gly Gly Ser Leu Arg Leu Ser Cys Ala
            35              40              45

Ala Ser Gly Phe Thr Phe Ser Ser Tyr Ala Met Asn Trp Val Arg Gln
            50              55              60

Ala Pro Gly Lys Gly Leu Glu Trp Val Ser Ala Ile Leu Ser Ser Gly
65              70              75              80

Gly Ser Thr Tyr Tyr Ala Asp Ser Val Lys Gly Arg Phe Thr Ile Ser
            85              90              95

Arg Asp Asn Ser Lys Asn Thr Leu Tyr Leu Gln Met Asn Ser Leu Arg
            100             105             110

Ala Glu Asp Thr Ala Val Tyr Tyr Cys Ala Arg Tyr Trp Pro Met Asp
            115             120             125
```

225

```
Ile Trp Gly Gln Gly Thr Leu Val Thr Val Ser Ser Gly Gly Gly Gly
    130             135             140

Ser Gly Gly Gly Gly Ser Gly Gly Gly Gly Ser Glu Ile Val Leu Thr
    145             150             155             160

Gln Ser Pro Gly Thr Leu Ser Leu Ser Pro Gly Glu Arg Ala Thr Leu
            165             170             175

Ser Cys Arg Gly Gly Gln Ser Val Ser Ser Ser Tyr Leu Ala Trp Tyr
        180             185             190

Gln Gln Lys Pro Gly Gln Ala Pro Arg Leu Leu Met Tyr Asp Ala Ser
        195             200             205

Ile Arg Ala Thr Gly Ile Pro Asp Arg Phe Ser Gly Ser Gly Ser Gly
    210             215             220

Thr Asp Phe Thr Leu Thr Ile Ser Arg Leu Glu Pro Glu Asp Phe Ala
225             230             235             240

Val Tyr Tyr Cys Gln Gln Tyr Gln Ser Trp Pro Leu Thr Phe Gly Gln
            245             250             255

Gly Thr Lys Val Glu Ile Lys Ser Asp Pro Gly Ser Gly Gly Gly Gly
        260             265             270

Ser Cys Pro Tyr Ser Asn Pro Ser Leu Cys Ser Gly Gly Gly Gly Ser
        275             280             285

Ala Pro Thr Thr Thr Pro Ala Pro Arg Pro Pro Thr Pro Ala Pro Thr
    290             295             300

Ile Ala Ser Gln Pro Leu Ser Leu Arg Pro Glu Ala Cys Arg Pro Ala
305             310             315             320

Ala Gly Gly Ala Val His Thr Arg Gly Leu Asp Phe Ala Cys Asp Ile
            325             330             335

Tyr Ile Trp Ala Pro Leu Ala Gly Thr Cys Gly Val Leu Leu Leu Ser
        340             345             350

Leu Val Ile Thr Leu Tyr Cys Arg Arg Gly Arg Lys Lys Leu Leu Tyr
        355             360             365

Ile Phe Lys Gln Pro Phe Met Arg Pro Val Gln Thr Thr Gln Glu Glu
    370             375             380
```

```
Asp Gly Cys Ser Cys Arg Phe Pro Glu Glu Glu Glu Gly Gly Cys Glu
385                 390             395                     400

Leu Arg Val Lys Phe Ser Arg Ser Ala Asp Ala Pro Ala Tyr Gln Gln
                405             410             415

Gly Gln Asn Gln Leu Tyr Asn Glu Leu Asn Leu Gly Arg Arg Glu Glu
            420             425             430

Tyr Asp Val Leu Asp Lys Arg Arg Gly Arg Asp Pro Glu Met Gly Gly
        435             440             445

Lys Pro Arg Arg Lys Asn Pro Gln Glu Gly Leu Tyr Asn Glu Leu Gln
    450             455             460

Lys Asp Lys Met Ala Glu Ala Tyr Ser Glu Ile Gly Met Lys Gly Glu
465             470             475                     480

Arg Arg Arg Gly Lys Gly His Asp Gly Leu Tyr Gln Gly Leu Ser Thr
            485             490                     495

Ala Thr Lys Asp Thr Tyr Asp Ala Leu His Met Gln Ala Leu Pro Pro
            500             505             510

Arg Glu
```

<210> 137
<211> 515
<212> PRT
<213> artificial sequence

<220>
<223> anti-BCMA-CAR

<400> 137

```
Gly Gly Gly Gly Ser Cys Pro Tyr Ser Asn Pro Ser Leu Cys Ser Gly
1               5               10              15

Gly Gly Gly Ser Gly Gly Gly Gly Ser Glu Val Gln Leu Leu Glu Ser
            20              25              30

Gly Gly Gly Leu Val Gln Pro Gly Gly Ser Leu Arg Leu Ser Cys Ala
        35              40              45

Ala Ser Gly Phe Thr Phe Ser Ser Tyr Ala Met Asn Trp Val Arg Gln
    50              55              60
```

Ala Pro Gly Lys Gly Leu Glu Trp Val Ser Ala Ile Leu Ser Ser Gly
65                  70                  75                  80

Gly Ser Thr Tyr Tyr Ala Asp Ser Val Lys Gly Arg Phe Thr Ile Ser
                85                  90                  95

Arg Asp Asn Ser Lys Asn Thr Leu Tyr Leu Gln Met Asn Ser Leu Arg
            100                 105                 110

Ala Glu Asp Thr Ala Val Tyr Tyr Cys Ala Arg Tyr Trp Pro Met Asp
        115                 120                 125

Ile Trp Gly Gln Gly Thr Leu Val Thr Val Ser Ser Gly Gly Ser Cys
    130                 135                 140

Pro Tyr Ser Asn Pro Ser Leu Cys Ser Gly Gly Ser Glu Ile Val Leu
145                 150                 155                 160

Thr Gln Ser Pro Gly Thr Leu Ser Leu Ser Pro Gly Glu Arg Ala Thr
                165                 170                 175

Leu Ser Cys Arg Gly Gly Gln Ser Val Ser Ser Ser Tyr Leu Ala Trp
            180                 185                 190

Tyr Gln Gln Lys Pro Gly Gln Ala Pro Arg Leu Leu Met Tyr Asp Ala
        195                 200                 205

Ser Ile Arg Ala Thr Gly Ile Pro Asp Arg Phe Ser Gly Ser Gly Ser
    210                 215                 220

Gly Thr Asp Phe Thr Leu Thr Ile Ser Arg Leu Glu Pro Glu Asp Phe
225                 230                 235                 240

Ala Val Tyr Tyr Cys Gln Gln Tyr Gln Ser Trp Pro Leu Thr Phe Gly
                245                 250                 255

Gln Gly Thr Lys Val Glu Ile Lys Ser Asp Pro Gly Ser Gly Gly Gly
            260                 265                 270

Gly Ser Cys Pro Tyr Ser Asn Pro Ser Leu Cys Ser Gly Gly Gly Gly
        275                 280                 285

Ser Ala Pro Thr Thr Thr Pro Ala Pro Arg Pro Pro Thr Pro Ala Pro
        290                 295                 300

Thr Ile Ala Ser Gln Pro Leu Ser Leu Arg Pro Glu Ala Cys Arg Pro
305                 310                 315                 320

228

```
Ala Ala Gly Gly Ala Val His Thr Arg Gly Leu Asp Phe Ala Cys Asp
            325             330             335

Ile Tyr Ile Trp Ala Pro Leu Ala Gly Thr Cys Gly Val Leu Leu Leu
            340             345             350

Ser Leu Val Ile Thr Leu Tyr Cys Arg Arg Gly Arg Lys Lys Leu Leu
            355             360             365

Tyr Ile Phe Lys Gln Pro Phe Met Arg Pro Val Gln Thr Thr Gln Glu
    370             375             380

Glu Asp Gly Cys Ser Cys Arg Phe Pro Glu Glu Glu Glu Gly Gly Cys
385             390             395             400

Glu Leu Arg Val Lys Phe Ser Arg Ser Ala Asp Ala Pro Ala Tyr Gln
            405             410             415

Gln Gly Gln Asn Gln Leu Tyr Asn Glu Leu Asn Leu Gly Arg Arg Glu
            420             425             430

Glu Tyr Asp Val Leu Asp Lys Arg Arg Gly Arg Asp Pro Glu Met Gly
            435             440             445

Gly Lys Pro Arg Arg Lys Asn Pro Gln Glu Gly Leu Tyr Asn Glu Leu
    450             455             460

Gln Lys Asp Lys Met Ala Glu Ala Tyr Ser Glu Ile Gly Met Lys Gly
465             470             475             480

Glu Arg Arg Arg Gly Lys Gly His Asp Gly Leu Tyr Gln Gly Leu Ser
            485             490             495

Thr Ala Thr Lys Asp Thr Tyr Asp Ala Leu His Met Gln Ala Leu Pro
            500             505             510

Pro Arg Glu
            515
```

<210> 138
<211> 521
<212> PRT
<213> artificial sequence

<220>
<223> anti-BCMA-CAR

<400> 138

```
Gly Gly Gly Gly Ser Cys Pro Tyr Ser Asn Pro Ser Leu Cys Ser Gly
1               5                   10              15

Gly Gly Gly Ser Gly Gly Gly Gly Ser Glu Val Gln Leu Leu Glu Ser
            20              25                  30

Gly Gly Gly Leu Val Gln Pro Gly Gly Ser Leu Arg Leu Ser Cys Ala
        35              40                  45

Ala Ser Gly Phe Thr Phe Ser Ser Tyr Ala Met Asn Trp Val Arg Gln
    50              55                  60

Ala Pro Gly Lys Gly Leu Glu Trp Val Ser Ala Ile Leu Ser Ser Gly
65              70                  75                  80

Gly Ser Thr Tyr Tyr Ala Asp Ser Val Lys Gly Arg Phe Thr Ile Ser
            85                  90                  95

Arg Asp Asn Ser Lys Asn Thr Leu Tyr Leu Gln Met Asn Ser Leu Arg
            100             105             110

Ala Glu Asp Thr Ala Val Tyr Tyr Cys Ala Arg Tyr Trp Pro Met Asp
        115             120             125

Ile Trp Gly Gln Gly Thr Leu Val Thr Val Ser Ser Gly Gly Gly Gly
    130             135             140

Gly Ser Cys Pro Tyr Ser Asn Pro Ser Leu Cys Ser Gly Gly Gly Gly
145             150             155             160

Gly Ser Glu Ile Val Leu Thr Gln Ser Pro Gly Thr Leu Ser Leu Ser
            165             170             175

Pro Gly Glu Arg Ala Thr Leu Ser Cys Arg Gly Gly Gln Ser Val Ser
        180             185             190

Ser Ser Tyr Leu Ala Trp Tyr Gln Gln Lys Pro Gly Gln Ala Pro Arg
    195             200             205

Leu Leu Met Tyr Asp Ala Ser Ile Arg Ala Thr Gly Ile Pro Asp Arg
    210             215             220

Phe Ser Gly Ser Gly Ser Gly Thr Asp Phe Thr Leu Thr Ile Ser Arg
225             230             235             240

Leu Glu Pro Glu Asp Phe Ala Val Tyr Tyr Cys Gln Gln Tyr Gln Ser
            245             250             255
```

230

```
Trp Pro Leu Thr Phe Gly Gln Gly Thr Lys Val Glu Ile Lys Ser Asp
        260                 265             270

Pro Gly Ser Gly Gly Gly Gly Ser Cys Pro Tyr Ser Asn Pro Ser Leu
        275             280                 285

Cys Ser Gly Gly Gly Gly Ser Ala Pro Thr Thr Thr Pro Ala Pro Arg
    290             295                 300

Pro Pro Thr Pro Ala Pro Thr Ile Ala Ser Gln Pro Leu Ser Leu Arg
305             310                 315                 320

Pro Glu Ala Cys Arg Pro Ala Ala Gly Gly Ala Val His Thr Arg Gly
            325             330                 335

Leu Asp Phe Ala Cys Asp Ile Tyr Ile Trp Ala Pro Leu Ala Gly Thr
        340                 345                 350

Cys Gly Val Leu Leu Leu Ser Leu Val Ile Thr Leu Tyr Cys Arg Arg
        355                 360                 365

Gly Arg Lys Lys Leu Leu Tyr Ile Phe Lys Gln Pro Phe Met Arg Pro
    370                 375                 380

Val Gln Thr Thr Gln Glu Glu Asp Gly Cys Ser Cys Arg Phe Pro Glu
385                 390                 395                 400

Glu Glu Glu Gly Gly Cys Glu Leu Arg Val Lys Phe Ser Arg Ser Ala
            405                 410                 415

Asp Ala Pro Ala Tyr Gln Gln Gly Gln Asn Gln Leu Tyr Asn Glu Leu
        420                 425                 430

Asn Leu Gly Arg Arg Glu Glu Tyr Asp Val Leu Asp Lys Arg Arg Gly
        435                 440                 445

Arg Asp Pro Glu Met Gly Gly Lys Pro Arg Arg Lys Asn Pro Gln Glu
    450             455                 460

Gly Leu Tyr Asn Glu Leu Gln Lys Asp Lys Met Ala Glu Ala Tyr Ser
465             470                 475                 480

Glu Ile Gly Met Lys Gly Glu Arg Arg Arg Gly Lys Gly His Asp Gly
            485                 490                 495

Leu Tyr Gln Gly Leu Ser Thr Ala Thr Lys Asp Thr Tyr Asp Ala Leu
```

```
                              500                    505                        510


                   His Met Gln Ala Leu Pro Pro Arg Glu
                               515                    520
```

<210> 139
<211> 504
<212> PRT
<213> artificial sequence

<220>
<223> anti-BCMA-CAR

<400> 139

```
Glu Val Gln Leu Leu Glu Ser Gly Gly Gly Leu Val Gln Pro Gly Gly
1               5                10                15

Ser Leu Arg Leu Ser Cys Ala Ala Ser Gly Phe Thr Phe Ser Ser Tyr
            20              25                30

Ala Met Asn Trp Val Arg Gln Ala Pro Gly Lys Gly Leu Glu Trp Val
        35                40                45

Ser Ala Ile Leu Ser Ser Gly Gly Ser Thr Tyr Tyr Ala Asp Ser Val
    50              55                60

Lys Gly Arg Phe Thr Ile Ser Arg Asp Asn Ser Lys Asn Thr Leu Tyr
65                  70              75                  80

Leu Gln Met Asn Ser Leu Arg Ala Glu Asp Thr Ala Val Tyr Tyr Cys
            85                90                95

Ala Arg Tyr Trp Pro Met Asp Ile Trp Gly Gln Gly Thr Leu Val Thr
        100                105                110

Val Ser Ser Gly Gly Gly Gly Ser Gly Gly Gly Gly Ser Gly Gly Gly
    115             120                125

Gly Ser Glu Ile Val Leu Thr Gln Ser Pro Gly Thr Leu Ser Leu Ser
    130             135                140

Pro Gly Glu Arg Ala Thr Leu Ser Cys Arg Gly Gly Gln Ser Val Ser
145             150                155                160

Ser Ser Tyr Leu Ala Trp Tyr Gln Gln Lys Pro Gly Gln Ala Pro Arg
            165                170                175

Leu Leu Met Tyr Asp Ala Ser Ile Arg Ala Thr Gly Ile Pro Asp Arg
            180                185                190
```

233

Phe Ser Gly Ser Gly Ser Gly Thr Asp Phe Thr Leu Thr Ile Ser Arg
195 200 205

Leu Glu Pro Glu Asp Phe Ala Val Tyr Tyr Cys Gln Gln Tyr Gln Ser
210 215 220

Trp Pro Leu Thr Phe Gly Gln Gly Thr Lys Val Glu Ile Lys Ser Asp
225 230 235 240

Pro Gly Ser Gly Gly Gly Gly Ser Cys Pro Tyr Ser Asn Pro Ser Leu
245 250 255

Cys Ser Gly Gly Gly Gly Ser Cys Pro Tyr Ser Asn Pro Ser Leu Cys
260 265 270

Ser Gly Gly Gly Gly Ser Ala Pro Thr Thr Thr Pro Ala Pro Arg Pro
275 280 285

Pro Thr Pro Ala Pro Thr Ile Ala Ser Gln Pro Leu Ser Leu Arg Pro
290 295 300

Glu Ala Cys Arg Pro Ala Ala Gly Gly Ala Val His Thr Arg Gly Leu
305 310 315 320

Asp Phe Ala Cys Asp Ile Tyr Ile Trp Ala Pro Leu Ala Gly Thr Cys
325 330 335

Gly Val Leu Leu Leu Ser Leu Val Ile Thr Leu Tyr Cys Arg Arg Gly
340 345 350

Arg Lys Lys Leu Leu Tyr Ile Phe Lys Gln Pro Phe Met Arg Pro Val
355 360 365

Gln Thr Thr Gln Glu Glu Asp Gly Cys Ser Cys Arg Phe Pro Glu Glu
370 375 380

Glu Glu Gly Gly Cys Glu Leu Arg Val Lys Phe Ser Arg Ser Ala Asp
385 390 395 400

Ala Pro Ala Tyr Gln Gln Gly Gln Asn Gln Leu Tyr Asn Glu Leu Asn
405 410 415

Leu Gly Arg Arg Glu Glu Tyr Asp Val Leu Asp Lys Arg Arg Gly Arg
420 425 430

Asp Pro Glu Met Gly Gly Lys Pro Arg Arg Lys Asn Pro Gln Glu Gly

234

435                         440                         445

Leu Tyr Asn Glu Leu Gln Lys Asp Lys Met Ala Glu Ala Tyr Ser Glu
        450                 455                 460

Ile Gly Met Lys Gly Glu Arg Arg Arg Gly Lys Gly His Asp Gly Leu
465                 470                 475                 480

Tyr Gln Gly Leu Ser Thr Ala Thr Lys Asp Thr Tyr Asp Ala Leu His
            485                 490                 495

Met Gln Ala Leu Pro Pro Arg Glu
            500

<210> 140
<211> 547
<212> PRT
<213> artificial sequence

<220>
<223> anti-BCMA-CAR

<400> 140

```
Gly Gly Gly Gly Ser Cys Pro Tyr Ser Asn Pro Ser Leu Cys Ser Gly
1               5                   10              15

Gly Gly Gly Ser Gly Gly Gly Gly Ser Glu Val Gln Leu Leu Glu Ser
            20                  25              30

Gly Gly Gly Leu Val Gln Pro Gly Gly Ser Leu Arg Leu Ser Cys Ala
            35              40              45

Ala Ser Gly Phe Thr Phe Ser Ser Tyr Ala Met Asn Trp Val Arg Gln
        50              55              60

Ala Pro Gly Lys Gly Leu Glu Trp Val Ser Ala Ile Leu Ser Ser Gly
65              70              75                  80

Gly Ser Thr Tyr Tyr Ala Asp Ser Val Lys Gly Arg Phe Thr Ile Ser
            85                  90              95

Arg Asp Asn Ser Lys Asn Thr Leu Tyr Leu Gln Met Asn Ser Leu Arg
        100             105             110

Ala Glu Asp Thr Ala Val Tyr Tyr Cys Ala Arg Tyr Trp Pro Met Asp
        115             120             125

Ile Trp Gly Gln Gly Thr Leu Val Thr Val Ser Ser Gly Gly Gly Gly
130             135             140
```

```
Ser Gly Gly Gly Gly Ser Gly Gly Gly Gly Ser Glu Ile Val Leu Thr
145             150             155             160

Gln Ser Pro Gly Thr Leu Ser Leu Ser Pro Gly Glu Arg Ala Thr Leu
            165             170             175

Ser Cys Arg Gly Gly Gln Ser Val Ser Ser Ser Tyr Leu Ala Trp Tyr
        180             185             190

Gln Gln Lys Pro Gly Gln Ala Pro Arg Leu Leu Met Tyr Asp Ala Ser
        195             200             205

Ile Arg Ala Thr Gly Ile Pro Asp Arg Phe Ser Gly Ser Gly Ser Gly
    210             215             220

Thr Asp Phe Thr Leu Thr Ile Ser Arg Leu Glu Pro Glu Asp Phe Ala
225             230             235             240

Val Tyr Tyr Cys Gln Gln Tyr Gln Ser Trp Pro Leu Thr Phe Gly Gln
            245             250             255

Gly Thr Lys Val Glu Ile Lys Ser Asp Pro Gly Ser Gly Gly Gly Gly
        260             265             270

Ser Cys Pro Tyr Ser Asn Pro Ser Leu Cys Ser Gly Gly Gly Gly Ser
        275             280             285

Glu Leu Pro Thr Gln Gly Thr Phe Ser Asn Val Ser Thr Asn Val Ser
    290             295             300

Pro Ala Lys Pro Thr Thr Thr Ala Cys Pro Tyr Ser Asn Pro Ser Leu
305             310             315             320

Cys Ala Pro Thr Thr Thr Pro Ala Pro Arg Pro Pro Thr Pro Ala Pro
            325             330             335

Thr Ile Ala Ser Gln Pro Leu Ser Leu Arg Pro Glu Ala Cys Arg Pro
        340             345             350

Ala Ala Gly Gly Ala Val His Thr Arg Gly Leu Asp Phe Ala Cys Asp
        355             360             365

Ile Tyr Ile Trp Ala Pro Leu Ala Gly Thr Cys Gly Val Leu Leu Leu
    370             375             380

Ser Leu Val Ile Thr Leu Tyr Cys Arg Arg Gly Arg Lys Lys Leu Leu
```

385             390             395             400

Tyr Ile Phe Lys Gln Pro Phe Met Arg Pro Val Gln Thr Thr Gln Glu
           405         410         415

Glu Asp Gly Cys Ser Cys Arg Phe Pro Glu Glu Glu Glu Gly Gly Cys
         420         425         430

Glu Leu Arg Val Lys Phe Ser Arg Ser Ala Asp Ala Pro Ala Tyr Gln
        435         440         445

Gln Gly Gln Asn Gln Leu Tyr Asn Glu Leu Asn Leu Gly Arg Arg Glu
        450         455         460

Glu Tyr Asp Val Leu Asp Lys Arg Arg Gly Arg Asp Pro Glu Met Gly
465           470         475         480

Gly Lys Pro Arg Arg Lys Asn Pro Gln Glu Gly Leu Tyr Asn Glu Leu
         485         490         495

Gln Lys Asp Lys Met Ala Glu Ala Tyr Ser Glu Ile Gly Met Lys Gly
        500         505         510

Glu Arg Arg Arg Gly Lys Gly His Asp Gly Leu Tyr Gln Gly Leu Ser
        515         520         525

Thr Ala Thr Lys Asp Thr Tyr Asp Ala Leu His Met Gln Ala Leu Pro
        530         535         540

Pro Arg Glu
545

<210> 141
<211> 523
<212> PRT
<213> artificial sequence

<220>
<223> anti-BCMA-CAR

<400> 141

Glu Val Gln Leu Leu Glu Ser Gly Gly Gly Leu Val Gln Pro Gly Gly
1                   5                   10                  15

Ser Leu Arg Leu Ser Cys Ala Ala Ser Gly Phe Thr Phe Ser Ser Tyr
                20                  25                  30

Ala Met Asn Trp Val Arg Gln Ala Pro Gly Lys Gly Leu Glu Trp Val
            35                  40                  45

Ser Ala Ile Leu Ser Ser Gly Gly Ser Thr Tyr Tyr Ala Asp Ser Val
    50              55             60

Lys Gly Arg Phe Thr Ile Ser Arg Asp Asn Ser Lys Asn Thr Leu Tyr
65             70             75             80

Leu Gln Met Asn Ser Leu Arg Ala Glu Asp Thr Ala Val Tyr Tyr Cys
          85          90             95

Ala Arg Tyr Trp Pro Met Asp Ile Trp Gly Gln Gly Thr Leu Val Thr
        100          105          110

Val Ser Ser Gly Gly Ser Cys Pro Tyr Ser Asn Pro Ser Leu Cys Ser
      115          120          125

Gly Gly Ser Glu Ile Val Leu Thr Gln Ser Pro Gly Thr Leu Ser Leu
      130          135          140

Ser Pro Gly Glu Arg Ala Thr Leu Ser Cys Arg Gly Gly Gln Ser Val
145             150          155          160

Ser Ser Ser Tyr Leu Ala Trp Tyr Gln Gln Lys Pro Gly Gln Ala Pro
        165          170          175

Arg Leu Leu Met Tyr Asp Ala Ser Ile Arg Ala Thr Gly Ile Pro Asp
      180          185          190

Arg Phe Ser Gly Ser Gly Ser Gly Thr Asp Phe Thr Leu Thr Ile Ser
      195          200          205

Arg Leu Glu Pro Glu Asp Phe Ala Val Tyr Tyr Cys Gln Gln Tyr Gln
    210             215          220

Ser Trp Pro Leu Thr Phe Gly Gln Gly Thr Lys Val Glu Ile Lys Ser
225             230          235          240

Asp Pro Gly Ser Gly Gly Gly Gly Ser Cys Pro Tyr Ser Asn Pro Ser
        245          250          255

Leu Cys Ser Gly Gly Gly Gly Ser Glu Leu Pro Thr Gln Gly Thr Phe
      260          265          270

Ser Asn Val Ser Thr Asn Val Ser Pro Ala Lys Pro Thr Thr Thr Ala
      275          280          285

Cys Pro Tyr Ser Asn Pro Ser Leu Cys Ala Pro Thr Thr Thr Pro Ala

                    290                     295                     300

Pro Arg Pro Pro Thr Pro Ala Pro Thr Ile Ala Ser Gln Pro Leu Ser
305             310             315             320

Leu Arg Pro Glu Ala Cys Arg Pro Ala Ala Gly Gly Ala Val His Thr
            325             330             335

Arg Gly Leu Asp Phe Ala Cys Asp Ile Tyr Ile Trp Ala Pro Leu Ala
            340             345             350

Gly Thr Cys Gly Val Leu Leu Leu Ser Leu Val Ile Thr Leu Tyr Cys
            355             360             365

Arg Arg Gly Arg Lys Lys Leu Leu Tyr Ile Phe Lys Gln Pro Phe Met
    370             375             380

Arg Pro Val Gln Thr Thr Gln Glu Glu Asp Gly Cys Ser Cys Arg Phe
385             390             395             400

Pro Glu Glu Glu Glu Gly Gly Cys Glu Leu Arg Val Lys Phe Ser Arg
            405             410             415

Ser Ala Asp Ala Pro Ala Tyr Gln Gln Gly Gln Asn Gln Leu Tyr Asn
            420             425             430

Glu Leu Asn Leu Gly Arg Arg Glu Glu Tyr Asp Val Leu Asp Lys Arg
    435             440             445

Arg Gly Arg Asp Pro Glu Met Gly Gly Lys Pro Arg Arg Lys Asn Pro
    450             455             460

Gln Glu Gly Leu Tyr Asn Glu Leu Gln Lys Asp Lys Met Ala Glu Ala
465             470             475             480

Tyr Ser Glu Ile Gly Met Lys Gly Glu Arg Arg Arg Gly Lys Gly His
            485             490             495

Asp Gly Leu Tyr Gln Gly Leu Ser Thr Ala Thr Lys Asp Thr Tyr Asp
            500             505             510

Ala Leu His Met Gln Ala Leu Pro Pro Arg Glu
            515             520

<210> 142
<211> 467
<212> PRT

<213> artificial sequence

<220>
<223> anti-CD123-CAR

<400> 142

```
Gln Ile Gln Leu Val Gln Ser Gly Pro Glu Leu Lys Lys Pro Gly Glu
1               5               10              15

Thr Val Lys Ile Ser Cys Lys Ala Ser Gly Tyr Ile Phe Thr Asn Tyr
            20              25              30

Gly Met Asn Trp Val Lys Gln Ala Pro Gly Lys Ser Phe Lys Trp Met
        35              40              45

Gly Trp Ile Asn Thr Tyr Thr Gly Glu Ser Thr Tyr Ser Ala Asp Phe
    50              55              60

Lys Gly Arg Phe Ala Phe Ser Leu Glu Thr Ser Ala Ser Thr Ala Tyr
65              70              75              80

Leu His Ile Asn Asp Leu Lys Asn Glu Asp Thr Ala Thr Tyr Phe Cys
            85              90              95

Ala Arg Ser Gly Gly Tyr Asp Pro Met Asp Tyr Trp Gly Gln Gly Thr
        100             105             110

Ser Val Thr Val Ser Ser Gly Gly Gly Ser Gly Gly Gly Gly Ser
        115             120             125

Gly Gly Gly Gly Ser Asp Ile Val Leu Thr Gln Ser Pro Ala Ser Leu
    130             135             140

Ala Val Ser Leu Gly Gln Arg Ala Thr Ile Ser Cys Arg Ala Ser Glu
145             150             155             160

Ser Val Asp Asn Tyr Gly Asn Thr Phe Met His Trp Tyr Gln Gln Lys
            165             170             175

Pro Gly Gln Pro Pro Lys Leu Leu Ile Tyr Arg Ala Ser Asn Leu Glu
        180             185             190

Ser Gly Ile Pro Ala Arg Phe Ser Gly Ser Gly Ser Arg Thr Asp Phe
    195             200             205

Thr Leu Thr Ile Asn Pro Val Glu Ala Asp Asp Val Ala Thr Tyr Tyr
    210             215             220

Cys Gln Gln Ser Asn Glu Asp Pro Pro Thr Phe Gly Ala Gly Thr Lys
```

225     230     235     240

Leu Glu Leu Lys Thr Thr Thr Pro Ala Pro Arg Pro Pro Thr Pro Ala
     245    250     255

Pro Thr Ile Ala Ser Gln Pro Leu Ser Leu Arg Pro Glu Ala Cys Arg
    260    265    270

Pro Ala Ala Gly Gly Ala Val His Thr Arg Gly Leu Asp Phe Ala Cys
    275    280    285

Asp Ile Tyr Ile Trp Ala Pro Leu Ala Gly Thr Cys Gly Val Leu Leu
   290    295    300

Leu Ser Leu Val Ile Thr Leu Tyr Cys Lys Arg Gly Arg Lys Lys Leu
305    310    315    320

Leu Tyr Ile Phe Lys Gln Pro Phe Met Arg Pro Val Gln Thr Thr Gln
    325    330    335

Glu Glu Asp Gly Cys Ser Cys Arg Phe Pro Glu Glu Glu Glu Gly Gly
    340    345    350

Cys Glu Leu Arg Val Lys Phe Ser Arg Ser Ala Asp Ala Pro Ala Tyr
   355    360    365

Gln Gln Gly Gln Asn Gln Leu Tyr Asn Glu Leu Asn Leu Gly Arg Arg
   370    375    380

Glu Glu Tyr Asp Val Leu Asp Lys Arg Arg Gly Arg Asp Pro Glu Met
385    390    395    400

Gly Gly Lys Pro Arg Arg Lys Asn Pro Gln Glu Gly Leu Tyr Asn Glu
    405    410    415

Leu Gln Lys Asp Lys Met Ala Glu Ala Tyr Ser Glu Ile Gly Met Lys
    420    425    430

Gly Glu Arg Arg Arg Gly Lys Gly His Asp Gly Leu Tyr Gln Gly Leu
    435    440    445

Ser Thr Ala Thr Lys Asp Thr Tyr Asp Ala Leu His Met Gln Ala Leu
   450    455    460

Pro Pro Arg
465

<210> 143
<211> 518
<212> PRT
<213> artificial sequence

<220>
<223> CD123 protein

<400> 143

```
Met Thr Lys Glu Asp Pro Asn Pro Pro Ile Thr Asn Leu Arg Met Lys
1               5               10                  15

Ala Lys Ala Gln Gln Leu Thr Trp Asp Leu Asn Arg Asn Val Thr Asp
            20              25                  30

Ile Glu Cys Val Lys Asp Ala Asp Tyr Ser Met Pro Ala Val Asn Asn
        35              40                  45

Ser Tyr Cys Gln Phe Gly Ala Ile Ser Leu Cys Glu Val Thr Asn Tyr
    50              55                  60

Thr Val Arg Val Ala Asn Pro Pro Phe Ser Thr Trp Ile Leu Phe Pro
65              70                  75                      80

Glu Asn Ser Gly Lys Pro Trp Ala Gly Ala Glu Asn Leu Thr Cys Trp
                85              90                  95

Ile His Asp Val Asp Phe Leu Ser Cys Ser Trp Ala Val Gly Pro Gly
            100             105                 110

Ala Pro Ala Asp Val Gln Tyr Asp Leu Tyr Leu Asn Val Ala Asn Arg
        115             120                 125

Arg Gln Gln Tyr Glu Cys Leu His Tyr Lys Thr Asp Ala Gln Gly Thr
    130             135                 140

Arg Ile Gly Cys Arg Phe Asp Asp Ile Ser Arg Leu Ser Ser Gly Ser
145             150                 155                 160

Gln Ser Ser His Ile Leu Val Arg Gly Arg Ser Ala Ala Phe Gly Ile
            165             170                 175

Pro Cys Thr Asp Lys Phe Val Val Phe Ser Gln Ile Glu Ile Leu Thr
        180             185                 190

Pro Pro Asn Met Thr Ala Lys Cys Asn Lys Thr His Ser Phe Met His
        195             200                 205

Trp Lys Met Arg Ser His Phe Asn Arg Lys Phe Arg Tyr Glu Leu Gln
```

```
                210                    215                     220

        Ile Gln Lys Arg Met Gln Pro Val Ile Thr Glu Gln Val Arg Asp Arg
        225                 230             235                     240

        Thr Ser Phe Gln Leu Leu Asn Pro Gly Thr Tyr Thr Val Gln Ile Arg
                        245             250                     255

        Ala Arg Glu Arg Val Tyr Glu Phe Leu Ser Ala Trp Ser Thr Pro Gln
                    260             265             270

        Arg Phe Glu Cys Asp Gln Glu Glu Gly Ala Asn Thr Arg Ala Trp Arg
                    275             280             285

        Gly Gly Gly Gly Ala Gly Gly Gly Gly Cys Lys Pro Cys Ile Cys Thr
            290             295             300

        Val Pro Glu Val Ser Ser Val Phe Ile Phe Pro Pro Lys Pro Lys Asp
        305             310             315                     320

        Val Leu Thr Ile Thr Leu Thr Pro Lys Val Thr Cys Val Val Val Asp
                        325             330                     335

        Ile Ser Lys Asp Asp Pro Glu Val Gln Phe Ser Trp Phe Val Asp Asp
                    340             345             350

        Val Glu Val His Thr Ala Gln Thr Gln Pro Arg Glu Glu Gln Phe Asn
                    355             360             365

        Ser Thr Phe Arg Ser Val Ser Glu Leu Pro Ile Met His Gln Asp Trp
            370             375             380

        Leu Asn Gly Lys Glu Phe Lys Cys Arg Val Asn Ser Ala Ala Phe Pro
        385             390             395                     400

        Ala Pro Ile Glu Lys Thr Ile Ser Lys Thr Lys Gly Arg Pro Lys Ala
                        405             410                     415

        Pro Gln Val Tyr Thr Ile Pro Pro Pro Lys Glu Gln Met Ala Lys Asp
                    420             425             430

        Lys Val Ser Leu Thr Cys Met Ile Thr Asp Phe Phe Pro Glu Asp Ile
                    435             440             445

        Thr Val Glu Trp Gln Trp Asn Gly Gln Pro Ala Glu Asn Tyr Lys Asn
            450             455             460
```

EP 3 250 604 B1

Thr Gln Pro Ile Met Asp Thr Asp Gly Ser Tyr Phe Val Tyr Ser Lys
465                 470                 475                 480


Leu Asn Val Gln Lys Ser Asn Trp Glu Ala Gly Asn Thr Phe Thr Cys
                485                 490                 495


Ser Val Leu His Glu Gly Leu His Asn His His Thr Glu Lys Ser Leu
                500                 505                 510


Ser His Ser Pro Gly Lys
                515

<210> 144
<211> 16
<212> PRT
<213> artificial sequence

<220>
<223> mAb-specific epitope

<400> 144

Glu Leu Pro Thr Gln Gly Thr Phe Ser Asn Val Ser Thr Asn Val Ser
1                   5                   10                  15

<210> 145
<211> 469
<212> PRT
<213> artificial sequence

<220>
<223> anti-BCMA-CAR

<400> 145

**248**

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Ser Phe Pro Asp Tyr
            20                  25                  30

Tyr Ile Asn Trp Val Arg Gln Ala Pro Gly Gln Gly Leu Glu Trp Met
            35                  40                  45

Gly Trp Ile Tyr Phe Ala Ser Gly Asn Ser Glu Tyr Asn Gln Lys Phe
    50                  55                  60

Thr Gly Arg Val Thr Met Thr Arg Asp Thr Ser Ser Ser Thr Ala Tyr
65                  70                  75                  80

Met Glu Leu Ser Ser Leu Arg Ser Glu Asp Thr Ala Val Tyr Phe Cys
                85                  90                  95
```

```
Ala Ser Leu Tyr Asp Tyr Asp Trp Tyr Phe Asp Val Trp Gly Gln Gly
            100                 105                 110

Thr Met Val Thr Val Ser Ser Gly Gly Gly Gly Ser Gly Gly Gly Gly
            115                 120                 125

Ser Gly Gly Gly Gly Ser Asp Ile Val Met Thr Gln Thr Pro Leu Ser
            130                 135                 140

Leu Ser Val Thr Pro Gly Glu Pro Ala Ser Ile Ser Cys Lys Ser Ser
145                 150                 155                 160

Gln Ser Leu Val His Ser Asn Gly Asn Thr Tyr Leu His Trp Tyr Leu
                165                 170                 175

Gln Lys Pro Gly Gln Ser Pro Gln Leu Leu Ile Tyr Lys Val Ser Asn
                180                 185                 190

Arg Phe Ser Gly Val Pro Asp Arg Phe Ser Gly Ser Gly Ser Gly Ala
        195                 200                 205

Asp Phe Thr Leu Lys Ile Ser Arg Val Glu Ala Glu Asp Val Gly Val
    210                 215                 220

Tyr Tyr Cys Ala Glu Thr Ser His Val Pro Trp Thr Phe Gly Gln Gly
225                 230                 235                 240

Thr Lys Leu Glu Ile Lys Thr Thr Thr Pro Ala Pro Arg Pro Pro Thr
                245                 250                 255

Pro Ala Pro Thr Ile Ala Ser Gln Pro Leu Ser Leu Arg Pro Glu Ala
                260                 265                 270

Cys Arg Pro Ala Ala Gly Gly Ala Val His Thr Arg Gly Leu Asp Phe
        275                 280                 285

Ala Cys Asp Ile Tyr Ile Trp Ala Pro Leu Ala Gly Thr Cys Gly Val
    290                 295                 300

Leu Leu Leu Ser Leu Val Ile Thr Leu Tyr Cys Lys Arg Gly Arg Lys
305                 310                 315                 320

Lys Leu Leu Tyr Ile Phe Lys Gln Pro Phe Met Arg Pro Val Gln Thr
                325                 330                 335

Thr Gln Glu Glu Asp Gly Cys Ser Cys Arg Phe Pro Glu Glu Glu Glu
                340                 345                 350
```

250

```
Gly Gly Cys Glu Leu Arg Val Lys Phe Ser Arg Ser Ala Asp Ala Pro
        355                 360             365

Ala Tyr Gln Gln Gly Gln Asn Gln Leu Tyr Asn Glu Leu Asn Leu Gly
    370             375                 380

Arg Arg Glu Glu Tyr Asp Val Leu Asp Lys Arg Arg Gly Arg Asp Pro
385                 390                 395                 400

Glu Met Gly Gly Lys Pro Arg Arg Lys Asn Pro Gln Glu Gly Leu Tyr
            405                 410                 415

Asn Glu Leu Gln Lys Asp Lys Met Ala Glu Ala Tyr Ser Glu Ile Gly
        420                 425                 430

Met Lys Gly Glu Arg Arg Arg Gly Lys Gly His Asp Gly Leu Tyr Gln
        435                 440                 445

Gly Leu Ser Thr Ala Thr Lys Asp Thr Tyr Asp Ala Leu His Met Gln
    450                 455                 460

Ala Leu Pro Pro Arg
465
```

<210> 146
<211> 485
<212> PRT
<213> artificial sequence

<220>
<223> BC30-LM

<400> 146

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Ser Phe Pro Asp Tyr
            20                  25                  30

Tyr Ile Asn Trp Val Arg Gln Ala Pro Gly Gln Gly Leu Glu Trp Met
            35                  40                  45

Gly Trp Ile Tyr Phe Ala Ser Gly Asn Ser Glu Tyr Asn Gln Lys Phe
        50                  55                  60

Thr Gly Arg Val Thr Met Thr Arg Asp Thr Ser Ser Ser Thr Ala Tyr
65                  70                  75                  80
```

```
Met Glu Leu Ser Ser Leu Arg Ser Glu Asp Thr Ala Val Tyr Phe Cys
             85              90                  95

Ala Ser Leu Tyr Asp Tyr Asp Trp Tyr Phe Asp Val Trp Gly Gln Gly
            100             105             110

Thr Met Val Thr Val Ser Ser Gly Gly Gly Gly Ser Gly Gly Gly Gly
        115             120             125

Ser Gly Gly Gly Gly Ser Asp Ile Val Met Thr Gln Thr Pro Leu Ser
    130             135             140

Leu Ser Val Thr Pro Gly Glu Pro Ala Ser Ile Ser Cys Lys Ser Ser
145             150             155             160

Gln Ser Leu Val His Ser Asn Gly Asn Thr Tyr Leu His Trp Tyr Leu
            165             170             175

Gln Lys Pro Gly Gln Ser Pro Gln Leu Leu Ile Tyr Lys Val Ser Asn
        180             185             190

Arg Phe Ser Gly Val Pro Asp Arg Phe Ser Gly Ser Gly Ser Gly Ala
    195             200             205

Asp Phe Thr Leu Lys Ile Ser Arg Val Glu Ala Glu Asp Val Gly Val
210             215             220

Tyr Tyr Cys Ala Glu Thr Ser His Val Pro Trp Thr Phe Gly Gln Gly
225             230             235             240

Thr Lys Leu Glu Ile Lys Gly Ser Gly Gly Gly Ser Cys Pro Tyr
            245             250             255

Ser Asn Pro Ser Leu Cys Thr Thr Thr Pro Ala Pro Arg Pro Pro Thr
        260             265             270

Pro Ala Pro Thr Ile Ala Ser Gln Pro Leu Ser Leu Arg Pro Glu Ala
    275             280             285

Cys Arg Pro Ala Ala Gly Gly Ala Val His Thr Arg Gly Leu Asp Phe
    290             295             300

Ala Cys Asp Ile Tyr Ile Trp Ala Pro Leu Ala Gly Thr Cys Gly Val
305             310             315             320

Leu Leu Leu Ser Leu Val Ile Thr Leu Tyr Cys Lys Arg Gly Arg Lys
            325             330             335
```

```
Lys Leu Leu Tyr Ile Phe Lys Gln Pro Phe Met Arg Pro Val Gln Thr
            340             345             350

Thr Gln Glu Glu Asp Gly Cys Ser Cys Arg Phe Pro Glu Glu Glu Glu
            355             360             365

Gly Gly Cys Glu Leu Arg Val Lys Phe Ser Arg Ser Ala Asp Ala Pro
    370             375             380

Ala Tyr Gln Gln Gly Gln Asn Gln Leu Tyr Asn Glu Leu Asn Leu Gly
385             390             395             400

Arg Arg Glu Glu Tyr Asp Val Leu Asp Lys Arg Arg Gly Arg Asp Pro
            405             410             415

Glu Met Gly Gly Lys Pro Arg Arg Lys Asn Pro Gln Glu Gly Leu Tyr
            420             425             430

Asn Glu Leu Gln Lys Asp Lys Met Ala Glu Ala Tyr Ser Glu Ile Gly
            435             440             445

Met Lys Gly Glu Arg Arg Arg Gly Lys Gly His Asp Gly Leu Tyr Gln
    450             455             460

Gly Leu Ser Thr Ala Thr Lys Asp Thr Tyr Asp Ala Leu His Met Gln
465             470             475             480

Ala Leu Pro Pro Arg
                485
```

<210> 147
<211> 491
<212> PRT
<213> artificial sequence

<220>
<223> BC30-LML

<400> 147

```
Gln Val Gln Leu Val Gln Ser Gly Ala Glu Val Lys Lys Pro Gly Ala
1               5               10              15

Ser Val Lys Val Ser Cys Lys Ala Ser Gly Tyr Ser Phe Pro Asp Tyr
            20              25              30

Tyr Ile Asn Trp Val Arg Gln Ala Pro Gly Gln Gly Leu Glu Trp Met
            35              40              45
```

```
Gly Trp Ile Tyr Phe Ala Ser Gly Asn Ser Glu Tyr Asn Gln Lys Phe
    50                  55                  60

Thr Gly Arg Val Thr Met Thr Arg Asp Thr Ser Ser Ser Thr Ala Tyr
65                  70                  75                  80

Met Glu Leu Ser Ser Leu Arg Ser Glu Asp Thr Ala Val Tyr Phe Cys
                85                  90                  95

Ala Ser Leu Tyr Asp Tyr Asp Trp Tyr Phe Asp Val Trp Gly Gln Gly
            100                 105                 110

Thr Met Val Thr Val Ser Ser Gly Gly Gly Ser Gly Gly Gly Gly
            115                 120                 125

Ser Gly Gly Gly Gly Ser Asp Ile Val Met Thr Gln Thr Pro Leu Ser
        130                 135                 140

Leu Ser Val Thr Pro Gly Glu Pro Ala Ser Ile Ser Cys Lys Ser Ser
145                 150                 155                 160

Gln Ser Leu Val His Ser Asn Gly Asn Thr Tyr Leu His Trp Tyr Leu
                165                 170                 175

Gln Lys Pro Gly Gln Ser Pro Gln Leu Leu Ile Tyr Lys Val Ser Asn
            180                 185                 190

Arg Phe Ser Gly Val Pro Asp Arg Phe Ser Gly Ser Gly Ser Gly Ala
        195                 200                 205

Asp Phe Thr Leu Lys Ile Ser Arg Val Glu Ala Glu Asp Val Gly Val
    210                 215                 220

Tyr Tyr Cys Ala Glu Thr Ser His Val Pro Trp Thr Phe Gly Gln Gly
225                 230                 235                 240

Thr Lys Leu Glu Ile Lys Gly Ser Gly Gly Gly Ser Cys Pro Tyr
            245                 250                 255

Ser Asn Pro Ser Leu Cys Ser Gly Gly Gly Gly Ser Thr Thr Thr Pro
        260                 265                 270

Ala Pro Arg Pro Pro Thr Pro Ala Pro Thr Ile Ala Ser Gln Pro Leu
        275                 280                 285

Ser Leu Arg Pro Glu Ala Cys Arg Pro Ala Ala Gly Gly Ala Val His
    290                 295                 300
```

254

EP 3 250 604 B1

Thr Arg Gly Leu Asp Phe Ala Cys Asp Ile Tyr Ile Trp Ala Pro Leu
305                 310             315                 320

Ala Gly Thr Cys Gly Val Leu Leu Leu Ser Leu Val Ile Thr Leu Tyr
                325             330                 335

Cys Lys Arg Gly Arg Lys Lys Leu Leu Tyr Ile Phe Lys Gln Pro Phe
                340             345                 350

Met Arg Pro Val Gln Thr Thr Gln Glu Glu Asp Gly Cys Ser Cys Arg
                355             360                 365

Phe Pro Glu Glu Glu Glu Gly Gly Cys Glu Leu Arg Val Lys Phe Ser
    370                 375             380

Arg Ser Ala Asp Ala Pro Ala Tyr Gln Gln Gly Gln Asn Gln Leu Tyr
385                 390                 395                 400

Asn Glu Leu Asn Leu Gly Arg Arg Glu Glu Tyr Asp Val Leu Asp Lys
                405             410                 415

Arg Arg Gly Arg Asp Pro Glu Met Gly Gly Lys Pro Arg Arg Lys Asn
                420             425                 430

Pro Gln Glu Gly Leu Tyr Asn Glu Leu Gln Lys Asp Lys Met Ala Glu
        435             440                 445

Ala Tyr Ser Glu Ile Gly Met Lys Gly Glu Arg Arg Arg Gly Lys Gly
    450             455                 460

His Asp Gly Leu Tyr Gln Gly Leu Ser Thr Ala Thr Lys Asp Thr Tyr
465             470                 475                 480

Asp Ala Leu His Met Gln Ala Leu Pro Pro Arg
                485                 490

<210> 148
<211> 500
<212> PRT
<213> artificial sequence

<220>
<223> BC30-LMLM

<400> 148

Gln Val Gln Leu Val Gln Ser Gly Ala Glu Val Lys Lys Pro Gly Ala
1               5               10                  15

255

```
Ser Val Lys Val Ser Cys Lys Ala Ser Gly Tyr Ser Phe Pro Asp Tyr
        20                  25                  30

Tyr Ile Asn Trp Val Arg Gln Ala Pro Gly Gln Gly Leu Glu Trp Met
        35                  40                  45

Gly Trp Ile Tyr Phe Ala Ser Gly Asn Ser Glu Tyr Asn Gln Lys Phe
        50                  55                  60

Thr Gly Arg Val Thr Met Thr Arg Asp Thr Ser Ser Ser Thr Ala Tyr
65                  70                  75                  80

Met Glu Leu Ser Ser Leu Arg Ser Glu Asp Thr Ala Val Tyr Phe Cys
                85                  90                  95

Ala Ser Leu Tyr Asp Tyr Asp Trp Tyr Phe Asp Val Trp Gly Gln Gly
            100                 105                 110

Thr Met Val Thr Val Ser Ser Gly Gly Gly Gly Ser Gly Gly Gly Gly
        115                 120                 125

Ser Gly Gly Gly Gly Ser Asp Ile Val Met Thr Gln Thr Pro Leu Ser
    130                 135                 140

Leu Ser Val Thr Pro Gly Glu Pro Ala Ser Ile Ser Cys Lys Ser Ser
145                 150                 155                 160

Gln Ser Leu Val His Ser Asn Gly Asn Thr Tyr Leu His Trp Tyr Leu
                165                 170                 175

Gln Lys Pro Gly Gln Ser Pro Gln Leu Leu Ile Tyr Lys Val Ser Asn
            180                 185                 190

Arg Phe Ser Gly Val Pro Asp Arg Phe Ser Gly Ser Gly Ser Gly Ala
        195                 200                 205

Asp Phe Thr Leu Lys Ile Ser Arg Val Glu Ala Glu Asp Val Gly Val
    210                 215                 220

Tyr Tyr Cys Ala Glu Thr Ser His Val Pro Trp Thr Phe Gly Gln Gly
225                 230                 235                 240

Thr Lys Leu Glu Ile Lys Gly Ser Gly Gly Gly Gly Ser Cys Pro Tyr
            245                 250                 255

Ser Asn Pro Ser Leu Cys Ser Gly Gly Gly Gly Ser Cys Pro Tyr Ser
        260                 265                 270
```

```
Asn Pro Ser Leu Cys Thr Thr Thr Pro Ala Pro Arg Pro Pro Thr Pro
    275             280             285

Ala Pro Thr Ile Ala Ser Gln Pro Leu Ser Leu Arg Pro Glu Ala Cys
    290             295             300

Arg Pro Ala Ala Gly Gly Ala Val His Thr Arg Gly Leu Asp Phe Ala
305             310             315             320

Cys Asp Ile Tyr Ile Trp Ala Pro Leu Ala Gly Thr Cys Gly Val Leu
            325             330             335

Leu Leu Ser Leu Val Ile Thr Leu Tyr Cys Lys Arg Gly Arg Lys Lys
            340             345             350

Leu Leu Tyr Ile Phe Lys Gln Pro Phe Met Arg Pro Val Gln Thr Thr
            355             360             365

Gln Glu Glu Asp Gly Cys Ser Cys Arg Phe Pro Glu Glu Glu Glu Gly
    370             375             380

Gly Cys Glu Leu Arg Val Lys Phe Ser Arg Ser Ala Asp Ala Pro Ala
385             390             395             400

Tyr Gln Gln Gly Gln Asn Gln Leu Tyr Asn Glu Leu Asn Leu Gly Arg
            405             410             415

Arg Glu Glu Tyr Asp Val Leu Asp Lys Arg Arg Gly Arg Asp Pro Glu
            420             425             430

Met Gly Gly Lys Pro Arg Arg Lys Asn Pro Gln Glu Gly Leu Tyr Asn
            435             440             445

Glu Leu Gln Lys Asp Lys Met Ala Glu Ala Tyr Ser Glu Ile Gly Met
    450             455             460

Lys Gly Glu Arg Arg Arg Gly Lys Gly His Asp Gly Leu Tyr Gln Gly
465             470             475             480

Leu Ser Thr Ala Thr Lys Asp Thr Tyr Asp Ala Leu His Met Gln Ala
            485             490             495

Leu Pro Pro Arg
            500
```

<210> 149
<211> 506

<212> PRT
<213> artificial sequence

<220>
<223> BC30-LMLML

<400> 149

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Ser Phe Pro Asp Tyr
            20                  25                  30

Tyr Ile Asn Trp Val Arg Gln Ala Pro Gly Gln Gly Leu Glu Trp Met
            35                  40                  45

Gly Trp Ile Tyr Phe Ala Ser Gly Asn Ser Glu Tyr Asn Gln Lys Phe
    50                  55                  60

Thr Gly Arg Val Thr Met Thr Arg Asp Thr Ser Ser Ser Thr Ala Tyr
65                  70                  75                  80

Met Glu Leu Ser Ser Leu Arg Ser Glu Asp Thr Ala Val Tyr Phe Cys
                85                  90                  95

Ala Ser Leu Tyr Asp Tyr Asp Trp Tyr Phe Asp Val Trp Gly Gln Gly
            100                 105                 110

Thr Met Val Thr Val Ser Ser Gly Gly Gly Ser Gly Gly Gly Gly
        115                 120                 125

Ser Gly Gly Gly Gly Ser Asp Ile Val Met Thr Gln Thr Pro Leu Ser
    130                 135                 140

Leu Ser Val Thr Pro Gly Glu Pro Ala Ser Ile Ser Cys Lys Ser Ser
145                 150                 155                 160

Gln Ser Leu Val His Ser Asn Gly Asn Thr Tyr Leu His Trp Tyr Leu
            165                 170                 175

Gln Lys Pro Gly Gln Ser Pro Gln Leu Leu Ile Tyr Lys Val Ser Asn
            180                 185                 190

Arg Phe Ser Gly Val Pro Asp Arg Phe Ser Gly Ser Gly Ser Gly Ala
        195                 200                 205

Asp Phe Thr Leu Lys Ile Ser Arg Val Glu Ala Glu Asp Val Gly Val
    210                 215                 220

259

```
Tyr Tyr Cys Ala Glu Thr Ser His Val Pro Trp Thr Phe Gly Gln Gly
225                 230             235                 240

Thr Lys Leu Glu Ile Lys Gly Ser Gly Gly Gly Gly Ser Cys Pro Tyr
                245             250                 255

Ser Asn Pro Ser Leu Cys Ser Gly Gly Gly Gly Ser Cys Pro Tyr Ser
            260             265                 270

Asn Pro Ser Leu Cys Ser Gly Gly Gly Gly Ser Thr Thr Thr Pro Ala
            275             280                 285

Pro Arg Pro Pro Thr Pro Ala Pro Thr Ile Ala Ser Gln Pro Leu Ser
            290             295                 300

Leu Arg Pro Glu Ala Cys Arg Pro Ala Ala Gly Gly Ala Val His Thr
305                 310             315                 320

Arg Gly Leu Asp Phe Ala Cys Asp Ile Tyr Ile Trp Ala Pro Leu Ala
                325             330                 335

Gly Thr Cys Gly Val Leu Leu Leu Ser Leu Val Ile Thr Leu Tyr Cys
            340             345                 350

Lys Arg Gly Arg Lys Lys Leu Leu Tyr Ile Phe Lys Gln Pro Phe Met
            355             360                 365

Arg Pro Val Gln Thr Thr Gln Glu Glu Asp Gly Cys Ser Cys Arg Phe
        370             375                 380

Pro Glu Glu Glu Glu Gly Gly Cys Glu Leu Arg Val Lys Phe Ser Arg
385                 390             395                 400

Ser Ala Asp Ala Pro Ala Tyr Gln Gln Gly Gln Asn Gln Leu Tyr Asn
                405             410                 415

Glu Leu Asn Leu Gly Arg Arg Glu Glu Tyr Asp Val Leu Asp Lys Arg
            420             425                 430

Arg Gly Arg Asp Pro Glu Met Gly Gly Lys Pro Arg Arg Lys Asn Pro
            435             440                 445

Gln Glu Gly Leu Tyr Asn Glu Leu Gln Lys Asp Lys Met Ala Glu Ala
    450             455                 460

Tyr Ser Glu Ile Gly Met Lys Gly Glu Arg Arg Arg Gly Lys Gly His
```

                465                    470                    475                    480

        Asp Gly Leu Tyr Gln Gly Leu Ser Thr Ala Thr Lys Asp Thr Tyr Asp
                        485             490             495

        Ala Leu His Met Gln Ala Leu Pro Pro Arg
                    500             505

<210> 150
<211> 136
<212> PRT
<213> artificial sequence

<220>
<223> BC30-RQR8

<400> 150

        Cys Pro Tyr Ser Asn Pro Ser Leu Cys Ser Gly Gly Gly Gly Ser Glu
        1               5               10              15

        Leu Pro Thr Gln Gly Thr Phe Ser Asn Val Ser Thr Asn Val Ser Pro
                    20              25              30

        Ala Lys Pro Thr Thr Thr Ala Cys Pro Tyr Ser Asn Pro Ser Leu Cys
                35              40              45

        Ser Gly Gly Gly Gly Ser Pro Ala Pro Arg Pro Pro Thr Pro Ala Pro
            50              55              60

        Thr Ile Ala Ser Gln Pro Leu Ser Leu Arg Pro Glu Ala Cys Arg Pro
        65              70              75              80

        Ala Ala Gly Gly Ala Val His Thr Arg Gly Leu Asp Phe Ala Cys Asp
                        85              90              95

        Ile Tyr Ile Trp Ala Pro Leu Ala Gly Thr Cys Gly Val Leu Leu Leu
                    100             105             110

        Ser Leu Val Ile Thr Leu Tyr Cys Asn His Arg Asn Arg Arg Arg Val
                115             120             125

        Cys Lys Cys Pro Arg Pro Val Val
            130             135

<210> 151
<211> 284
<212> PRT
<213> artificial sequence

261

<220>
<223> BCMA protein

<400> 151

```
Met Leu Gln Met Ala Gly Gln Cys Ser Gln Asn Glu Tyr Phe Asp Ser
1               5               10              15

Leu Leu His Ala Cys Ile Pro Cys Gln Leu Arg Cys Ser Ser Asn Thr
        20              25              30

Pro Pro Leu Thr Cys Gln Arg Tyr Cys Asn Ala Ser Val Thr Asn Ser
        35              40              45

Val Lys Gly Thr Asn Ala Gly Gly Gly Gly Ala Gly Gly Gly Gly Cys
    50              55              60

Lys Pro Cys Ile Cys Thr Val Pro Glu Val Ser Ser Val Phe Ile Phe
65              70              75              80

Pro Pro Lys Pro Lys Asp Val Leu Thr Ile Thr Leu Thr Pro Lys Val
            85              90              95

Thr Cys Val Val Val Asp Ile Ser Lys Asp Asp Pro Glu Val Gln Phe
        100             105             110

Ser Trp Phe Val Asp Asp Val Glu Val His Thr Ala Gln Thr Gln Pro
        115             120             125

Arg Glu Glu Gln Phe Asn Ser Thr Phe Arg Ser Val Ser Glu Leu Pro
    130             135             140

Ile Met His Gln Asp Trp Leu Asn Gly Lys Glu Phe Lys Cys Arg Val
145             150             155             160

Asn Ser Ala Ala Phe Pro Ala Pro Ile Glu Lys Thr Ile Ser Lys Thr
            165             170             175

Lys Gly Arg Pro Lys Ala Pro Gln Val Tyr Thr Ile Pro Pro Pro Lys
            180             185             190

Glu Gln Met Ala Lys Asp Lys Val Ser Leu Thr Cys Met Ile Thr Asp
        195             200             205

Phe Phe Pro Glu Asp Ile Thr Val Glu Trp Gln Trp Asn Gly Gln Pro
    210             215             220

Ala Glu Asn Tyr Lys Asn Thr Gln Pro Ile Met Asp Thr Asp Gly Ser
225             230             235             240
```

```
Tyr Phe Val Tyr Ser Lys Leu Asn Val Gln Lys Ser Asn Trp Glu Ala
                245                 250                 255

Gly Asn Thr Phe Thr Cys Ser Val Leu His Glu Gly Leu His Asn His
                260                 265                 270

His Thr Glu Lys Ser Leu Ser His Ser Pro Gly Lys
                275                 280
```

<210> 152
<211> 490
<212> PRT
<213> artificial sequence

<220>
<223> polypeptide

<400> 152

```
Gln Ile Gln Leu Val Gln Ser Gly Pro Glu Leu Lys Lys Pro Gly Glu
1               5                   10                  15

Thr Val Lys Ile Ser Cys Lys Ala Ser Gly Tyr Ile Phe Thr Asn Tyr
                20                  25                  30

Gly Met Asn Trp Val Lys Gln Ala Pro Gly Lys Ser Phe Lys Trp Met
                35                  40                  45

Gly Trp Ile Asn Thr Tyr Thr Gly Glu Ser Thr Tyr Ser Ala Asp Phe
                50                  55                  60

Lys Gly Arg Phe Ala Phe Ser Leu Glu Thr Ser Ala Ser Thr Ala Tyr
65                  70                  75                  80

Leu His Ile Asn Asp Leu Lys Asn Glu Asp Thr Ala Thr Tyr Phe Cys
                85                  90                  95

Ala Arg Ser Gly Gly Tyr Asp Pro Met Asp Tyr Trp Gly Gln Gly Thr
                100                 105                 110

Ser Val Thr Val Ser Ser Gly Gly Gly Gly Ser Gly Gly Gly Gly Ser
                115                 120                 125

Gly Gly Gly Gly Ser Asp Ile Val Leu Thr Gln Ser Pro Ala Ser Leu
                130                 135                 140

Ala Val Ser Leu Gly Gln Arg Ala Thr Ile Ser Cys Arg Ala Ser Glu
145                 150                 155                 160
```

Ser Val Asp Asn Tyr Gly Asn Thr Phe Met His Trp Tyr Gln Gln Lys
165 170 175

Pro Gly Gln Pro Pro Lys Leu Leu Ile Tyr Arg Ala Ser Asn Leu Glu
180 185 190

Ser Gly Ile Pro Ala Arg Phe Ser Gly Ser Gly Ser Arg Thr Asp Phe
195 200 205

Thr Leu Thr Ile Asn Pro Val Glu Ala Asp Asp Val Ala Thr Tyr Tyr
210 215 220

Cys Gln Gln Ser Asn Glu Asp Pro Pro Thr Phe Gly Ala Gly Thr Lys
225 230 235 240

Leu Glu Leu Lys Arg Ser Asp Pro Gly Ser Gly Gly Gly Gly Ser Cys
245 250 255

Pro Tyr Ser Asn Pro Ser Leu Cys Ala Pro Thr Thr Thr Pro Ala Pro
260 265 270

Arg Pro Pro Thr Pro Ala Pro Thr Ile Ala Ser Gln Pro Leu Ser Leu
275 280 285

Arg Pro Glu Ala Cys Arg Pro Ala Ala Gly Gly Ala Val His Thr Arg
290 295 300

Gly Leu Asp Phe Ala Cys Asp Ile Tyr Ile Trp Ala Pro Leu Ala Gly
305 310 315 320

Thr Cys Gly Val Leu Leu Leu Ser Leu Val Ile Thr Leu Tyr Cys Arg
325 330 335

Arg Gly Arg Lys Lys Leu Leu Tyr Ile Phe Lys Gln Pro Phe Met Arg
340 345 350

Pro Val Gln Thr Thr Gln Glu Glu Asp Gly Cys Ser Cys Arg Phe Pro
355 360 365

Glu Glu Glu Glu Gly Gly Cys Glu Leu Arg Val Lys Phe Ser Arg Ser
370 375 380

Ala Asp Ala Pro Ala Tyr Gln Gln Gly Gln Asn Gln Leu Tyr Asn Glu
385 390 395 400

Leu Asn Leu Gly Arg Arg Glu Glu Tyr Asp Val Leu Asp Lys Arg Arg
405 410 415

265

```
Gly Arg Asp Pro Glu Met Gly Gly Lys Pro Arg Arg Lys Asn Pro Gln
            420                 425                 430

Glu Gly Leu Tyr Asn Glu Leu Gln Lys Asp Lys Met Ala Glu Ala Tyr
            435                 440                 445

Ser Glu Ile Gly Met Lys Gly Glu Arg Arg Arg Gly Lys Gly His Asp
            450                 455                 460

Gly Leu Tyr Gln Gly Leu Ser Thr Ala Thr Lys Asp Thr Tyr Asp Ala
465                 470                 475                 480

Leu His Met Gln Ala Leu Pro Pro Arg Glu
                485                 490
```

<210> 153
<211> 496
<212> PRT
<213> artificial sequence

<220>
<223> polypeptide

<400> 153

```
Gln Ile Gln Leu Val Gln Ser Gly Pro Glu Leu Lys Lys Pro Gly Glu
1               5                   10                  15

Thr Val Lys Ile Ser Cys Lys Ala Ser Gly Tyr Ile Phe Thr Asn Tyr
            20                  25                  30

Gly Met Asn Trp Val Lys Gln Ala Pro Gly Lys Ser Phe Lys Trp Met
            35                  40                  45

Gly Trp Ile Asn Thr Tyr Thr Gly Glu Ser Thr Tyr Ser Ala Asp Phe
        50                  55                  60

Lys Gly Arg Phe Ala Phe Ser Leu Glu Thr Ser Ala Ser Thr Ala Tyr
65                  70                  75                  80

Leu His Ile Asn Asp Leu Lys Asn Glu Asp Thr Ala Thr Tyr Phe Cys
                85                  90                  95

Ala Arg Ser Gly Gly Tyr Asp Pro Met Asp Tyr Trp Gly Gln Gly Thr
            100                 105                 110

Ser Val Thr Val Ser Ser Gly Gly Gly Gly Ser Gly Gly Gly Gly Ser
            115                 120                 125
```

```
Gly Gly Gly Gly Ser Asp Ile Val Leu Thr Gln Ser Pro Ala Ser Leu
    130             135             140

Ala Val Ser Leu Gly Gln Arg Ala Thr Ile Ser Cys Arg Ala Ser Glu
145             150             155             160

Ser Val Asp Asn Tyr Gly Asn Thr Phe Met His Trp Tyr Gln Gln Lys
                165             170             175

Pro Gly Gln Pro Pro Lys Leu Leu Ile Tyr Arg Ala Ser Asn Leu Glu
            180             185             190

Ser Gly Ile Pro Ala Arg Phe Ser Gly Ser Gly Ser Arg Thr Asp Phe
        195             200             205

Thr Leu Thr Ile Asn Pro Val Glu Ala Asp Asp Val Ala Thr Tyr Tyr
    210             215             220

Cys Gln Gln Ser Asn Glu Asp Pro Pro Thr Phe Gly Ala Gly Thr Lys
225             230             235             240

Leu Glu Leu Lys Arg Ser Asp Pro Gly Ser Gly Gly Gly Gly Ser Cys
            245             250             255

Pro Tyr Ser Asn Pro Ser Leu Cys Ser Gly Gly Gly Gly Ser Ala Pro
            260             265             270

Thr Thr Thr Pro Ala Pro Arg Pro Pro Thr Pro Ala Pro Thr Ile Ala
        275             280             285

Ser Gln Pro Leu Ser Leu Arg Pro Glu Ala Cys Arg Pro Ala Ala Gly
    290             295             300

Gly Ala Val His Thr Arg Gly Leu Asp Phe Ala Cys Asp Ile Tyr Ile
305             310             315             320

Trp Ala Pro Leu Ala Gly Thr Cys Gly Val Leu Leu Leu Ser Leu Val
            325             330             335

Ile Thr Leu Tyr Cys Arg Arg Gly Arg Lys Lys Leu Leu Tyr Ile Phe
        340             345             350

Lys Gln Pro Phe Met Arg Pro Val Gln Thr Thr Gln Glu Glu Asp Gly
        355             360             365

Cys Ser Cys Arg Phe Pro Glu Glu Glu Glu Gly Gly Cys Glu Leu Arg
    370             375             380
```

267

```
Val Lys Phe Ser Arg Ser Ala Asp Ala Pro Ala Tyr Gln Gln Gly Gln
385             390             395             400

Asn Gln Leu Tyr Asn Glu Leu Asn Leu Gly Arg Arg Glu Glu Tyr Asp
                405             410             415

Val Leu Asp Lys Arg Arg Gly Arg Asp Pro Glu Met Gly Gly Lys Pro
            420             425             430

Arg Arg Lys Asn Pro Gln Glu Gly Leu Tyr Asn Glu Leu Gln Lys Asp
            435             440             445

Lys Met Ala Glu Ala Tyr Ser Glu Ile Gly Met Lys Gly Glu Arg Arg
    450             455             460

Arg Gly Lys Gly His Asp Gly Leu Tyr Gln Gly Leu Ser Thr Ala Thr
465             470             475             480

Lys Asp Thr Tyr Asp Ala Leu His Met Gln Ala Leu Pro Pro Arg Glu
            485             490             495
```

<210> 154
<211> 505
<212> PRT
<213> artificial sequence

<220>
<223> polypeptide

<400> 154

```
Gln Ile Gln Leu Val Gln Ser Gly Pro Glu Leu Lys Lys Pro Gly Glu
1               5               10              15

Thr Val Lys Ile Ser Cys Lys Ala Ser Gly Tyr Ile Phe Thr Asn Tyr
            20              25              30

Gly Met Asn Trp Val Lys Gln Ala Pro Gly Lys Ser Phe Lys Trp Met
    35              40              45

Gly Trp Ile Asn Thr Tyr Thr Gly Glu Ser Thr Tyr Ser Ala Asp Phe
    50              55              60

Lys Gly Arg Phe Ala Phe Ser Leu Glu Thr Ser Ala Ser Thr Ala Tyr
65              70              75              80

Leu His Ile Asn Asp Leu Lys Asn Glu Asp Thr Ala Thr Tyr Phe Cys
                85              90              95
```

268

Ala Arg Ser Gly Gly Tyr Asp Pro Met Asp Tyr Trp Gly Gln Gly Thr
            100                 105                 110

Ser Val Thr Val Ser Ser Gly Gly Gly Gly Ser Gly Gly Gly Gly Ser
            115                 120                 125

Gly Gly Gly Gly Ser Asp Ile Val Leu Thr Gln Ser Pro Ala Ser Leu
            130                 135                 140

Ala Val Ser Leu Gly Gln Arg Ala Thr Ile Ser Cys Arg Ala Ser Glu
145                 150                 155                 160

Ser Val Asp Asn Tyr Gly Asn Thr Phe Met His Trp Tyr Gln Gln Lys
                165                 170                 175

Pro Gly Gln Pro Pro Lys Leu Leu Ile Tyr Arg Ala Ser Asn Leu Glu
            180                 185                 190

Ser Gly Ile Pro Ala Arg Phe Ser Gly Ser Gly Ser Arg Thr Asp Phe
            195                 200                 205

Thr Leu Thr Ile Asn Pro Val Glu Ala Asp Asp Val Ala Thr Tyr Tyr
            210                 215                 220

Cys Gln Gln Ser Asn Glu Asp Pro Pro Thr Phe Gly Ala Gly Thr Lys
225                 230                 235                 240

Leu Glu Leu Lys Arg Ser Asp Pro Gly Ser Gly Gly Gly Gly Ser Cys
                245                 250                 255

Pro Tyr Ser Asn Pro Ser Leu Cys Ser Gly Gly Gly Gly Ser Cys Pro
                260                 265                 270

Tyr Ser Asn Pro Ser Leu Cys Ala Pro Thr Thr Thr Pro Ala Pro Arg
                275                 280                 285

Pro Pro Thr Pro Ala Pro Thr Ile Ala Ser Gln Pro Leu Ser Leu Arg
            290                 295                 300

Pro Glu Ala Cys Arg Pro Ala Ala Gly Gly Ala Val His Thr Arg Gly
305                 310                 315                 320

Leu Asp Phe Ala Cys Asp Ile Tyr Ile Trp Ala Pro Leu Ala Gly Thr
                325                 330                 335

Cys Gly Val Leu Leu Leu Ser Leu Val Ile Thr Leu Tyr Cys Arg Arg
            340                 345                 350

```
Gly Arg Lys Lys Leu Leu Tyr Ile Phe Lys Gln Pro Phe Met Arg Pro
        355             360             365

Val Gln Thr Thr Gln Glu Glu Asp Gly Cys Ser Cys Arg Phe Pro Glu
        370             375             380

Glu Glu Glu Gly Gly Cys Glu Leu Arg Val Lys Phe Ser Arg Ser Ala
    385             390             395             400

Asp Ala Pro Ala Tyr Gln Gln Gly Gln Asn Gln Leu Tyr Asn Glu Leu
                405             410             415

Asn Leu Gly Arg Arg Glu Glu Tyr Asp Val Leu Asp Lys Arg Arg Gly
            420             425             430

Arg Asp Pro Glu Met Gly Gly Lys Pro Arg Arg Lys Asn Pro Gln Glu
        435             440             445

Gly Leu Tyr Asn Glu Leu Gln Lys Asp Lys Met Ala Glu Ala Tyr Ser
    450             455             460

Glu Ile Gly Met Lys Gly Glu Arg Arg Arg Gly Lys Gly His Asp Gly
    465             470             475             480

Leu Tyr Gln Gly Leu Ser Thr Ala Thr Lys Asp Thr Tyr Asp Ala Leu
                485             490             495

His Met Gln Ala Leu Pro Pro Arg Glu
            500             505
```

<210> 155
<211> 511
<212> PRT
<213> artificial sequence

<220>
<223> polypeptide

<400> 155

```
Gln Ile Gln Leu Val Gln Ser Gly Pro Glu Leu Lys Lys Pro Gly Glu
1               5               10              15

Thr Val Lys Ile Ser Cys Lys Ala Ser Gly Tyr Ile Phe Thr Asn Tyr
            20              25              30

Gly Met Asn Trp Val Lys Gln Ala Pro Gly Lys Ser Phe Lys Trp Met
            35              40              45
```

Gly Trp Ile Asn Thr Tyr Thr Gly Glu Ser Thr Tyr Ser Ala Asp Phe
    50              55              60

Lys Gly Arg Phe Ala Phe Ser Leu Glu Thr Ser Ala Ser Thr Ala Tyr
65              70              75              80

Leu His Ile Asn Asp Leu Lys Asn Glu Asp Thr Ala Thr Tyr Phe Cys
                85              90              95

Ala Arg Ser Gly Gly Tyr Asp Pro Met Asp Tyr Trp Gly Gln Gly Thr
            100             105             110

Ser Val Thr Val Ser Ser Gly Gly Gly Ser Gly Gly Gly Gly Ser
        115             120             125

Gly Gly Gly Gly Ser Asp Ile Val Leu Thr Gln Ser Pro Ala Ser Leu
        130             135             140

Ala Val Ser Leu Gly Gln Arg Ala Thr Ile Ser Cys Arg Ala Ser Glu
145             150             155             160

Ser Val Asp Asn Tyr Gly Asn Thr Phe Met His Trp Tyr Gln Gln Lys
            165             170             175

Pro Gly Gln Pro Pro Lys Leu Leu Ile Tyr Arg Ala Ser Asn Leu Glu
        180             185             190

Ser Gly Ile Pro Ala Arg Phe Ser Gly Ser Gly Ser Arg Thr Asp Phe
        195             200             205

Thr Leu Thr Ile Asn Pro Val Glu Ala Asp Asp Val Ala Thr Tyr Tyr
    210             215             220

Cys Gln Gln Ser Asn Glu Asp Pro Pro Thr Phe Gly Ala Gly Thr Lys
225             230             235             240

Leu Glu Leu Lys Arg Ser Asp Pro Gly Ser Gly Gly Gly Gly Ser Cys
            245             250             255

Pro Tyr Ser Asn Pro Ser Leu Cys Ser Gly Gly Gly Gly Ser Cys Pro
        260             265             270

Tyr Ser Asn Pro Ser Leu Cys Ser Gly Gly Gly Gly Ser Ala Pro Thr
        275             280             285

Thr Thr Pro Ala Pro Arg Pro Pro Thr Pro Ala Pro Thr Ile Ala Ser
    290             295             300

271

EP 3 250 604 B1

Gln Pro Leu Ser Leu Arg Pro Glu Ala Cys Arg Pro Ala Ala Gly Gly
305                310                315                320

Ala Val His Thr Arg Gly Leu Asp Phe Ala Cys Asp Ile Tyr Ile Trp
325                330                335

Ala Pro Leu Ala Gly Thr Cys Gly Val Leu Leu Leu Ser Leu Val Ile
340                345                350

Thr Leu Tyr Cys Arg Arg Gly Arg Lys Lys Leu Leu Tyr Ile Phe Lys
355                360                365

Gln Pro Phe Met Arg Pro Val Gln Thr Thr Gln Glu Glu Asp Gly Cys
370                375                380

Ser Cys Arg Phe Pro Glu Glu Glu Glu Gly Gly Cys Glu Leu Arg Val
385                390                395                400

Lys Phe Ser Arg Ser Ala Asp Ala Pro Ala Tyr Gln Gln Gly Gln Asn
405                410                415

Gln Leu Tyr Asn Glu Leu Asn Leu Gly Arg Arg Glu Glu Tyr Asp Val
420                425                430

Leu Asp Lys Arg Arg Gly Arg Asp Pro Glu Met Gly Gly Lys Pro Arg
435                440                445

Arg Lys Asn Pro Gln Glu Gly Leu Tyr Asn Glu Leu Gln Lys Asp Lys
450                455                460

Met Ala Glu Ala Tyr Ser Glu Ile Gly Met Lys Gly Glu Arg Arg Arg
465                470                475                480

Gly Lys Gly His Asp Gly Leu Tyr Gln Gly Leu Ser Thr Ala Thr Lys
485                490                495

Asp Thr Tyr Asp Ala Leu His Met Gln Ala Leu Pro Pro Arg Glu
500                505                510

<210> 156
<211> 474
<212> PRT
<213> artificial sequence

<220>
<223> polypeptide

<400> 156

272

```
Gln Ile Gln Leu Val Gln Ser Gly Pro Glu Leu Lys Lys Pro Gly Glu
1               5                   10                  15

Thr Val Lys Ile Ser Cys Lys Ala Ser Gly Tyr Ile Phe Thr Asn Tyr
            20                  25                  30

Gly Met Asn Trp Val Lys Gln Ala Pro Gly Lys Ser Phe Lys Trp Met
        35                  40                  45

Gly Trp Ile Asn Thr Tyr Thr Gly Glu Ser Thr Tyr Ser Ala Asp Phe
        50                  55                  60

Lys Gly Arg Phe Ala Phe Ser Leu Glu Thr Ser Ala Ser Thr Ala Tyr
65                  70                  75                  80

Leu His Ile Asn Asp Leu Lys Asn Glu Asp Thr Ala Thr Tyr Phe Cys
                85                  90                  95

Ala Arg Ser Gly Gly Tyr Asp Pro Met Asp Tyr Trp Gly Gln Gly Thr
            100                 105                 110

Ser Val Thr Val Ser Ser Gly Gly Gly Cys Pro Tyr Ser Asn Pro Ser
        115                 120                 125

Leu Cys Gly Gly Gly Gly Ser Asp Ile Val Leu Thr Gln Ser Pro Ala
        130                 135                 140

Ser Leu Ala Val Ser Leu Gly Gln Arg Ala Thr Ile Ser Cys Arg Ala
145                 150                 155                 160

Ser Glu Ser Val Asp Asn Tyr Gly Asn Thr Phe Met His Trp Tyr Gln
                165                 170                 175

Gln Lys Pro Gly Gln Pro Pro Lys Leu Leu Ile Tyr Arg Ala Ser Asn
            180                 185                 190

Leu Glu Ser Gly Ile Pro Ala Arg Phe Ser Gly Ser Gly Ser Arg Thr
            195                 200                 205

Asp Phe Thr Leu Thr Ile Asn Pro Val Glu Ala Asp Asp Val Ala Thr
        210                 215                 220

Tyr Tyr Cys Gln Gln Ser Asn Glu Asp Pro Pro Thr Phe Gly Ala Gly
225                 230                 235                 240

Thr Lys Leu Glu Leu Lys Arg Ser Asp Pro Thr Thr Thr Pro Ala Pro
                245                 250                 255
```

273

```
Arg Pro Pro Thr Pro Ala Pro Thr Ile Ala Ser Gln Pro Leu Ser Leu
        260         265                 270

Arg Pro Glu Ala Cys Arg Pro Ala Ala Gly Gly Ala Val His Thr Arg
        275             280             285

Gly Leu Asp Phe Ala Cys Asp Ile Tyr Ile Trp Ala Pro Leu Ala Gly
        290             295             300

Thr Cys Gly Val Leu Leu Leu Ser Leu Val Ile Thr Leu Tyr Cys Arg
305             310             315             320

Arg Gly Arg Lys Lys Leu Leu Tyr Ile Phe Lys Gln Pro Phe Met Arg
            325             330             335

Pro Val Gln Thr Thr Gln Glu Glu Asp Gly Cys Ser Cys Arg Phe Pro
        340             345             350

Glu Glu Glu Glu Gly Gly Cys Glu Leu Arg Val Lys Phe Ser Arg Ser
        355             360             365

Ala Asp Ala Pro Ala Tyr Gln Gln Gly Gln Asn Gln Leu Tyr Asn Glu
        370             375             380

Leu Asn Leu Gly Arg Arg Glu Glu Tyr Asp Val Leu Asp Lys Arg Arg
385             390             395             400

Gly Arg Asp Pro Glu Met Gly Gly Lys Pro Arg Arg Lys Asn Pro Gln
            405             410             415

Glu Gly Leu Tyr Asn Glu Leu Gln Lys Asp Lys Met Ala Glu Ala Tyr
        420             425             430

Ser Glu Ile Gly Met Lys Gly Glu Arg Arg Arg Gly Lys Gly His Asp
        435             440             445

Gly Leu Tyr Gln Gly Leu Ser Thr Ala Thr Lys Asp Thr Tyr Asp Ala
        450             455             460

Leu His Met Gln Ala Leu Pro Pro Arg Glu
465             470
```

<210> 157
<211> 480
<212> PRT
<213> artificial sequence

<220>

<223> polypeptide

<400> 157

```
Gln Ile Gln Leu Val Gln Ser Gly Pro Glu Leu Lys Lys Pro Gly Glu
1               5                   10                  15

Thr Val Lys Ile Ser Cys Lys Ala Ser Gly Tyr Ile Phe Thr Asn Tyr
                20                  25                  30

Gly Met Asn Trp Val Lys Gln Ala Pro Gly Lys Ser Phe Lys Trp Met
                35                  40                  45

Gly Trp Ile Asn Thr Tyr Thr Gly Glu Ser Thr Tyr Ser Ala Asp Phe
        50                  55                  60

Lys Gly Arg Phe Ala Phe Ser Leu Glu Thr Ser Ala Ser Thr Ala Tyr
65                  70                  75                  80

Leu His Ile Asn Asp Leu Lys Asn Glu Asp Thr Ala Thr Tyr Phe Cys
                85                  90                  95

Ala Arg Ser Gly Gly Tyr Asp Pro Met Asp Tyr Trp Gly Gln Gly Thr
            100                 105                 110

Ser Val Thr Val Ser Ser Gly Gly Gly Gly Gly Gly Cys Pro Tyr Ser
            115                 120                 125

Asn Pro Ser Leu Cys Gly Gly Gly Gly Gly Gly Gly Ser Asp Ile Val
    130                 135                 140

Leu Thr Gln Ser Pro Ala Ser Leu Ala Val Ser Leu Gly Gln Arg Ala
145                 150                 155                 160

Thr Ile Ser Cys Arg Ala Ser Glu Ser Val Asp Asn Tyr Gly Asn Thr
            165                 170                 175

Phe Met His Trp Tyr Gln Gln Lys Pro Gly Gln Pro Pro Lys Leu Leu
            180                 185                 190

Ile Tyr Arg Ala Ser Asn Leu Glu Ser Gly Ile Pro Ala Arg Phe Ser
        195                 200                 205

Gly Ser Gly Ser Arg Thr Asp Phe Thr Leu Thr Ile Asn Pro Val Glu
    210                 215                 220

Ala Asp Asp Val Ala Thr Tyr Tyr Cys Gln Gln Ser Asn Glu Asp Pro
225                 230                 235                 240
```

276

```
Pro Thr Phe Gly Ala Gly Thr Lys Leu Glu Leu Lys Arg Ser Asp Pro
            245             250             255

Thr Thr Thr Pro Ala Pro Arg Pro Pro Thr Pro Ala Pro Thr Ile Ala
            260             265             270

Ser Gln Pro Leu Ser Leu Arg Pro Glu Ala Cys Arg Pro Ala Ala Gly
            275             280             285

Gly Ala Val His Thr Arg Gly Leu Asp Phe Ala Cys Asp Ile Tyr Ile
            290             295             300

Trp Ala Pro Leu Ala Gly Thr Cys Gly Val Leu Leu Leu Ser Leu Val
305             310             315             320

Ile Thr Leu Tyr Cys Arg Arg Gly Arg Lys Lys Leu Leu Tyr Ile Phe
            325             330             335

Lys Gln Pro Phe Met Arg Pro Val Gln Thr Thr Gln Glu Glu Asp Gly
            340             345             350

Cys Ser Cys Arg Phe Pro Glu Glu Glu Glu Gly Gly Cys Glu Leu Arg
            355             360             365

Val Lys Phe Ser Arg Ser Ala Asp Ala Pro Ala Tyr Gln Gln Gly Gln
            370             375             380

Asn Gln Leu Tyr Asn Glu Leu Asn Leu Gly Arg Arg Glu Glu Tyr Asp
385             390             395             400

Val Leu Asp Lys Arg Arg Gly Arg Asp Pro Glu Met Gly Gly Lys Pro
            405             410             415

Arg Arg Lys Asn Pro Gln Glu Gly Leu Tyr Asn Glu Leu Gln Lys Asp
            420             425             430

Lys Met Ala Glu Ala Tyr Ser Glu Ile Gly Met Lys Gly Glu Arg Arg
            435             440             445

Arg Gly Lys Gly His Asp Gly Leu Tyr Gln Gly Leu Ser Thr Ala Thr
            450             455             460

Lys Asp Thr Tyr Asp Ala Leu His Met Gln Ala Leu Pro Pro Arg Glu
465             470             475             480
```

<210> 158
<211> 487

277

<212> PRT
<213> artificial sequence

<220>
<223> polypeptide

<400> 158

Gly Gly Gly Gly Ser Cys Pro Tyr Ser Asn Pro Ser Leu Cys Gly Gln
1           5           10          15

Ile Gln Leu Val Gln Ser Gly Pro Glu Leu Lys Lys Pro Gly Glu Thr
        20          25          30

Val Lys Ile Ser Cys Lys Ala Ser Gly Tyr Ile Phe Thr Asn Tyr Gly
        35          40          45

Met Asn Trp Val Lys Gln Ala Pro Gly Lys Ser Phe Lys Trp Met Gly
    50          55          60

Trp Ile Asn Thr Tyr Thr Gly Glu Ser Thr Tyr Ser Ala Asp Phe Lys
65          70          75          80

Gly Arg Phe Ala Phe Ser Leu Glu Thr Ser Ala Ser Thr Ala Tyr Leu
        85          90          95

His Ile Asn Asp Leu Lys Asn Glu Asp Thr Ala Thr Tyr Phe Cys Ala
        100         105         110

Arg Ser Gly Gly Tyr Asp Pro Met Asp Tyr Trp Gly Gln Gly Thr Ser
    115         120         125

Val Thr Val Ser Ser Gly Gly Gly Ser Gly Gly Gly Gly Ser Gly
    130         135         140

Gly Gly Gly Ser Asp Ile Val Leu Thr Gln Ser Pro Ala Ser Leu Ala
145         150         155         160

Val Ser Leu Gly Gln Arg Ala Thr Ile Ser Cys Arg Ala Ser Glu Ser
        165         170         175

Val Asp Asn Tyr Gly Asn Thr Phe Met His Trp Tyr Gln Gln Lys Pro
        180         185         190

Gly Gln Pro Pro Lys Leu Leu Ile Tyr Arg Ala Ser Asn Leu Glu Ser
    195         200         205

Gly Ile Pro Ala Arg Phe Ser Gly Ser Gly Ser Arg Thr Asp Phe Thr
    210         215         220

```
Leu Thr Ile Asn Pro Val Glu Ala Asp Asp Val Ala Thr Tyr Tyr Cys
225             230             235             240

Gln Gln Ser Asn Glu Asp Pro Pro Thr Phe Gly Ala Gly Thr Lys Leu
                245             250             255

Glu Leu Lys Arg Ser Asp Pro Thr Thr Thr Pro Ala Pro Arg Pro Pro
            260             265             270

Thr Pro Ala Pro Thr Ile Ala Ser Gln Pro Leu Ser Leu Arg Pro Glu
            275             280             285

Ala Cys Arg Pro Ala Ala Gly Gly Ala Val His Thr Arg Gly Leu Asp
            290             295             300

Phe Ala Cys Asp Ile Tyr Ile Trp Ala Pro Leu Ala Gly Thr Cys Gly
305             310             315             320

Val Leu Leu Leu Ser Leu Val Ile Thr Leu Tyr Cys Arg Arg Gly Arg
                325             330             335

Lys Lys Leu Leu Tyr Ile Phe Lys Gln Pro Phe Met Arg Pro Val Gln
            340             345             350

Thr Thr Gln Glu Glu Asp Gly Cys Ser Cys Arg Phe Pro Glu Glu Glu
            355             360             365

Glu Gly Gly Cys Glu Leu Arg Val Lys Phe Ser Arg Ser Ala Asp Ala
            370             375             380

Pro Ala Tyr Gln Gln Gly Gln Asn Gln Leu Tyr Asn Glu Leu Asn Leu
385             390             395             400

Gly Arg Arg Glu Glu Tyr Asp Val Leu Asp Lys Arg Arg Gly Arg Asp
                405             410             415

Pro Glu Met Gly Gly Lys Pro Arg Arg Lys Asn Pro Gln Glu Gly Leu
            420             425             430

Tyr Asn Glu Leu Gln Lys Asp Lys Met Ala Glu Ala Tyr Ser Glu Ile
            435             440             445

Gly Met Lys Gly Glu Arg Arg Arg Gly Lys Gly His Asp Gly Leu Tyr
            450             455             460

Gln Gly Leu Ser Thr Ala Thr Lys Asp Thr Tyr Asp Ala Leu His Met
465             470             475             480
```

```
                    Gln Ala Leu Pro Pro Arg Glu
                                485
```

<210> 159

<211> 493

<212> PRT

<213> artificial sequence


<220>

<223> polypeptide


<400> 159

```
        Gly Gly Gly Gly Ser Cys Pro Tyr Ser Asn Pro Ser Leu Cys Ser Gly
        1               5                   10                  15


        Gly Gly Gly Ser Gly Gln Ile Gln Leu Val Gln Ser Gly Pro Glu Leu
                    20                  25                  30


        Lys Lys Pro Gly Glu Thr Val Lys Ile Ser Cys Lys Ala Ser Gly Tyr
                    35                  40                  45


        Ile Phe Thr Asn Tyr Gly Met Asn Trp Val Lys Gln Ala Pro Gly Lys
            50                  55                  60


        Ser Phe Lys Trp Met Gly Trp Ile Asn Thr Tyr Thr Gly Glu Ser Thr
        65                  70                  75                  80


        Tyr Ser Ala Asp Phe Lys Gly Arg Phe Ala Phe Ser Leu Glu Thr Ser
                        85                  90                  95


        Ala Ser Thr Ala Tyr Leu His Ile Asn Asp Leu Lys Asn Glu Asp Thr
                    100                 105                 110


        Ala Thr Tyr Phe Cys Ala Arg Ser Gly Gly Tyr Asp Pro Met Asp Tyr
                    115                 120                 125


        Trp Gly Gln Gly Thr Ser Val Thr Val Ser Ser Gly Gly Gly Gly Ser
                130                 135                 140


        Gly Gly Gly Gly Ser Gly Gly Gly Gly Ser Asp Ile Val Leu Thr Gln
        145                 150                 155                 160


        Ser Pro Ala Ser Leu Ala Val Ser Leu Gly Gln Arg Ala Thr Ile Ser
                        165                 170                 175


        Cys Arg Ala Ser Glu Ser Val Asp Asn Tyr Gly Asn Thr Phe Met His
                    180                 185                 190
```

Trp Tyr Gln Gln Lys Pro Gly Gln Pro Pro Lys Leu Leu Ile Tyr Arg
        195                 200             205

Ala Ser Asn Leu Glu Ser Gly Ile Pro Ala Arg Phe Ser Gly Ser Gly
        210                 215             220

Ser Arg Thr Asp Phe Thr Leu Thr Ile Asn Pro Val Glu Ala Asp Asp
225                 230             235                 240

Val Ala Thr Tyr Tyr Cys Gln Gln Ser Asn Glu Asp Pro Pro Thr Phe
                245             250             255

Gly Ala Gly Thr Lys Leu Glu Leu Lys Arg Ser Asp Pro Thr Thr Thr
            260             265             270

Pro Ala Pro Arg Pro Pro Thr Pro Ala Pro Thr Ile Ala Ser Gln Pro
            275             280             285

Leu Ser Leu Arg Pro Glu Ala Cys Arg Pro Ala Ala Gly Gly Ala Val
        290             295             300

His Thr Arg Gly Leu Asp Phe Ala Cys Asp Ile Tyr Ile Trp Ala Pro
305                 310             315                 320

Leu Ala Gly Thr Cys Gly Val Leu Leu Leu Ser Leu Val Ile Thr Leu
                325             330             335

Tyr Cys Arg Arg Gly Arg Lys Lys Leu Leu Tyr Ile Phe Lys Gln Pro
            340             345             350

Phe Met Arg Pro Val Gln Thr Thr Gln Glu Glu Asp Gly Cys Ser Cys
            355             360             365

Arg Phe Pro Glu Glu Glu Glu Gly Gly Cys Glu Leu Arg Val Lys Phe
    370             375             380

Ser Arg Ser Ala Asp Ala Pro Ala Tyr Gln Gln Gly Gln Asn Gln Leu
385             390             395                 400

Tyr Asn Glu Leu Asn Leu Gly Arg Arg Glu Glu Tyr Asp Val Leu Asp
            405             410             415

Lys Arg Arg Gly Arg Asp Pro Glu Met Gly Gly Lys Pro Arg Arg Lys
            420             425             430

Asn Pro Gln Glu Gly Leu Tyr Asn Glu Leu Gln Lys Asp Lys Met Ala
            435             440             445

282

```
Glu Ala Tyr Ser Glu Ile Gly Met Lys Gly Glu Arg Arg Arg Gly Lys
    450             455             460

Gly His Asp Gly Leu Tyr Gln Gly Leu Ser Thr Ala Thr Lys Asp Thr
465             470             475             480

Tyr Asp Ala Leu His Met Gln Ala Leu Pro Pro Arg Glu
                485             490
```

<210> 160
<211> 502
<212> PRT
<213> artificial sequence

<220>
<223> polypeptide

<400> 160

```
Gly Gly Gly Gly Ser Cys Pro Tyr Ser Asn Pro Ser Leu Cys Ser Gly
1           5           10              15

Gly Gly Gly Ser Cys Pro Tyr Ser Asn Pro Ser Leu Cys Gly Gln Ile
            20          25              30

Gln Leu Val Gln Ser Gly Pro Glu Leu Lys Lys Pro Gly Glu Thr Val
            35          40              45

Lys Ile Ser Cys Lys Ala Ser Gly Tyr Ile Phe Thr Asn Tyr Gly Met
        50              55              60

Asn Trp Val Lys Gln Ala Pro Gly Lys Ser Phe Lys Trp Met Gly Trp
65              70              75              80

Ile Asn Thr Tyr Thr Gly Glu Ser Thr Tyr Ser Ala Asp Phe Lys Gly
                85              90              95

Arg Phe Ala Phe Ser Leu Glu Thr Ser Ala Ser Thr Ala Tyr Leu His
            100             105             110

Ile Asn Asp Leu Lys Asn Glu Asp Thr Ala Thr Tyr Phe Cys Ala Arg
        115             120             125

Ser Gly Gly Tyr Asp Pro Met Asp Tyr Trp Gly Gln Gly Thr Ser Val
    130             135             140

Thr Val Ser Ser Gly Gly Gly Gly Ser Gly Gly Gly Gly Ser Gly Gly
145             150             155             160
```

283

```
Gly Gly Ser Asp Ile Val Leu Thr Gln Ser Pro Ala Ser Leu Ala Val
                165             170             175

Ser Leu Gly Gln Arg Ala Thr Ile Ser Cys Arg Ala Ser Glu Ser Val
                180             185             190

Asp Asn Tyr Gly Asn Thr Phe Met His Trp Tyr Gln Gln Lys Pro Gly
                195             200             205

Gln Pro Pro Lys Leu Leu Ile Tyr Arg Ala Ser Asn Leu Glu Ser Gly
    210             215             220

Ile Pro Ala Arg Phe Ser Gly Ser Gly Ser Arg Thr Asp Phe Thr Leu
225             230             235             240

Thr Ile Asn Pro Val Glu Ala Asp Asp Val Ala Thr Tyr Tyr Cys Gln
                245             250             255

Gln Ser Asn Glu Asp Pro Pro Thr Phe Gly Ala Gly Thr Lys Leu Glu
                260             265             270

Leu Lys Arg Ser Asp Pro Thr Thr Thr Pro Ala Pro Arg Pro Pro Thr
        275             280             285

Pro Ala Pro Thr Ile Ala Ser Gln Pro Leu Ser Leu Arg Pro Glu Ala
        290             295             300

Cys Arg Pro Ala Ala Gly Gly Ala Val His Thr Arg Gly Leu Asp Phe
305             310             315             320

Ala Cys Asp Ile Tyr Ile Trp Ala Pro Leu Ala Gly Thr Cys Gly Val
                325             330             335

Leu Leu Leu Ser Leu Val Ile Thr Leu Tyr Cys Arg Arg Gly Arg Lys
                340             345             350

Lys Leu Leu Tyr Ile Phe Lys Gln Pro Phe Met Arg Pro Val Gln Thr
        355             360             365

Thr Gln Glu Glu Asp Gly Cys Ser Cys Arg Phe Pro Glu Glu Glu Glu
        370             375             380

Gly Gly Cys Glu Leu Arg Val Lys Phe Ser Arg Ser Ala Asp Ala Pro
385             390             395             400

Ala Tyr Gln Gln Gly Gln Asn Gln Leu Tyr Asn Glu Leu Asn Leu Gly
                405             410             415
```

284

```
Arg Arg Glu Glu Tyr Asp Val Leu Asp Lys Arg Arg Gly Arg Asp Pro
        420             425             430

Glu Met Gly Gly Lys Pro Arg Arg Lys Asn Pro Gln Glu Gly Leu Tyr
        435             440             445

Asn Glu Leu Gln Lys Asp Lys Met Ala Glu Ala Tyr Ser Glu Ile Gly
    450             455             460

Met Lys Gly Glu Arg Arg Arg Gly Lys Gly His Asp Gly Leu Tyr Gln
465             470             475             480

Gly Leu Ser Thr Ala Thr Lys Asp Thr Tyr Asp Ala Leu His Met Gln
            485             490             495

Ala Leu Pro Pro Arg Glu
            500
```

<210> 161
<211> 508
<212> PRT
<213> artificial sequence

<220>
<223> polypeptide

<400> 161

```
Gly Gly Gly Gly Ser Cys Pro Tyr Ser Asn Pro Ser Leu Cys Ser Gly
1               5               10              15

Gly Gly Gly Ser Cys Pro Tyr Ser Asn Pro Ser Leu Cys Ser Gly Gly
        20              25              30

Gly Gly Ser Gly Gln Ile Gln Leu Val Gln Ser Gly Pro Glu Leu Lys
        35              40              45

Lys Pro Gly Glu Thr Val Lys Ile Ser Cys Lys Ala Ser Gly Tyr Ile
    50              55              60

Phe Thr Asn Tyr Gly Met Asn Trp Val Lys Gln Ala Pro Gly Lys Ser
65              70              75              80

Phe Lys Trp Met Gly Trp Ile Asn Thr Tyr Thr Gly Glu Ser Thr Tyr
            85              90              95

Ser Ala Asp Phe Lys Gly Arg Phe Ala Phe Ser Leu Glu Thr Ser Ala
            100             105             110
```

```
Ser Thr Ala Tyr Leu His Ile Asn Asp Leu Lys Asn Glu Asp Thr Ala
    115             120             125

Thr Tyr Phe Cys Ala Arg Ser Gly Gly Tyr Asp Pro Met Asp Tyr Trp
    130             135             140

Gly Gln Gly Thr Ser Val Thr Val Ser Ser Gly Gly Gly Gly Ser Gly
    145             150             155             160

Gly Gly Gly Ser Gly Gly Gly Gly Ser Asp Ile Val Leu Thr Gln Ser
            165             170             175

Pro Ala Ser Leu Ala Val Ser Leu Gly Gln Arg Ala Thr Ile Ser Cys
            180             185             190

Arg Ala Ser Glu Ser Val Asp Asn Tyr Gly Asn Thr Phe Met His Trp
            195             200             205

Tyr Gln Gln Lys Pro Gly Gln Pro Pro Lys Leu Leu Ile Tyr Arg Ala
    210             215             220

Ser Asn Leu Glu Ser Gly Ile Pro Ala Arg Phe Ser Gly Ser Gly Ser
225             230             235             240

Arg Thr Asp Phe Thr Leu Thr Ile Asn Pro Val Glu Ala Asp Asp Val
            245             250             255

Ala Thr Tyr Tyr Cys Gln Gln Ser Asn Glu Asp Pro Pro Thr Phe Gly
            260             265             270

Ala Gly Thr Lys Leu Glu Leu Lys Arg Ser Asp Pro Thr Thr Thr Pro
            275             280             285

Ala Pro Arg Pro Pro Thr Pro Ala Pro Thr Ile Ala Ser Gln Pro Leu
    290             295             300

Ser Leu Arg Pro Glu Ala Cys Arg Pro Ala Ala Gly Gly Ala Val His
305             310             315             320

Thr Arg Gly Leu Asp Phe Ala Cys Asp Ile Tyr Ile Trp Ala Pro Leu
            325             330             335

Ala Gly Thr Cys Gly Val Leu Leu Leu Ser Leu Val Ile Thr Leu Tyr
            340             345             350

Cys Arg Arg Gly Arg Lys Lys Leu Leu Tyr Ile Phe Lys Gln Pro Phe
    355             360             365
```

```
Met Arg Pro Val Gln Thr Thr Gln Glu Glu Asp Gly Cys Ser Cys Arg
    370             375             380

Phe Pro Glu Glu Glu Glu Gly Gly Cys Glu Leu Arg Val Lys Phe Ser
385             390             395             400

Arg Ser Ala Asp Ala Pro Ala Tyr Gln Gln Gly Gln Asn Gln Leu Tyr
                405             410             415

Asn Glu Leu Asn Leu Gly Arg Arg Glu Glu Tyr Asp Val Leu Asp Lys
            420             425             430

Arg Arg Gly Arg Asp Pro Glu Met Gly Gly Lys Pro Arg Arg Lys Asn
            435             440             445

Pro Gln Glu Gly Leu Tyr Asn Glu Leu Gln Lys Asp Lys Met Ala Glu
    450             455             460

Ala Tyr Ser Glu Ile Gly Met Lys Gly Glu Arg Arg Arg Gly Lys Gly
465             470             475             480

His Asp Gly Leu Tyr Gln Gly Leu Ser Thr Ala Thr Lys Asp Thr Tyr
            485             490             495

Asp Ala Leu His Met Gln Ala Leu Pro Pro Arg Glu
            500             505
```

<210> 162
<211> 558
<212> PRT
<213> artificial sequence

<220>
<223> polypeptide

<400> 162

```
Gly Gly Gly Gly Ser Cys Pro Tyr Ser Asn Pro Ser Leu Cys Ser Gly
1               5               10              15

Gly Gly Gly Ser Gly Gly Gly Gly Ser Glu Val Gln Leu Gln Gln Ser
            20              25              30

Gly Pro Glu Leu Ile Lys Pro Gly Ala Ser Val Lys Met Ser Cys Lys
            35              40              45

Ala Ser Gly Tyr Thr Phe Thr Ser Tyr Val Met His Trp Val Lys Gln
    50              55              60
```

```
Lys Pro Gly Gln Gly Leu Glu Trp Ile Gly Tyr Ile Asn Pro Tyr Asn
65              70              75              80

Asp Gly Thr Lys Tyr Asn Glu Lys Phe Lys Gly Lys Ala Thr Leu Thr
            85              90              95

Ser Asp Lys Ser Ser Ser Thr Ala Tyr Met Glu Leu Ser Ser Leu Thr
        100             105             110

Ser Glu Asp Ser Ala Val Tyr Tyr Cys Ala Arg Gly Thr Tyr Tyr Tyr
        115             120             125

Gly Ser Arg Val Phe Asp Tyr Trp Gly Gln Gly Thr Thr Leu Thr Val
    130             135             140

Ser Ser Gly Gly Gly Gly Ser Gly Gly Gly Ser Gly Gly Gly Gly
145             150             155             160

Ser Asp Ile Val Met Thr Gln Ala Ala Pro Ser Ile Pro Val Thr Pro
            165             170             175

Gly Glu Ser Val Ser Ile Ser Cys Arg Ser Ser Lys Ser Leu Leu Asn
        180             185             190

Ser Asn Gly Asn Thr Tyr Leu Tyr Trp Phe Leu Gln Arg Pro Gly Gln
    195             200             205

Ser Pro Gln Leu Leu Ile Tyr Arg Met Ser Asn Leu Ala Ser Gly Val
    210             215             220

Pro Asp Arg Phe Ser Gly Ser Gly Ser Gly Thr Ala Phe Thr Leu Arg
225             230             235             240

Ile Ser Arg Val Glu Ala Glu Asp Val Gly Val Tyr Tyr Cys Met Gln
            245             250             255

His Leu Glu Tyr Pro Phe Thr Phe Gly Ala Gly Thr Lys Leu Glu Leu
        260             265             270

Lys Arg Ala Asp Pro Gly Ser Gly Gly Gly Gly Ser Cys Pro Tyr Ser
        275             280             285

Asn Pro Ser Leu Cys Ser Gly Gly Gly Gly Ser Glu Leu Pro Thr Gln
    290             295             300

Gly Thr Phe Ser Asn Val Ser Thr Asn Val Ser Pro Ala Lys Pro Thr
305             310             315             320
```

```
Thr Thr Ala Cys Pro Tyr Ser Asn Pro Ser Leu Cys Ala Pro Thr Thr
                325             330             335

Thr Pro Ala Pro Arg Pro Pro Thr Pro Ala Pro Thr Ile Ala Ser Gln
            340             345             350

Pro Leu Ser Leu Arg Pro Glu Ala Cys Arg Pro Ala Ala Gly Gly Ala
        355             360             365

Val His Thr Arg Gly Leu Asp Phe Ala Cys Asp Ile Tyr Ile Trp Ala
    370             375             380

Pro Leu Ala Gly Thr Cys Gly Val Leu Leu Leu Ser Leu Val Ile Thr
385             390             395             400

Leu Tyr Cys Arg Arg Gly Arg Lys Lys Leu Leu Tyr Ile Phe Lys Gln
            405             410             415

Pro Phe Met Arg Pro Val Gln Thr Thr Gln Glu Glu Asp Gly Cys Ser
            420             425             430

Cys Arg Phe Pro Glu Glu Glu Glu Gly Gly Cys Glu Leu Arg Val Lys
        435             440             445

Phe Ser Arg Ser Ala Asp Ala Pro Ala Tyr Gln Gln Gly Gln Asn Gln
    450             455             460

Leu Tyr Asn Glu Leu Asn Leu Gly Arg Arg Glu Glu Tyr Asp Val Leu
465             470             475             480

Asp Lys Arg Arg Gly Arg Asp Pro Glu Met Gly Gly Lys Pro Arg Arg
            485             490             495

Lys Asn Pro Gln Glu Gly Leu Tyr Asn Glu Leu Gln Lys Asp Lys Met
            500             505             510

Ala Glu Ala Tyr Ser Glu Ile Gly Met Lys Gly Glu Arg Arg Arg Gly
        515             520             525

Lys Gly His Asp Gly Leu Tyr Gln Gly Leu Ser Thr Ala Thr Lys Asp
        530             535             540

Thr Tyr Asp Ala Leu His Met Gln Ala Leu Pro Pro Arg Glu
545             550             555
```

<210> 163
<211> 536

<212> PRT
<213> artificial sequence

<220>
<223> polypeptide

<400> 163

```
Glu  Val  Gln  Leu  Gln  Gln  Ser  Gly  Pro  Glu  Leu  Ile  Lys  Pro  Gly  Ala
1              5                   10                  15

Ser  Val  Lys  Met  Ser  Cys  Lys  Ala  Ser  Gly  Tyr  Thr  Phe  Thr  Ser  Tyr
          20                  25                  30

Val  Met  His  Trp  Val  Lys  Gln  Lys  Pro  Gly  Gln  Gly  Leu  Glu  Trp  Ile
          35                  40                  45

Gly  Tyr  Ile  Asn  Pro  Tyr  Asn  Asp  Gly  Thr  Lys  Tyr  Asn  Glu  Lys  Phe
          50                  55                  60

Lys  Gly  Lys  Ala  Thr  Leu  Thr  Ser  Asp  Lys  Ser  Ser  Ser  Thr  Ala  Tyr
65                  70                  75                  80

Met  Glu  Leu  Ser  Ser  Leu  Thr  Ser  Glu  Asp  Ser  Ala  Val  Tyr  Tyr  Cys
                85                  90                  95

Ala  Arg  Gly  Thr  Tyr  Tyr  Tyr  Gly  Ser  Arg  Val  Phe  Asp  Tyr  Trp  Gly
          100                 105                 110

Gln  Gly  Thr  Thr  Leu  Thr  Val  Ser  Ser  Ser  Ser  Gly  Gly  Ser  Cys  Pro
          115                 120                 125

Tyr  Ser  Asn  Pro  Ser  Leu  Cys  Ser  Gly  Gly  Ser  Asp  Ile  Val  Met  Thr
     130                 135                 140

Gln  Ala  Ala  Pro  Ser  Ile  Pro  Val  Thr  Pro  Gly  Glu  Ser  Val  Ser  Ile
145                 150                 155                 160

Ser  Cys  Arg  Ser  Ser  Lys  Ser  Leu  Leu  Asn  Ser  Asn  Gly  Asn  Thr  Tyr
               165                 170                 175

Leu  Tyr  Trp  Phe  Leu  Gln  Arg  Pro  Gly  Gln  Ser  Pro  Gln  Leu  Leu  Ile
          180                 185                 190

Tyr  Arg  Met  Ser  Asn  Leu  Ala  Ser  Gly  Val  Pro  Asp  Arg  Phe  Ser  Gly
          195                 200                 205

Ser  Gly  Ser  Gly  Thr  Ala  Phe  Thr  Leu  Arg  Ile  Ser  Arg  Val  Glu  Ala
     210                 215                 220
```

```
Glu Asp Val Gly Val Tyr Tyr Cys Met Gln His Leu Glu Tyr Pro Phe
225                 230             235             240

Thr Phe Gly Ala Gly Thr Lys Leu Glu Leu Lys Arg Ala Asp Pro Gly
                245             250             255

Ser Gly Gly Gly Gly Ser Cys Pro Tyr Ser Asn Pro Ser Leu Cys Ser
                260             265             270

Gly Gly Gly Gly Ser Glu Leu Pro Thr Gln Gly Thr Phe Ser Asn Val
        275             280             285

Ser Thr Asn Val Ser Pro Ala Lys Pro Thr Thr Thr Ala Cys Pro Tyr
    290             295             300

Ser Asn Pro Ser Leu Cys Ala Pro Thr Thr Thr Pro Ala Pro Arg Pro
305             310             315             320

Pro Thr Pro Ala Pro Thr Ile Ala Ser Gln Pro Leu Ser Leu Arg Pro
                325             330             335

Glu Ala Cys Arg Pro Ala Ala Gly Gly Ala Val His Thr Arg Gly Leu
            340             345             350

Asp Phe Ala Cys Asp Ile Tyr Ile Trp Ala Pro Leu Ala Gly Thr Cys
        355             360             365

Gly Val Leu Leu Leu Ser Leu Val Ile Thr Leu Tyr Cys Arg Arg Gly
    370             375             380

Arg Lys Lys Leu Leu Tyr Ile Phe Lys Gln Pro Phe Met Arg Pro Val
385             390             395             400

Gln Thr Thr Gln Glu Glu Asp Gly Cys Ser Cys Arg Phe Pro Glu Glu
                405             410             415

Glu Glu Gly Gly Cys Glu Leu Arg Val Lys Phe Ser Arg Ser Ala Asp
            420             425             430

Ala Pro Ala Tyr Gln Gln Gly Gln Asn Gln Leu Tyr Asn Glu Leu Asn
        435             440             445

Leu Gly Arg Arg Glu Glu Tyr Asp Val Leu Asp Lys Arg Arg Gly Arg
    450             455             460

Asp Pro Glu Met Gly Gly Lys Pro Arg Arg Lys Asn Pro Gln Glu Gly
```

```
              465                    470                    475                    480


      Leu Tyr Asn Glu Leu Gln Lys Asp Lys Met Ala Glu Ala Tyr Ser Glu
                      485                    490                    495


      Ile Gly Met Lys Gly Glu Arg Arg Arg Gly Lys Gly His Asp Gly Leu
                      500                    505                    510


      Tyr Gln Gly Leu Ser Thr Ala Thr Lys Asp Thr Tyr Asp Ala Leu His
                      515                    520                    525


      Met Gln Ala Leu Pro Pro Arg Glu
              530                    535
```

<210> 164
<211> 554
<212> PRT
<213> artificial sequence

<220>
<223> polypeptide

<400> 164

```
Gly Gly Gly Gly Ser Cys Pro Tyr Ser Asn Pro Ser Leu Cys Ser Gly
1               5                   10              15

Gly Gly Gly Ser Gly Gly Gly Gly Ser Gln Ile Gln Leu Val Gln Ser
            20                  25              30

Gly Pro Glu Leu Lys Lys Pro Gly Glu Thr Val Lys Ile Ser Cys Lys
        35                  40              45

Ala Ser Gly Tyr Ile Phe Thr Asn Tyr Gly Met Asn Trp Val Lys Gln
    50              55                  60

Ala Pro Gly Lys Ser Phe Lys Trp Met Gly Trp Ile Asn Thr Tyr Thr
65              70                  75                  80

Gly Glu Ser Thr Tyr Ser Ala Asp Phe Lys Gly Arg Phe Ala Phe Ser
            85                  90                  95

Leu Glu Thr Ser Ala Ser Thr Ala Tyr Leu His Ile Asn Asp Leu Lys
            100                 105                 110

Asn Glu Asp Thr Ala Thr Tyr Phe Cys Ala Arg Ser Gly Gly Tyr Asp
        115                 120                 125

Pro Met Asp Tyr Trp Gly Gln Gly Thr Ser Val Thr Val Ser Ser Gly
    130                 135                 140
```

Gly Gly Gly Ser Gly Gly Gly Gly Ser Gly Gly Gly Gly Ser Asp Ile
145             150             155             160

Val Leu Thr Gln Ser Pro Ala Ser Leu Ala Val Ser Leu Gly Gln Arg
            165             170             175

Ala Thr Ile Ser Cys Arg Ala Ser Glu Ser Val Asp Asn Tyr Gly Asn
            180             185             190

Thr Phe Met His Trp Tyr Gln Gln Lys Pro Gly Gln Pro Pro Lys Leu
            195             200             205

Leu Ile Tyr Arg Ala Ser Asn Leu Glu Ser Gly Ile Pro Ala Arg Phe
            210             215             220

Ser Gly Ser Gly Ser Arg Thr Asp Phe Thr Leu Thr Ile Asn Pro Val
225             230             235             240

Glu Ala Asp Asp Val Ala Thr Tyr Tyr Cys Gln Gln Ser Asn Glu Asp
            245             250             255

Pro Pro Thr Phe Gly Ala Gly Thr Lys Leu Glu Leu Lys Arg Ser Asp
            260             265             270

Pro Gly Ser Gly Gly Gly Gly Ser Cys Pro Tyr Ser Asn Pro Ser Leu
            275             280             285

Cys Ser Gly Gly Gly Gly Ser Glu Leu Pro Thr Gln Gly Thr Phe Ser
            290             295             300

Asn Val Ser Thr Asn Val Ser Pro Ala Lys Pro Thr Thr Thr Ala Cys
305             310             315             320

Pro Tyr Ser Asn Pro Ser Leu Cys Ala Pro Thr Thr Thr Pro Ala Pro
            325             330             335

Arg Pro Pro Thr Pro Ala Pro Thr Ile Ala Ser Gln Pro Leu Ser Leu
            340             345             350

Arg Pro Glu Ala Cys Arg Pro Ala Ala Gly Gly Ala Val His Thr Arg
            355             360             365

Gly Leu Asp Phe Ala Cys Asp Ile Tyr Ile Trp Ala Pro Leu Ala Gly
            370             375             380

Thr Cys Gly Val Leu Leu Leu Ser Leu Val Ile Thr Leu Tyr Cys Arg

```
        385                    390                    395                    400
```

Arg Gly Arg Lys Lys Leu Leu Tyr Ile Phe Lys Gln Pro Phe Met Arg
            405                 410                 415

Pro Val Gln Thr Thr Gln Glu Glu Asp Gly Cys Ser Cys Arg Phe Pro
            420                 425                 430

Glu Glu Glu Glu Gly Gly Cys Glu Leu Arg Val Lys Phe Ser Arg Ser
            435                 440                 445

Ala Asp Ala Pro Ala Tyr Gln Gln Gly Gln Asn Gln Leu Tyr Asn Glu
        450                 455                 460

Leu Asn Leu Gly Arg Arg Glu Glu Tyr Asp Val Leu Asp Lys Arg Arg
465                 470                 475                 480

Gly Arg Asp Pro Glu Met Gly Gly Lys Pro Arg Arg Lys Asn Pro Gln
                485                 490                 495

Glu Gly Leu Tyr Asn Glu Leu Gln Lys Asp Lys Met Ala Glu Ala Tyr
            500                 505                 510

Ser Glu Ile Gly Met Lys Gly Glu Arg Arg Arg Gly Lys Gly His Asp
            515                 520                 525

Gly Leu Tyr Gln Gly Leu Ser Thr Ala Thr Lys Asp Thr Tyr Asp Ala
        530                 535                 540

Leu His Met Gln Ala Leu Pro Pro Arg Glu
545                 550

<210> 165
<211> 532
<212> PRT
<213> artificial sequence

<220>
<223> polypeptide

<400> 165

```
Gln Ile Gln Leu Val Gln Ser Gly Pro Glu Leu Lys Lys Pro Gly Glu
1                   5                   10                  15

Thr Val Lys Ile Ser Cys Lys Ala Ser Gly Tyr Ile Phe Thr Asn Tyr
              20                  25                  30

Gly Met Asn Trp Val Lys Gln Ala Pro Gly Lys Ser Phe Lys Trp Met
              35                  40                  45
```

Gly Trp Ile Asn Thr Tyr Thr Gly Glu Ser Thr Tyr Ser Ala Asp Phe
    50                  55                  60

Lys Gly Arg Phe Ala Phe Ser Leu Glu Thr Ser Ala Ser Thr Ala Tyr
65                  70                  75                  80

Leu His Ile Asn Asp Leu Lys Asn Glu Asp Thr Ala Thr Tyr Phe Cys
                85                  90                  95

Ala Arg Ser Gly Gly Tyr Asp Pro Met Asp Tyr Trp Gly Gln Gly Thr
            100             105             110

Ser Val Thr Val Ser Ser Ser Ser Gly Gly Ser Cys Pro Tyr Ser Asn
        115             120             125

Pro Ser Leu Cys Ser Gly Gly Ser Asp Ile Val Leu Thr Gln Ser Pro
    130             135             140

Ala Ser Leu Ala Val Ser Leu Gly Gln Arg Ala Thr Ile Ser Cys Arg
145             150             155             160

Ala Ser Glu Ser Val Asp Asn Tyr Gly Asn Thr Phe Met His Trp Tyr
            165             170             175

Gln Gln Lys Pro Gly Gln Pro Pro Lys Leu Leu Ile Tyr Arg Ala Ser
        180             185             190

Asn Leu Glu Ser Gly Ile Pro Ala Arg Phe Ser Gly Ser Gly Ser Arg
        195             200             205

Thr Asp Phe Thr Leu Thr Ile Asn Pro Val Glu Ala Asp Asp Val Ala
    210             215             220

Thr Tyr Tyr Cys Gln Gln Ser Asn Glu Asp Pro Pro Thr Phe Gly Ala
225             230             235             240

Gly Thr Lys Leu Glu Leu Lys Arg Ser Asp Pro Gly Ser Gly Gly Gly
            245             250             255

Gly Ser Cys Pro Tyr Ser Asn Pro Ser Leu Cys Ser Gly Gly Gly Gly
            260             265             270

Ser Glu Leu Pro Thr Gln Gly Thr Phe Ser Asn Val Ser Thr Asn Val
    275             280             285

Ser Pro Ala Lys Pro Thr Thr Thr Ala Cys Pro Tyr Ser Asn Pro Ser

```
          290                        295                          300
```

```
Leu Cys Ala Pro Thr Thr Thr Pro Ala Pro Arg Pro Pro Thr Pro Ala
305             310             315             320
```

```
Pro Thr Ile Ala Ser Gln Pro Leu Ser Leu Arg Pro Glu Ala Cys Arg
            325             330             335
```

```
Pro Ala Ala Gly Gly Ala Val His Thr Arg Gly Leu Asp Phe Ala Cys
            340             345             350
```

```
Asp Ile Tyr Ile Trp Ala Pro Leu Ala Gly Thr Cys Gly Val Leu Leu
            355             360             365
```

```
Leu Ser Leu Val Ile Thr Leu Tyr Cys Arg Arg Gly Arg Lys Lys Leu
            370             375             380
```

```
Leu Tyr Ile Phe Lys Gln Pro Phe Met Arg Pro Val Gln Thr Thr Gln
385             390             395             400
```

```
Glu Glu Asp Gly Cys Ser Cys Arg Phe Pro Glu Glu Glu Glu Gly Gly
            405             410             415
```

```
Cys Glu Leu Arg Val Lys Phe Ser Arg Ser Ala Asp Ala Pro Ala Tyr
            420             425             430
```

```
Gln Gln Gly Gln Asn Gln Leu Tyr Asn Glu Leu Asn Leu Gly Arg Arg
            435             440             445
```

```
Glu Glu Tyr Asp Val Leu Asp Lys Arg Arg Gly Arg Asp Pro Glu Met
            450             455             460
```

```
Gly Gly Lys Pro Arg Arg Lys Asn Pro Gln Glu Gly Leu Tyr Asn Glu
465             470             475             480
```

```
Leu Gln Lys Asp Lys Met Ala Glu Ala Tyr Ser Glu Ile Gly Met Lys
            485             490             495
```

```
Gly Glu Arg Arg Arg Gly Lys Gly His Asp Gly Leu Tyr Gln Gly Leu
            500             505             510
```

```
Ser Thr Ala Thr Lys Asp Thr Tyr Asp Ala Leu His Met Gln Ala Leu
            515             520             525
```

```
Pro Pro Arg Glu
            530
```

<210> 166
<211> 553
<212> PRT
<213> artificial sequence

<220>
<223> polypeptide

<400> 166

Gly Gly Gly Gly Ser Cys Pro Tyr Ser Asn Pro Ser Leu Cys Ser Gly
1               5                   10              15

Gly Gly Gly Ser Gly Gly Gly Gly Ser Glu Val Gln Leu Val Glu Ser
          20                  25              30

Gly Gly Gly Leu Val Gln Pro Gly Arg Ser Leu Arg Leu Ser Cys Ala
          35                  40              45

Ala Ser Gly Phe Thr Phe Asn Asp Tyr Ala Met His Trp Val Arg Gln
    50                  55              60

Ala Pro Gly Lys Gly Leu Glu Trp Val Ser Thr Ile Ser Trp Asn Ser
65                  70              75                  80

Gly Ser Ile Gly Tyr Ala Asp Ser Val Lys Gly Arg Phe Thr Ile Ser
              85                  90                  95

Arg Asp Asn Ala Lys Lys Ser Leu Tyr Leu Gln Met Asn Ser Leu Arg
          100             105             110

Ala Glu Asp Thr Ala Leu Tyr Tyr Cys Ala Lys Asp Ile Gln Tyr Gly
          115             120             125

Asn Tyr Tyr Tyr Gly Met Asp Val Trp Gly Gln Gly Thr Thr Val Thr
    130             135             140

Val Ser Ser Gly Gly Gly Gly Ser Gly Gly Gly Ser Gly Gly Gly
145             150             155                 160

Gly Ser Glu Ile Val Leu Thr Gln Ser Pro Ala Thr Leu Ser Leu Ser
          165             170             175

Pro Gly Glu Arg Ala Thr Leu Ser Cys Arg Ala Ser Gln Ser Val Ser
          180             185             190

Ser Tyr Leu Ala Trp Tyr Gln Gln Lys Pro Gly Gln Ala Pro Arg Leu
    195             200             205

Leu Ile Tyr Asp Ala Ser Asn Arg Ala Thr Gly Ile Pro Ala Arg Phe

301

```
                 210                    215                     220

        Ser Gly Ser Gly Ser Gly Thr Asp Phe Thr Leu Thr Ile Ser Ser Leu
        225                 230             235                     240

        Glu Pro Glu Asp Phe Ala Val Tyr Tyr Cys Gln Gln Arg Ser Asn Trp
                        245             250                     255

        Pro Ile Thr Phe Gly Gln Gly Thr Arg Leu Glu Ile Lys Ser Asp Pro
                        260             265                     270

        Gly Ser Gly Gly Gly Gly Ser Cys Pro Tyr Ser Asn Pro Ser Leu Cys
                        275             280                     285

        Ser Gly Gly Gly Gly Ser Glu Leu Pro Thr Gln Gly Thr Phe Ser Asn
                290                 295             300

        Val Ser Thr Asn Val Ser Pro Ala Lys Pro Thr Thr Thr Ala Cys Pro
        305                 310                 315                     320

        Tyr Ser Asn Pro Ser Leu Cys Ala Pro Thr Thr Thr Pro Ala Pro Arg
                        325             330                     335

        Pro Pro Thr Pro Ala Pro Thr Ile Ala Ser Gln Pro Leu Ser Leu Arg
                        340             345                     350

        Pro Glu Ala Cys Arg Pro Ala Ala Gly Gly Ala Val His Thr Arg Gly
                355                 360                 365

        Leu Asp Phe Ala Cys Asp Ile Tyr Ile Trp Ala Pro Leu Ala Gly Thr
                370                 375             380

        Cys Gly Val Leu Leu Leu Ser Leu Val Ile Thr Leu Tyr Cys Arg Arg
        385                 390                 395                     400

        Gly Arg Lys Lys Leu Leu Tyr Ile Phe Lys Gln Pro Phe Met Arg Pro
                        405             410                     415

        Val Gln Thr Thr Gln Glu Glu Asp Gly Cys Ser Cys Arg Phe Pro Glu
                        420             425                     430

        Glu Glu Glu Gly Gly Cys Glu Leu Arg Val Lys Phe Ser Arg Ser Ala
                435                 440                 445

        Asp Ala Pro Ala Tyr Gln Gln Gly Gln Asn Gln Leu Tyr Asn Glu Leu
                450                 455                 460
```

```
Asn Leu Gly Arg Arg Glu Glu Tyr Asp Val Leu Asp Lys Arg Arg Gly
465                 470             475                 480


Arg Asp Pro Glu Met Gly Gly Lys Pro Arg Arg Lys Asn Pro Gln Glu
                485             490                 495


Gly Leu Tyr Asn Glu Leu Gln Lys Asp Lys Met Ala Glu Ala Tyr Ser
            500             505             510


Glu Ile Gly Met Lys Gly Glu Arg Arg Arg Gly Lys Gly His Asp Gly
            515             520             525


Leu Tyr Gln Gly Leu Ser Thr Ala Thr Lys Asp Thr Tyr Asp Ala Leu
    530             535             540


His Met Gln Ala Leu Pro Pro Arg Glu
545             550
```

<210> 167
<211> 531
<212> PRT
<213> artificial sequence

<220>
<223> polypeptide

<400> 167

```
Glu Val Gln Leu Val Glu Ser Gly Gly Gly Leu Val Gln Pro Gly Arg
1               5               10              15

Ser Leu Arg Leu Ser Cys Ala Ala Ser Gly Phe Thr Phe Asn Asp Tyr
            20              25              30

Ala Met His Trp Val Arg Gln Ala Pro Gly Lys Gly Leu Glu Trp Val
        35              40              45

Ser Thr Ile Ser Trp Asn Ser Gly Ser Ile Gly Tyr Ala Asp Ser Val
    50              55              60

Lys Gly Arg Phe Thr Ile Ser Arg Asp Asn Ala Lys Lys Ser Leu Tyr
65              70              75              80

Leu Gln Met Asn Ser Leu Arg Ala Glu Asp Thr Ala Leu Tyr Tyr Cys
            85              90              95

Ala Lys Asp Ile Gln Tyr Gly Asn Tyr Tyr Tyr Gly Met Asp Val Trp
        100             105             110

Gly Gln Gly Thr Thr Val Thr Val Ser Ser Ser Ser Gly Gly Ser Cys
```

```
                    115                     120                      125


        Pro Tyr Ser Asn Pro Ser Leu Cys Ser Gly Gly Ser Glu Ile Val Leu
            130                 135                 140


        Thr Gln Ser Pro Ala Thr Leu Ser Leu Ser Pro Gly Glu Arg Ala Thr
            145                 150                 155                 160


        Leu Ser Cys Arg Ala Ser Gln Ser Val Ser Ser Tyr Leu Ala Trp Tyr
                        165                 170                 175


        Gln Gln Lys Pro Gly Gln Ala Pro Arg Leu Leu Ile Tyr Asp Ala Ser
                        180                 185                 190


        Asn Arg Ala Thr Gly Ile Pro Ala Arg Phe Ser Gly Ser Gly Ser Gly
                        195                 200                 205


        Thr Asp Phe Thr Leu Thr Ile Ser Ser Leu Glu Pro Glu Asp Phe Ala
            210                 215                 220


        Val Tyr Tyr Cys Gln Gln Arg Ser Asn Trp Pro Ile Thr Phe Gly Gln
        225                 230                 235                 240


        Gly Thr Arg Leu Glu Ile Lys Ser Asp Pro Gly Ser Gly Gly Gly Gly
                        245                 250                 255


        Ser Cys Pro Tyr Ser Asn Pro Ser Leu Cys Ser Gly Gly Gly Gly Ser
                        260                 265                 270


        Glu Leu Pro Thr Gln Gly Thr Phe Ser Asn Val Ser Thr Asn Val Ser
                        275                 280                 285


        Pro Ala Lys Pro Thr Thr Thr Ala Cys Pro Tyr Ser Asn Pro Ser Leu
            290                 295                 300


        Cys Ala Pro Thr Thr Thr Pro Ala Pro Arg Pro Pro Thr Pro Ala Pro
        305                 310                 315                 320


        Thr Ile Ala Ser Gln Pro Leu Ser Leu Arg Pro Glu Ala Cys Arg Pro
                        325                 330                 335


        Ala Ala Gly Gly Ala Val His Thr Arg Gly Leu Asp Phe Ala Cys Asp
                        340                 345                 350


        Ile Tyr Ile Trp Ala Pro Leu Ala Gly Thr Cys Gly Val Leu Leu Leu
                        355                 360                 365
```

```
Ser Leu Val Ile Thr Leu Tyr Cys Arg Arg Gly Arg Lys Lys Leu Leu
    370             375             380

Tyr Ile Phe Lys Gln Pro Phe Met Arg Pro Val Gln Thr Thr Gln Glu
385             390             395             400

Glu Asp Gly Cys Ser Cys Arg Phe Pro Glu Glu Glu Glu Gly Gly Cys
                405             410             415

Glu Leu Arg Val Lys Phe Ser Arg Ser Ala Asp Ala Pro Ala Tyr Gln
            420             425             430

Gln Gly Gln Asn Gln Leu Tyr Asn Glu Leu Asn Leu Gly Arg Arg Glu
            435             440             445

Glu Tyr Asp Val Leu Asp Lys Arg Arg Gly Arg Asp Pro Glu Met Gly
    450             455             460

Gly Lys Pro Arg Arg Lys Asn Pro Gln Glu Gly Leu Tyr Asn Glu Leu
465             470             475             480

Gln Lys Asp Lys Met Ala Glu Ala Tyr Ser Glu Ile Gly Met Lys Gly
            485             490             495

Glu Arg Arg Arg Gly Lys Gly His Asp Gly Leu Tyr Gln Gly Leu Ser
            500             505             510

Thr Ala Thr Lys Asp Thr Tyr Asp Ala Leu His Met Gln Ala Leu Pro
            515             520             525

Pro Arg Glu
    530
```

<210> 168
<211> 552
<212> PRT
<213> artificial sequence

<220>
<223> polypeptide

<400> 168

```
Pro Gly Gly Gly Gly Ser Cys Pro Tyr Ser Asn Pro Ser Leu Cys Ser
1               5               10              15

Gly Gly Gly Gly Ser Gly Gly Gly Gly Ser Glu Val Lys Leu Gln Glu
        20              25              30

Ser Gly Pro Gly Leu Val Ala Pro Ser Gln Ser Leu Ser Val Thr Cys
```

        35                      40                    45

Thr Val Ser Gly Val Ser Leu Pro Asp Tyr Gly Val Ser Trp Ile Arg
     50              55                60

Gln Pro Pro Arg Lys Gly Leu Glu Trp Leu Gly Val Ile Trp Gly Ser
65              70              75            80

Glu Thr Thr Tyr Tyr Asn Ser Ala Leu Lys Ser Arg Leu Thr Ile Ile
          85              90              95

Lys Asp Asn Ser Lys Ser Gln Val Phe Leu Lys Met Asn Ser Leu Gln
        100            105          110

Thr Asp Asp Thr Ala Ile Tyr Tyr Cys Ala Lys His Tyr Tyr Tyr Gly
        115            120          125

Gly Ser Tyr Ala Met Asp Tyr Trp Gly Gln Gly Thr Ser Val Thr Val
        130            135          140

Ser Ser Gly Gly Gly Gly Ser Gly Gly Gly Gly Ser Gly Gly Gly Gly
145            150            155          160

Ser Asp Ile Gln Met Thr Gln Thr Thr Ser Ser Leu Ser Ala Ser Leu
        165            170          175

Gly Asp Arg Val Thr Ile Ser Cys Arg Ala Ser Gln Asp Ile Ser Lys
        180            185          190

Tyr Leu Asn Trp Tyr Gln Gln Lys Pro Asp Gly Thr Val Lys Leu Leu
        195            200          205

Ile Tyr His Thr Ser Arg Leu His Ser Gly Val Pro Ser Arg Phe Ser
        210            215          220

Gly Ser Gly Ser Gly Thr Asp Tyr Ser Leu Thr Ile Ser Asn Leu Glu
225            230            235          240

Gln Glu Asp Ile Ala Thr Tyr Phe Cys Gln Gln Gly Asn Thr Leu Pro
        245            250          255

Tyr Thr Phe Gly Gly Gly Thr Lys Leu Glu Ile Thr Ser Asp Pro Gly
        260            265          270

Ser Gly Gly Gly Gly Ser Cys Pro Tyr Ser Asn Pro Ser Leu Cys Ser
        275            280          285

```
Gly Gly Gly Gly Ser Glu Leu Pro Thr Gln Gly Thr Phe Ser Asn Val
    290             295             300

Ser Thr Asn Val Ser Pro Ala Lys Pro Thr Thr Thr Ala Cys Pro Tyr
305             310             315             320

Ser Asn Pro Ser Leu Cys Ala Pro Thr Thr Thr Pro Ala Pro Arg Pro
            325             330             335

Pro Thr Pro Ala Pro Thr Ile Ala Ser Gln Pro Leu Ser Leu Arg Pro
        340             345             350

Glu Ala Cys Arg Pro Ala Ala Gly Gly Ala Val His Thr Arg Gly Leu
    355             360             365

Asp Phe Ala Cys Asp Ile Tyr Ile Trp Ala Pro Leu Ala Gly Thr Cys
    370             375             380

Gly Val Leu Leu Leu Ser Leu Val Ile Thr Leu Tyr Cys Arg Arg Gly
385             390             395             400

Arg Lys Lys Leu Leu Tyr Ile Phe Lys Gln Pro Phe Met Arg Pro Val
            405             410             415

Gln Thr Thr Gln Glu Glu Asp Gly Cys Ser Cys Arg Phe Pro Glu Glu
        420             425             430

Glu Glu Gly Gly Cys Glu Leu Arg Val Lys Phe Ser Arg Ser Ala Asp
        435             440             445

Ala Pro Ala Tyr Gln Gln Gly Gln Asn Gln Leu Tyr Asn Glu Leu Asn
    450             455             460

Leu Gly Arg Arg Glu Glu Tyr Asp Val Leu Asp Lys Arg Arg Gly Arg
465             470             475             480

Asp Pro Glu Met Gly Gly Lys Pro Arg Arg Lys Asn Pro Gln Glu Gly
            485             490             495

Leu Tyr Asn Glu Leu Gln Lys Asp Lys Met Ala Glu Ala Tyr Ser Glu
            500             505             510

Ile Gly Met Lys Gly Glu Arg Arg Arg Gly Lys Gly His Asp Gly Leu
    515             520             525

Tyr Gln Gly Leu Ser Thr Ala Thr Lys Asp Thr Tyr Asp Ala Leu His
    530             535             540
```

309

```
                    Met Gln Ala Leu Pro Pro Arg Glu
                    545                 550
```

<210> 169
<211> 529
<212> PRT
<213> artificial sequence

<220>
<223> polypeptide

<400> 169

```
        Glu Val Lys Leu Gln Glu Ser Gly Pro Gly Leu Val Ala Pro Ser Gln
        1               5               10                  15


        Ser Leu Ser Val Thr Cys Thr Val Ser Gly Val Ser Leu Pro Asp Tyr
                        20                  25                  30


        Gly Val Ser Trp Ile Arg Gln Pro Pro Arg Lys Gly Leu Glu Trp Leu
                    35                  40                  45


        Gly Val Ile Trp Gly Ser Glu Thr Thr Tyr Tyr Asn Ser Ala Leu Lys
                    50                  55                  60


        Ser Arg Leu Thr Ile Ile Lys Asp Asn Ser Lys Ser Gln Val Phe Leu
        65                  70                  75                  80


        Lys Met Asn Ser Leu Gln Thr Asp Asp Thr Ala Ile Tyr Tyr Cys Ala
                        85                  90                  95


        Lys His Tyr Tyr Tyr Gly Gly Ser Tyr Ala Met Asp Tyr Trp Gly Gln
                        100                 105                 110


        Gly Thr Ser Val Thr Val Ser Ser Ser Gly Gly Ser Cys Pro Tyr
                    115                 120                 125


        Ser Asn Pro Ser Leu Cys Ser Gly Gly Ser Asp Ile Gln Met Thr Gln
                    130                 135                 140


        Thr Thr Ser Ser Leu Ser Ala Ser Leu Gly Asp Arg Val Thr Ile Ser
        145                 150                 155                 160


        Cys Arg Ala Ser Gln Asp Ile Ser Lys Tyr Leu Asn Trp Tyr Gln Gln
                        165                 170                 175


        Lys Pro Asp Gly Thr Val Lys Leu Leu Ile Tyr His Thr Ser Arg Leu
                    180                 185                 190
```

```
His Ser Gly Val Pro Ser Arg Phe Ser Gly Ser Gly Ser Gly Thr Asp
    195                 200             205

Tyr Ser Leu Thr Ile Ser Asn Leu Glu Gln Glu Asp Ile Ala Thr Tyr
    210                 215             220

Phe Cys Gln Gln Gly Asn Thr Leu Pro Tyr Thr Phe Gly Gly Gly Thr
225             230             235                 240

Lys Leu Glu Ile Thr Ser Asp Pro Gly Ser Gly Gly Gly Gly Ser Cys
            245             250                 255

Pro Tyr Ser Asn Pro Ser Leu Cys Ser Gly Gly Gly Gly Ser Glu Leu
            260             265                 270

Pro Thr Gln Gly Thr Phe Ser Asn Val Ser Thr Asn Val Ser Pro Ala
            275             280             285

Lys Pro Thr Thr Thr Ala Cys Pro Tyr Ser Asn Pro Ser Leu Cys Ala
    290             295             300

Pro Thr Thr Thr Pro Ala Pro Arg Pro Pro Thr Pro Ala Pro Thr Ile
305             310             315                 320

Ala Ser Gln Pro Leu Ser Leu Arg Pro Glu Ala Cys Arg Pro Ala Ala
            325             330                 335

Gly Gly Ala Val His Thr Arg Gly Leu Asp Phe Ala Cys Asp Ile Tyr
            340             345             350

Ile Trp Ala Pro Leu Ala Gly Thr Cys Gly Val Leu Leu Leu Ser Leu
    355             360             365

Val Ile Thr Leu Tyr Cys Arg Arg Gly Arg Lys Lys Leu Leu Tyr Ile
    370             375             380

Phe Lys Gln Pro Phe Met Arg Pro Val Gln Thr Thr Gln Glu Glu Asp
385             390             395                 400

Gly Cys Ser Cys Arg Phe Pro Glu Glu Glu Glu Gly Gly Cys Glu Leu
            405             410                 415

Arg Val Lys Phe Ser Arg Ser Ala Asp Ala Pro Ala Tyr Gln Gln Gly
            420             425                 430

Gln Asn Gln Leu Tyr Asn Glu Leu Asn Leu Gly Arg Arg Glu Glu Tyr
            435             440                 445
```

```
Asp Val Leu Asp Lys Arg Arg Gly Arg Asp Pro Glu Met Gly Gly Lys
    450             455             460

Pro Arg Arg Lys Asn Pro Gln Glu Gly Leu Tyr Asn Glu Leu Gln Lys
465             470             475             480

Asp Lys Met Ala Glu Ala Tyr Ser Glu Ile Gly Met Lys Gly Glu Arg
            485             490             495

Arg Arg Gly Lys Gly His Asp Gly Leu Tyr Gln Gly Leu Ser Thr Ala
        500             505             510

Thr Lys Asp Thr Tyr Asp Ala Leu His Met Gln Ala Leu Pro Pro Arg
        515             520             525

Glu
```

<210> 170
<211> 124
<212> PRT
<213> artificial sequence

<220>
<223> Humanized F19 VH chain

<400> 170

```
Gln Val Gln Leu Val Gln Ser Gly Ala Glu Val Lys Lys Pro Gly Ala
1               5               10              15

Ser Val Lys Val Ser Cys Lys Thr Ser Arg Tyr Thr Phe Thr Glu Tyr
        20              25              30

Thr Ile His Trp Val Arg Gln Ala Pro Gly Gln Arg Leu Glu Trp Ile
        35              40              45

Gly Gly Ile Asn Pro Asn Asn Gly Ile Pro Asn Tyr Asn Gln Lys Phe
        50              55              60

Lys Gly Arg Val Thr Ile Thr Val Asp Thr Ser Ala Ser Thr Ala Tyr
65              70              75              80

Met Glu Leu Ser Ser Leu Arg Ser Glu Asp Thr Ala Val Tyr Tyr Cys
            85              90              95

Ala Arg Arg Arg Ile Ala Tyr Gly Tyr Asp Glu Gly His Ala Met Asp
        100             105             110
```

```
                    Tyr Trp Gly Gln Gly Thr Leu Val Thr Val Ser Ser
                        115                 120
```

<210> 171
<211> 113
<212> PRT
<213> artificial sequence

<220>
<223> Humanized F19 VL chain

<400> 171

```
        Asp Ile Val Met Thr Gln Ser Pro Asp Ser Leu Ala Val Ser Leu Gly
        1                   5               10                  15


        Glu Arg Ala Thr Ile Asn Cys Lys Ser Ser Gln Ser Leu Leu Tyr Ser
                    20                  25                  30


        Arg Asn Gln Lys Asn Tyr Leu Ala Trp Tyr Gln Gln Lys Pro Gly Gln
                    35                  40                  45


        Pro Pro Lys Leu Leu Ile Phe Trp Ala Ser Thr Arg Glu Ser Gly Val
            50                  55                  60


        Pro Asp Arg Phe Ser Gly Ser Gly Phe Gly Thr Asp Phe Thr Leu Thr
        65                  70                  75                  80


        Ile Ser Ser Leu Gln Ala Glu Asp Val Ala Val Tyr Tyr Cys Gln Gln
                        85                  90                  95


        Tyr Phe Ser Tyr Pro Leu Thr Phe Gly Gln Gly Thr Lys Val Glu Ile
                    100                 105                 110


        Lys
```

<210> 172
<211> 119
<212> PRT
<213> artificial sequence

<220>
<223> FAP5 VH chain

<400> 172

```
        Gln Val Gln Leu Gln Gln Ser Gly Ala Glu Leu Ala Arg Pro Gly Ala
        1                   5               10                  15


        Ser Val Asn Leu Ser Cys Lys Ala Ser Gly Tyr Thr Phe Thr Asn Asn
                    20                  25                  30
```

```
        Gly Ile Asn Trp Leu Lys Gln Arg Thr Gly Gln Gly Leu Glu Trp Ile
                35                  40                  45

        Gly Glu Ile Tyr Pro Arg Ser Thr Asn Thr Leu Tyr Asn Glu Lys Phe
                50                  55                  60

        Lys Gly Lys Ala Thr Leu Thr Ala Asp Arg Ser Ser Asn Thr Ala Tyr
                65                  70                  75                  80

        Met Glu Thr Glu Leu Arg Ser Leu Thr Ser Glu Asp Ser Ala Val Tyr
                            85                  90                  95

        Phe Cys Ala Arg Thr Leu Thr Ala Pro Phe Ala Phe Trp Gly Gln Gly
                    100                 105                 110

        Thr Leu Val Thr Val Ser Ala
                    115
```

<210> 173
<211> 107
<212> PRT
<213> artificial sequence

<220>
<223> FAP5 VL chain

<400> 173

```
        Gln Ile Val Leu Thr Gln Ser Pro Ala Ile Met Ser Ala Ser Pro Gly
        1               5                   10                  15

        Glu Lys Val Thr Met Thr Cys Ser Ala Ser Ser Gly Val Asn Phe Met
                    20                  25                  30

        His Trp Tyr Gln Gln Lys Ser Gly Thr Ser Pro Lys Arg Trp Ile Phe
                35                  40                  45

        Asp Thr Ser Lys Leu Ala Ser Gly Val Pro Ala Arg Phe Ser Gly Ser
                50                  55                  60

        Gly Ser Gly Thr Ser Tyr Ser Leu Thr Ile Ser Ser Met Glu Ala Glu
        65                  70                  75                  80

        Asp Ala Ala Thr Tyr Tyr Cys Gln Gln Trp Ser Phe Asn Pro Pro Thr
                            85                  90                  95

        Phe Gly Gly Gly Thr Lys Leu Glu Ile Lys Arg
                    100                 105
```

<210> 174
<211> 12
<212> PRT
<213> artificial sequence

<220>
<223> mAb-specific epitope

<400> 174

```
        Gly Gln Asn Asp Thr Ser Gln Thr Ser Ser Pro Ser
        1               5               10
```

<210> 175
<211> 112
<212> PRT
<213> artificial sequence

<220>
<223> CD3zeta intracellular signaling domain (ISD)

<400> 175

```
        Arg Val Lys Phe Ser Arg Ser Ala Asp Ala Pro Ala Tyr Gln Gln Gly
        1               5               10                  15

        Gln Asn Gln Leu Tyr Asn Glu Leu Asn Leu Gly Arg Arg Glu Glu Tyr
                    20              25                  30

        Asp Val Leu Asp Lys Arg Arg Gly Arg Asp Pro Glu Met Gly Gly Lys
                    35              40              45

        Pro Arg Arg Lys Asn Pro Gln Glu Gly Leu Tyr Asn Glu Leu Gln Lys
                50              55                  60

        Asp Lys Met Ala Glu Ala Tyr Ser Glu Ile Gly Met Lys Gly Glu Arg
        65              70                  75                  80

        Arg Arg Gly Lys Gly His Asp Gly Leu Tyr Gln Gly Leu Ser Thr Ala
                    85                  90                  95

        Thr Lys Asp Thr Tyr Asp Ala Leu His Met Gln Ala Leu Pro Pro Arg
                    100                 105                 110
```

<210> 176
<211> 42
<212> PRT
<213> artificial sequence

<220>
<223> 41BB intracellular signaling domain (ISD)

<400> 176

```
Lys Arg Gly Arg Lys Lys Leu Leu Tyr Ile Phe Lys Gln Pro Phe Met

1           5               10              15


Arg Pro Val Gln Thr Thr Gln Glu Glu Asp Gly Cys Ser Cys Arg Phe
            20              25              30


Pro Glu Glu Glu Glu Gly Gly Cys Glu Leu
        35              40
```

<210> 177
<211> 42
<212> PRT
<213> artificial sequence

<220>
<223> 41BB-IC (41BB co-stimulatory domain)

<400> 177

```
Lys Arg Gly Arg Lys Lys Leu Leu Tyr Ile Phe Lys Gln Pro Phe Met
1           5               10              15


Arg Pro Val Gln Thr Thr Gln Glu Glu Asp Gly Cys Ser Cys Arg Phe
            20              25              30


Pro Glu Glu Glu Glu Gly Gly Cys Glu Leu
        35              40
```

<210> 178
<211> 21
<212> PRT
<213> artificial sequence

<220>
<223> CD8alpha signal peptide

<400> 178

```
Met Ala Leu Pro Val Thr Ala Leu Leu Leu Pro Leu Ala Leu Leu Leu
1           5               10              15


His Ala Ala Arg Pro
            20
```

<210> 179
<211> 16
<212> PRT
<213> artificial sequence

<220>
<223> FcgammaRIIIalpha hinge

<400> 179

```
        Gly Leu Ala Val Ser Thr Ile Ser Ser Phe Phe Pro Pro Gly Tyr Gln
        1               5               10                  15
```

<210> 180
<211> 45
<212> PRT
<213> artificial sequence

<220>
<223> CD8alpha hinge

<400> 180

```
        Thr Thr Thr Pro Ala Pro Arg Pro Pro Thr Pro Ala Pro Thr Ile Ala
        1               5               10                  15

        Ser Gln Pro Leu Ser Leu Arg Pro Glu Ala Cys Arg Pro Ala Ala Gly
                    20              25                  30

        Gly Ala Val His Thr Arg Gly Leu Asp Phe Ala Cys Asp
                    35              40                  45
```

<210> 181
<211> 231
<212> PRT
<213> artificial sequence

<220>
<223> IgG1 hinge

<400> 181

```
Glu Pro Lys Ser Pro Asp Lys Thr His Thr Cys Pro Pro Cys Pro Ala
1               5                   10              15


Pro Pro Val Ala Gly Pro Ser Val Phe Leu Phe Pro Pro Lys Pro Lys
            20              25              30


Asp Thr Leu Met Ile Ala Arg Thr Pro Glu Val Thr Cys Val Val Val
        35              40              45


Asp Val Ser His Glu Asp Pro Glu Val Lys Phe Asn Trp Tyr Val Asp
    50              55              60


Gly Val Glu Val His Asn Ala Lys Thr Lys Pro Arg Glu Glu Gln Tyr
65              70              75              80


Asn Ser Thr Tyr Arg Val Val Ser Val Leu Thr Val Leu His Gln Asp
            85              90              95


Trp Leu Asn Gly Lys Glu Tyr Lys Cys Lys Val Ser Asn Lys Ala Leu
        100             105             110


Pro Ala Pro Ile Glu Lys Thr Ile Ser Lys Ala Lys Gly Gln Pro Arg


            115             120             125


Glu Pro Gln Val Tyr Thr Leu Pro Pro Ser Arg Asp Glu Leu Thr Lys
    130             135             140


Asn Gln Val Ser Leu Thr Cys Leu Val Lys Gly Phe Tyr Pro Ser Asp
145             150             155             160


Ile Ala Val Glu Trp Glu Ser Asn Gly Gln Pro Glu Asn Asn Tyr Lys
            165             170             175


Thr Thr Pro Pro Val Leu Asp Ser Asp Gly Ser Phe Phe Leu Tyr Ser
            180             185             190


Lys Leu Thr Val Asp Lys Ser Arg Trp Gln Gln Gly Asn Val Phe Ser
        195             200             205


Cys Ser Val Met His Glu Ala Leu His Asn His Tyr Thr Gln Lys Ser
    210             215             220


Leu Ser Leu Ser Pro Gly Lys
225             230
```

<210> 182
<211> 24
<212> PRT
<213> artificial sequence

<220>
<223> CD8alpha transmembrane (TM) domain

<400> 182

```
        Ile Tyr Ile Trp Ala Pro Leu Ala Gly Thr Cys Gly Val Leu Leu Leu
        1               5                   10                  15

        Ser Leu Val Ile Thr Leu Tyr Cys
                        20
```

<210> 183
<211> 52
<212> PRT
<213> artificial sequence

<220>
<223> FcepsilonRI alpha-TM-IC (FcepsilonRI alpha chain transmembrane and intracellular domain)

<400> 183

```
        Phe Phe Ile Pro Leu Leu Val Val Ile Leu Phe Ala Val Asp Thr Gly
        1               5                   10                  15

        Leu Phe Ile Ser Thr Gln Gln Gln Val Thr Phe Leu Leu Lys Ile Lys
                        20                  25                  30

        Arg Thr Arg Lys Gly Phe Arg Leu Leu Asn Pro His Pro Lys Pro Asn
                        35                  40                  45

        Pro Lys Asn Asn
                50
```

<210> 184
<211> 215
<212> PRT
<213> artificial sequence

<220>
<223> FcepsilonRIbeta-(-)ITAM (FcepsilonRI beta chain without ITAM)

<400> 184

```
Met Asp Thr Glu Ser Asn Arg Arg Ala Asn Leu Ala Leu Pro Gln Glu
1               5                   10                  15

Pro Ser Ser Val Pro Ala Phe Glu Val Leu Glu Ile Ser Pro Gln Glu
            20                  25                  30

Val Ser Ser Gly Arg Leu Leu Lys Ser Ala Ser Ser Pro Pro Leu His
            35                  40                  45

Thr Trp Leu Thr Val Leu Lys Lys Glu Gln Glu Phe Leu Gly Val Thr
    50                  55                  60

Gln Ile Leu Thr Ala Met Ile Cys Leu Cys Phe Gly Thr Val Val Cys
65                  70                  75                  80

Ser Val Leu Asp Ile Ser His Ile Glu Gly Asp Ile Phe Ser Ser Phe
                85                  90                  95

Lys Ala Gly Tyr Pro Phe Trp Gly Ala Ile Phe Phe Ser Ile Ser Gly
            100                 105                 110

Met Leu Ser Ile Ile Ser Glu Arg Arg Asn Ala Thr Tyr Leu Val Arg
        115                 120                 125

Gly Ser Leu Gly Ala Asn Thr Ala Ser Ser Ile Ala Gly Gly Thr Gly
    130                 135                 140

Ile Thr Ile Leu Ile Ile Asn Leu Lys Lys Ser Leu Ala Tyr Ile His
145                 150                 155                 160

Ile His Ser Cys Gln Lys Phe Phe Glu Thr Lys Cys Phe Met Ala Ser
            165                 170                 175

Phe Ser Thr Glu Ile Val Val Met Met Leu Phe Leu Thr Ile Leu Gly
        180                 185                 190

Leu Gly Ser Ala Val Ser Leu Thr Ile Cys Gly Ala Gly Glu Glu Leu
    195                 200                 205

Lys Gly Asn Lys Val Pro Glu
    210                 215
```

<210> 185
<211> 41
<212> PRT
<213> artificial sequence

<220>

<223> CD28-IC (CD28 co-stimulatory domain)

<400> 185

```
Arg Ser Lys Arg Ser Arg Gly Gly His Ser Asp Tyr Met Asn Met Thr
1               5                   10                  15


Pro Arg Arg Pro Gly Pro Thr Arg Lys His Tyr Gln Pro Tyr Ala Pro
                20                  25                  30


Pro Arg Asp Phe Ala Ala Tyr Arg Ser
        35                  40
```

**Claims**

1. A polypeptide encoding a chimeric antigen receptor (CAR) comprising at least one extracellular binding domain that comprises a scFv formed by at least a VH chain and a VL chain specific to an antigen, wherein said extracellular binding domain comprises at least one inserted mAb-specific epitope having the amino acid sequence of SEQ ID NO 35.

2. The polypeptide according to claim 1, wherein said mAb-specific epitope is located between the VH and VL chains.

3. The polypeptide according to claim 1 or 2, wherein said VH and VL chains, and mAb specific-epitope are bound together by at least one linker and to the transmembrane domain of said CAR by a hinge.

4. The polypeptide according to any one of claims 1 to 3, wherein the extracellular binding domain further comprises at least one mAb-specific epitope having the amino acid sequence of SEQ ID NO 144.

5. The polypeptide according to any one of claims 1 to 3, wherein the extracellular binding domain comprises the following sequence
$V_1$-$L_1$-$V_2$-$(L)_x$-Epitope1-$(L)_x$-;
$V_1$-$L_1$-$V_2$-$(L)_x$-Epitope1-$(L)_x$-Epitope2-$(L)_x$-;
$V_1$-$L_1$-$V_2$-$(L)_x$-Epitope1-$(L)_x$-Epitope2-$(L)_x$-Epitope3-$(L)_x$-;
$(L)_x$-Epitope1-$(L)_x$-$V_1$-$L_1$-$V_2$;
$(L)_x$-Epitope1-$(L)_x$-Epitope2-$(L)_x$-$V_1$-$L_1$-$V_2$;
Epitope1-$(L)_x$-Epitope2-$(L)_x$-Epitope3-$(L)_x$-$V_1$-$L_1$-$V_2$;
$(L)_x$-Epitope1-$(L)_x$-$V_1$-$L_1$-$V_2$-$(L)_x$-Epitope2-$(L)_x$;
$(L)_x$-Epitope1-$(L)_x$-$V_1$-$L_1$-$V_2$-$(L)_x$-Epitope2-$(L)_x$-Epitope3-$(L)_x$-;
$(L)_x$-Epitope1-$(L)_x$-$V_1$-$L_1$-$V_2$-$(L)_x$-Epitope2-$(L)_x$-Epitope3-$(L)_x$-Epitope4-$(L)_x$-;
$(L)_x$-Epitope1-$(L)_x$-Epitope2-$(L)_x$-$V_1$-$L_1$-$V_2$-$(L)_x$-Epitope3-$(L)_x$-;
$(L)_x$-Epitope1-$(L)_x$-Epitope2-$(L)_x$-$V_1$-$L_1$-$V_2$-$(L)_x$-Epitope3-$(L)_x$-Epitope4-$(L)_x$-;
$V_1$-$(L)_x$-Epitope1-$(L)_x$-$V_2$;
$V_1$-$(L)_x$-Epitope1-$(L)_x$-$V_2$-$(L)_x$-Epitope2-$(L)_x$;
$V_1$-$(L)_x$-Epitope1-$(L)_x$-$V_2$-$(L)_x$-Epitope2-$(L)_x$-Epitope3-$(L)_x$;
$V_1$-$(L)_x$-Epitope1-$(L)_x$-$V_2$-$(L)_x$-Epitope2-$(L)_x$-Epitope3-$(L)_x$-Epitope4-$(L)_x$;
$(L)_x$-Epitope1-$(L)_x$-$V_1$-$(L)_x$-Epitope2-$(L)_x$-$V_2$;
$(L)_x$-Epitope$_1$-$(L)_x$-$V_1$-$(L)_x$-Epitope2-$(L)_x$-$V_2$-$(L)_x$-Epitope3-$(L)_x$; or,
$V_1$-$L_1$-$V_2$-L-Epitope1; $V_1$-$L_1$-$V_2$-L-Epitope1-L; $V_1$-$L_1$-$V_2$-L-Epitope1-L-Epitope2; $V_1$-$L_1$-$V_2$-L-Epitope1-L-Epitope2-L;
$V_1$-$L_1$-$V_2$-L-Epitope1-L-Epitope2-L-Epitope3; $V_1$-$L_2$-$V_2$-L-Epitope1-L-Epitope2-L-Epitope3-L; $V_1$-$L_1$-$V_2$-Epitope1;
$V_1$-$L_1$-$V_2$-Epitope1-L; $V_1$-$L_1$-$V_2$-Epitope1-L-Epitope2; $V_1$-$L_1$-$V_2$-Epitope1-L-Epitope2-L; $V_1$-$L_1$-$V_2$-Epitope1-L-Epitope2-L-Epitope3; $V_1$-$L_1$-$V_2$-Epitope1-L-Epitope2-L-Epitope3-L; Epitope1-$V_1$-$L_1$-$V_2$; Epitope1-L-$V_1$-$L_1$-$V_2$; L-Epitope1-$V_1$-$L_1$-$V_2$; L-Epitope1-L-$V_1$-$L_1$-$V_2$; Epitope1-L-Epitope2-$V_1$-$L_1$-$V_2$; Epitope1-L-Epitope2-L-$V_1$-$L_1$-$V_2$; L-Epitope1-L-Epitope2-$V_1$-$L_1$-$V_2$; L-Epitope1-L-Epitope2-L-$V_1$-$L_1$-$V_2$; Epitope1-L-Epitope2-L-Epitope3-$V_1$-$L_1$-$V_2$; Epitope1-L-Epitope2-L-Epitope3-L-$V_1$-$L_1$-$V_2$; L-Epitope1-L-Epitope2-L-Epitope3-$V_1$-$L_1$-$V_2$; L-Epitope1-L-Epitope2-L-Epitope3-L-$V_1$-$L_1$-$V_2$; $V_1$-L-Epitope$_1$-L-$V_2$; L-Epitope1-L-$V_1$-L-Epitope2-L-$V_2$; $V_1$-L-Epitope1-L-$V_2$-L-Epitope2-L;

V$_1$-L-Epitope1-L-V$_2$-L-Epitope2-L-Epitope3;    V$_1$-L-Epitope1-L-V$_2$-L-Epitope2-Epitope3;    V$_1$-L-Epitope1-L-V$_2$-L-Epitope2-L-Epitope3-Epitope4; L-Epitope1-L-V$_1$-L-Epitope2-L-V$_2$-L-Epitope3-L; Epitope1-L-V$_1$-L-Epitope2-L-V$_2$-L-Epitope3-L; L-Epitope1-L-V$_1$-L-Epitope2-L-V$_2$-L-Epitope3; L-Epitope1-L-V$_1$-L$_1$-V$_2$-L-Epitope2-L; L-Epitope1-L-V$_1$-L$_1$-V$_2$-L-Epitope2-Epitope3; L-Epitope1-L-V$_1$-L$_1$-V$_2$-L-Epitope2-Epitope3, or Epitope1-L-V$_1$-L$_1$-V$_2$-L-Epitope2-L-Epitope3-Epitope4

wherein,

V$_1$ is V$_L$ and V$_2$ is V$_H$ or V$_1$ is V$_H$ and V$_2$ is V$_L$;

L$_1$ is a linker suitable to link the V$_H$ chain to the V$_L$ chain;

L is a linker comprising glycine and serine residues, and each occurrence of L in the extracellular binding domain can be identical or different to other occurrence of L in the same extracellular binding domain, and,

x is 0 or 1 and each occurrence of x is selected independently from the others; and

Epitope 1, Epitope 2 and Epitope 4 are a mAb-specific epitope having the amino acid sequence of SEQ ID NO 35; and Epitope 3 is selected from mAb-specific epitopes having the amino acid sequence of SEQ ID NO 35 or SEQ ID NO 144.

6.  The polypeptide according to claim 5, wherein Epitope 1, Epitope 2 and Epitope 4 are a mAb-specific epitope having the amino acid sequence of SEQ ID NO 35 and Epitope 3 is a mAb-specific epitope having the amino acid sequence of SEQ ID NO 144.

7.  The polypeptide according to claim 5 or 6, wherein L$_1$ is a linker comprising Glycine and/or Serine.

8.  The polypeptide according to claim 7, wherein L$_1$ is a linker comprising the amino acid sequence (Gly-Gly-Gly-Ser)$_n$ or (Gly-Gly-Gly-Gly-Ser)$_n$, where n is 1, 2, 3, 4 or 5 or a linker comprising the amino acid sequence (Gly$_4$Ser)$_4$ or (Gly$_4$Ser)$_3$.

9.  The polypeptide according to any one of claims 5 to 8 wherein L is a linker comprising Glycine and/or Serine.

10. The polypeptide according to claim 9 wherein L is a linker having an amino acid sequence selected from SGG, GGS, SGGS, SSGGS, GGGG, SGGGG, GGGGS, SGGGGS, GGGGGS, SGGGGGS, SGGGGG, GSGGGGS, GGGGGGGS, SGGGGGGG, SGGGGGGGS, or SGGGGSGGGGS.

11. The polypeptide according to anyone of claim 1 to 10, wherein said VH and VL chains have an antigenic target sequence of over 80% identity, preferably over 90%, and more preferably over 95% with SEQ ID NO 43 (CD19 antigen), SEQ ID NO 44 (CD38 antigen), SEQ ID NO 45 (CD123 antigen), SEQ ID NO 46 (CS1 antigen), SEQ ID NO 47 (BCMA antigen), SEQ ID NO 48 (FLT-3 antigen), SEQ ID NO 49 (CD33 antigen), SEQ ID NO 50 (CD70 antigen), SEQ ID NO 51 (EGFR-3v antigen) and SEQ ID NO 52 (WT1 antigen).

12. The polypeptide according to any one of claims 1 to 10, wherein said antigen is selected from ErbB2 (HER2/neu), carcinoembryonic antigen (CEA), epithelial cell adhesion molecule (EpCAM), epidermal growth factor receptor (EGFR), EGFR variant III (EGFRvIII), CD19, CD20, CD30, CD40, disialoganglioside GD2, GD3, C-type lectin-like molecule-1 (CLL-1), ductal-epithelial mucine, gp36, TAG-72, glycosphingolipids, glioma-associated antigen, β-human chorionic gonadotropin, alphafetoprotein (AFP), lectin-reactive AFP, thyroglobulin, RAGE-1, MN-CA IX, human telomerase reverse transcriptase, RU1, RU2 (AS), intestinal carboxyl esterase, mut hsp70-2, M-CSF, prostase, prostase specific antigen (PSA), PAP, NY-ESO-1, LAGA-1a, p53, prostein, PSMA, survivin and telomerase, prostate-carcinoma tumor antigen-1 (PCTA-1), MAGE, ELF2M, neutrophil elastase, ephrin B2, CD22, insulin growth factor (IGF1)-I, IGF-II, IGFI receptor, mesothelin, a major histocompatibility complex (MHC) molecule presenting a tumor-specific peptide epitope, 5T4, ROR1, Nkp30, NKG2D, tumor stromal antigens, the extra domain A (EDA) and extra domain B (EDB) of fibronectin and the A1 domain of tenascin-C (TnC A1) and fibroblast associated protein (fap), LRP6, melamona-associated Chondroitin Sulfate Proteoglycan (MCSP), CD38/CS1, MART1, WT1, MUC1, LMP2, Idiotype, NY-ESO-1, Ras mutant, gp100, proteinase 3, bcr-abl, tyrosinase, hTERT, EphA2, ML-TAP, ERG, NA17, PAX3, ALK, Androgen receptor ; a lineage-specific or tissue specific antigen such as CD3, CD4, CD8, CD24, CD25, CD33, CD34, CD70, CD79, CD116, CD117, CD135, CD123, CD133, CD138, CTLA-4, B7-1 (CD80), B7-2 (CD86), endoglin, a major histocompatibility complex (MHC) molecule, BCMA (CD269, TNFRSF 17) or FLT-3.

13. The polypeptide according to any one of claims 1 to 10, wherein VH and VL are selected from a VH of SEQ ID NO 65 and a VL of SEQ ID NO 66;- a VH of SEQ ID NO 67 and a VL of SEQ ID NO 68; a VH of SEQ ID NO 69 and a VL of SEQ ID NO 70; a VH of SEQ ID NO 71 and a VL of SEQ ID NO 72; a VH of SEQ ID NO 77 and a VL of SEQ ID NO 78; a VH of SEQ ID NO 79 and a VL of SEQ ID NO 80; a VH of SEQ ID NO 81 and a VL of SEQ ID NO 82; a VH of SEQ ID NO 83 and a VL of SEQ ID NO 84; a VH of SEQ ID NO 85 and a VL of SEQ ID NO 86; a VH of SEQ ID NO 87 and a VL of SEQ ID NO 88; a VH of SEQ ID NO 89 and a VL of SEQ ID NO 90; a VH of SEQ ID NO 91 and a VL of SEQ ID NO 92; a VH of SEQ ID NO 93 and a VL of SEQ ID NO 94; a VH of SEQ ID NO 95 and a VL of SEQ ID NO 96; a VH of SEQ ID NO 97 and a VL of SEQ ID NO 98; a VH of SEQ ID NO 99 and a VL of SEQ ID NO 100; a VH of SEQ ID NO 101 and a VL of SEQ ID NO 102; a VH of SEQ ID NO 103 and a VL of SEQ ID NO 104; a VH of SEQ ID NO 105 and a VL of SEQ ID NO 106; a VH of SEQ ID NO 107 and a VL of SEQ ID NO 108; a VH of SEQ ID NO 109 and a VL of SEQ ID NO 110; a VH of SEQ ID NO 111 and a VL of SEQ ID NO 112; a VH of SEQ ID NO 113 and a VL of SEQ ID NO 114; a VH of SEQ ID NO 115 and a VL of SEQ ID NO 116; a VH of SEQ ID NO 117 and a VL of SEQ ID NO 118; a VH of SEQ ID NO 119 and a VL of SEQ ID NO 120; a VH of SEQ ID NO 121 and a VL of SEQ ID NO 122; or, a VH of SEQ ID NO 123 and a VL of SEQ ID NO 124, a VH of SEQ ID NO 170 and a VL of SEQ ID NO 171; a VH of SEQ ID NO 172 and a VL of SEQ ID NO 173; or, a VH of SEQ ID NO 186 and a VL of SEQ ID NO 187.

14. The polypeptide according to any one of claims 1 to 13, wherein the polypeptide comprises one extracellular binding domain, wherein said extracellular binding domain further comprises a hinge, and said polypeptide further comprises

    - a transmembrane domain, and,
    - an intracellular domain.

15. The polypeptide according to claim 14, wherein the hinge comprises an IgG1 hinge, an IgG4 hinge, a CD8alpha hinge or a FcyRII1 alpha hinge.

16. The polypeptide according to claim 14 or 15, wherein the transmembrane domain comprises the transmembrane region(s) of the alpha, beta or zeta chain of the T-cell receptor, PD-1, 4-1BB, OX40, ICOS, CTLA-4, LAG3, 2B4, BTLA4, TIM-3, TIGIT, SIRPA, CD28, CD3 epsilon, CD45, CD4, CD5, CD8, CD9, CD16, CD22, CD33, CD37, CD64, CD80, CD86, CD134, CD137 or CD154.

17. The polypeptide according to any one of claims 14 to 16, wherein the transmembrane domain comprises the transmembrane region(s) of CD8 alpha.

18. The polypeptide according to any one of claims 14 to 17, wherein the intracellular domain comprises a CD3zeta signalling domain.

19. The polypeptide according to any one of claims 14 to 18, wherein the intracellular domain comprises a 4-1BB domain.

20. A polypeptide according to any one of claim 1-19, wherein the CAR is a single-chain CAR.

21. A polypeptide according to claim 1, wherein the said polypeptide shares over 80% identity, over 90%, over 95% with or is identical to SEQ ID NO 1 to 10, SEQ ID NO 126 to 141 or SEQ ID no 145 to 149 or SEQ ID NO 152 to 169.

22. A polypeptide according to any one of claim 1-19, wherein the CAR is a multi-chain CAR.

23. A polynucleotide encoding a polypeptide according to any one of claims 1 to 22.

24. An expression vector comprising a polynucleotide of claim 23.

25. An engineered immune cell expressing at its cell surface a polypeptide according to any one of claims 1 to 24.

26. The engineered immune cell according to claim 25, wherein said cell is derived from inflammatory T-lymphocytes, cytotoxic T-lymphocytes, regulatory T-lymphocytes or helper T-lymphocytes.

27. The engineered immune cell according to claim 25 or 26 for use as a medicament.

28. The engineered immune cell according to claim 25 or 26 for use in the treatment of cancer in a patient in need thereof.

29. An ex vivo method for engineering an immune cell of any one of claim 25-28, comprising:

(a) Introducing into an immune cell that has been provided at least one polynucleotide encoding the chimeric antigen receptor according to any one of claim 1-22; and

(b) Expressing said polynucleotide into said cell.

**30.** The method for engineering an immune cell of claim 29, wherein immune cell is a T-cell.

**31.** An epitope-specific monoclonal antibody (mAb) which specifically recognizes the mAb-specific epitope having the amino acid sequence of SEQ ID NO 35 for use in a method for *in vivo* depleting an engineered immune cell expressing at its cell surface a CAR polypeptide comprising at least one mAb-specific epitope according to any one of claims 1 to 22, in a patient, said method comprising contacting said engineered immune cell with said epitope-specific mAb.

**32.** The epitope-specific mAb for use according to claim 31 wherein the epitope-specific mAb is rituximab.

**33.** The epitope-specific mAb for use according to claim 31 or 32 wherein the epitope-specific mAb is conjugated with a molecule able to activate the complement system or wherein a cytotoxic drug is coupled to the epitope-specific mAb.

**34.** A bi-specific mAb (BsAb) able to bind both an mAb-specific epitope having the amino acid sequence of SEQ ID NO 35 borne on an engineered immune cell expressing at its cell surface a CAR polypeptide according to any one of claims 1 to 22, and a surface antigen borne on an effector cell for use in a method for *in vivo* depleting said engineered immune cell in a patient, said method comprising contacting said engineered immune cell with said bi-specific mAb (BsAb).

**Patentansprüche**

**1.** Polypeptid, das für einen chimären Antigenrezeptor (CAR) kodiert, umfassend mindestens eine extrazelluläre Bindungsdomäne, die ein scFv umfasst, gebildet durch mindestens eine VH-Kette und eine VL-Kette spezifisch für ein Antigen, wobei die extrazelluläre Bindungsdomäne mindestens ein eingefügtes mAb-spezifisches Epitop mit der Aminosäuresequenz gemäß SEQ ID NO 35 umfasst.

**2.** Polypeptid nach Anspruch 1, wobei das mAb-spezifische Epitop zwischen der VH-Kette und der VL-Kette angeordnet ist.

**3.** Polypeptid nach Anspruch 1 oder 2, wobei die VH- und VL-Kette und das mAb-spezifische Epitop durch mindestens einen Linker zusammengebunden sind und durch ein Gelenk mit der Transmembrandomäne des chimären Antigenrezeptors CAR verknüpft sind.

**4.** Polypeptid nach einem der Ansprüche 1 bis 3, wobei die extrazelluläre Bindungsdomäne ferner mindestens ein mAb-spezifisches Epitop mit der Aminosäuresequenz gemäß SEQ ID NO 144 umfasst.

**5.** Polypeptid nach einem der Ansprüche 1 bis 3, wobei die extrazelluläre Bindungsdomäne die folgende Sequenz umfasst

$V_1$-$L_1$-$V_2$-$(L)_x$-Epitop1-$(L)_x$-,
$V_1$-$L_1$-$V_2$-$(L)_x$-Epitop1-$(L)_x$-Epitop2-$(L)_x$-;
$V_1$-$L_1$-$V_2$-$(L)_x$-Epitop1-$(L)_x$-Epitop2-$(L)_x$-Epitop3-$(L)_x$-,
$(L)_x$-Epitop1-$(L)_x$-$V_1$-$L_1$-$V_2$;
$(L)_x$-Epitop1-$(L)_x$-Epitop2-$(L)_x$-$V_1$-$L_1$-$V_2$;
Epitop1-$(L)_x$-Epitop2-$(L)_x$-Epitop3-$(L)_x$-$V_1$-$L_1$-$V_2$;
$(L)_x$-Epitop1-$(L)_x$-$V_1$-$L_1$-$V_2$-$(L)_x$-Epitop2-$(L)_x$;
$(L)_x$-Epitop1-$(L)_x$-$V_1$-$L_1$-$V_2$-$(L)_x$-Epitop2-$(L)_x$-Epitop3-$(L)_x$-,
$(L)_x$-Epitop1-$(L)_x$-$V_1$-$L_1$-$V_2$-$(L)_x$-Epitop2-$(L)_x$-Epitop3-$(L)_x$-Epitop4-$(L)_x$-,
$(L)_x$-Epitop1-$(L)_x$-Epitop2-$(L)_x$-$V_1$-$L_1$-$V_2$-$(L)_x$-Epitop3-$(L)_x$-;
$(L)_x$-Epitop1-$(L)_x$-Epitop2-$(L)_x$-$V_1$-$L_1$-$V_2$-$(L)_x$-Epitop3-$(L)_x$-Epitop4-$(L)_x$-,
$V_1$-$(L)_x$-Epitop1-$(L)_x$-$V_2$;
$V_1$-$(L)_x$-Epitop1-$(L)_x$-$V_2$-$(L)_x$-Epitop2-$(L)_x$;
$V_1$-$(L)_x$-Epitop1-$(L)_x$-$V_2$-$(L)_x$-Epitop2-$(L)_x$-Epitop3-$(L)_x$;
$V_1$-$(L)_x$-Epitop1-$(L)_x$-$V_2$-$(L)_x$-Epitop2-$(L)_x$-Epitop3-$(L)_x$-Epitop4-$(L)_x$;
$(L)_x$-Epitop1-$(L)_x$-$V_1$-$(L)_x$-Epitop2-$(L)_x$-$V_2$;

$(L)_x$-Epitop1-$(L)_x$-$V_1$-$(L)_x$-Epitop2-$(L)_x$-$V_2$-$(L)_x$-Epitop3-$(L)_x$; oder

$V_1$-$L_1$-$V_2$-$L$-Epitop1;  $V_1$-$L_1$-$V_2$-$L$-Epitop1-$L$;  $V_1$-$L_1$-$V_2$-$L$-Epitop1-$L$-Epitop2;  $V_1$-$L_1$-$V_2$-$L$-Epitop1-$L$-Epitop2-$L$; $V_1$-$L_1$-$V_2$-$L$-Epitop1-$L$-Epitop2-$L$-Epitop3;  $V_1$-$L_1$-$V_2$-$L$-Epitop1-$L$-Epitop2-$L$-Epitop3-$L$;  $V_1$-$L_1$-$V_2$-Epitop1; $V_1$-$L_1$-$V_2$-Epitop1-$L$;  $V_1$-$L_1$-$V_2$-Epitop1-$L$-Epitop2;  $V_1$-$L_1$-$V_2$-Epitop1-$L$-Epitop2-$L$;  $V_1$-$L_1$-$V_2$-Epitop1-$L$-Epitop2-Epitop3; $V_1$-$L_1$-$V_2$-Epitop1-$L$-Epitop2-$L$-Epitop3-$L$; Epitop1-$V_1$-$L_1$-$V_2$; Epitop1-$L$-$V_1$-$L_1$-$V_2$; $L$-Epitop1-$V_1$-$L_1$-$V_2$; $L$-Epitop1-$L$-$V_1$-$L_1$-$V_2$; Epitop1-$L$-Epitop2-$V_1$-$L_1$-$V_2$; Epitop1-$L$-Epitop2-$L$-$V_1$-$L_1$-$V_2$; $L$-Epitop1-$L$-Epitop2-$V_1$-$L_1$-$V_2$; $L$-Epitop1-$L$-Epitop2-$L$-$V_1$-$L_1$-$V_2$; Epitop1-$L$-Epitop2-$L$-Epitop3-$V_1$-$L_1$-$V_2$; Epitop1-$L$-Epitop2-$L$-Epitop3-$L$-$V_1$-$L_1$-$V_2$; Epitop1-$L$-Epitop2-$L$-Epitop3-$V_1$-$L_1$-$V_2$; $L$-Epitop1-$L$-Epitop2-$L$-Epitop3-$L$-$V_1$-$L_1$-$V_2$; $V_1$-$L$-Epitop1-$L$-$V_2$; $L$-Epitop1-$L$-$V_1$-$L$-Epitop2-$L$-$V_2$;  $V_1$-$L$-Epitop1-$L$-$V_2$-$L$-Epitop2-$L$;  $V_1$-$L$-Epitop1-$L$-$V_2$-$L$-Epitop2-Epitop3;  $V_1$-$L$-Epitop1-$L$-$V_2$-$L$-Epitop2-Epitop3; $V_1$-$L$-Epitop1-$L$-$V_2$-$L$-Epitop2-Epitop3-Epitop4; $L$-Epitop1-$L$-$V_1$-$L$-Epitop2-$L$-$V_2$-$L$-Epitop3-$L$;  Epitop1-$L$-$V_1$-$L$-Epitop2-$L$-$V_2$-$L$-Epitop3-$L$;  $L$-Epitop1-$L$-$V_1$-$L$-Epitop2-$L$-$V_2$-$L$-Epitop3;  $L$-Epitop1-$L$-$V_1$-$L_1$-$V_2$-$L$-Epitop2-$L$;  $L$-Epitop1-$L$-$V_1$-$L_1$-$V_2$-$L$-Epitop2-$L$-Epitop3;  $L$-Epitop1-$L$-$V_1$-$L_1$-$V_2$-$L$-Epitop2-Epitop3 oder  Epitop1-$L$-$V_1$-$L_1$-$V_2$-$L$-Epitop2-$L$-Epitop3-Epitop4

wobei,

$V_1$ $V_L$ ist und $V_2$ $V_H$ ist oder $V_1$ $V_H$ ist und $V_2$ $V_L$ ist;

$L_1$ ein Linker ist, der zum Verbinden der $V_H$-Kette mit der $V_L$-Kette geeignet ist; $L$ ein Linker, umfassend Glycin- und Serinreste, ist und jedes Vorkommen von $L$ in der extrazellulären Bindungsdomäne identisch oder verschieden von anderem Vorkommen von $L$ in der gleichen extrazellulären Bindungsdomäne sein kann und

x 0 oder 1 ist und jedes Vorkommen von x unabhängig von den anderen ausgewählt ist; und

Epitop 1, Epitop 2 und Epitop 4 ein mAb-spezifisches Epitop mit der Aminosäuresequenz gemäß SEQ ID NO 35 sind; und Epitop 3 ausgewählt ist aus mAb-spezifischen Epitopen mit der Aminosäuresequenz gemäß SEQ ID NO 35 oder SEQ ID NO 144.

6. Polypeptid nach Anspruch 5, wobei Epitop 1, Epitop 2 und Epitop 4 ein mAb-spezifisches Epitop mit der Aminosäuresequenz gemäß SEQ ID NO 35 sind, und Epitop 3 ein mAb-spezifisches Epitop gemäß der Aminosäuresequenz mit SEQ ID NO 144 ist.

7. Polypeptid nach Anspruch 5 oder 6, wobei $L_1$ ein Linker umfassend Glycin und/oder Serin ist.

8. Polypeptid nach Anspruch 7, wobei $L_1$ ein Linker umfassend die Aminosäuresequenz $(Gly$-$Gly$-$Gly$-$Ser)_n$ oder $(Gly$-$Gly$-$Gly$-$Gly$-$Ser)_n$ ist, wobei n 1, 2, 3, 4 oder 5 oder ein Linker umfassend die Aminosäuresequenz $(Gly_4Ser)_4$ oder $(Gly_4Ser)_3$ ist.

9. Polypeptid nach einem der Ansprüche 5 bis 8, wobei $L$ ein Linker umfassend Glycin und/oder Serin ist.

10. Polypeptid nach Anspruch 9, wobei $L$ ein Linker mit einer Aminosäuresequenz ausgewählt aus SGG, GGS, SGGS, SSGGS, GGGG, SGGGG, GGGGS, SGGGGS, GGGGGS, SGGGGGS, SGGGGG, GSGGGGS, GGGGGGGS, SGGGGGGGG, SGGGGGGGS oder SGGGGSGGGGS ist.

11. Polypeptid nach einem der Ansprüche 1 bis 10, wobei die VH- und VL-Kette eine Antigenzielsequenz mit mehr als 80% Identität, vorzugsweise 90%, und eher bevorzugt mehr als 95% mit SEQ ID NO 43 (CD19-Antigen), SEQ ID NO 44 (CD38-Antigen), SEQ ID NO 45 (CD123-Antigen), SEQ ID NO 46 (CS1-Antigen), SEQ ID NO 47 (BCMA-Antigen), SEQ ID NO 48 (FLT-3-Antigen), SEQ ID NO 49 (CD33-Antigen), SEQ ID NO 50 (CD70-Antigen), SEQ ID NO 51 (EGFR-3v-Antigen) und SEQ ID NO 52 (WT1-Antigen) aufweisen.

12. Polypeptid nach einem der Ansprüche 1 bis 10, wobei das Antigen ausgewählt ist aus ErbB2 (HER2/neu), karzinoembryonalem Antigen (CEA), Epithelzellenhaftungsmolekül (EpCAM), epidermalem Wachstumsfaktorrezeptor (EGFR), EGFR-Variant III (EGFRvIII), CD19, CD20, CD30, CD40, Disialogangliosid GD2, GD3, C-Typ Lektin-artigem Molekül-1 (CLL-1), Ductal-Epithelmuzin, gp36, TAG-72, Glycosphingolipiden, Gliom-assoziiertem Antigen, β-humanem Choriongonadotropin, Alphafetoprotein (AFP), Lektin-reaktivem AFP, Thyroglobulin, RAGE-1, MN-CA IX, humaner Telomeraseumkehrtranskriptase, RU1, RU2 (AS), intestinaler Carboxylesterase, Mut hsp70-2, M-CSF, Prostase, prostasepezifischem Antigen (PSA), PAP, NY-ESO-1, LAGA-1a, p53, Prostein, PSMA, Survivin und Telomerase, Prostatakarzinomtumor-Antigen-1 (PCTA-1), MAGE, ELF2M, neutrophiler Elastase, Ephrin B2, CD22, Insulinwachstumsfaktor (IGF1)-I, IGF-II, IGFI-Rezeptor, Mesothelin, einem Haupthistokompatibilitätskomplex (MHC)-Molekül, das ein Tumor-spezifisches Peptidepitop darstellt, 5T4, ROR1, Nkp30, NKG2D, Tumorstromaantigenen, der Extradomäne A (EDA) und Extradomäne B (EDB) von Fibronektin und der A1-Domäne von Tenascin-C (TnC A1) und Fibroblast-assoziiertem Protein (fap), LRP6, Melamon-assoziiertem Chondroitin-Sulfat-Proteoglycan (MCSP), CD38/CS1, MART1, WT1, MUC1, LMP2, Idiotyp, NY-ESO-1, Ras-Mutant, gp100, Proteinase 3, bcr-abl, Tyrosinase, hTERT, EphA2, ML-TAP, ERG, NA17, PAX3, ALK, Androgen-Rezeptor; einem Linie-spezifischem oder gewebespezifischem Antigen wie CD3, CD4, CD8, CD24, CD25, CD33, CD34, CD70, CD79, CD116, CD117, CD135, CD123, CD133, CD138, CTLA-4, B7-1 (CD80), B7-2 (CD86), Endoglin, einem Haupthistokompatibilitätskomplex (MHC)-Molekül, BCMA (CD269, TNFRSF 17) oder FLT-3.

13. Polypeptid nach einem der Ansprüche 1 bis 10, wobei VH und VL ausgewählt sind aus einer VH mit SEQ ID NO 65 und einer VL mit SEQ ID NO 66; einer VH mit SEQ ID NO 67 und einer VL mit SEQ ID NO 68; einer VH mit SEQ ID NO 69 und einer VL mit SEQ ID NO 70; einer VH mit SEQ ID NO 71 und einer VL mit SEQ ID NO 72; einer VH mit SEQ ID NO 77 und einer VL mit SEQ ID NO 78; einer VH mit SEQ ID NO 79 und einer VL mit SEQ ID NO 80; einer VH mit SEQ ID NO 81 und einer VL mit SEQ ID NO 82; einer VH mit SEQ ID NO 83 und einer VL mit SEQ ID NO 84; einer VH mit SEQ ID NO 85 und einer VL mit SEQ ID NO 86; einer VH mit SEQ ID NO 87 und einer VL mit SEQ ID NO 88; einer VH mit SEQ ID NO 89 und einer VL mit SEQ ID NO 90; einer VH mit SEQ ID NO 91 und einer VL mit SEQ ID NO 92; einer VH mit SEQ ID NO 93 und einer VL mit SEQ ID NO 94; einer VH mit SEQ ID NO 95 und einer VL mit SEQ ID NO 96; einer VH mit SEQ ID NO 97 und einer VL mit SEQ ID NO 98; einer VH mit SEQ ID NO 99 und einer VL mit SEQ ID NO 100; einer VH mit SEQ ID NO 101 und einer VL mit SEQ ID NO 102; einer VH mit SEQ ID NO 103 und einer VL mit SEQ ID NO 104; einer VH mit SEQ ID NO 105 und einer VL mit SEQ ID NO 106; einer VH mit SEQ ID NO 107 und einer VL mit SEQ ID NO 108; einer VH mit SEQ ID NO 109 und einer VL mit SEQ ID NO 110; einer VH mit SEQ ID NO 111 und einer VL mit SEQ ID NO 112; einer VH mit SEQ ID NO 113 und einer VL mit SEQ ID NO 114; einer VH mit SEQ ID NO 115 und einer VL mit SEQ ID NO 116; einer VH mit SEQ ID NO 117 und einer VL mit SEQ ID NO 118; einer VH mit SEQ ID NO 119 und einer VL mit SEQ ID NO 120; einer VH mit SEQ ID NO 121 und einer VL mit SEQ ID NO 122; oder einer VH mit SEQ ID NO 123 und einer VL mit SEQ ID NO 124, einer VH mit SEQ ID NO 170 und einer VL mit SEQ ID NO 171; einer VH mit SEQ ID NO 172 und einer VL mit SEQ ID NO 173; oder einer VH mit SEQ ID NO 186 und einer VL mit SEQ ID NO 187.

14. Polypeptid nach einem der Ansprüche 1 bis 13, wobei das Polypeptid eine extrazelluläre Bindungsdomäne umfasst, wobei die extrazelluläre Bindungsdomäne ferner ein Gelenk umfasst, und das Polypeptid ferner Folgendes umfasst

   - eine Transmembrandomäne und
   - eine intrazelluläre Domäne.

15. Polypeptid nach Anspruch 14, wobei das Gelenk ein IgG1-Gelenk, ein IgG4-Gelenk, ein CD8alpha-Gelenk oder ein FcγRIII-Alpha-Gelenk umfasst.

16. Polypeptid nach Anspruch 14 oder 15, wobei die Transmembrandomäne die Transmembranregion(en) der Alpha-, Beta- oder Zeta-Kette des T-Zellenrezeptors, PD-1, 4-1BB, OX40, ICOS, CTLA-4, LAG3, 2B4, BTLA4, TIM-3, TIGIT, SIRPA, CD28, CD3-Epsilon, CD45, CD4, CD5, CD8, CD9, CD16, CD22, CD33, CD37, CD64, CD80, CD86, CD134, CD137 oder CD154 umfasst.

17. Polypeptid nach einem der Ansprüche 14 bis 16, wobei die Transmembrandomäne die Transmembranregion(en) von CD8-Alpha umfasst.

18. Polypeptid nach einem der Ansprüche 14 bis 17, wobei die intrazelluläre Domäne eine CD3zeta-Signalisierungdomäne umfasst.

19. Polypeptid nach einem der Ansprüche 14 bis 18, wobei die intrazelluläre Domäne eine 4-1BB-Domäne umfasst.

20. Polypeptid nach einem der Ansprüche 1-19, wobei der CAR ein Einzelketten-CAR ist.

21. Polypeptid nach Anspruch 1, wobei das Polypeptid mehr als 80%, mehr als 90%, mehr als 95% Identität mit SEQ ID NO 1 bis 10, SEQ ID NO 126 bis 141 oder SEQ ID NO 145 bis 149 oder SEQ ID NO 152 bis 169 aufweist oder dazu identisch ist.

22. Polypeptid nach einem der Ansprüche 1-19, wobei der CAR ein Multiketten-CAR ist.

23. Polynukleotid, das für ein Polypeptid nach einem der Ansprüche 1 bis 22 kodiert.

24. Expressionsvektor, der ein Polynukleotid nach Anspruch 23 umfasst.

**25.** Geänderte Immunzelle, die an der Zelloberfläche ein Polypeptid nach einem der Ansprüche 1 bis 24 exprimiert.

**26.** Geänderte Immunzelle nach Anspruch 25, wobei die Zelle von inflammatorischen T-Lymphozyten, zytotoxischen T-Lymphozyten, regulatorischen T-Lymphozyten oder Helfer-T-Lymphozyten abgeleitet ist.

**27.** Geänderte Immunzelle nach Anspruch 25 oder 26 zur Verwendung als ein Arzneimittel.

**28.** Geänderte Immunzelle nach Anspruch 25 oder 26 zur Verwendung bei der Behandlung von Krebs bei einem danach bedürftigen Patienten.

**29.** Ex-vivo-Verfahren zum Ändern einer Immunzelle nach einem der Ansprüche 25-28, umfassend:

(a) Einführen von mindestens einem Polynukleotid, das für den chimären Antigenrezeptor nach einem der Ansprüche 1-22 kodiert, in eine bereitgestellte Immunzelle; und
(b) Exprimieren des Polynukleotids in die Zelle.

**30.** Verfahren zum Ändern einer Immunzelle nach Anspruch 29, wobei die Immunzelle eine T-Zelle ist.

**31.** Epitop-spezifischer monoklonaler Antikörper (mAb), der das mAb-spezifische Epitop mit der Aminosäuresequenz gemäß SEQ ID NO 35 spezifisch erkennt, zur Verwendung in einem Verfahren zur In vivo-Depletion einer geänderten Immunzelle, die an der Zelloberfläche ein CAR-Polypeptid, das mindestens ein mAb-spezifisches Epitop umfasst, nach einem der Ansprüche 1 bis 22 exprimiert, in einem Patienten, wobei das Verfahren das Inkontaktbringen der geänderten Immunzelle mit dem Epitop-spezifischen mAb umfasst.

**32.** Epitop-spezifischer mAb zur Verwendung nach Anspruch 31, wobei der Epitop-spezifische mAb Rituximab ist.

**33.** Epitop-spezifischer mAb zur Verwendung nach Anspruch 31 oder 32, wobei der Epitop-spezifische mAb mit einem Molekül konjugiert ist, das das Komplementsystem aktivieren kann, oder wobei ein zytotoxisches Arzneimittel mit dem Epitop-spezifischen mAb verbunden ist.

**34.** Bispezifischer mAb (BsAb), der sowohl ein mAb-spezifisches Epitop mit der Aminosäuresequenz gemäß SEQ ID NO 35, getragen auf einer geänderten Immunzelle, die an der Zelloberfläche ein CAR-Polypeptid nach einem der Ansprüche 1 bis 22 exprimiert, als auch ein Oberflächenantigen, getragen auf einer Effektorzelle, binden kann, zur Verwendung in einem Verfahren zur *In vivo*-Depletion der geänderten Immunzelle in einem Patienten, wobei das Verfahren das Inkontaktbringen der geänderten Immunzelle mit dem bispezifischen mAb (BsAb) umfasst.

## Revendications

**1.** Polypeptide codant pour un récepteur d'antigène chimérique (CAR) comprenant au moins un domaine de liaison extracellulaire qui comprend un scFv formé par au moins une chaîne VH et une chaîne VL spécifique d'un antigène, dans lequel ledit domaine de liaison extracellulaire comprend au moins un épitope spécifique de mAb inséré ayant la séquence d'acide aminé de SEQ ID NO 35.

**2.** Polypeptide selon la revendication 1, dans lequel ledit épitope spécifique de mAb est situé entre les chaînes VH et VL.

**3.** Polypeptide selon la revendication 1 ou 2, dans lequel lesdites chaînes VH et VL, et l'épitope spécifique de mAb sont liés ensemble par au moins un lieur et au domaine transmembranaire dudit CAR par une charnière.

**4.** Polypeptide selon l'une quelconque des revendications 1 à 3, dans lequel le domaine de liaison extracellulaire inclut au moins un épitope spécifique de mAb ayant une séquence d'acide aminé de SEQ ID NO 144.

**5.** Polypeptide selon l'une quelconque des revendications 1 à 3, dans lequel le domaine de liaison extracellulaire comprend la séquence suivante

$V_1$-$L_1$-$V_2$-$(L)_x$-Epitope1-$(L)_x$-;
$V_1$-$L_1$-$V_2$-$(L)_x$-Epitope1-$(L)_x$-Epitope2-$(L)_x$;
$V_1$-$L_1$-$V_2$-$(L)_x$-Epitope1-$(L)_x$-Epitope2-$(L)_x$-Epitope3-$(L)_x$-,
$(L)_x$-Epitope1-$(L)_x$-$V_1$-$L_1$-$V_2$;

$(L)_x$-Epitope1-$(L)_x$-Epitope2-$(L)_x$-$V_1$-$L_1$-$V_2$;
Epitope1-$(L)_x$-Epitope2-$(L)_x$-Epitope3-$(L)_x$-$V_1$-$L_1$-$V_2$;
$(L)_x$-Epitope1-$(L)_x$-$V_1$-$L_1$-$V_2$-$(L)_x$-Epitope2-$(L)_x$;
$(L)_x$-Epitope1-$(L)_x$-$V_1$-$L_1$-$V_2$-$(L)_x$-Epitope2-$(L)_x$-Epitope3-$(L)_x$-,
$(L)_x$-Epitope1-$(L)_x$-$V_1$-$L_1$-$V_2$-$(L)_x$-Epitope2-$(L)_x$-Epitope3-$(L)_x$-Epitope4-$(L)_x$;
$(L)_x$-Epitope1-$(L)_x$-Epitope2-$(L)_x$-$V_1$-$L_1$-$V_2$-$(L)_x$-Epitope3-$(L)_x$-,
$(L)_x$-Epitope1-$(L)_x$-Epitope2-$(L)_x$-$V_1$-$L_1$-$V_2$-$(L)_x$-Epitope3-$(L)_x$-Epitope4-$(L)_x$;
$V_1$-$(L)_x$-Epitope1-$(L)_x$-$V_2$;
$V_1$-$(L)_x$-Epitope1-$(L)_x$-$V_2$-$(L)_x$-Epitope2-$(L)_x$;
$V_1$-$(L)_x$-Epitope1-$(L)_x$-$V_2$-$(L)_x$-Epitope2-$(L)_x$-Epitope3-$(L)_x$;
$V_1$-$(L)_x$-Epitope1-$(L)_x$-$V_2$-$(L)_x$-Epitope2-$(L)_x$-Epitope3-$(L)_x$-Epitope4-$(L)_x$;
$(L)_x$-Epitope1-$(L)_x$-$V_1$-$(L)_x$-Epitope2-$(L)_x$-$V_2$;
$(L)_x$-Epitope1-$(L)_x$-$V_1$-$(L)_x$-Epitope2-$(L)_x$-$V_2$-$(L)_x$-Epitope3-$(L)_x$; ou,
$V_1$-$L_1$-$V_2$-L-Epitope1; $V_1$-$L_1$-$V_2$-L-Epitope1-L; $V_1$-$L_1$-$V_2$-L-Epitope1-L-Epitope2; $V_1$-$L_1$-$V_2$-L-Epitope1-L-Epitope2-L;
$V_1$-$L_1$-$V_2$-L-Epitope1-L-Epitope2-L-Epitope3; $V_1$-$L_1$-$V_2$-L-Epitope1-L-Epitope2-L-Epitope3-L; $V_1$-$L_1$-$V_2$-Epitope1;
$V_1$-$L_1$-$V_2$-Epitope1-L; $V_1$-$L_1$-$V_2$-Epitope1-L-Epitope2; $V_1$-$L_1$-$V_2$-Epitope1-L-Epitope2-L; $V_1$-$L_1$-$V_2$-Epitope1-L-Epitope2-L-Epitope3; $V_1$-$L_1$-$V_2$-Epitope1-L-Epitope2-L-Epitope3-L; Epitope1-$V_1$-$L_1$-$V_2$; Epitope1-L-$V_1$-$L_1$-$V_2$; L-Epitope1-$V_1$-$L_1$-$V_2$; L-Epitope1-L-$V_1$-$L_1$-$V_2$; Epitope1-L-Epitope2-$V_1$-$L_1$-$V_2$; Epitope1-L-Epitope2-L-$V_1$-$L_1$-$V_2$; L-Epitope1-L-Epitope2-$V_1$-$L_1$-$V_2$; L-Epitope1-L-Epitope2-L-$V_1$-$L_1$-$V_2$; Epitope1-L-Epitope2-L-Epitope3-$V_1$-$L_1$-$V_2$; Epitope1-L-Epitope2-L-Epitope3-L-$V_1$-$L_1$-$V_2$; L-Epitope1-L-Epitope2-L-Epitope3-$V_1$-$L_1$-$V_2$; L-Epitope1-L-Epitope2-L-Epitope3-L-$V_1$-$L_1$-$V_2$; $V_1$-L-Epitope1-L-$V_2$; L-Epitope1-L-$V_1$-L-Epitope2-L-$V_2$; $V_1$-L-Epitope1-L-$V_2$-L-Epitope2-L; $V_1$-L-Epitope1-L-$V_2$-L-Epitope2-L-Epitope3; $V_1$-L-Epitope1-L-$V_2$-L-Epitope2-Epitope3; $V_1$-L-Epitope1-L-$V_2$-L-Epitope2-L-Epitope3-Epitope4; L-Epitope1-L-$V_1$-L-Epitope2-L-$V_2$-L-Epitope3-L; Epitope1-L-$V_1$-L-Epitope2-L-$V_2$-L-Epitope3-L; L-Epitope1-L-$V_1$-L-Epitope2-L-$V_2$-L-Epitope3; L-Epitope1-L-$V_1$-$L_1$-$V_2$-L-Epitope2-L; Epitope1-L-$V_1$-$L_1$-$V_2$-L-Epitope2-L-Epitope3; L-Epitope1-L-$V_1$-$L_1$-$V_2$-L-Epitope2-Epitope3, ou Epitope1-L-$V_1$-$L_1$-$V_2$-L-Epitope2-L-Epitope3-Epitope4
dans laquelle,

$V_1$ est $V_L$, et $V_2$ est $V_H$, ou $V_1$ est $V_H$, et $V_2$ est $V_L$ ;
$L_1$ est un lieur apte à lier la chaîne $V_H$ à la chaîne $V_L$ ;
L est un lieur comprenant des résidus de glycine et de sérine, et chaque occurrence de L dans le domaine de liaison extracellulaire peut être identique à ou différente d'une autre occurrence de L dans le même domaine de liaison extracellulaire, et
x est 0 ou 1 et chaque occurrence de x est choisie indépendamment des autres ; et
Epitope 1, Epitope 2 et Epitope 4 sont des épitopes spécifiques de mAb ayant la séquence d'acide aminé de SEQ ID NO 35 ; et Epitope 3 est sélectionné parmi les épitopes spécifiques de mAb ayant la séquence d'acide aminé de SEQ ID NO 35 ou SEQ ID NO 144.

6. Polypeptide selon la revendication 5, dans lequel Epitope 1, Epitope 2 et Epitope 4 sont des épitopes spécifiques de mAb ayant la séquence d'acide aminé de SEQ ID NO 35, et Epitope 3 est un épitope spécifique de mAb ayant la séquence d'acide aminé de SEQ ID NO 144.

7. Polypeptide selon la revendication 5 ou 6, dans lequel $L_1$ est un lieur comprenant de la glycine et / ou de la sérine.

8. Polypeptide selon la revendication 7, dans lequel $L_1$ est un lieur comprenant la séquence d'acide aminé (Gly-Gly-Gly-Ser)$_n$ ou (Gly-Gly-Gly-Gly-Ser)$_n$, dans laquelle n est 1, 2, 3, 4 ou 5 ou un lieur comprenant la séquence d'acide aminé (Gly$_4$Ser)$_4$ ou (Gly$_4$Ser)$_3$.

9. Polypeptide selon l'une quelconque des revendications 5 à 8 dans lequel L est un lieur comprenant de la glycine et / ou de la sérine.

10. Polypeptide selon la revendication 9, dans lequel L est un lieur ayant une séquence d'acides aminés choisie parmi SGG, GGS, SGGS, SSGGS, GGGG, SGGGG, GGGGS, SGGGGS, GGGGGS, SGGGGGS, SGGGGG, GSGGGGS, GGGGGGGS, SGGGGGGG, SGGGGGGGS ou SGGGGSGGGGS.

11. Polypeptide selon l'une quelconque des revendications 1 à 10, dans lequel lesdites chaînes VH et VL ont une séquence cible antigénique de plus de 80% d'identité, de préférence de plus de 90%, et plus préférablement de plus de 95% avec SEQ ID NO 43 (antigène CD19), SEQ ID NO 44 (antigène CD38), SEQ ID NO 45 (antigène CD123), SEQ ID NO 46 (antigène CS1), SEQ ID NO 47 (antigène BCMA), SEQ ID NO 48 (antigène FLT-3), SEQ ID NO 49 (antigène CD33), SEQ ID NO 50 (antigène CD70), SEQ ID NO 51 (antigène EGFR-3v) et SEQ ID NO 52 (antigène WT1).

**12.** Polypeptide selon l'une quelconque des revendications 1 à 10, dans lequel ledit antigène est choisi parmi ErbB2 (HER2 / neu), l'antigène carcino-embryonnaire (CEA), la molécule d'adhésion des cellules épithéliales (EpCAM), le récepteur du facteur de croissance épidermique (EGFR), la variante III de l'EGFR (EGFRvIII), CD19, CD20, CD30, CD40, disialoganglioside GD2, GD3, molécule de type lectine-1 de type C (CLL-1), mucine épithéliale canalaire, gp36, TAG-72, glycosphingolipides, antigène associé au gliome, β-gonadotrophine chorionique humaine, alphafétoprotéine (AFP), AFP réactif à la lectine, thyroglobuline, RAGE-1, MN-CA IX, transcriptase inverse de télomérase humaine, RU1, RU2 (AS), carboxylestérase intestinale, mut hsp70-2, M-CSF, prostase, antigène spécifique de la prostase (PSA), PAP, NY-ESO-1, LAGA-1a, p53, prostein, PSMA, survivine et télomérase, antigène tumoral du carcinome prostatique-1 (PCTA-1), MAGE, ELF2M, neutrophile élastase, éphrine B2, CD22, facteur de croissance de l'insuline (IGF1)-I, IGF-II, récepteur IGFI, mésothéline, une molécule de complexe majeur d'histocompatibilité (MHC) présentant une tumeur-specifique d'épitope peptidique, 5T4, ROR1, Nkp30, NKG2D, antigènes stromaux tumoraux, le domaine supplémentaire A (EDA) et le domaine supplémentaire B (EDB) de la fibronectine et le domaine A1 de la ténascine-C (TnC A1) et de la protéine associée aux fibroblastes (fap), LRP6, protéoglycane de sulfate de chondroïtine associé à la mélamona (MCSP), CD38/CS1, MART1, WT1, MUC1, LMP2, Idiotype, NY-ESO-1, mutant Ras, gp100, protéinase 3, bcr-abl, tyrosinase, hTERT, EphA2, ML-TAP, ERG, NA17, PAX3, ALK, récepteur androgène ; un antigène spécifique à une lignée ou à un tissu tel que CD3, CD4, CD8, CD24, CD25, CD33, CD34, CD70, CD79, CD116, CD117, CD135, CD123, CD133, CD138, CTLA-4, B7-1 (CD80) , B7-2 (CD86), endogline, une molécule de complexe majeur d'histocompatibilité (CMH), BCMA (CD269, TNFRSF 17) ou FLT-3.

**13.** Polypeptide selon l'une quelconque des revendications 1 à 10, dans lequel VH et VL sont choisis parmi un VH de SEQ ID NO 65 et un VL de SEQ ID NO 66 ; un VH de SEQ ID NO 67 et un VL de SEQ ID NO 68 ; un VH de SEQ ID NO 69 et un VL de SEQ ID NO 70 ; un VH de SEQ ID NO 71 et un VL de SEQ ID NO 72 ; un VH de SEQ ID NO 77 et un VL de SEQ ID NO 78 ; un VH de SEQ ID NO 79 et un VL de SEQ ID NO 80 ; un VH de SEQ ID NO 81 et un VL de SEQ ID NO 82; un VH de SEQ ID NO 83 et un VL de SEQ ID NO 84 ; un VH de SEQ ID NO 85 et un VL de SEQ ID NO 86 ; un VH de SEQ ID NO 87 et un VL de SEQ ID NO 88 ; un VH de SEQ ID NO 89 et un VL de SEQ ID NO 90 ; un VH de SEQ ID NO 91 et un VL de SEQ ID NO 92 ; un VH de SEQ ID NO 93 et un VL de SEQ ID NO 94 ; un VH de SEQ ID NO 95 et un VL de SEQ ID NO 96 ; un VH de SEQ ID NO 97 et un VL de SEQ ID NO 98 ; un VH de SEQ ID NO 99 et un VL de SEQ ID NO 100 ; un VH de SEQ ID NO 101 et un VL de SEQ ID NO 102 ; un VH de SEQ ID NO 103 et un VL de SEQ ID NO 104 ; un VH de SEQ ID NO 105 et un VL de SEQ ID NO 106 ; un VH de SEQ ID NO 107 et un VL de SEQ ID NO 108 ; un VH de SEQ ID NO 109 et un VL de SEQ ID NO 110 ; un VH de SEQ ID NO 111 et un VL de SEQ ID NO 112 ; un VH de SEQ ID NO 113 et un VL de SEQ ID NO 114 ; un VH de SEQ ID NO 115 et un VL de SEQ ID NO 116 ; un VH de SEQ ID NO 117 et un VL de SEQ ID NO 118 ; un VH de SEQ ID NO 119 et un VL de SEQ ID NO 120 ; un VH de SEQ ID NO 121 et un VL de SEQ ID NO 122 ; ou un VH de SEQ ID NO 123 et un VL de SEQ ID NO 124, un VH de SEQ ID NO 170 et un VL de SEQ ID NO 171 ; un VH de SEQ ID NO 172 et un VL de SEQ ID NO 173 ; ou un VH de SEQ ID NO 186 et un VL de SEQ ID NO 187.

**14.** Polypeptide selon l'une quelconque des revendications 1 à 13, dans lequel le polypeptide comprend un domaine de liaison extracellulaire, ledit domaine de liaison extracellulaire comprenant une charnière, et ledit polypeptide comprend en outre

- un domaine transmembranaire, et
- un domaine intracellulaire.

**15.** Polypeptide selon la revendication 14, dans lequel la charnière comprend une charnière IgG1, une charnière IgG4, une charnière CD8alpha ou une charnière FcγRIII alpha.

**16.** Polypeptide selon l'une quelconque des revendications 14 à 15, dans lequel le domaine transmembranaire comprend la ou les régions transmembranaires de la chaîne alpha, bêta ou zêta du récepteur des cellules T, PD-1, 4-1BB, OX40, ICOS, CTLA-4, LAG 3, 2B4, BTLA4, TIM-3, TIGIT, SIRPA, CD28, CD3 epsilon, CD45, CD4, CD5, CD8, CD9, CD16, CD22, CD33, CD37, CD64, CD80, CD86, CD134, CD137 ou CD154.

**17.** Polypeptide selon l'une quelconque des revendications 14 à 16, dans lequel le domaine transmembranaire comprend la ou les régions transmembranaires de CD8 alpha.

**18.** Polypeptide selon l'une quelconque des revendications 14 à 17, dans lequel le domaine intracellulaire comprend un domaine de signalisation CD3zeta.

**19.** Polypeptide selon l'une quelconque des revendications 14 à 18, dans lequel le domaine intracellulaire comprend un domaine 4-1BB.

**20.** Polypeptide selon l'une quelconque des revendications 1 à 19, dans lequel le CAR est un CAR à chaîne unique.

**21.** Polypeptide selon la revendication 1, dans lequel ledit polypeptide partage plus de 80% d'identité, plus de 90%, plus de 95% avec ou est identique à SEQ ID NO 1 à 10, SEQ ID NO 126 à 141 ou SEQ ID NO 145 à 149 ou SEQ ID NO 152 à 169.

**22.** Polypeptide selon l'une quelconque des revendications 1 à 19, dans lequel le CAR est un CAR à chaînes multiples.

**23.** Polynucléotide codant pour un polypeptide selon l'une quelconque des revendications 1 à 22.

**24.** Vecteur d'expression comprenant un polynucléotide selon la revendication 23.

**25.** Cellule immunitaire modifiée exprimant à sa surface cellulaire un polypeptide selon l'une quelconque des revendications 1 à 24.

**26.** Cellule immunitaire modifiée selon la revendication 25, dans laquelle ladite cellule est dérivée de lymphocytes T inflammatoires, de lymphocytes T cytotoxiques, de lymphocytes T régulateurs ou de lymphocytes T auxiliaires.

**27.** Cellule immunitaire modifiée selon la revendication 25 ou 26 pour son utilisation en tant que médicament.

**28.** Cellule immunitaire modifiée selon la revendication 25 ou 26 pour son utilisation dans le traitement de cancer chez un patient en ayant besoin.

**29.** Procédé ex vivo d'ingénierie d'une cellule immunitaire selon l'une quelconque des revendications 25 à 28, comprenant :

(a) l'introduction dans une cellule immunitaire qui a été pourvue d'au moins un polynucléotide codant pour le récepteur d'antigène chimérique selon l'une quelconque des revendications 1 à 22 ; et
(b) l'expression dudit polynucléotide dans ladite cellule.

**30.** Procédé pour l'ingénierie d'une cellule immunitaire selon la revendication 29, dans lequel la cellule immunitaire est une cellule T.

**31.** Anticorps monoclonal spécifique de l'épitope (mAb) qui reconnaît spécifiquement l'épitope spécifique de mAb ayant la séquence d'acide aminée de SEQ ID NO 35 à utiliser dans un procédé *in vivo* d'appauvrissement d'une cellule immunitaire modifiée exprimant à sa surface cellulaire un polypeptide comprenant au moins un épitope spécifique de mAb selon l'une quelconque des revendications 1 à 22 chez un patient, comprenant la mise en contact de ladite cellule immunitaire modifiée avec ledit mAb spécifique de l'épitope.

**32.** mAb spécifique de l'épitope pour l'usage selon la revendication 31, dans lequel le mAb spécifique de l'épitope est rituximab.

**33.** mAb spécifique de l'épitope pour l'usage selon la revendication 31 ou 32, dans lequel le mAb spécifique de l'épitope est conjugué avec une molécule capable d'activer le système du complément ou dans lequel un médicament cytotoxique est couplé au mAb spécifique de l'épitope.

**34.** mAb bi-spécifique (BsAb) capable de lier à la fois un épitope spécifique de mAb ayant la séquence d'acides aminés de SEQ ID NO 35 portée sur une cellule immunitaire modifiée exprimant à sa surface cellulaire un polypeptide CAR selon l'une quelconque des revendications 1 à 22, et un antigène de surface porté sur une cellule effectrice pour une utilisation dans un procédé *in vivo* d'appauvrissement de ladite cellule immunitaire modifiée chez un patient, ledit procédé comprenant la mise en contact de ladite cellule immunitaire modifiée avec ledit mAb bi-spécifique (BsAb).

Chimeric scFv

CAR-T cell

CAR-T cell

CAR-T cell

TM/stimulatory domains

Linker

$V_2$

$V_1$

mAb - specific epitope X

**Figure 1**

Figure 2

Cell depletion

Effector cells

DART

Purified CAR⁺ and depletion system⁺ cells

Specific cell lysis of CAR⁺/depletion system⁺ cells by effector cell

**Figure 3**

Figure 4

Figure 5

Figure 6

Figure 7A

Figure 7B

Figure 7C

Figure 8A

Figure 8B

Figure 9

A

B

Figure 10

Figure 11

Figure 12

**Figure 13**

Figure 14A

Figure 14B

Figure 15

EP 3 250 604 B1

Figure 16

Figure 17

EP 3 250 604 B1

Figure 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20140286987 A
- WO 2014152177 A
- EP 239400 A
- WO 9109967 A
- US 5225539 A
- US 5530101 A
- US 5585089 A
- EP 592106 A
- EP 519596 A
- US 5565332 A
- US 20050042664 A
- US 20050048617 A
- US 6407213 B
- US 5766886 A
- WO 9317105 A
- WO 2012138475 A
- WO 2014039523 A
- US 7446190 B
- WO 2008121420 A
- US 8252592 B
- US 20140024809 A
- WO 2012079000 A
- WO 2014153270 A
- WO 2012099973 A
- WO 2014011988 A

- WO 2014011987 A
- WO 2013067492 A
- WO 2013070468 A
- WO 2013040557 A
- WO 2013126712 A
- WO 2013126729 A
- WO 2013126726 A
- WO 2013126733 A
- US 8399645 B
- US 20130266551 A
- US 20140023674 A
- US 7514537 B
- US 8324353 B
- WO 2010025177 A
- US 7446179 B
- WO 2012031744 A
- WO 2012136231 A1
- WO 2012050374 A2
- WO 2013074916 A
- WO 2009091826 A3
- WO 2013176915 A
- WO 2013059593 A
- US 4975278 A
- WO 2014191128 A
- PA 201570044

### Non-patent literature cited in the description

- *CHEMICAL ABSTRACTS, 943609-66-3*
- *CHEMICAL ABSTRACTS, 679818-59-8*
- *CHEMICAL ABSTRACTS, 946414-94-4*
- PADLAN. *Molecular Immunology,* 1991, vol. 28 (4/5), 489-498
- STUDNICKA et al. *Protein Engineering,* 1994, vol. 7 (6), 805-814
- ROGUSKA et al. *PNAS,* 1994, vol. 91, 969-973
- TAN et al. *J. Immunol.,* 2002, vol. 169, 1119-25
- CALDAS et al. *Protein Eng.,* 2000, vol. 13 (5), 353-60
- MOREA et al. *Methods,* 2000, vol. 20 (3), 267-79
- BACA et al. *J. Biol. Chem.,* 1997, vol. 272 (16), 10678-84
- ROGUSKA et al. *Protein Eng.,* 1996, vol. 9 (10), 895-904

- COUTO et al. *Cancer Res.,* 1995, vol. 55 (23), 5973s-5977s
- COUTO et al. *Cancer Res.,* 1995, vol. 55 (8), 1717-22
- SANDHU J S. *Gene,* 1994, vol. 150 (2), 409-10
- PEDERSEN et al. *J. Mol. Biol.,* 1994, vol. 235 (3), 959-73
- RIECHMANN et al. *Nature,* 1988, vol. 332, 323
- SHEN Z ; MERNAUGH RL ; YAN H ; YU L ; ZHANG Y ; ZENG X. *Anal. Chem.,* 2005, vol. 77, 6834-6842
- SHEN Z ; STRYKER GA ; MERNAUGH RL ; YU L ; YAN H ; ZENG X. *Anal. Chem.,* 2005, vol. 77, 797-805
- ZHIHONG SHEN ; HEPING YAN ; YING ZHANG ; RAYMOND L. MERNAUGH ; XIANGQUN ZENG. *Anal Chem.,* 2008, vol. 80 (6), 1910-1917

- **BAUER ; GROH et al.** *Science,* 1999, vol. 285 (5428), 727-9
- **WU, SONG et al.** *Science,* 1999, vol. 285 (5428), 730-2
- **CHEN ; FLIES.** *Nat Rev Immunol,* 2013, vol. 13 (4), 227-42
- **BASU S ; CAMPBELL HM ; DITTEL BN ; RAY A.** Purification of specific cell population by fluorescence activated cell sorting (FACS). *J Vis Exp.,* vol. 41, 1546
- **MÜLLER D ; KONTERMANN R.E.** Bispecific Antibodies for Cancer Immunotherapy. *BioDrugs,* 2010, vol. 24 (2), 89-98
- **DEO Y M ; SUNDARAPANDIYAN K ; KELER T ; WALLACE PK ; GRAZIANO RF.** *Journal of Immunology,* 2000, vol. 165 (10), 5954-5961
- **PAYNE, G.** *Cancer Cell,* 2003, vol. 3, 207-212
- **TRAIL et al.** *Cancer Immunol. Immunother.,* 2003, vol. 52, 328-337
- **SYRIGOS ; EPENETOS.** *Anticancer Research,* 1999, vol. 19, 605-614
- **NICULESCU-DUVAZ ; SPRINGER.** *Adv. Drug Del. Rev.,* 1997, vol. 26, 151-172
- **COURTOIS, A ; GAC-BRETON, S. ; BERTHOU, C. ; GUÉZENNEC, J. ; BORDRON, A. ; BOISSET, C.** Complement dependent cytotoxicity activity of therapeutic antibody fragments is acquired by immunogenic glycan coupling. *Electronic Journal of Biotechnology,* 2012, ISSN 0717-3458, http://www.ejbiotechnology.info
- **HENDERSON ; NAYA et al.** *Immunology,* 1991, vol. 73 (3), 316-21
- **BIERER ; HOLLANDER et al.** *Curr Opin Immunol,* 1993, vol. 5 (5), 763-73
- Retroviridae: The viruses and their replication. **COFFIN, J. M. et al.** Fundamental Virology. Lippincott-Raven Publishers, 1996
- **VALTON.** *Mol Ther,* 2015, vol. 23 (9), 1507-1518
- **ARBIZA J. ; TAYLOR G. ; LÓPEZ J.A. ; FURZE J. ; WYLD S. ; WHYTE P. ; STOTT E.J. ; WERTZ G. ; SULLENDER W. ; TRUDEL M. et al.** Characterization of two antigenic sites recognized by neutralizing monoclonal antibodies directed against the fusion glycoprotein of human respiratory syncytial virus. *J Gen Virol.,* 1992, vol. 73 (9), 2225-34
- **BENJAMIN, RJ ; WALDMANN, H.** Induction of tolerance by monoclonal antibody therapy. *Nature,* 1986, vol. 520, 449-451
- **BOCH, J. ; H. SCHOLZE et al.** Breaking the code of DNA binding specificity of TAL-type III effectors. *Science,* 2009, vol. 326 (5959), 1509-12
- **BUDDE, L.E. ; BERGER C ; LIN Y ; WANG J ; LIN X ; FRAYO SE ; BROUNS SA ; SPENCER DM ; TILL BG ; JENSEN MC.** Combining a CD20 chimeric antigen receptor and an inducible caspase 9 suicide switch to improve the efficacy and safety of T cell adoptive immunotherapy for lymphoma. *PLoS One,* 17 December 2013, vol. 8 (12), e82742
- **BULLER R.M. ; HOLMES K.L. ; HÜGIN A. ; FREDRICKSON T.N. ; MORSE H.C.** Induction of cytotoxic T cell responses in vivo in the absence of CD4 helper cells. *Nature,* 1987, vol. 328, 77-79
- **COBBOLD, S.P. ; MARTIN, G. ; QIN, S. ; WALDMANN, H.** Monoclonal antibodies to promote marrow engraftment and tissue graft rejection. *Nature,* 1986, vol. 323, 164-166
- **DOLAN DE ; GUPTA S.** PD-1 pathway inhibitors: changing the landscape of cancer immunotherapy. *Cancer Control.,* 2014, vol. 21 (3), 231-7
- **CARTELLIERI. M. ; KORISTKA, S. ; ARNDT, C. ; FELDMANN, A. ; STAMOVA, S. ; VON BONIN, M. ; TÖPFER, K. ; KRÜGER, T. ; GEIB, M. ; MICHALK, I.** A novel ex vivo isolation and expansion procedure for chimeric antigen receptor engrafted human T cells. *PloS One,* 2014, vol. 9 (4), e93745
- **EPA, V. C. ; O. DOLEZAL et al.** Structural model for the interaction of a designed Ankyrin Repeat Protein with the human epidermal growth factor receptor 2. *PLoS One,* 2013, vol. 8 (3), e59163
- **FEDOROV V.D. ; THEMELI M ; SADELAIN M.** PD-1- and CTLA-4-Based Inhibitory Chimeric Antigen Receptors (iCARs) Divert Off-Target Immunotherapy Responses. *Sci Transl Med,* 2013, vol. 5 (215), 215
- **FRIEDRICH, K. ; J. R. HANAUER et al.** DARPin-targeting of measles virus: unique bispecificity, effective oncolysis, and enhanced safety. *Mol Ther,* 2013, vol. 21 (4), 849-59
- **JENA, B. ; G. DOTTI et al.** Redirecting T-cell specificity by introducing a tumor-specific chimeric antigen receptor. *Blood,* 2010, vol. 116 (7), 1035-44
- **JOST, C. ; J. SCHILLING et al.** Structural Basis for Eliciting a Cytotoxic Effect in HER2-Overexpressing Cancer Cells via Binding to the Extracellular Domain of HER2. *Structure,* 2013, vol. 21 (11), 1979-91
- **MOSCOU, M. J. ; A. J. BOGDANOVE.** A simple cipher governs DNA recognition by TAL effectors. *Science,* 2009, vol. 326 (5959), 1501
- **PARK, T. S. ; S. A. ROSENBERG et al.** Treating cancer with genetically engineered T cells. *Trends Biotechnol,* 2011, vol. 29 (11), 550-7
- **PHILIP B ; KOKALAKI E ; MEKKAOUI L ; THOMAS S ; STRAATHOF K ; FLUTTER B ; MARIN V ; MARAFIOTI T ; CHAKRAVERTY R ; LINCH D.** A highly compact epitope-based marker/suicide gene for easier and safer T-cell therapy. *Blood,* 2014, vol. 124 (8), 1277-87

- **RIEMER A.B. ; KURZ H. ; KLINGER, M. ; SCHEIN-ER, O. ; ZIELINSKI, C. ; JENSEN-JAROLIM, E.** Vaccination with cetuximab mimotopes and biological properties of induced anti-epidermal growth factor receptor antibodies. *J Natl Cancer Inst.,* 2005, vol. 97 (22), 1663-70

- **VALTON J. ; GUYOT V. ; MARECHAL A. ; FILHOL JM. ; JUILLERAT A. ; DUCLERT A. ; DUCHATEAU P. ; POIROT L.** A multidrug resistant engineered CAR T cell for allogeneic combination immunotherapy. *Mol Ther,* 2015, vol. 23 (9), 1507-1518
- **PHILIP B ; KOKALAKI E ; MEKKAOUI L ; THOMAS S et al.** A highly compact epitope-based marker/suicide gene for easier and safer T-cell therapy. *Blood,* 2014, vol. 124 (8), 1277-87